# EUROPEAN PATENT APPLICATION

(11) **EP 4 653 010 A1**
(43) Date of publication of application: **26.11.2025**
(21) Application number: 25176511.1
(22) Date of filing: 14.05.2025
(51) Int. Cl.: A61K 38/17, A61P 3/04, A61P 21/00

(54) **ACTIVIN RECEPTOR TYPE IIB TRAPS FOR USE IN IMPROVING BODY COMPOSITION**

(30) Priority: 14.05.2024 US 202463647538 P
(71) Applicant: 35Pharma Inc., Montreal, QC H2Y 2Z4 (CA)
(72) Inventor: O Connor-McCourt, Maureen, Montreal (CA); Ganesh, Vannakambadi, Montreal (CA); Tremblay, Gilles, Montreal (CA); Schang, Gauthier, Montreal (CA)
(74) Representative: Cooley (UK) LLP

(57) **Abstract**

There are provided polypeptides that include an Activin receptor type IIB (ActRIIB) ectodomain (ECD) variant. In some embodiments, a polypeptide of the disclosure includes an ActRIIB-ECD variant fused to an Fc domain moiety. The disclosure also provides pharmaceutical compositions and methods of using the polypeptides to treat diseases and conditions associated with TGFβ superfamily ligand signaling, such as metabolic disorders, diabetes, obesity, cardiometabolic disease, pulmonary hypertension, fibrosis, muscle weakness and atrophy, bone damage, and/or low red blood cell levels (such as anemia).

## Description

### CROSS-REFERENCE TO RELATED APPLICATIONS

This application claims priority to US Provisional Application No. 63/647,538, filed on May 14, 2024, the content of which is incorporated herein by reference in its entirety.

### REFERENCE TO AN ELECTRONIC SEQUENCE LISTING

The content of the electronic sequence listing (35PH_009_01EP_SeqList_ST26.xml; Size: 451,004 bytes; and Date of Creation: May 13, 2025) are herein incorporated by reference in its entirety.

### FIELD

The present disclosure relates to polypeptides that include an Activin receptor type IIB (ActRIIB) ectodomain (ECD) variant and uses thereof for binding and neutralizing TGFβ superfamily ligands, particularly for the treatment of diseases and conditions associated with TGFβ superfamily signaling such as metabolic disorders, cardiometabolic disease, pulmonary hypertension, fibrosis, muscle weakness and atrophy, bone damage, and low red blood cell levels.

### BACKGROUND

The transforming growth factor beta (TGFβ) superfamily includes 35 ligands that regulate several physiological processes, including cell proliferation, migration and differentiation, muscle growth, vascular homeostasis, and osteogenesis. Perturbation of their levels and/or signaling pathways gives rise to significant pathological effects. For instance, TGFβ and activin ligands have been implicated in the pathogenesis of multiple human disorders and play critical pathogenic roles in many diseases. Examples of TGFβ-superfamily associated disorders include metabolic and cardiometabolic disorders (including diabetes and obesity), pulmonary hypertension (including pulmonary arterial hypertension), hematologic malignancies, solid tumors, bone marrow failure states, muscle weakness, and a wide variety of disorders characterized by uncontrolled fibrosis such as pulmonary, liver, renal and cardiac fibrosis, and systemic sclerosis (SSc; also called scleroderma) (Nanthakumar, D.B. et al., 2015; Meng, X.-M. et al., 2016). There remains a need in the art for therapeutics effective in the treatment of TGFβ-superfamily associated disorders.

### SUMMARY

There are provided herein activin receptor type IIB (ActRIIB)-ectodomain (ECD) based traps having a tailored ligand specificity profile for binding and neutralization of TGFβ superfamily ligands, and pharmaceutical compositions and methods of use thereof in the treatment of diseases and conditions associated with or mediated by TGFβ superfamily signaling.

ActRIIB-ECD traps provided herein comprise an ActRIIB-ECD variant fused to an Fc domain monomer that can function to assemble two polypeptides together. ActRIIB-ECD variants provided herein have been designed to tailor ligand specificity, in order to maximize therapeutic efficacy in certain disease indications while minimizing adverse effects. The ActRIIB-ECD variants provided herein are constructed by introducing novel amino acid substitutions into the ActRIIB-ECD, with the goal of preventing or reducing disruption of endogenous BMP-9 and/or BMP-10 signaling, while maintaining and/or increasing neutralization of other TGFβ superfamily ligands such as activin A, activin B, GDF-8, and/or GDF-11. Without wishing to be limited by theory, the goal of sparing BMP-9 and/or BMP-10 signaling is based on the finding that these ligands are important for the maintenance of vascular quiescence and homeostasis (Desroches-Castan, A. et al., 2022). Wild type ActRIIB binds to BMP-9 and BMP-10; therefore, ActRIIB-ECD-based traps have the potential to disrupt vascular homeostasis which may result in bleeding concerns. In support of this concept, telangiectasias, epistaxis and gingival bleeding were observed in clinical studies of a non-mutated ActRIIB-ECD trap (called ACE-031) (Campbell, C. et al., 2017). It was suggested that these vascular effects may have resulted from inhibition of the BMP-9 pathway.

The preferred ActRIIB-ECD variants provided herein exhibit: (1) similar or improved binding to activin A, activin B, GDF-8, and/or GDF-11 compared to wild type ActRIIB, which allows them to compete with endogenous activin receptors for ligand binding and reduce or inhibit endogenous ligand-stimulated receptor signaling; and (2) reduced or removed binding to BMP-9 and/or BMP-10 compared to wild type ActRIIB, which allows homeostatic BMP-9 and/or BMP-10 signaling to be maintained. These variants can be used to treat diseases and conditions in which activin receptor signaling is elevated, such as pulmonary hypertension (PH) (e.g., PAH, venous PH, hypoxic PH, thromboembolic PH, or miscellaneous PH), metabolic disease, bone disease, muscle disease, fibrosis, and/or low red blood cell levels (e.g., anemia). The variants can for example lead to a reduction in the symptoms or progression of PH (e.g., PAH, venous PH, hypoxic PH, thromboembolic PH, or miscellaneous PH), a reduction in bone resorption or osteoclast activity, an increase in bone formation or bone mineral density, an increase in muscle mass or strength, a reduction in adipose tissue, a reduction in fibrosis (e.g., reduced fibrosis or a slowing or stopping of the progression of fibrosis), and/or an increase in red blood cell levels (e.g., an increase in hemoglobin levels, hematocrit, or red blood cell counts).

In some embodiments, the present disclosure provides A method of improving body composition in a subject comprising administering a TGFβ superfamily ligand binding agent comprising a first and a second polypeptide, wherein each of the first and second polypeptides comprise: an Activin receptor type IIB (ActRIIB) ectodomain (ECD) variant comprising an amino acid substitution at the position corresponding to position 33 of SEQ ID NO: 2; a peptide linker; and an Fc domain monomer. In some embodiments, an improvement in body composition comprises an increase in lean muscle mass and/or decrease in fat mass.

In some embodiments, the present disclosure provides a method of improving exercise tolerance in a subject comprising administering a TGFβ superfamily ligand binding agent comprising a first and a second polypeptide, wherein each of the first and second polypeptides comprise: an Activin receptor type IIB (ActRIIB) ectodomain (ECD) variant comprising an amino acid substitution at the position corresponding to position 33 of SEQ ID NO: 2; a peptide linker; and an Fc domain monomer. In some embodiments, an improvement in exercise tolerance comprises reduced Left ventricular end-diastolic pressure (LVEDP) and/or an increased 6-minute walk distance.

In some embodiments, the subject suffers from a metabolic disorder. In some embodiments, the metabolic disorder is obesity. In some embodiments, the subject does not suffer from a cardiometabolic disorder.

In some embodiments, the ActRIIB ECD variant comprises the amino acid substitution L33E. In some embodiments, the ActRIIB ECD variant comprises an amino acid sequence that is at least 85%, at least 90%, at least 95%, at least 96%, at least 97%, at least 98%, or at least 99% identical to SEQ ID NO: 16; or comprises or consists of the amino acid sequence of SEQ ID NO: 16.

In some embodiments, the first and second polypeptides comprise the following structure, from N- to C-terminus: ActRIIB-ECD - peptide linker - Fc domain monomer. In some embodiments, the Fc domain monomer is an IgG1 isotype In some embodiments, the Fc domain monomer comprises or consists of the amino acid sequence set forth in SEQ ID NO: 253, SEQ ID NO: 255, or SEQ ID NO: 256. In some embodiments, the Fc domain monomer forms a dimer.

In some embodiments, the peptide linker is Glycine-rich. In some embodiments, the peptide linker is between 10 and 40 amino acids long. In some embodiments, the peptide linker is 14 amino acids long.

In some embodiments, the first and second polypeptides comprise or consist of the amino acid sequence of SEQ ID NO: 231, or an amino acid sequence that is at least 95%, 96%, 97%, 98%, or 99% identical thereto.

Further scope, applicability and advantages of the present technology will become apparent from the non-restrictive detailed description given hereinafter. It should be understood, however, that this detailed description, while indicating exemplary embodiments of the technology, is given by way of example only, with reference to the accompanying drawings.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.

For a better understanding of the technology and to show more clearly how it may be carried into effect, reference will now be made by way of example to the accompanying drawings, which illustrate aspects and features according to non-limiting embodiments of the present technology.

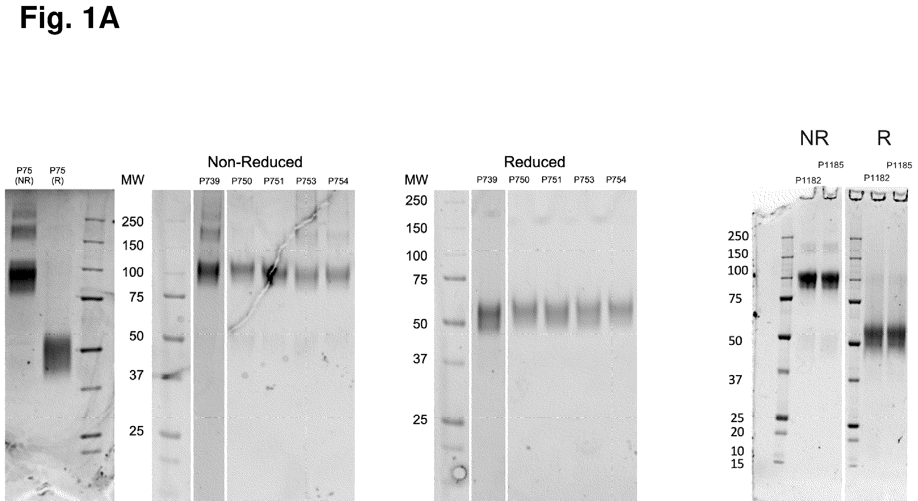

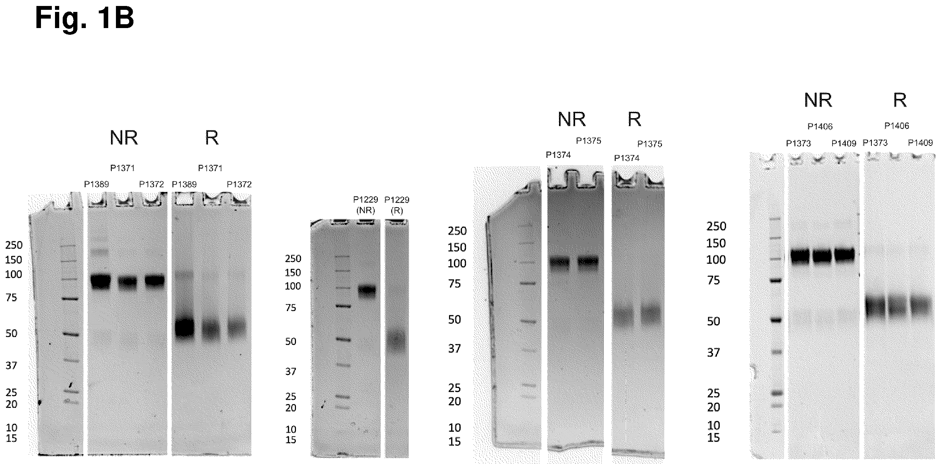

**FIG. 1A-1B** shows polyacrylamide gel electrophoresis analysis under non-reducing and reducing conditions of representative ActRIIB-ECD polypeptide constructs. After expression and purification, 1 µg of each protein was loaded on the gel, as indicated: P75: Protein 75; P739: Protein 739; P750: Protein 750; P751: Protein 751; P753: Protein 753; P754: Protein 754; P1182: Protein 1182; P1185: Protein 1185; P1229: Protein 1229; P1371: Protein 1371; P1372: Protein 1372; P1373: Protein 1373; P1374: Protein 1374; P1375: Protein 1375; P1389: Protein 1389; P1406: Protein 1406; P1409: Protein 1409. "NR": non-reducing conditions; "R": reducing conditions.

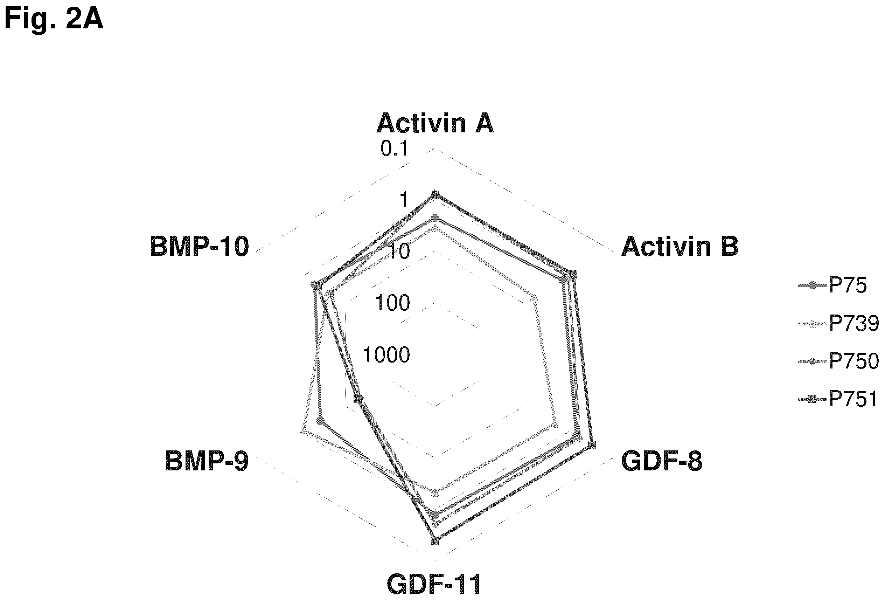

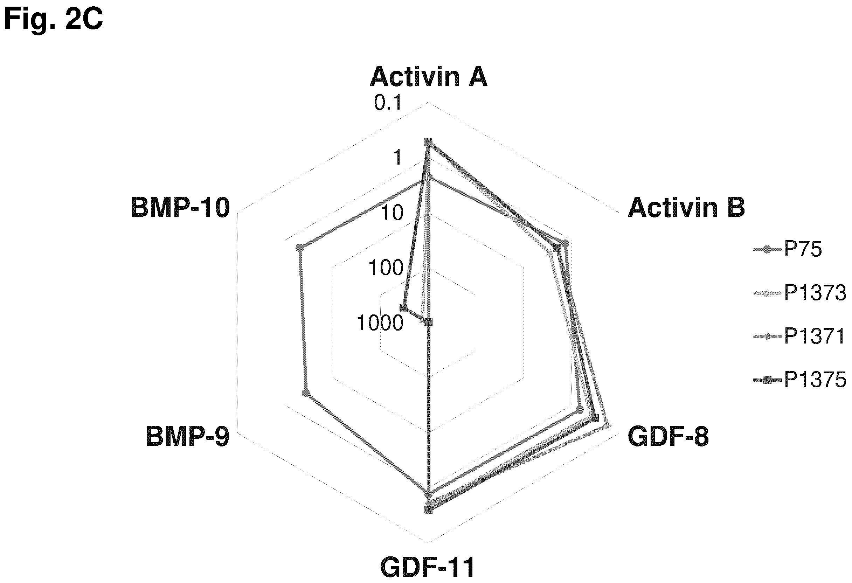

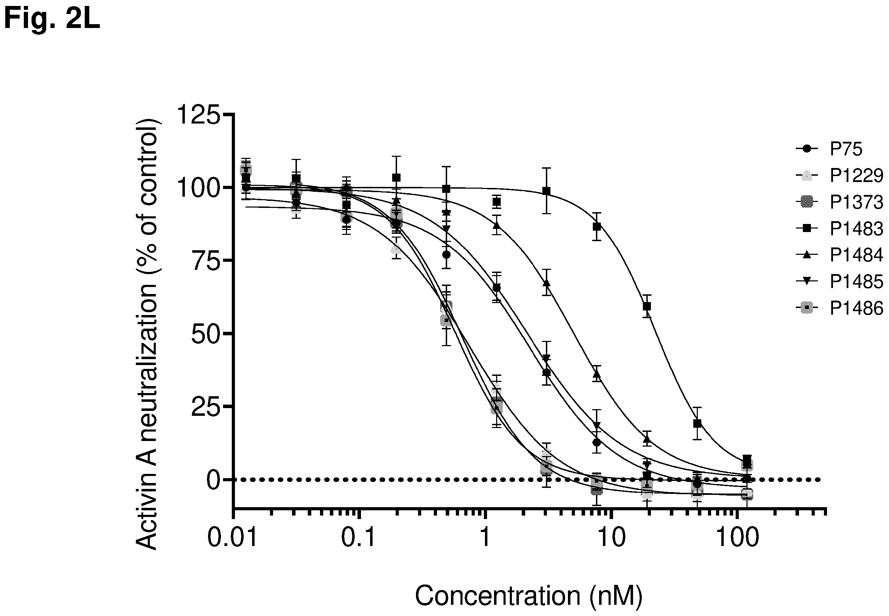

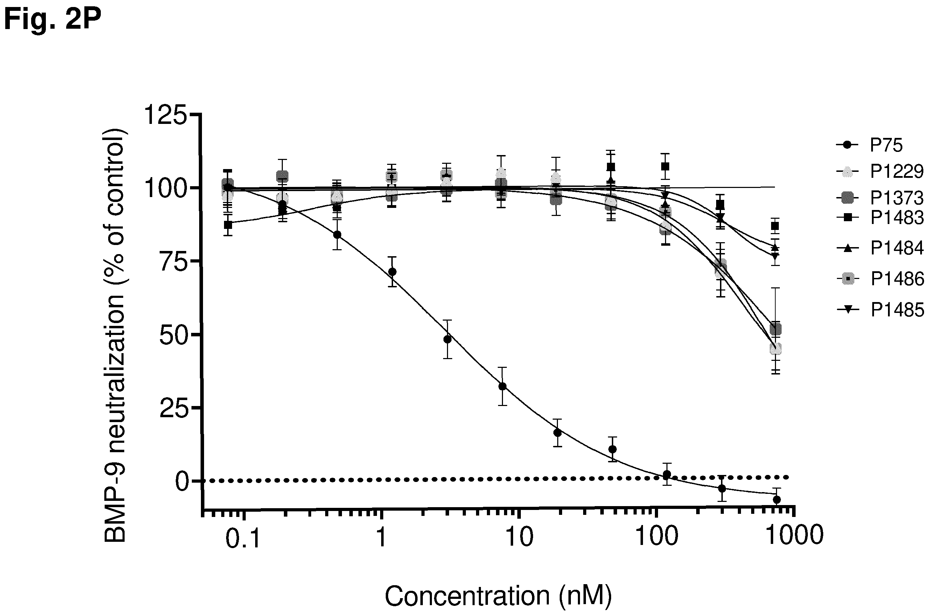

**FIG. 2A** **-** **FIG. 2Q** show comparative charts (radar plots) in which IC50 values for neutralization of TGFβ superfamily ligands (activin A, activin B, GDF-8, GDF-11, BMP-9, and BMP-10) are displayed for exemplary test proteins, as indicated. Points at the center of the chart indicate low neutralization potency for a given cytokine (high IC50 value), whereas points at the edge of the chart indicate high neutralization potency for a given cytokine (low IC50 value). Exemplary test proteins displayed are (**FIG**. **2A**) P739, P750, and P751, (**FIG**. **2B**) P753, P754, and P1229, (**FIG. 2C**) P1373, P1371, and P1375, (**FIG**. **2D**) P1182 and P1185, and (**FIG**. **2E**) P1389, P1406, and P1409. All agents are compared to wild type ActRIIB-ECD (P75). **FIG**. **2F** - **FIG**. **2I** show representative results in the HEK-Blue cell-based assay for inhibition of activin A, activin B, GDF-8, and GDF-11, respectively, for exemplary proteins P75, P1229, P1371, P1372, P1373, P1374, and P1375. **FIG**. **2J** - **FIG**. **2K** show representative results in the HepG2 cell-based assay for inhibition of BMP-9 and BMP-10, respectively, for exemplary proteins P75, P1229, P1372, P1373, P1374, and P1375. **FIG. 2L** **-** **FIG. 2O** show representative results in the HEK-Blue cell-based assay for inhibition of activin A, activin B, GDF-8, and GDF-11, respectively, for exemplary proteins P75, P1229, P1373, P1483, P1484, P1485, and P1486. **FIG. 2P** **-** **FIG. 2Q** show representative results in the HepG2 cell-based assay for inhibition of BMP-9 and BMP-10, respectively, for exemplary proteins P75, P1229, P1373, P1483, P1484, P1485, and P1486. Error bars indicate standard error of the mean (SEM).

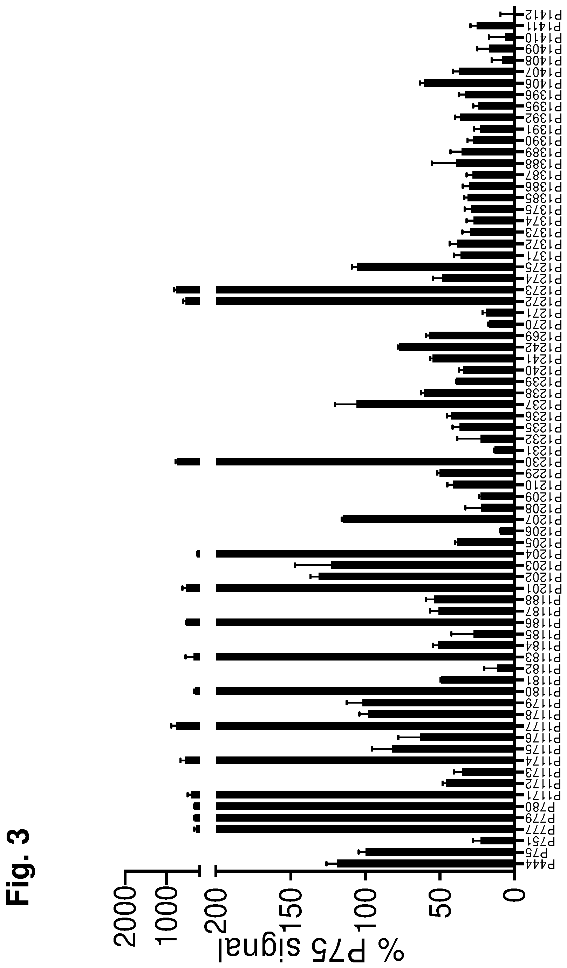

**FIG. 3** shows results from the activin A ELISA using supernatants from the small-scale production of ActRIIB-ECD fusion proteins. Results are expressed as a % of the signal obtained with wild-type ActRIIB-ECD-Fc (P75). In this assay, a loss of signal suggests increased binding of the exemplary test agent to activin A. Error bars indicate standard error of the mean (SEM). This graph was generated using GraphPad Prism 9.0.

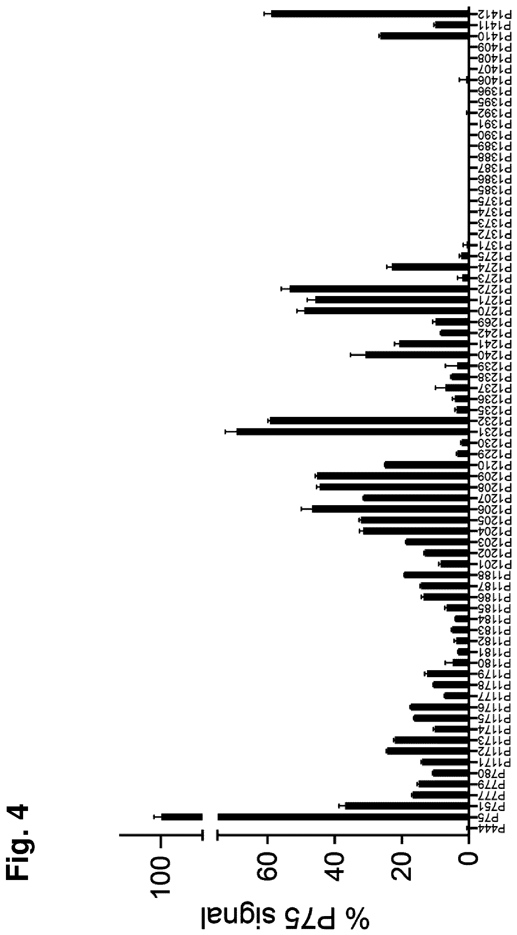

**FIG. 4** shows results from the BMP-9 ELISA using supernatants from the small-scale production of ActRIIB-ECD fusion proteins. Results are expressed as a % of the signal obtained with wild-type ActRIIB-ECD-Fc (P75). In this assay, a loss of signal suggests decreased binding of the exemplary test agent to BMP-9. Error bars indicate standard error of the mean (SEM). This graph was generated using GraphPad Prism 9.0.

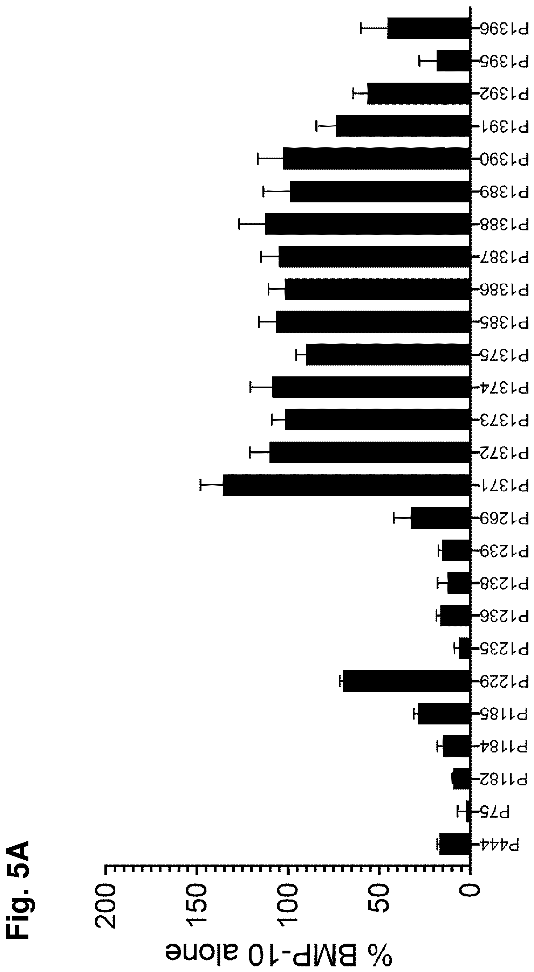

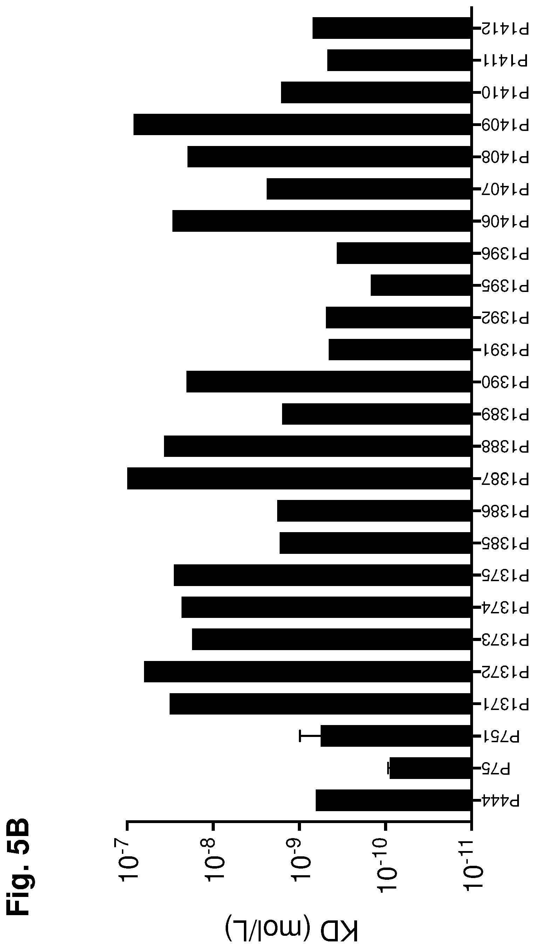

**FIG. 5A** **-** **FIG. 5B** show results from the BMP-10 assays using supernatants from the small-scale production of ActRIIB-ECD fusion proteins. (**FIG. 5A**) Results from the cell-based assays are expressed as a % of the signal obtained with BMP-10 alone. In this assay, a loss of signal suggests increased binding of the exemplary test agent to BMP-10. Error bars indicate standard error of the mean (SEM). (**FIG. 5B**) Bio-layer interferometry was used to derive KD values for exemplary test agents. These graphs were generated using GraphPad Prism 9.0.

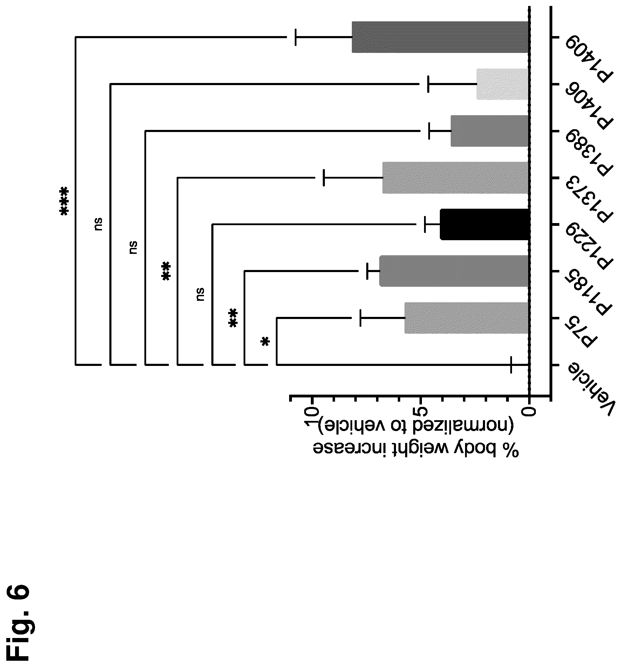

**FIG. 6** shows results from single-injection experiments in wild-type mice. Male mice were injected with a single dose of test agent as indicated (25 mg/kg, subcutaneous), and body weight gain was evaluated after 4 days. Results were normalized to the vehicle control, and error bars indicate standard error of the mean (SEM). Results were analyzed by one-way ANOVA followed by post-hoc Bonferroni-corrected multiple comparison test; *p<0.05, **p<0.01, ***p<0.001, ***p<0.0001 relative to the vehicle group.

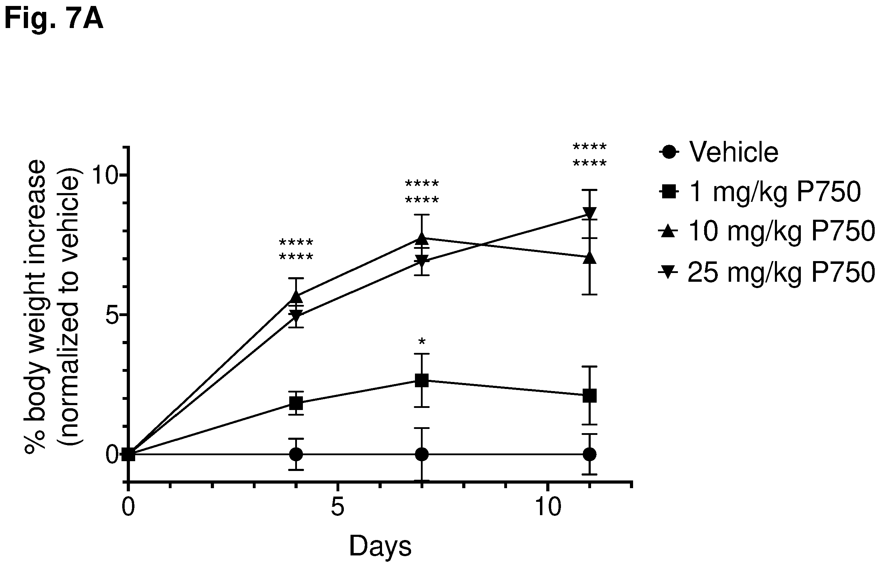

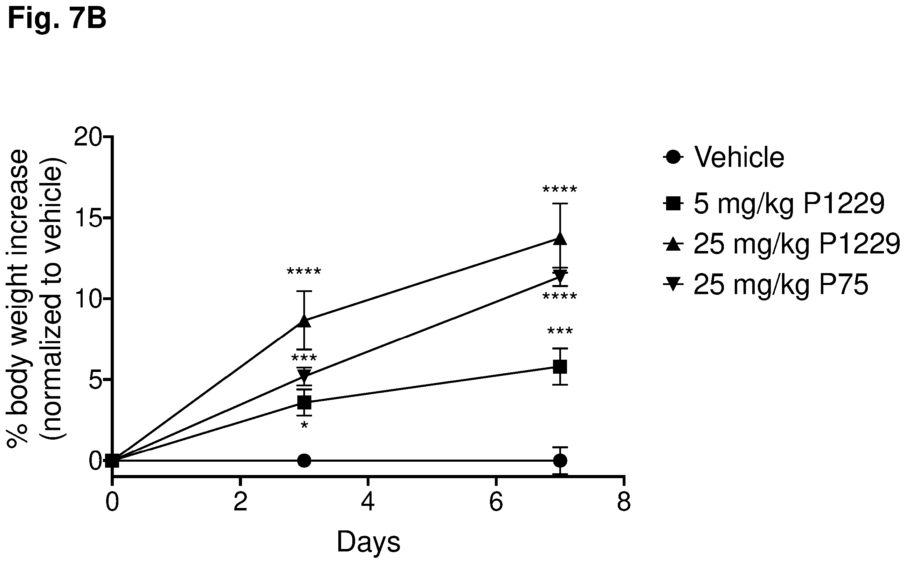

**FIG. 7A** **-** **FIG. 7B** show body weight gain in male mice injected (subcutaneous) with test proteins. **FIG. 7A** shows results for P750 (1, 5, or 25 mg/kg) and **FIG. 7B** shows results for P1229 (5 or 25 mg/kg) and P75 (25 mg/kg). Injections were performed twice weekly for 11 days (P750) or 7 days (P1229 and P75). Results were normalized to the vehicle control, and error bars indicate standard error of the mean (SEM). Results were analyzed by two-way ANOVA followed by post-hoc Bonferroni-corrected multiple comparison test; *p<0.05, ***p<0.001, ***p<0.0001 relative to the vehicle group.

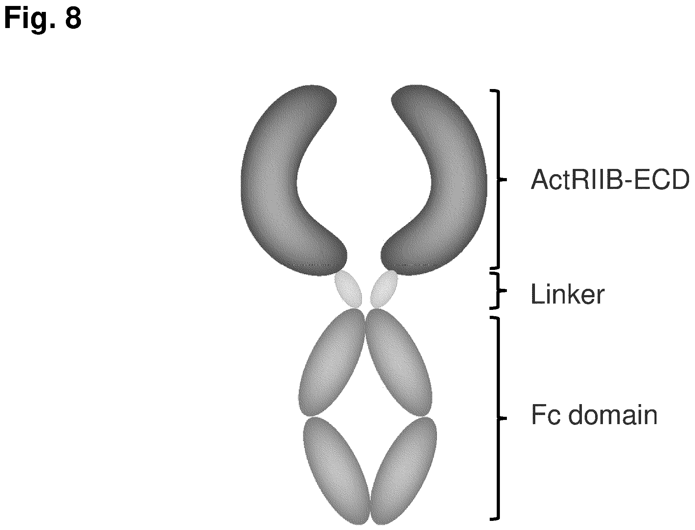

**FIG. 8** provides an exemplary schematic of binding agents described herein.

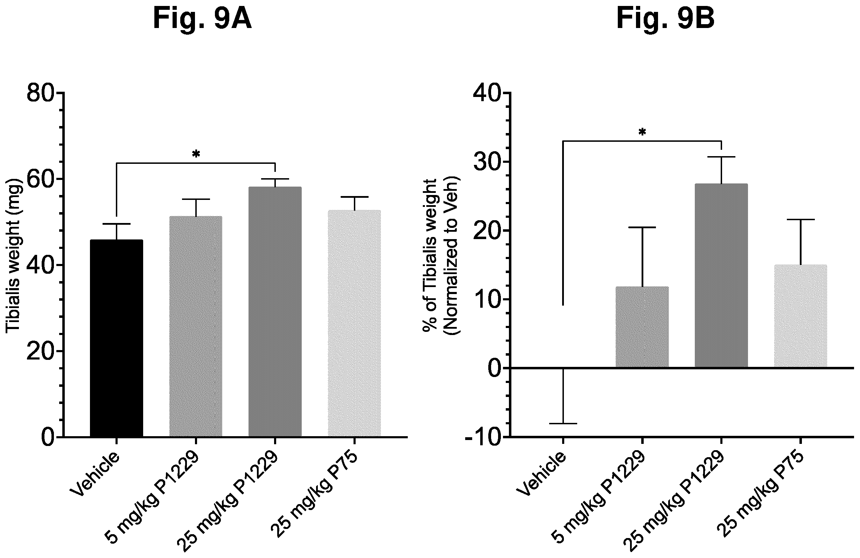

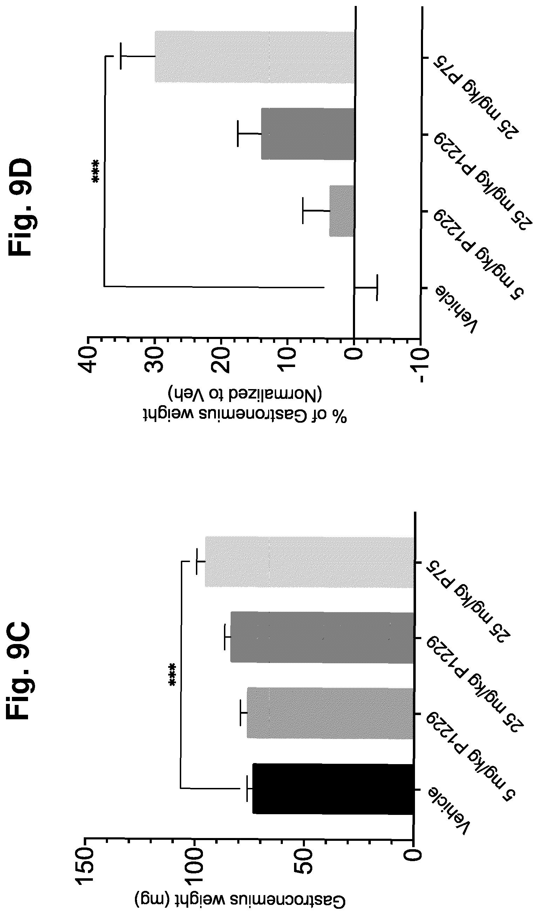

**FIG. 9A** **-** **FIG. 9D** illustrate changes in skeletal muscle mass after a week of treatment with vehicle, P1229 (5 or 25 mg/kg), and P75 (25 mg/kg). Injections were done subcutaneously, twice weekly. **FIG. 9A** shows average tibialis anterior weight. **FIG. 9B** shows tibialis weight as normalized to vehicle control. **FIG. 9C** shows average gastrocnemius weight. **FIG. 9D** shows gastrocnemius weight as normalized to vehicle control. Results were analyzed by one-way ANOVA followed by post-hoc Bonferroni-corrected multiple comparison test; *p<0.05, ***p<0.001 relative to the vehicle group.

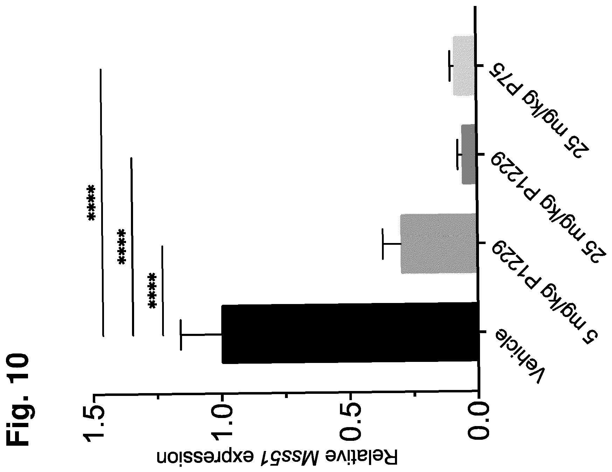

**FIG. 10** illustrates changes in *Mss51* expression in tibialis anterior after a week of treatment with vehicle, P1229 (5 or 25 mg/kg), and P75 (25 mg/kg). Injections were done subcutaneously, twice weekly. Results were analyzed by one-way ANOVA followed by post-hoc Bonferroni-corrected multiple comparison test; ****p<0.0001 relative to the vehicle group.

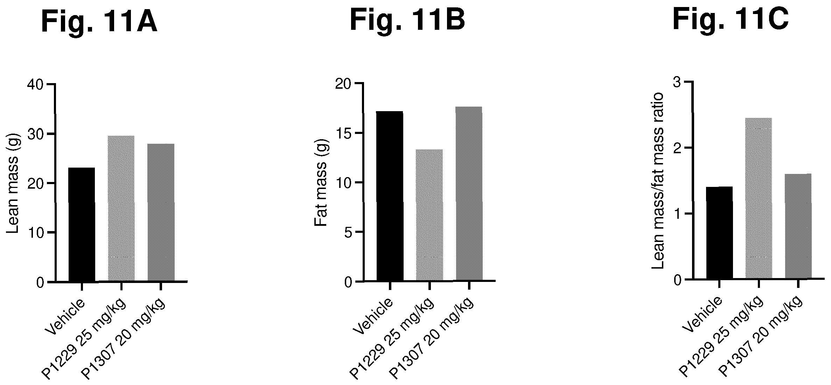

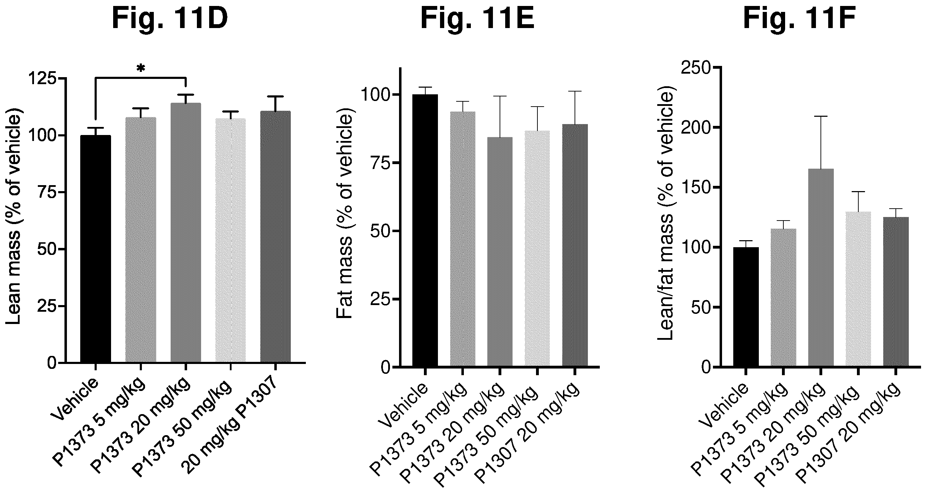

**FIG. 11A** **-** **FIG. 11F** illustrate changes in body composition of diet-induced obese (DIO) mice injected subcutaneously with vehicle, P1229 (25 mg/kg, twice weekly), P75 (25 mg/kg, twice weekly), P1373 (5, 20, or 50 mg/kg twice weekly), or P1307 (CDD866, murine bimagrumab (an antibody targeting ActRIIB and ActRIIA), 20 mg/kg once weekly). **FIGs. 11A** and **11D** show average lean mass, **FIG. 11B** and **11E** show average fat mass, and **FIG. 11C** and **11F** show the ratio of lean mass over fat mass. Parameters were assessed by echoMRI. Results were analyzed by one-way ANOVA followed by post-hoc Bonferroni-corrected multiple comparison test; **p*<0.05 relative to the vehicle group.

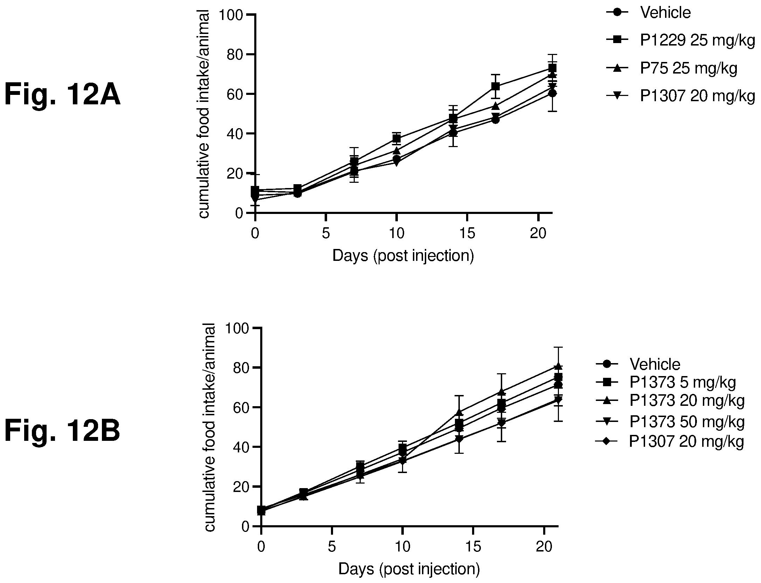

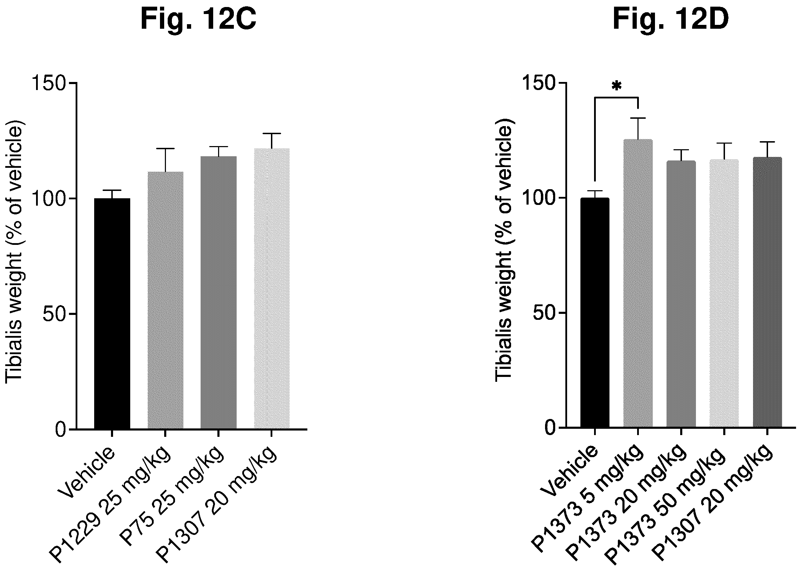

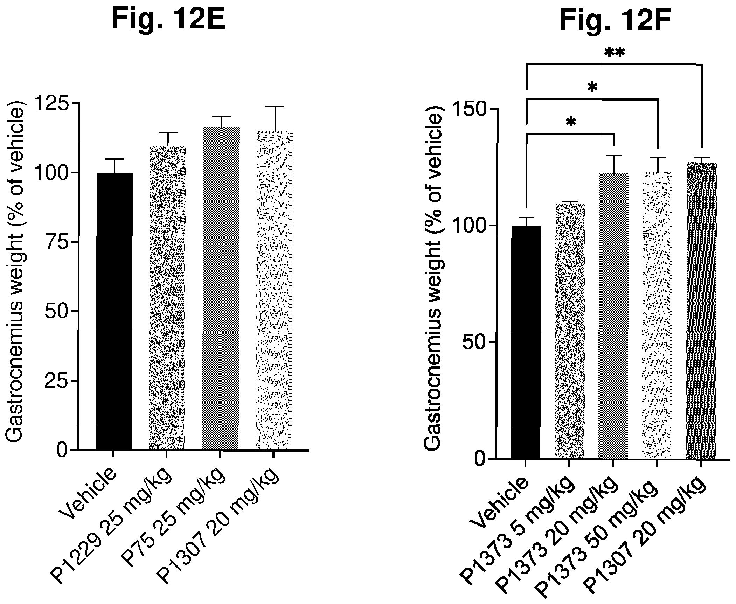

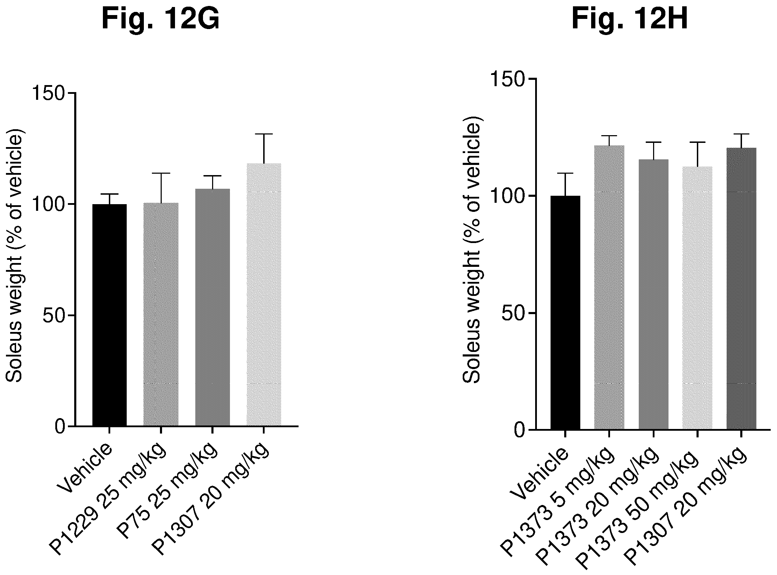

**FIG. 12A** **-** **FIG. 12B** illustrate food consumption over time of DIO mice injected subcutaneously with vehicle, P1229 (25 mg/kg, twice weekly), P75 (25 mg/kg, twice weekly), P1373 (5, 20, or 50 mg/kg twice weekly), or P1307 (CDD866, or murine bimagrumab, 20 mg/kg once weekly). **FIGs. 12C-H** show skeletal muscle weights at the end of the study; **FIG. 12C** **-** **FIG. 12D****,** **FIG. 12E** **-** **FIG. 12F****,** and **FIG. 12G** **-** **FIG. 12H** show tibialis anterior, gastrocnemius, and soleus weights, respectively. Results were analyzed by one-way ANOVA followed by post-hoc Bonferroni-corrected multiple comparison test; **p*<0.05, ***p*<0.01 relative to the vehicle group.

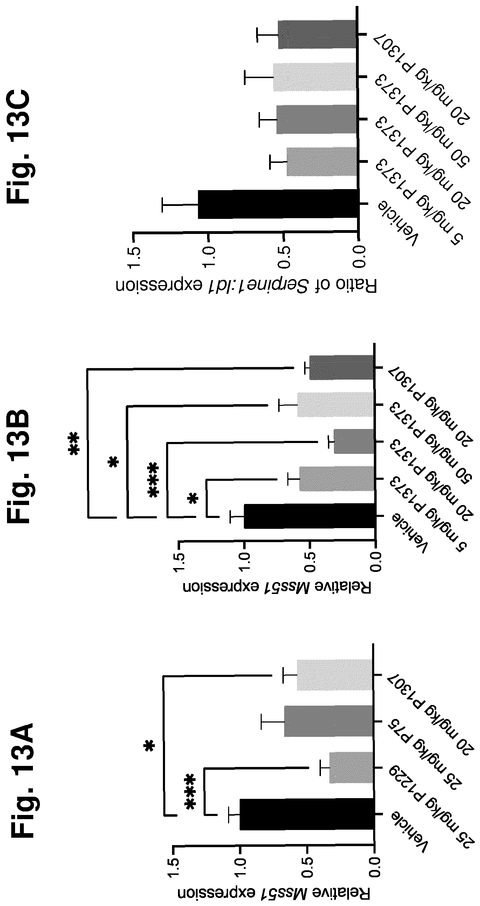

**FIG. 13A** **-** **FIG. 13C** illustrate changes in skeletal muscle gene expression in DIO mice after 3 weeks of subcutaneous injections with vehicle, P1229 (25 mg/kg, twice weekly), P75 (25 mg/kg, twice weekly), P1373 (5, 20, or 50 mg/kg twice weekly), or P1307 (CDD866, or murine bimagrumab, 20 mg/kg once weekly). **FIG. 13A** **-** **FIG. 13B** illustrate *Mss51* expression in gastrocnemius muscle. **FIG. 13C** illustrates changes in the ratio of *Serpine1* to *Id1* expression ratio in soleus muscle. Results were analyzed by one-way ANOVA followed by post-hoc Bonferroni-corrected multiple comparison test; **p<0.05, **p<0.01, ***p<0.001* relative to the vehicle group.

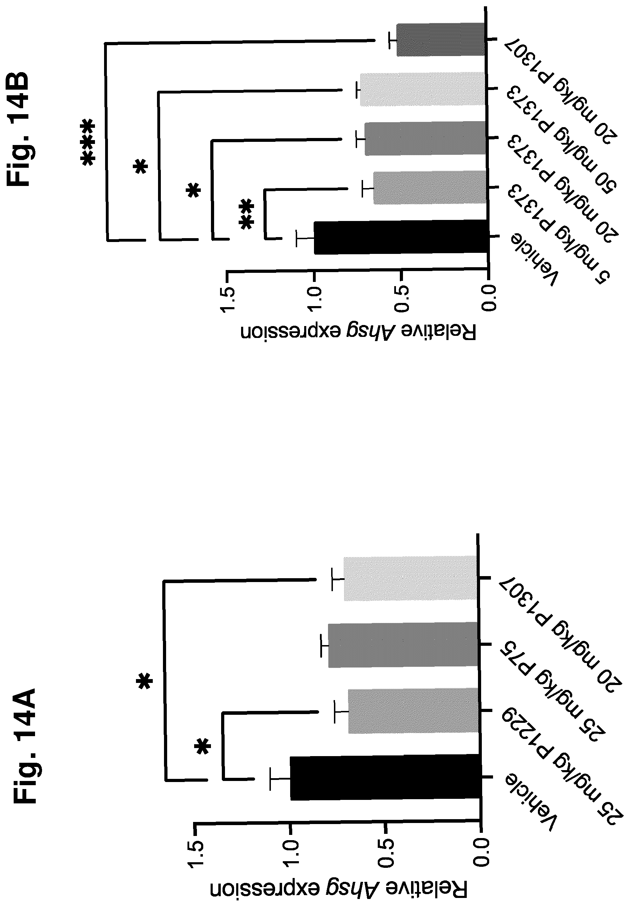

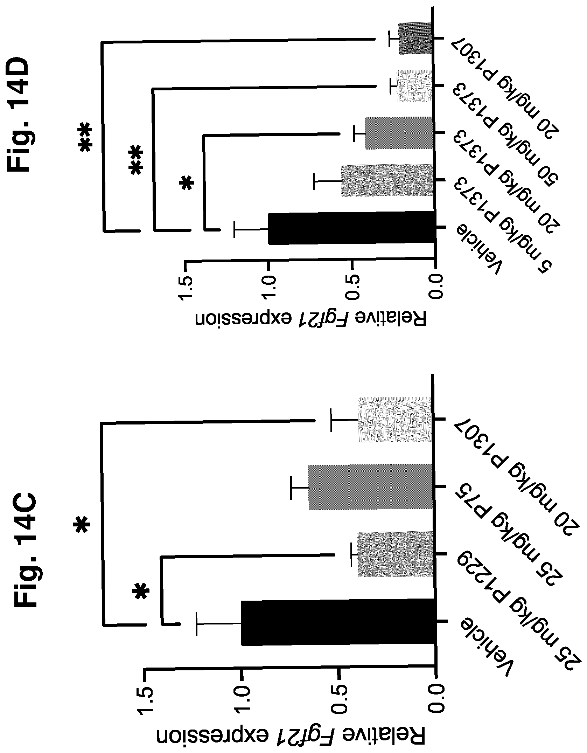

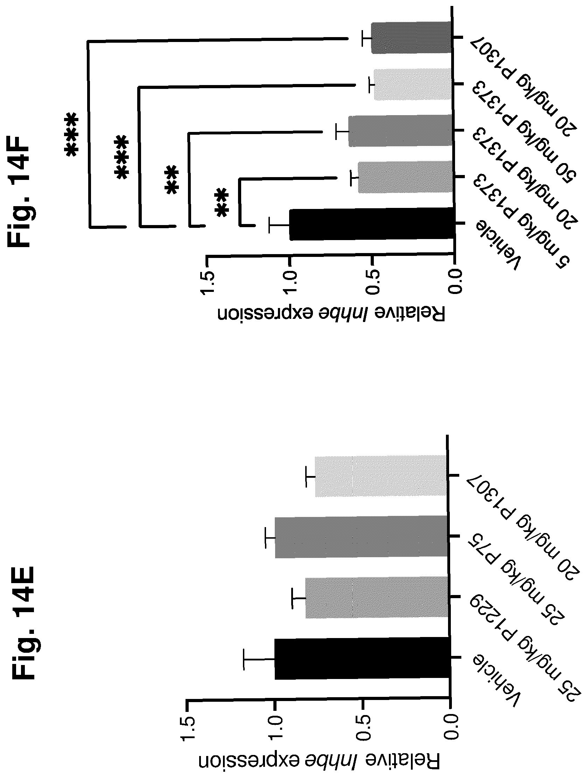

**FIG. 14A** **-** **FIG. 14F** illustrate changes in hepatic gene expression in DIO mice after subcutaneous injections with vehicle, P1229 (25 mg/kg, twice weekly), P75 (25 mg/kg, twice weekly), P1373 (5, 20, or 50 mg/kg twice weekly), or P1307 (CDD866, or murine bimagrumab, 20 mg/kg once weekly). **FIG. 14A** **-** **FIG. 14B** show *Ahsg* gene expression, encoding for fetuin-A. **FIG. 14C** **-** **FIG. 14D** show *Fgf21* gene expression, encoding for FGF21. **FIG. 14E** **-** **FIG. 14F** Show *Inhbe* gene expression, encoding for activin E. Results were analyzed by one-way ANOVA followed by post-hoc Bonferroni-corrected multiple comparison test; *p<0.05, ***p*<0.01, *****p<0.001 relative to the vehicle group.

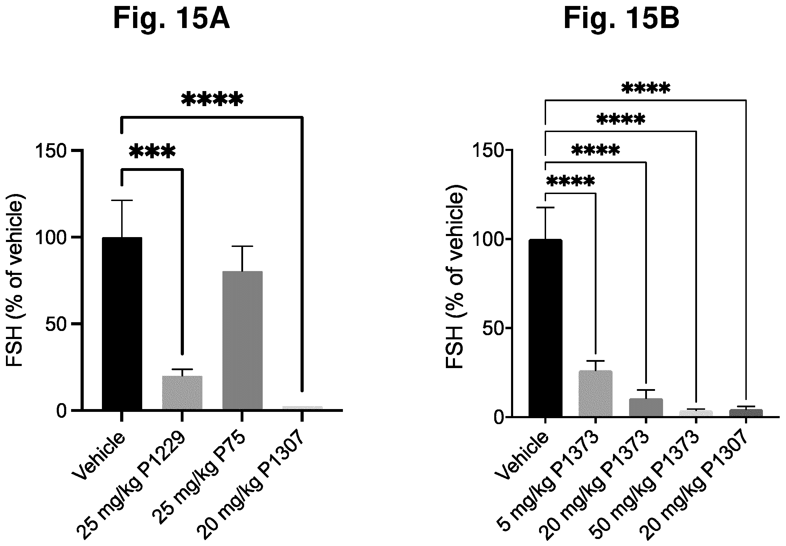

**FIG. 15A** **-** **FIG. 15B** illustrate changes in follicle-stimulating hormone (FSH) production in DIO mice after subcutaneous injections with vehicle, P1229 (25 mg/kg, twice weekly), P75 (25 mg/kg, twice weekly), P1373 (5, 20, or 50 mg/kg twice weekly), or P1307 (CDD866, or murine bimagrumab, 20 mg/kg once weekly). Results were analyzed by one-way ANOVA followed by post-hoc Bonferroni-corrected multiple comparison test; ****p<*0.001, *****p*<0.0001 relative to the vehicle group.

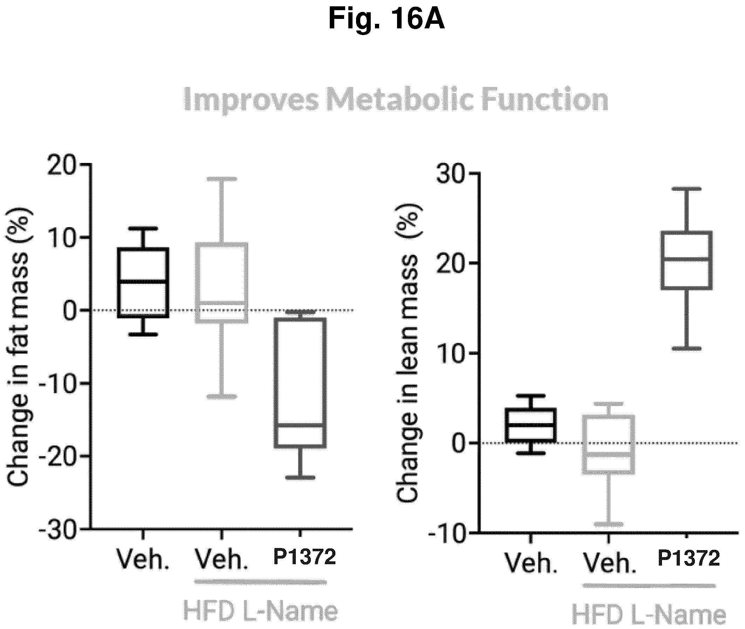

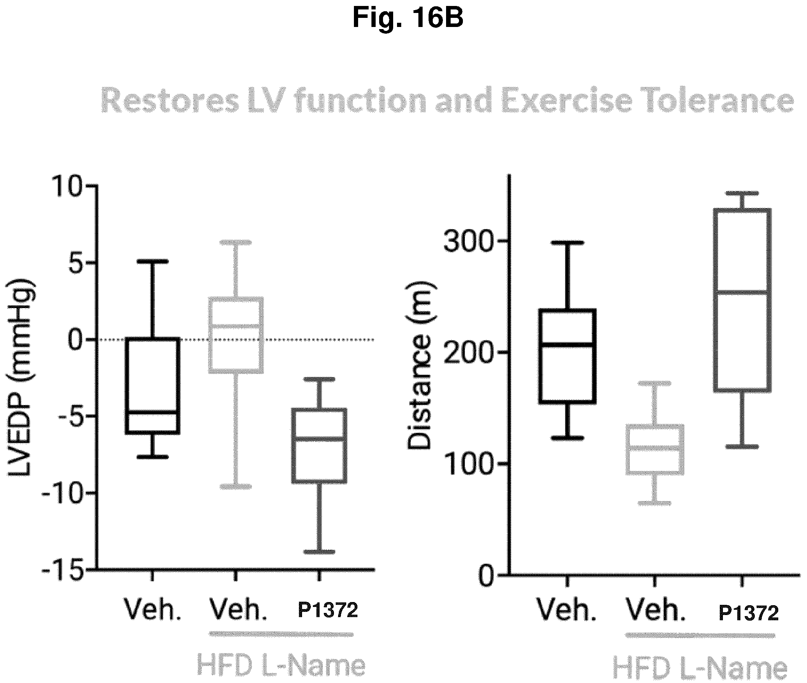

**FIG. 16A** depicts that the administration of P1372 improved the body composition - that is, decreased the % fat mass and increased the % lean mass - in the mice model of HFpEF. **FIG. 16B** depicts that the administration of P1372 to mice model of HFpEF restored the left ventricle function by decreasing the LVEDP, and improved exercise tolerance by increasing the distance covered.

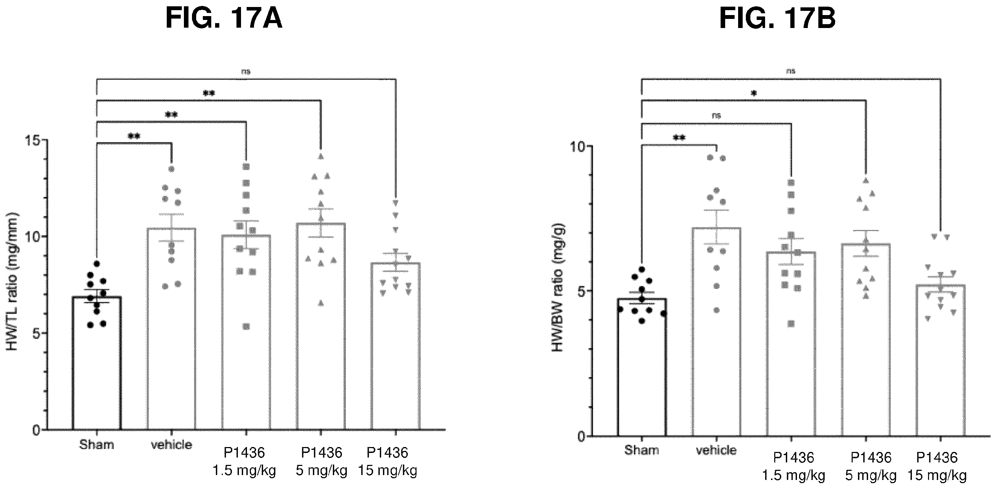

**FIG. 17A** depicts the weight of the total heart (HW) normalized to tibia length (TL). **FIG. 17B** depicts HW normalized to body weight (BW). Each symbol represents one animal, and bars represent the average values per group ± SEM. Data were analyzed by Kruskal-Wallis followed by post-hoc Dunn's multiple comparison test; **p*<0.05*,* ***p*<0.01.

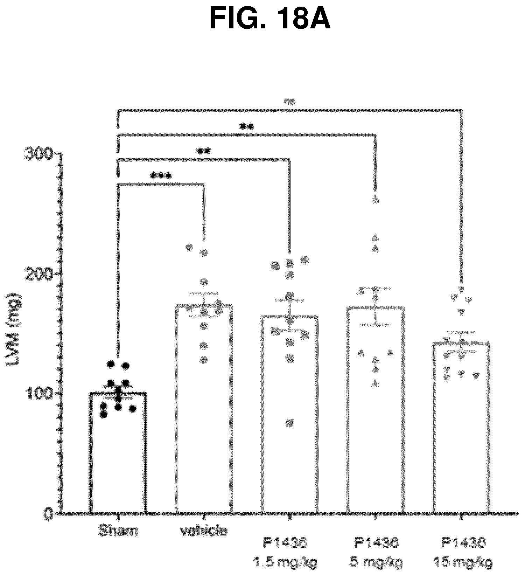

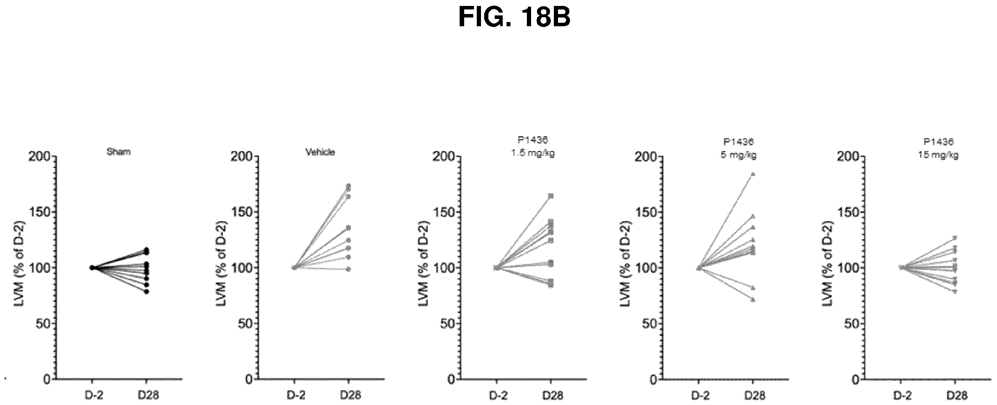

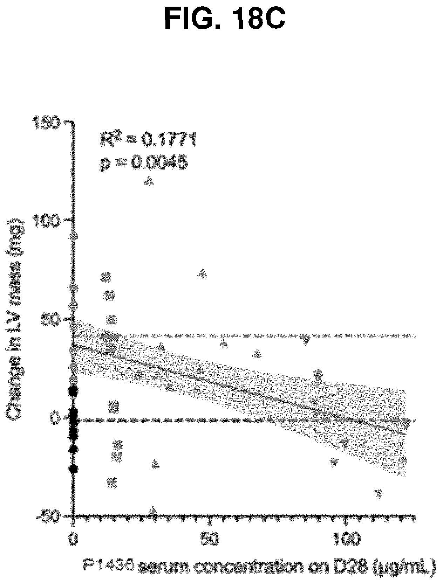

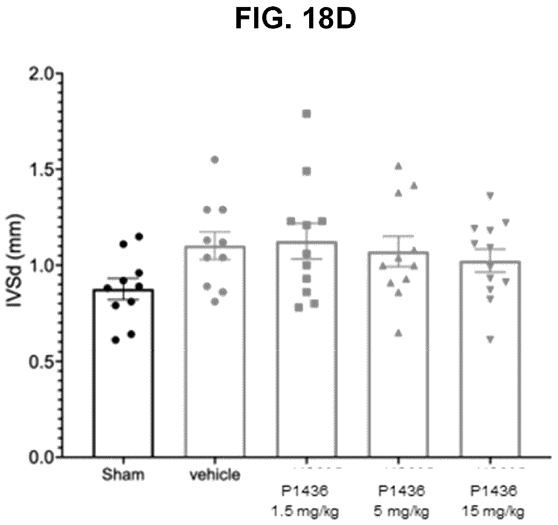

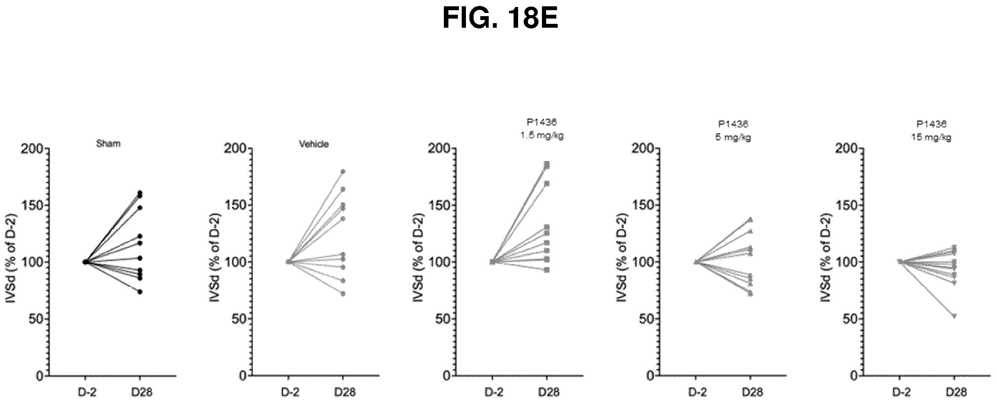

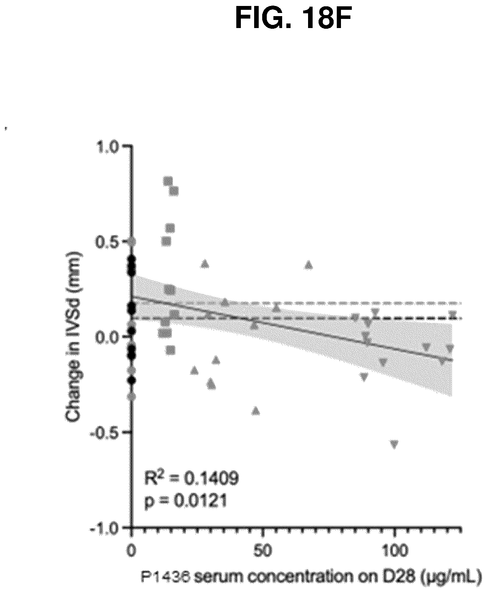

**FIG. 18A** **-** **FIG. 18C** depict left ventricular mass (LVM) and **FIG. 18D** **-** **FIG. 18F** depict intra-ventricular septum diameter (IVSd). Data (**FIG. 18A** and **FIG. 18D**) were obtained by echocardiography on Day 28. Each symbol represents one animal and bars represent the average values per group ± SEM. Data were analyzed by Kruskal-Wallis followed by post-hoc Dunn's multiple comparison test: **p<0.01, ***p<0.001. LVM (**FIG. 18B**) and IVSd (**FIG. 18E**) echocardiography measures were obtained on Day -2 and Day 28. Data were normalized on D-2 for each animal. LVM (**FIG. 18C**) and IVSd (**FIG. 18F**) change measured by echocardiography (Day 28 vs Day -2) correlation with circulating concentration of P1436 measured at Day 28.

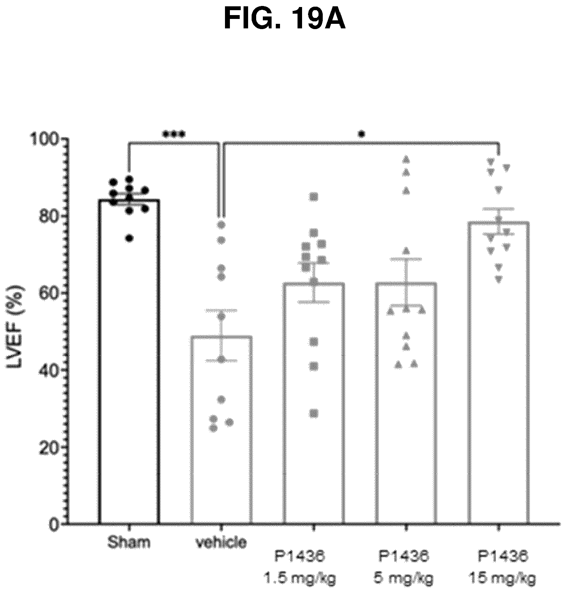

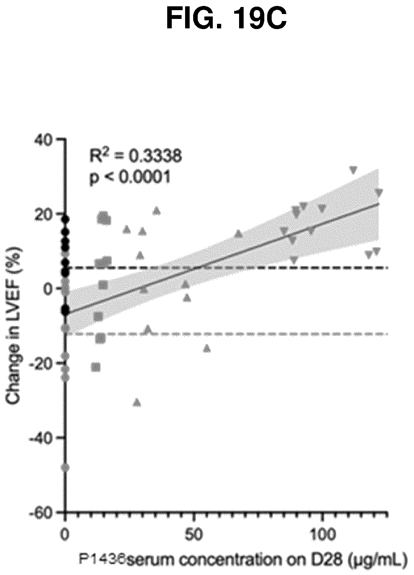

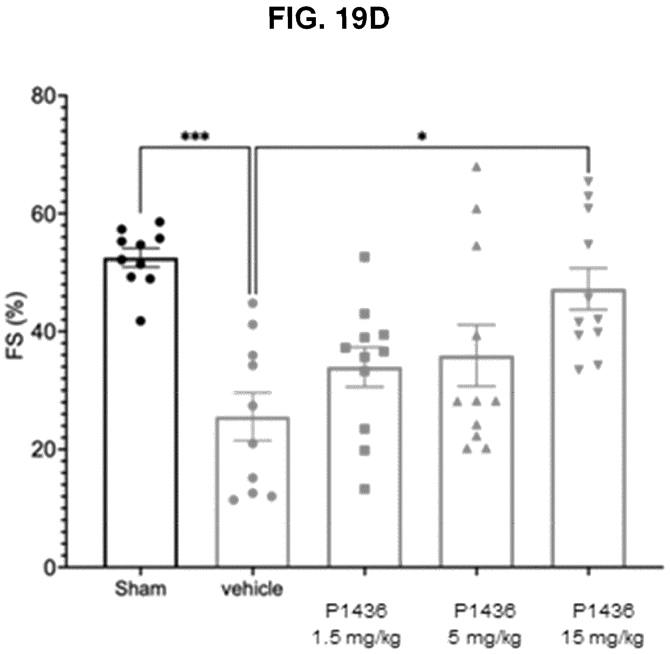

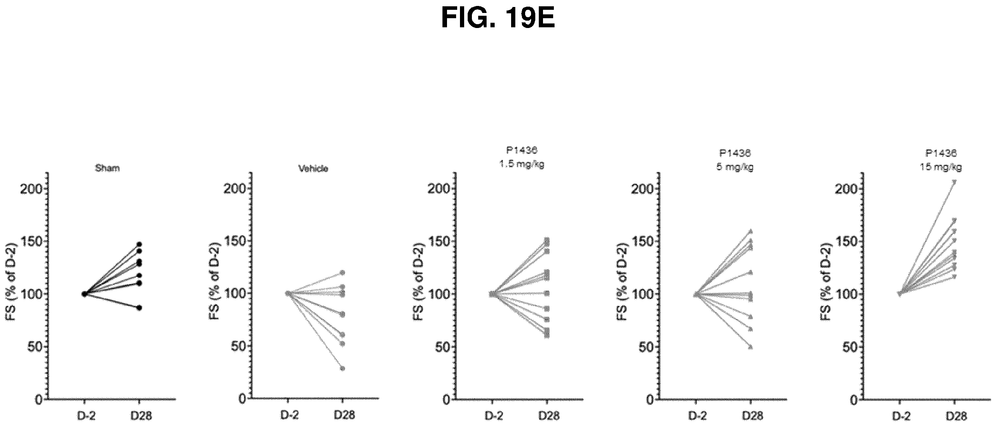

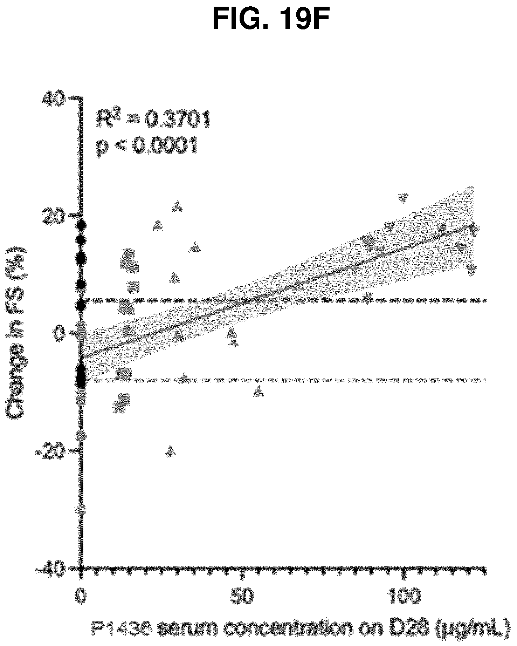

**FIG. 19A** **-** **FIG. 19C** depict left ventricular ejection fraction (LVEF) and **FIG. 19D** **-** **FIG. 19F** depict left ventricular fractional shortening (FS). Data (**FIG. 19A** and **FIG. 19D**) were obtained by echocardiography on Day 28. Each symbol represents one animal and bars represent the average values per group ± SEM. Data were analyzed by Kruskal-Wallis followed by post-hoc Dunn's multiple comparison test: **p<0.01, ***p<0.001. LVEF (**FIG**. **19B**) and FS (**FIG. 19E**) echocardiography measures were obtained on Day -2 and Day 28. Data were normalized on D-2 for each animal. LVEF (**FIG. 19C**) and FS (**FIG. 19F**) change measured by echocardiography (Day 28 vs Day -2) correlation with circulating concentration of P1436 measured at Day 28.

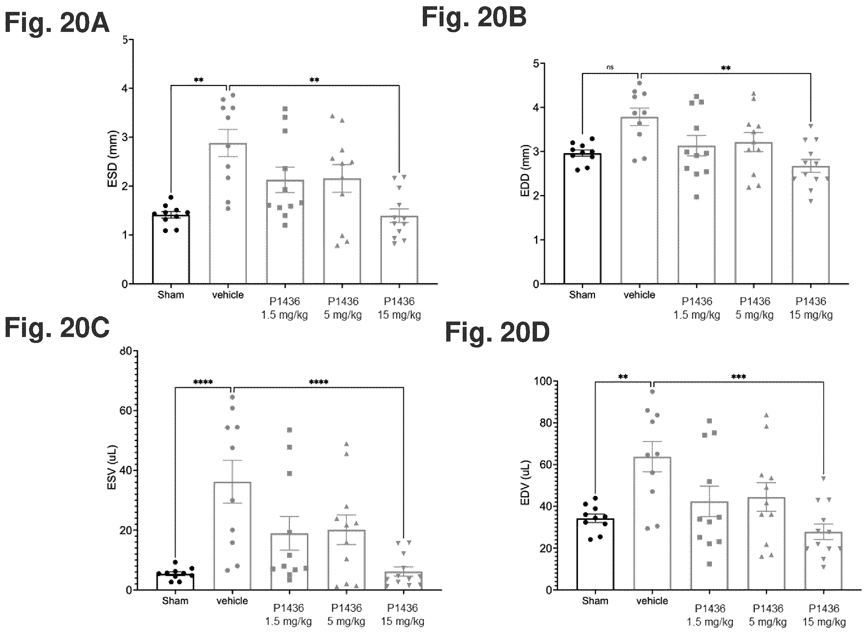

**FIG. 20A** depicts end systolic diameter (ESD) at Day 28. **FIG. 20B** depicts end diastolic diameter (EDD). **FIG. 20C** depicts end systolic volume (ESV). **FIG. 20D** depicts end diastolic volume (EDD). Data were measured by echocardiography. Data were analyzed by Kruskal-Wallis followed by post-hoc Dunn's multiple comparison test: **p<0.01, ***p<0.001, ****p<0.0001.

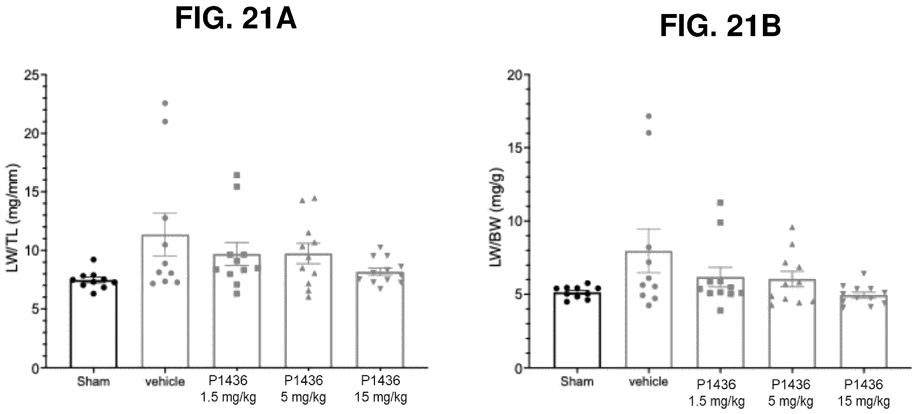

**FIG. 21A** depicts the weight of the lungs (LW) normalized to tibia length (TL). **FIG. 21B** depicts LW normalized to body weight (BW). Each symbol represents one animal, and bars represent the average values per group ± SEM.

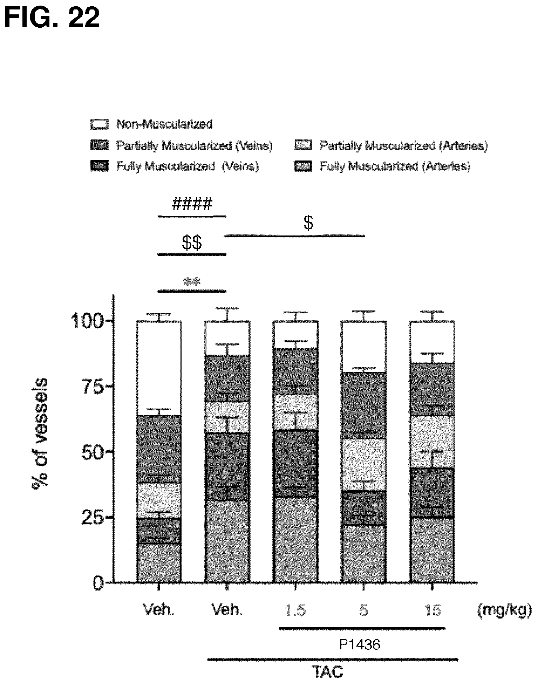

**FIG. 22** depicts vessel muscularization in lungs collected at the end of the study (Day 28). Non-, partially-, and fully-muscularized arteries and veins were scored in 25-30 vessels per animal from αSMA- Verhoeff-stained left lung sections. Vessel exterior diameter ranged from 10 - 50 µm. Bars represent average values across all animals ± SEM. Data were analyzed by two-way ANOVA followed by post-hoc Tukey-corrected multiple comparison test; * p<0.05, ** p<0.05, ****p<0.001. # show statistical comparisons for non-muscularized vessels, * for fully-muscularized arteries, or $ for fully-muscularized veins. Statistical comparisons shown for Sham-Vehicle vs. TAC-vehicle and TAC-vehicle vs. P1436.

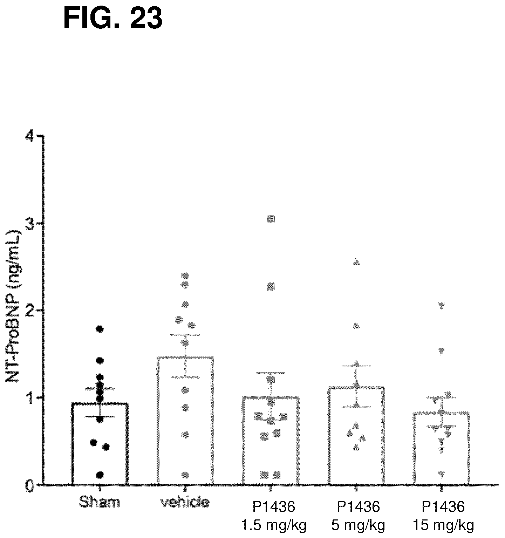

**FIG. 23** depicts circulating levels of NT-proBNP in serum on Day 28. Each symbol represents an animal, and bars represent the average values per group ± SEM.

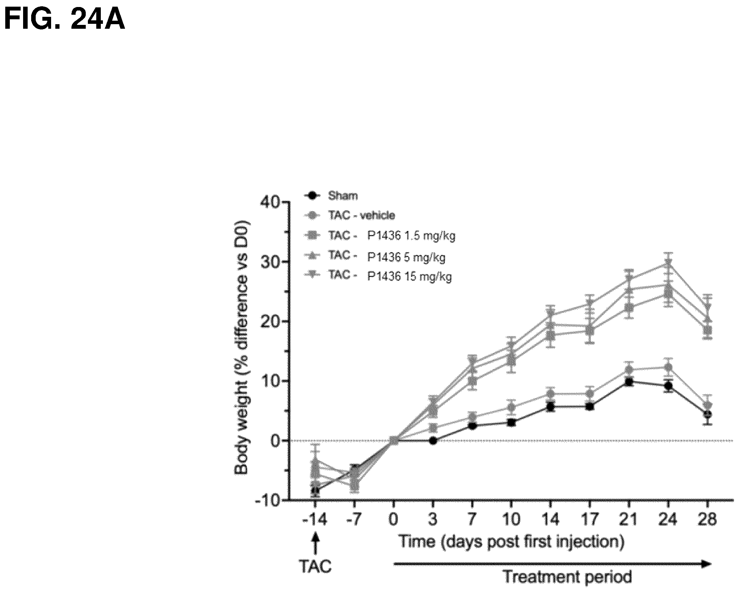

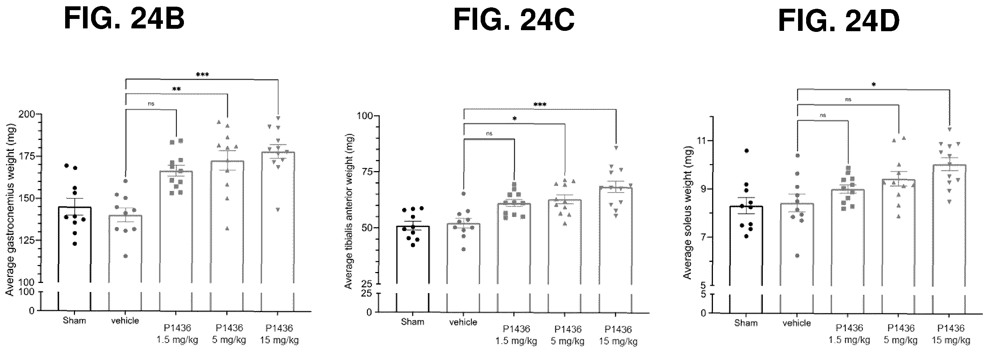

**FIG. 24A** depicts body weight change over the course of the study. Body weight change was normalized to Day 0, corresponding to the 1st injection day, i.e., 2 weeks post-TAC surgery. Data is shown as mean ± SEM. **FIG. 24B** **-** **FIG. 24D** depict skeletal muscle weights. Bilateral skeletal muscles were collected and weighed at Day 28. Animals treated with P1436 showed an exposure-dependent increase in muscle weights relative to vehicle in the gastrocnemius, tibialis anterior, and soleus. Each symbol represents the average of left and right muscles for one animal and bars represent the average values per group ± SEM. Data were analyzed by Kruskal-Wallis followed by post-hoc Dunn's multiple comparison test: * p<0.05, **p<0.01, ***p<0.001.

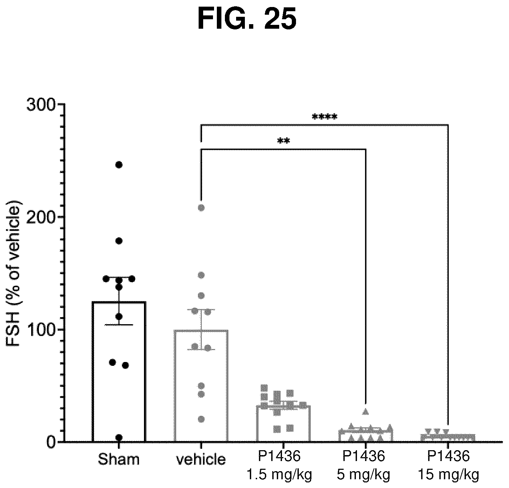

**FIG. 25** depicts levels of circulating follicle-stimulating hormone (FSH) at the end of life by treatment group. Each symbol represents one animal, and bars represent the average values per group ± SEM. Data were analyzed by Kruskal-Wallis followed by post-hoc Dunn's multiple comparison test: **p<0.01, ****p<0.0001.

### DETAILED DESCRIPTION

### Overview

Activin type II receptors are single transmembrane domain receptors that modulate signals for ligands in the TGFβ superfamily. There exist two types of activin type II receptors: ActRIIA and ActRIIB. Examples of ligands in the TGFβ superfamily include activin (e.g., activin A and activin B), inhibin, growth differentiation factors (GDFs) (e.g., GDF-8, also known as myostatin and GDF-11), and bone morphogenetic proteins (BMPs) (e.g., BMP-9, BMP-10). Activity of TGFβ superfamily ligands has been implicated in a variety of diseases and disorders including pulmonary hypertension (PH), fibrosis, muscular diseases (including muscular dystrophy), metabolic disorders (including Type II diabetes), bone diseases, and anemia.

One approach to developing therapeutic agents that inhibit TGFβ superfamily ligand function has been to use soluble decoy receptors (also termed receptor ectodomain (ECD)-based ligand traps) to bind and sequester ligands, thereby blocking access to the cell surface receptors. In general, receptor ECD-based traps are a class of therapeutic agents that are able to selectively sequester ligands, and that can be optimized using protein-engineering approaches. For example, polypeptide fusions based on a TGFβ receptor ectodomain that binds or "traps" the TGFβ1 and/or TGFβ2 and/or TGFβ3 ligand isoforms have been used to inhibit TGFβ signaling (see for example, WO01/83525; WO2005/028517; WO2008/113185; WO2008/157367; WO2010/0031168; WO2010/099219; WO2012/071649; WO2012/142515; WO2013/000234; WO2018/158727; US5693607; US2005/0203022; US2007/0244042; US8318135; US8658135; US8815247; US2015/0225483; US2015/0056199; and WO2017/037634).

In the endothelium and vasculature of the lung, bone morphogenetic proteins (BMPs) can induce anti-proliferative effects in smooth muscle cells (SMCs) and survival of endothelial cells (ECs), while activins and growth differentiation factors (GDFs) can induce opposing effects, i.e., pro-proliferative effects in SMCs and apoptosis of ECs (Yung, L.M. et al., 2020; Ryanto, G.R.T. et al., 2021). Under physiological conditions, these ligands act in concert to maintain homeostasis. However, in certain disease conditions such as PAH these pathways become unbalanced. For example, nearly ~80% of familial and ~20% of idiopathic cases of PAH are caused by mutations in the bone morphogenetic protein (BMP) type 2 receptor (BMPR2) (Austin, E.D. and Loyd, J.E., 2007; Quarck, R. and Perros, F., 2017). This results in an imbalance between activin/GDF and BMP signaling pathways (Ryanto, G.R.T. et al., 2021). It is desirable therefore to provide a receptor ectodomain-based trap that can neutralize certain ligands and not others, in order to rebalance the pathways and to re-establish vascular homeostasis.

The present application therefore provides TGFβ superfamily ligand binding agents that demonstrate improved ligand binding profiles and therapeutic efficacy.

The present technology is explained in greater detail below. This description is not intended to be a detailed catalog of all the different ways in which the technology may be implemented, or all the features that may be added to the instant technology. For examples, features illustrated with respect to one embodiment may be incorporated into other embodiments, and features illustrated with respect to a particular embodiment may be deleted from that embodiment. In addition, numerous variations and additions to the various embodiments suggested herein will be apparent to those skilled in the art in light of the instant disclosure, which variations and additions do not depart from the present technology. Hence, the following description is intended to illustrate some particular embodiments of the technology, and not to exhaustively specify all permutations, combinations and variations thereof.

### Definitions

In order to provide a clear and consistent understanding of the terms used in the present specification, a number of definitions are provided below. Moreover, unless defined otherwise, all technical and scientific terms as used herein have the same meaning as commonly understood to one of ordinary skill in the art to which this invention pertains.

The use of the terms "a" and "an" and "the" when used in conjunction with the term "comprising" in the claims and/or the specification may mean "one", but it is also consistent with the meaning of "one or more", "at least one", and "one or more than one". Similarly, the term "another" may mean at least a second or more. These terms are to be construed to cover both the singular and the plural, unless otherwise indicated herein or clearly contradicted by context.

As used herein, the terms "comprising" (and any form of comprising, such as "comprise" and "comprises"), "having" (and any form of having, such as "have" and "has"), "including" (and any form of including, such as "include" and "includes") or "containing" (and any form of containing, such as "contain" and "contains"), are inclusive or open-ended and do not exclude additional, unrecited elements or process steps. The term "consisting of" is to be construed as close-ended.

The term "about" is used to indicate that a value or quantity refers to the actual given value and also the approximation of such given value that would reasonably be inferred based on the ordinary skill in the art, including equivalents and approximations due to the experimental and/or measurement conditions for such given value. For example, the term "about" in the context of a given value or range refers to a value or range that is within 20%, preferably within 15%, more preferably within 10%, more preferably within 9%, more preferably within 8%, more preferably within 7%, more preferably within 6%, and more preferably within 5% of the given value or range.

The expression "and/or" where used herein is to be taken as specific disclosure of each of the specified features or components with or without the other. For example, "A and/or B" is to be taken as specific disclosure of each of (i) A, (ii) B, and (iii) A and B, just as if each is set out individually herein. Unless specifically stated or obvious from context, as used herein the term "or" is understood to be inclusive and covers both "or" and "and". For example, an embodiment of "a composition comprising A or B" would typically present an aspect with a composition comprising both A and B. "Or" should, however, be construed to exclude those aspects presented that cannot be combined without contradiction (e.g., a composition pH that is between 9 and 10 or between 7 and 8).

It is to be understood herein that terms such as "from 1 to 20" include any individual values comprised within and including 1 and 20. Therefore, the term "from 1 to 20" includes 1, 2, 3, 4, 5, 6, 7, 8, 9, 10, 11, 12, 13, 14, 15, 16, 17, 18, 19 and/or 20. Terms such as "from 1 to 20" also include any individual sub-ranges comprised within and including from 1 to 20. The term "from 1 to 20" therefore also includes sub-ranges such as "from 1 to 9", "from 2 to 9", "from 3 to 5", from 5 to 9", "from 5 to 20", "from 8 to 20" etc. The same applies for similar expressions such as and not limited to "from 1 to 19", "from 1 to 18", "from 1 to 10", "from 1 to 9", "from 5 to 15", etc.

It is to be understood herein that terms such as "from about 15 to about 35" include any individual values comprised within and including 15 and 35. Therefore, terms such as "from about 15 to about 35" include any number between and including 15 and 35 such as 15, 16, 17, 18, 19, 20, 21, 22, 23, 24, 25, 26, 27, 28, 29, 30, 31, 32, 33, 34 and/or 35. Terms such as "from about 15 to about 35" also include any individual sub-ranges comprised within and including from 15 to 35, "from about 16 to about 34", "from about 16 to about 24", from about 24 to about 34" and the like. The term "about" in the context of the number of amino acids means that the specified number of amino acids is specifically encompassed and allows a variation of +/- 2 in the number of amino acid residues. As such, the terms such as "from about 15 to about 35" also includes "from 13 to 37", "from 13 to 35", "from 17 to 37", from 17 to 35", etc. The same applies for similar expressions such as and not limited to "from about 16 to about 34", "from about 16 to about 24", from about 24 to about 34" and the like.

It is to be understood herein that terms such as "at least 80% identical" include any individual values comprised within and including from 80% to 100% and including 80%, 81%, 82%, 83%, 84%, 85%, 86%, 87%, 88%, 89%, 90%, 91%, 92%, 93%, 94%, 95%, 96%, 97%, 98%, 99% and 100%. The term "at least 80% identical" also includes any individual sub-ranges comprised within and including from 80% to 100%, such as for example, "from 85% to 99%", "from 97% to 100%", "from 90% to 100%", etc. The same applies for similar expressions such as, and not limited to, expressions such as "at least 70% identical", "at least 90% identical", and the like.

As used herein, the term "IC50" refers to the half maximal inhibitory concentration (i.e., the concentration of a substance that is required for 50% inhibition in vitro). It is a measure of the potency or effectiveness of a substance in inhibiting a specific biological or biochemical function. IC50 values are typically expressed as molar concentration. The IC50 of an inhibitor can be determined by constructing a dose-response curve and examining the effect of different concentrations of inhibitor on the specific biological or biochemical function in question.

As used herein, the term "inhibition potency" refers to effectiveness of a substance in inhibiting a specific biological or biochemical function such as, without limitation, binding between a protein receptor and its ligand, or activation of a cell receptor by its ligand. In some embodiments, potency of inhibition is determined by measuring the IC50 of an inhibitor for a particular ligand or substrate. In that case, relative inhibition potency for different inhibitors and/or ligands may be assessed by comparing IC50 values. For example, a relative inhibition potency of 3:1 means the ratio of IC50 values for two substances being compared is 3:1, wherein the first substance has a lower inhibition potency (i.e., a greater IC50) than the second substance. A relative inhibition potency of 1:3 means the ratio of IC50 values for two substances being compared is 1:3, wherein the first substance has a greater inhibition potency (i.e., a lower IC50) than the second substance. As the IC50 of an inhibitor can vary depending on the assay conditions, relative inhibition potency for different inhibitors and/or ligands is generally determined by comparing IC50 values obtained under the same assay conditions. The terms "inhibition potency", "inhibitory potency", "potency of inhibition" and "neutralization potency" are used interchangeably herein.

As used herein, the term "substantially the same" in reference to relative inhibition potency means that two proteins have a relative inhibition potency that is about the same, e.g., no more than about 2-fold different (+/- 2-fold) under the same experimental conditions, e.g., the ratio of IC50 values for the two proteins is about 2:1, 1:2, or 1:1.

As used herein, the term "functionally equivalent" refers to variant sequences that have the same or substantially the same biological activity or function as the original sequence from which it is derived, e.g., no significant change in physiological, chemical, physiochemical or functional properties compared to the original sequence. The term "substantially identical" refers to sequences that are functionally equivalent to the original or reference sequence and have a high degree of sequence identity thereto. Generally, a substantially identical sequence is at least about 80%, at least about 85%, at least about 90%, at least about 95%, at least about 96%, at least about 97%, at least about 98%, or at least about 99% identical to the original or reference sequence and has the same function. In some cases when referring to nucleic acid sequences, a substantially identical sequence hybridizes to the original sequence under high stringency conditions, for example at salt and temperature conditions substantially equivalent to 0.5 X SSC to about 5 X SSC and 65° C for both hybridization and wash.

The term "dimeric" refers to the presence of two polypeptides as described herein in a TGFβ superfamily ligand binding agent (also referred to herein as a "binding agent"). "Homodimeric" means the two polypeptides have the same amino acid sequence, whereas "heterodimeric" means the two polypeptides have different amino acid sequences.

The term "divalent" refers to the presence of two TGFβR superfamily ligand binding regions (e.g., the ectodomains) in a TGFβ superfamily ligand binding agent.

As used herein, a "recombinant polypeptide" is a polypeptide made through the use of recombinant DNA technology or genetic engineering. In the context of the present disclosure, recombinant polypeptides are often referred to as "polypeptide constructs" or simply as "polypeptides".

Proteins (including fragments thereof, preferably biologically active fragments, and peptides, usually having less than 30 amino acids) comprise two or more amino acids coupled to each other via a covalent peptide bond (resulting in a chain of amino acids). The term "polypeptide" as used herein describes a group of molecules, which usually consist of more than 10 amino acids. The terms "polypeptide", "polypeptide chain" and "chain" are used interchangeably herein. Polypeptides may further form multimers such as dimers, trimers, and higher oligomers, i.e., consisting of more than one polypeptide molecule. Polypeptide molecules forming such dimers, trimers etc. may be identical or non-identical. The corresponding higher order structures of such multimers are, consequently, termed homo- or heterodimers, homo- or heterotrimers etc. An example for a heteromultimer is an antibody molecule, which, in its naturally occurring form, consists of two identical light polypeptide chains and two identical heavy polypeptide chains. The terms "peptide", "polypeptide" and "protein" also refer to naturally modified peptides/polypeptides/proteins wherein the modification is effected, e.g. by post-translational modifications like glycosylation, acetylation, phosphorylation and the like. A "peptide", "polypeptide" or "protein" when referred to herein may also be chemically modified such as pegylated. Such modifications are well known in the art and described herein.

As used herein, the terms "(specifically) binds to", (specifically) recognizes", "specific for", "is (specifically) directed to", and "(specifically) reacts with" mean that a polypeptide interacts or specifically interacts with a given target(s), such as a specific member(s) of the TGFβ superfamily of ligands. Specific binding is believed to be effected by specific motifs in the amino acid sequence of a polypeptide. Thus, binding is achieved as a result of their primary, secondary and/or tertiary structure as well as the result of secondary modifications of said structures. The specific interaction of the target-interaction-site with its specific target may result in a simple binding of said site to the target. Moreover, the specific interaction of the target-interaction-site with its specific target may alternatively or additionally result in the initiation of a signal, e.g. due to the induction of a change of the conformation of the target, an oligomerization of the target, etc., or may block the target from performing another activity, such as binding to an endogenous receptor.

Generally, binding is considered specific when the binding affinity is about 10-12 to 10-9 M, 10-12 to 10-19 M, 10-11 to 10-9 M, or of about 10-11 to 10-9 M. Whether a polypeptide or binding agent specifically reacts with or binds to a target can be tested readily by, inter alia, comparing the reaction of the polypeptide or binding agent with a target with the reaction of the polypeptide or binding agent with other proteins. In some embodiments, a polypeptide or binding agent of the disclosure does not substantially bind to TGFβ superfamily ligands other than the desired ligands, e.g., does not substantially bind to BMP-9.

As used herein, the term "does not substantially bind" or "is not capable of binding" means that a polypeptide or binding agent of the present disclosure does not demonstrate detectable binding to a given target, e.g., does not show reactivity of more than 30%, not more than 20%, not more than 10%, or not more than 9%, 8%, 7%, 6%, 5% or 3% with the given target.

As used herein, the term "selectively binds" is used to mean that a polypeptide binds to a target site that is not shared with other proteins. In general, a selective binding agent will not cross-react with other proteins and exclusively binds to the designated target protein(s). In the context of the present disclosure, "selective for activin A and GDF-8" means that a polypeptide or binding agent binds or neutralizes the activin A and GDF-8 ligands exclusively, without substantially binding or neutralizing other TGFβ superfamily ligands such as, e.g., BMP-9.

"Half-life" means the time where 50% of an administered drug is eliminated through biological processes, e.g., metabolism, excretion, etc.

"Hepatic first-pass metabolism" refers to the propensity of a drug to be metabolized upon first contact with the liver, i.e., during its first pass through the liver.

"Volume of distribution" refers to the degree of retention of a drug throughout the various compartments of the body, such as, e.g., intracellular and extracellular spaces, tissues and organs, etc. and the distribution of the drug within these compartments.

"Degree of blood serum binding" refers to the propensity of a drug to interact with and bind to blood serum proteins, such as albumin, leading to a reduction or loss of biological activity of the drug.

The term "amino acid" or "amino acid residue" typically refers to an amino acid having its art recognized definition such as an amino acid selected from the group consisting of: alanine (Ala or A); arginine (Arg or R); asparagine (Asn or N); aspartic acid (Asp or D); cysteine (Cys or C); glutamine (Gln or Q); glutamic acid (Glu or E); glycine (Gly or G); histidine (His or H); isoleucine (He or I): leucine (Leu or L); lysine (Lys or K); methionine (Met or M); phenylalanine (Phe or F); pro line (Pro or P); serine (Ser or S); threonine (Thr or T); tryptophan (Trp or W); tyrosine (Tyr or Y); and valine (Val or V), although modified, synthetic, or rare amino acids may be used as desired. Generally, amino acids can be grouped as having a nonpolar side chain (e.g., Ala, Cys, He, Leu, Met, Phe, Pro, Val); a negatively charged side chain (e.g., Asp, Glu); a positively charged sidechain (e.g., Arg, His, Lys); or an uncharged polar side chain (e.g., Asn, Cys, Gin, Gly, His, Met, Phe, Ser, Thr, Trp, and Tyr).

In a similar manner, "percent (%) nucleic acid sequence identity" with respect to the nucleic acid sequence of the polypeptides or binding agents identified herein is defined as the percentage of nucleotide residues in a candidate sequence that are identical with the nucleotide residues in the coding sequence of the polypeptides or binding agents. A specific method utilizes the BLASTN module of WU-BLAST-2 set to the default parameters, with overlap span and overlap fraction set to 1 and 0.125, respectively.

### TGFβ superfamily ligand binding agents

In some embodiments, the present disclosure provides TGFβ superfamily ligand binding agents comprising an ActRIIB-ECD region, a linker region, and an Fc domain (also referred to herein as "binding agents" or "TGFβ ligand binding agents"). The individual components of the binding agents described herein are described in further detail in the following sections. In general, however, the binding agents described herein are dimeric proteins comprising two polypeptides each comprising an ActRIIB-ECD, a peptide linker, and an Fc domain monomer. The two polypeptides assemble via the Fc domain monomers to form the dimeric binding agents described herein. See schematic in Fig. 8. When assembled, the Fc domain monomers in each of the polypeptides form a dimeric Fc domain at one terminus and a divalent ActRIIB-ECD region at the other terminus. Binding agents of the present disclosure can bind to one or more ligand selected from activin A, activin B, GDF-8, and GDF-11 and inhibit signaling of the one or more ligand through their respective receptors, without substantially binding to BMP-9 and/or BMP-10, and/or inhibiting BMP-9 and/or BMP-10 signaling through its receptor. Binding agents may also have further biological activities or functions such as binding to other ligands or targets and the like, as further described herein.

In some embodiments, the binding agents of the present disclosure include two polypeptide chains that are associated via an Fc domain monomer of an antibody or via a constant CH2 domain, a constant CH3 domain and/or via a combination of CH2 and CH3. The constant region of the antibody may be from a human IgG1, IgG2, IgG3 or IgG4 antibody, or substantially identical thereto. The association of both polypeptide chains generally occurs during expression and secretion of the protein, e.g. in mammalian cells. The Fc domain monomer generally comprises a CH2, a CH3, or a CH2 and a CH3 from an antibody heavy chain that is of human origin and typically provides for disulfide crosslinking between single chain polypeptides. In an embodiment, the Fc domain monomer provides for at least one disulfide link between single chain polypeptides. In another embodiment, the Fc domain monomer provides for at least two disulfide links between single chain polypeptides. In some cases, the antibody heavy chain also provides for Protein A-based isolation of the dimeric polypeptide, e.g. after production in host cells.

As noted above, certain TGFβ superfamily ligand binding agents and point mutations in the ECD are described in the art. See *e.g.,* WO 2021/158675; WO 2022/150590; WO 2021/158675; WO 2022/072882; WO 2021/189019; and WO 2021/189010. Although point mutations in the ActRIIB ECD have been described in the context of other TGFβ superfamily ligand binding agents, the effects of these mutations in the context of these previously described agents do not predict the effects of these same mutations in the context of the binding agents described herein. See e.g.*,* PCT/CA2023/050116 describing the unpredictability of point mutations in the ActRIIB ECD when combined with linkers of varying lengths. As such, the efficacy of a particular binding agent described herein is determined not only by the mutations comprised in the extracellular ligand binding domain, but also by the length of the linker used. As shown herein, the length of the linker connecting the ActRIIB ECD to the Fc domain has unpredictable effects on binding to and inhibition of TGFβ superfamily ligands. The present application therefore provides TGFβ superfamily ligand binding agents that demonstrate improved ligand binding profiles and therapeutic efficacy. These compounds are useful in the treatment of various diseases and disorders driven by TGFβ superfamily ligands including pulmonary hypertension, muscular diseases, metabolic disorders, bone diseases, anemia, and fibrosis.

Additional ECD-based traps have been evaluated clinically, *e.g*., luspatercept and sotatercept. Luspatercept (also known as ACE-536, REBLOZYL^{®}) is a soluble fusion protein composed of a modified form of the extracellular domain of the activin receptor type IIB (ActRIIB) linked to the Fc portion of human IgG1. Luspatercept inhibits several endogenous TGFβ superfamily ligands, thereby diminishing Smad2/3 signaling. It is used for the treatment of anemia in beta thalassemia and myelodysplastic syndromes. For a description of luspatercept and other related fusion proteins, see for example U.S. Patent Nos. 7,842,663; 8,058,229; 8,216,997; 8,252,900; 8,343,933; 8,361,957; 8,703,927; 9,138,459; 9,399,669; 9,439,945; 9,932,379; 10,131,700; 10,259,861; 10,689,427; and 10,829,532.

Sotatercept (also known as ACE-011) is a soluble decoy receptor that is composed of the extracellular domain of the activin receptor type IIA (ActRIIA) linked to the Fc portion of human IgG1, and that is able to bind and neutralize activins and GDFs. Sotatercept has been evaluated in healthy volunteers, and in patients with conditions characterized by dysfunctional TGF-β superfamily signaling including hematologic disorders, bone loss, chemotherapy-induced anemia, multiple myeloma, myelodysplastic syndromes, β-thalassemia, and end-stage kidney disease (Raftopoulos, H. et al., 2016; Abdulkadyrov, K.M. et al., 2014; Ruckle, J. et al. 2009; Komrokji, R. et al., 2018; Cappellini, M.D. et al., 2019; Coyne, D.W. et al., 2019; Sherman, M.L. et al., 2013). Most recently, sotatercept has been evaluated for the treatment of pulmonary arterial hypertension (PAH).

By acting as a ligand trap for activins and GDFs, sotatercept may correct the imbalance between the growth-promoting activin/growth differentiation factor pathway and the growth-inhibiting BMP pathway that occurs in PAH. In a Phase 2 trial in PAH patients, sotatercept was shown to reduce pulmonary vascular resistance (Humbert, M. et al., 2021). Additional PH trials, including a Phase 3 trial, are ongoing or planned. For a description of sotatercept and other related fusion proteins, see for example U.S. Patent Nos. 7,612,041; 7,709,605; 7,951,771; 7,988,973; 8,007,809; 8,629,109; 8,895,016; and 9,163,075. However, in some clinical studies of sotatercept, vascular and hematologic side effects have been found to be dose limiting, restricting the potential therapeutic efficacy. For example, a multiple ascending dose study in healthy, postmenopausal women planned to evaluate four doses of 0.1, 0.3, 1 mg/kg and 2 mg/kg but was terminated early at the 1 mg/kg level as increases in hemoglobin, hematocrit and red blood cell counts were found to be dose limiting (Sherman, M.L. et al., 2013). In a Phase 2 clinical trial in PAH patients, thrombocytopenia and an increased hemoglobin level were the most common hematologic adverse events, with 17% of patients who received a dose of 0.7 mg/kg experiencing an adverse event of increased hemoglobin (Humbert, M. et al., 2021). Such vascular and hematologic side effects are dose-limiting as they may not allow for administration of dosages required for maximal efficacy, restricting the potential to achieve maximum therapeutic effect (Humbert, M. et al., 2021). In contrast, the binding agents provided herein do not induce hematological effects in non-human primates, suggesting that these agents may have a broader therapeutic window than sotatercept.

In some embodiments, binding agents of the present disclosure comprise homodimers, i.e., dimers of a polypeptide having the sequence set forth in any one of SEQ ID NOs: 174-251, or a sequence at least 85%, at least 90%, at least 95%, at least 96%, at least 97%, at least 98%, or at least 99% identical thereto, or a functionally equivalent variant thereof. In other embodiments, binding agents comprise heterodimers, i.e., dimers of two different polypeptides, at least one of the polypeptides having the sequence set forth in any one of SEQ ID NOs: 174-251, or a sequence at least 85%, at least 90%, at least 95%, at least 96%, at least 97%, at least 98%, or at least 99% identical thereto, or a functionally equivalent variant thereof.

In some embodiments, the binding agents of the present disclosure comprise a dimer of a polypeptide comprising an ActRIIB-ECD comprising an L33W mutation and a long peptide linker. In some embodiments, the peptide linker is, or is at least, 10 amino acids in length. In some embodiments, the peptide linker is, or is at least, 14 amino acids in length. In some embodiments, the peptide linker is, or is at least, 19 amino acids in length. In some embodiments, the peptide linker is, or is at least, 39 amino acids in length. In some embodiments, the binding agents of the present disclosure comprise a dimer of a polypeptide comprising or consisting of an amino acid sequence selected from SEQ ID NOs: 211 and 230-234, or a sequence that is at least 85%, at least 90%, at least 95%, at least 96%, at least 97%, at least 98%, or at least 99% identical thereto, or a functionally equivalent variant thereof.

In some embodiments, the binding agents of the present disclosure comprise a dimer of a polypeptide comprising or consisting of SEQ ID NO: 211, or a sequence that is at least 85%, at least 90%, at least 95%, at least 96%, at least 97%, at least 98%, or at least 99% identical thereto, or a functionally equivalent variant thereof. In some embodiments, the binding agents of the present disclosure comprise a dimer of a polypeptide comprising or consisting of SEQ ID NO: 230, or a sequence that is at least 85%, at least 90%, at least 95%, at least 96%, at least 97%, at least 98%, or at least 99% identical thereto, or a functionally equivalent variant thereof. In some embodiments, the binding agents of the present disclosure comprise a dimer of a polypeptide comprising or consisting of SEQ ID NO: 231, or a sequence that is at least 85%, at least 90%, at least 95%, at least 96%, at least 97%, at least 98%, or at least 99% identical thereto, or a functionally equivalent variant thereof. In some embodiments, the binding agents of the present disclosure comprise a dimer of a polypeptide comprising or consisting of SEQ ID NO: 232, or a sequence that is at least 85%, at least 90%, at least 95%, at least 96%, at least 97%, at least 98%, or at least 99% identical thereto, or a functionally equivalent variant thereof. In some embodiments, the binding agents of the present disclosure comprise a dimer of a polypeptide comprising or consisting of SEQ ID NO: 233, or a sequence that is at least 85%, at least 90%, at least 95%, at least 96%, at least 97%, at least 98%, or at least 99% identical thereto, or a functionally equivalent variant thereof. In some embodiments, the binding agents of the present disclosure comprise a dimer of a polypeptide comprising or consisting of SEQ ID NO: 234, or a sequence that is at least 85%, at least 90%, at least 95%, at least 96%, at least 97%, at least 98%, or at least 99% identical thereto, or a functionally equivalent variant thereof.

### Activin receptor type IIB ectodomain variants

As used herein, the term "Activin receptor type IIB ectodomain variants" or "ActRIIB-ECD variants" refers to a polypeptide comprising the soluble, extracellular portion of the single transmembrane receptor, ActRIIB, that has at least one amino acid substitution relative to a wild type extracellular ActRIIB. The sequence of the wild type human ActRIIB-ECD is shown in SEQ ID NO: 2 (**Table 1**). Unless otherwise noted, indicated positions for amino acid substitutions are numbered according to the amino acid sequence of SEQ ID NO: 2. For the purposes of this disclosure, the "human wild type ActRIIB-ECD" refers to SEQ ID NO: 2.

In some embodiments, the ActRIIB-ECD variant comprises one or more amino acid substitutions at a position selected from L14, G27, L33, L55, and T69. In some embodiments, the ActRIIB-ECD variant polypeptide comprises one or more amino acid substitutions selected from L14E, L14H, L14S, L14N, L14Q, L14D, G27E, G27D, G27N, G27Q, G27K, G27T, G27M, L33R, L33Y, L33F, L33Q, L33W, L33E, L33K, L33M, L55Y, L55Q, L55M, L55I, L69H, L69Q, L69E, L69R, L69Y, and L69W. In some embodiments, the ActRIIB-ECD variant comprises one or more amino acid substitutions selected from L33R, L33Y, L33F, L33Q, L33W, L33E, L33K, and L33M. In some embodiments, the ActRIIB-ECD variant comprises an amino acid substitution of L33Y. In some embodiments, the ActRIIB-ECD variant comprises an amino acid substitution of L33W. Other amino acid substitutions in the ActRIIB-ECD are known in the art (*e.g*., WO 2021/158675; WO 2022/150590; WO 2021/158675; WO 2022/072882; WO 2021/189019; and WO 2021/189010, each of which are incorporated herein by reference). These additional mutations can be used in combination with the linkers described herein, and incorporated into the binding agents described herein, to alter ligand binding properties of the ActRIIB-ECD.

In some embodiments, the ActRIIB-ECD variant comprises the amino acid sequence set forth in any one of SEQ ID NOs: 4-62. In some embodiments, an ActRIIB-ECD variant comprises at least 85% (e.g., at least 85%, at least 87%, at least 90%, at least 92%, at least 95%, at least 96%, at least 97%, at least 98%, at least 99%, or greater) amino acid sequence identity to the sequence of a wild type human ActRIIB-ECD. In some embodiments, an ActRIIB-ECD variant may have at least 85% (e.g., at least 85%, at least 87%, at least 90%, at least 92%, at least 95%, at least 96%, at least 97%, at least 98%, at least 99%, or greater) amino acid sequence identity to the sequence set forth in SEQ ID NO: 2.

In some embodiments, the amino acid sequence of the ActRIIB-ECD variant comprises at least 95%, at least 96%, at least 97%, at least 98%, or at least 99% sequence identity to any one of SEQ ID NOs: 4-62.

In some embodiments, the ActRIIB-ECD variant comprises an amino acid substitution at position G27 and comprises an amino acid sequence that is at least 95%, at least 96%, at least 97%, at least 98%, or at least 99% sequence identity to any one of SEQ ID NOs: 4-9. In some embodiments, the ActRIIB-ECD variant comprises an amino acid substitution at position G27 and comprises or consists of an amino acid sequence selected from SEQ ID NOs: 4-9. In some embodiments, the ActRIIB-ECD variant comprises an amino acid substitution at position G27E and comprises or consists of the amino acid sequence of SEQ ID NO: 4. In some embodiments, the ActRIIB-ECD variant comprises an amino acid substitution at position G27N and comprises or consists of the amino acid sequence of SEQ ID NO: 5. In some embodiments, the ActRIIB-ECD variant comprises an amino acid substitution at position G27Q and comprises or consists of the amino acid sequence of SEQ ID NO: 6. In some embodiments, the ActRIIB-ECD variant comprises an amino acid substitution at position G27K and comprises or consists of the amino acid sequence of SEQ ID NO: 7. In some embodiments, the ActRIIB-ECD variant comprises an amino acid substitution at position G27T and comprises or consists of the amino acid sequence of SEQ ID NO: 8. In some embodiments, the ActRIIB-ECD variant comprises an amino acid substitution at position G27M and comprises or consists of the amino acid sequence of SEQ ID NO: 9.

In some embodiments, the ActRIIB-ECD variant comprises an amino acid substitution at position L33 and comprises an amino acid sequence that is at least 95%, at least 96%, at least 97%, at least 98%, or at least 99% sequence identity to any one of SEQ ID NOs: 10-18. In some embodiments, the ActRIIB-ECD variant comprises an amino acid substitution at position L33 and comprises or consists of an amino acid sequence selected from SEQ ID NOs: 10-18. In some embodiments, the ActRIIB-ECD variant comprises an amino acid substitution at position L33F and comprises or consists of the amino acid sequence of SEQ ID NO: 10. In some embodiments, the ActRIIB-ECD variant comprises an amino acid substitution at position L33Q and comprises or consists of the amino acid sequence of SEQ ID NO: 11. In some embodiments, the ActRIIB-ECD variant comprises an amino acid substitution at position L33Y and comprises or consists of the amino acid sequence of SEQ ID NO: 12. In some embodiments, the ActRIIB-ECD variant comprises an amino acid substitution at position L33W and comprises or consists of the amino acid sequence of SEQ ID NO: 13. In some embodiments, the ActRIIB-ECD variant comprises an amino acid substitution at position L33H and comprises or consists of the amino acid sequence of SEQ ID NO: 14. In some embodiments, the ActRIIB-ECD variant comprises an amino acid substitution at position L33R and comprises or consists of the amino acid sequence of SEQ ID NO: 15. In some embodiments, the ActRIIB-ECD variant comprises an amino acid substitution at position L33E and comprises or consists of the amino acid sequence of SEQ ID NO: 16. In some embodiments, the ActRIIB-ECD variant comprises an amino acid substitution at position L33K and comprises or consists of the amino acid sequence of SEQ ID NO: 17. In some embodiments, the ActRIIB-ECD variant comprises an amino acid substitution at position L33M and comprises or consists of the amino acid sequence of SEQ ID NO: 18.

In some embodiments, the ActRIIB-ECD variant comprises an amino acid substitution at position T69 and comprises an amino acid sequence that is at least 95%, at least 96%, at least 97%, at least 98%, or at least 99% sequence identity to SEQ ID NOs: 19-24. In some embodiments, the ActRIIB-ECD variant comprises an amino acid substitution at position G27 and comprises or consists of SEQ ID NO: 19-24. In some embodiments, the ActRIIB-ECD variant comprises an amino acid substitution at position T69H and comprises or consists of the amino acid sequence of SEQ ID NO: 19. In some embodiments, the ActRIIB-ECD variant comprises an amino acid substitution at position T69Q and comprises or consists of the amino acid sequence of SEQ ID NO: 20. In some embodiments, the ActRIIB-ECD variant comprises an amino acid substitution at position T69E and comprises or consists of the amino acid sequence of SEQ ID NO: 21. In some embodiments, the ActRIIB-ECD variant comprises an amino acid substitution at position T69R and comprises or consists of the amino acid sequence of SEQ ID NO: 22. In some embodiments, the ActRIIB-ECD variant comprises an amino acid substitution at position T69Y and comprises or consists of the amino acid sequence of SEQ ID NO: 23. In some embodiments, the ActRIIB-ECD variant comprises an amino acid substitution at position T69W and comprises or consists of the amino acid sequence of SEQ ID NO: 24.

In some embodiments, the ActRIIB-ECD variant comprises an amino acid substitution at position L33 and at position T69 and comprises an amino acid sequence that is at least 95%, at least 96%, at least 97%, at least 98%, or at least 99% sequence identity to SEQ ID NOs: 25-31. In some embodiments, the ActRIIB-ECD variant comprises an amino acid substitution at position L33 and at position T69 and comprises or consists of SEQ ID NO: 25-31. In some embodiments, the ActRIIB-ECD variant comprises an amino acid substitution at position L33Y and T69R and comprises or consists of the amino acid sequence of SEQ ID NO: 25. In some embodiments, the ActRIIB-ECD variant comprises an amino acid substitution at position L33Y and T69Y and comprises or consists of the amino acid sequence of SEQ ID NO: 26. In some embodiments, the ActRIIB-ECD variant comprises an amino acid substitution at position L33Y and T69W and comprises or consists of the amino acid sequence of SEQ ID NO: 27. In some embodiments, the ActRIIB-ECD variant comprises an amino acid substitution at position L33Y and T69H and comprises or consists of the amino acid sequence of SEQ ID NO: 28. In some embodiments, the ActRIIB-ECD variant comprises an amino acid substitution at position L33Y and T69Q and comprises or consists of the amino acid sequence of SEQ ID NO: 29. In some embodiments, the ActRIIB-ECD variant comprises an amino acid substitution at position L33Y and T69E and comprises or consists of the amino acid sequence of SEQ ID NO: 30. In some embodiments, the ActRIIB-ECD variant comprises an amino acid substitution at position L33F and T69Q and comprises or consists of the amino acid sequence of SEQ ID NO: 31.

In some embodiments, the ActRIIB-ECD variant comprises an amino acid substitution at position G27 and at position L33 and comprises an amino acid sequence that is at least 95%, at least 96%, at least 97%, at least 98%, or at least 99% sequence identity to SEQ ID NOs: 32-48. In some embodiments, the ActRIIB-ECD variant comprises an amino acid substitution at position G27 and at position L33 and comprises or consists of SEQ ID NO: 32-48. In some embodiments, the ActRIIB-ECD variant comprises an amino acid substitution at position G27E and L33Y and comprises or consists of the amino acid sequence of SEQ ID NO: 32. In some embodiments, the ActRIIB-ECD variant comprises an amino acid substitution at position G27D and L33Y and comprises or consists of the amino acid sequence of SEQ ID NO: 33. In some embodiments, the ActRIIB-ECD variant comprises an amino acid substitution at position G27N and L33Y and comprises or consists of the amino acid sequence of SEQ ID NO: 34. In some embodiments, the ActRIIB-ECD variant comprises an amino acid substitution at position G27Q and L33Y and comprises or consists of the amino acid sequence of SEQ ID NO: 35. In some embodiments, the ActRIIB-ECD variant comprises an amino acid substitution at position G27K and L33Y and comprises or consists of the amino acid sequence of SEQ ID NO: 36. In some embodiments, the ActRIIB-ECD variant comprises an amino acid substitution at position G27T and L33Y and comprises or consists of the amino acid sequence of SEQ ID NO: 37. In some embodiments, the ActRIIB-ECD variant comprises an amino acid substitution at position G27M and L33Y and comprises or consists of the amino acid sequence of SEQ ID NO: 38. In some embodiments, the ActRIIB-ECD variant comprises an amino acid substitution at position G27D and L33Y and comprises or consists of the amino acid sequence of SEQ ID NO: 39. In some embodiments, the ActRIIB-ECD variant comprises an amino acid substitution at position G27E and L33Y and comprises or consists of the amino acid sequence of SEQ ID NO: 40. In some embodiments, the ActRIIB-ECD variant comprises an amino acid substitution at position G27N and L33Y and comprises or consists of the amino acid sequence of SEQ ID NO: 41. In some embodiments, the ActRIIB-ECD variant comprises an amino acid substitution at position G27Q and L33Y and comprises or consists of the amino acid sequence of SEQ ID NO: 42. In some embodiments, the ActRIIB-ECD variant comprises an amino acid substitution at position G27D and L33W and comprises or consists of the amino acid sequence of SEQ ID NO: 43. In some embodiments, the ActRIIB-ECD variant comprises an amino acid substitution at position G27E and L33W and comprises or consists of the amino acid sequence of SEQ ID NO: 44. In some embodiments, the ActRIIB-ECD variant comprises an amino acid substitution at position G27N and L33W and comprises or consists of the amino acid sequence of SEQ ID NO: 45. In some embodiments, the ActRIIB-ECD variant comprises an amino acid substitution at position G27Q and L33W and comprises or consists of the amino acid sequence of SEQ ID NO: 46. In some embodiments, the ActRIIB-ECD variant comprises an amino acid substitution at position G27T and L33W and comprises or consists of the amino acid sequence of SEQ ID NO: 47. In some embodiments, the ActRIIB-ECD variant comprises an amino acid substitution at position G27M and L33W and comprises or consists of the amino acid sequence of SEQ ID NO: 48.

In some embodiments, the ActRIIB-ECD variant comprises an amino acid substitution at position L14 and at position L33 and comprises an amino acid sequence that is at least 95%, at least 96%, at least 97%, at least 98%, or at least 99% sequence identity to SEQ ID NOs: 49-58. In some embodiments, the ActRIIB-ECD variant comprises an amino acid substitution at position L14 and at position L33 and comprises or consists of SEQ ID NO: 49-58. In some embodiments, the ActRIIB-ECD variant comprises an amino acid substitution at position L14Q and L33Y and comprises or consists of the amino acid sequence of SEQ ID NO: 49. In some embodiments, the ActRIIB-ECD variant comprises an amino acid substitution at position L14D and L33Y and comprises or consists of the amino acid sequence of SEQ ID NO: 50. In some embodiments, the ActRIIB-ECD variant comprises an amino acid substitution at position L14N and L33Y and comprises or consists of the amino acid sequence of SEQ ID NO: 51. In some embodiments, the ActRIIB-ECD variant comprises an amino acid substitution at position L14E and L33Y and comprises or consists of the amino acid sequence of SEQ ID NO: 52. In some embodiments, the ActRIIB-ECD variant comprises an amino acid substitution at position L14H and L33Y and comprises or consists of the amino acid sequence of SEQ ID NO: 53. In some embodiments, the ActRIIB-ECD variant comprises an amino acid substitution at position L14S and L33Y and comprises or consists of the amino acid sequence of SEQ ID NO: 54. In some embodiments, the ActRIIB-ECD variant comprises an amino acid substitution at position L14E and L33W and comprises or consists of the amino acid sequence of SEQ ID NO: 55. In some embodiments, the ActRIIB-ECD variant comprises an amino acid substitution at position L14D and L33W and comprises or consists of the amino acid sequence of SEQ ID NO: 56. In some embodiments, the ActRIIB-ECD variant comprises an amino acid substitution at position L14N and L33W and comprises or consists of the amino acid sequence of SEQ ID NO: 57. In some embodiments, the ActRIIB-ECD variant comprises an amino acid substitution at position L14Q and L33W and comprises or consists of the amino acid sequence of SEQ ID NO: 58.

In some embodiments, the ActRIIB-ECD variant comprises an amino acid substitution at position L33 and at position L55 and comprises an amino acid sequence that is at least 95%, at least 96%, at least 97%, at least 98%, or at least 99% sequence identity to SEQ ID NOs: 59-62. In some embodiments, the ActRIIB-ECD variant comprises an amino acid substitution at position L33 and at position L55 and comprises or consists of SEQ ID NO: 59-62. In some embodiments, the ActRIIB-ECD variant comprises an amino acid substitution at position L33Y and L55Y and comprises or consists of the amino acid sequence of SEQ ID NO: 59. In some embodiments, the ActRIIB-ECD variant comprises an amino acid substitution at position L33Y and L55Q and comprises or consists of the amino acid sequence of SEQ ID NO: 60. In some embodiments, the ActRIIB-ECD variant comprises an amino acid substitution at position L33Y and L55M and comprises or consists of the amino acid sequence of SEQ ID NO: 61. In some embodiments, the ActRIIB-ECD variant comprises an amino acid substitution at position L33Y and L55I and comprises or consists of the amino acid sequence of SEQ ID NO: 62.

Exemplary ActRIIB ECDs are provided in Table 1. Amino acid substitutions are indicated by bold and enlarged text.

**Table 1: Exemplary ActRIIB and ActRIIB variant ECDs**

| **ECD** | **AA Sequence** | **SEQ ID** |
|---|---|---|
| wild type ActRIIB-ECD | ETRECIYYNANWELERTNQSGLERCEGEQDKRLHCYASWRNSSGTIELVKK GCWLDDFNCYDRQECVATEENPQVYFCCCEGNFCNERFTHLPEAGGPEVTY EPPPT | 2 |
| wild type ActRIIA-ECD | ILGRSETQECLFFNANWEKDRTNQTGVEPCYGDKDKRRHCFATWKNISGSI EIVKQGCWLDDINCYDRTDCVEKKDSPEVYFCCCEGNMCNEKFSYFPEMEV TQPTSNPVTPKPPT | 3 |
| ActRIIB-ECD G27E | ETRECIYYNANWELERTNQSGLERCEEEQDKRLHCYASWRNSSGTIELVKK GCWLDDFNCYDRQECVATEENPQVYFCCCEGNFCNERFTHLPEAGGPEVTY EPPPT | 4 |
| ActRIIB-ECD G27N | ETRECIYYNANWELERTNQSGLERCENEQDKRLHCYASWRNSSGTIELVKK GCWLDDFNCYDRQECVATEENPQVYFCCCEGNFCNERFTHLPEAGGPEVTY EPPPT | 5 |
| ActRIIB-ECD G27Q | ETRECIYYNANWELERTNQSGLERCEQEQDKRLHCYASWRNSSGTIELVKK GCWLDDFNCYDRQECVATEENPQVYFCCCEGNFCNERFTHLPEAGGPEVTY EPPPT | 6 |
| ActRIIB-ECD G27K | ETRECIYYNANWELERTNQSGLERCEKEQDKRLHCYASWRNSSGTIELVKK GCWLDDFNCYDRQECVATEENPQVYFCCCEGNFCNERFTHLPEAGGPEVTY EPPPT | 7 |
| ActRIIB-ECD G27T | ETRECIYYNANWELERTNQSGLERCETEQDKRLHCYASWRNSSGTIELVKK GCWLDDFNCYDRQECVATEENPQVYFCCCEGNFCNERFTHLPEAGGPEVTY EPPPT | 8 |
| ActRIIB-ECD G27M | ETRECIYYNANWELERTNQSGLERCEMEQDKRLHCYASWRNSSGTIELVKK GCWLDDFNCYDRQECVATEENPQVYFCCCEGNFCNERFTHLPEAGGPEVTY EPPPT | 9 |
| ActRIIB-ECD L33F | ETRECIYYNANWELERTNQSGLERCEGEQDKRFHCYASWRNSSGTIELVKK GCWLDDFNCYDRQECVATEENPQVYFCCCEGNFCNERFTHLPEAGGPEVTY EPPPT | 10 |
| ActRIIB-ECD L33Q | ETRECIYYNANWELERTNQSGLERCEGEQDKRQHCYASWRNSSGTIELVKK GCWLDDFNCYDRQECVATEENPQVYFCCCEGNFCNERFTHLPEAGGPEVTY EPPPT | 11 |
| ActRIIB-ECD L33Y | ETRECIYYNANWELERTNQSGLERCEGEQDKRYHCYASWRNSSGTIELVKK GCWLDDFNCYDRQECVATEENPQVYFCCCEGNFCNERFTHLPEAGGPEVTY EPPPT | 12 |
| ActRIIB-ECD L33W | ETRECIYYNANWELERTNQSGLERCEGEQDKRWHCYASWRNSSGTIELVKK GCWLDDFNCYDRQECVATEENPQVYFCCCEGNFCNERFTHLPEAGGPEVTY EPPPT | 13 |
| ActRIIB-ECD L33H | ETRECIYYNANWELERTNQSGLERCEGEQDKRHHCYASWRNSSGTIELVKK GCWLDDFNCYDRQECVATEENPQVYFCCCEGNFCNERFTHLPEAGGPEVTY EPPPT | 14 |
| ActRIIB-ECD L33R | ETRECIYYNANWELERTNQSGLERCEGEQDKRRHCYASWRNSSGTIELVKK GCWLDDFNCYDRQECVATEENPQVYFCCCEGNFCNERFTHLPEAGGPEVTY EPPPT | 15 |
| ActRIIB-ECD L33E | ETRECIYYNANWELERTNQSGLERCEGEQDKREHCYASWRNSSGTIELVKK GCWLDDFNCYDRQECVATEENPQVYFCCCEGNFCNERFTHLPEAGGPEVTY EPPPT | 16 |
| ActRIIB-ECD L33K | ETRECIYYNANWELERTNQSGLERCEGEQDKRKHCYASWRNSSGTIELVKK GCWLDDFNCYDRQECVATEENPQVYFCCCEGNFCNERFTHLPEAGGPEVTY EPPPT | 17 |
| ActRIIB-ECD L33M | ETRECIYYNANWELERTNQSGLERCEGEQDKRMHCYASWRNSSGTIELVKK GCWLDDFNCYDRQECVATEENPQVYFCCCEGNFCNERFTHLPEAGGPEVTY EPPPT | 18 |
| ActRIIB-ECD T69H | ETRECIYYNANWELERTNQSGLERCEGEQDKRLHCYASWRNSSGTIELVKK GCWLDDFNCYDRQECVAHEENPQVYFCCCEGNFCNERFTHLPEAGGPEVTY EPPPT | 19 |
| ActRIIB-ECD T69Q | ETRECIYYNANWELERTNQSGLERCEGEQDKRLHCYASWRNSSGTIELVKK GCWLDDFNCYDRQECVAQEENPQVYFCCCEGNFCNERFTHLPEAGGPEVTY EPPPT | 20 |
| ActRIIB-ECD T69E | ETRECIYYNANWELERTNQSGLERCEGEQDKRLHCYASWRNSSGTIELVKK GCWLDDFNCYDRQECVAEEENPQVYFCCCEGNFCNERFTHLPEAGGPEVTY EPPPT | 21 |
| ActRIIB-ECD T69R | ETRECIYYNANWELERTNQSGLERCEGEQDKRLHCYASWRNSSGTIELVKK GCWLDDFNCYDRQECVAREENPQVYFCCCEGNFCNERFTHLPEAGGPEVTY EPPPT | 22 |
| ActRIIB-ECD T69Y | ETRECIYYNANWELERTNQSGLERCEGEQDKRLHCYASWRNSSGTIELVKK GCWLDDFNCYDRQECVAYEENPQVYFCCCEGNFCNERFTHLPEAGGPEVTY EPPPT | 23 |
| ActRIIB-ECD T69W | ETRECIYYNANWELERTNQSGLERCEGEQDKRLHCYASWRNSSGTIELVKK GCWLDDFNCYDRQECVAWEENPQVYFCCCEGNFCNERFTHLPEAGGPEVTY EPPPT | 24 |
| ActRIIB-ECD L33Y T69R | ETRECIYYNANWELERTNQSGLERCEGEQDKRYHCYASWRNSSGTIELVKK GCWLDDFNCYDRQECVAREENPQVYFCCCEGNFCNERFTHLPEAGGPEVTY EPPPT | 25 |
| ActRIIB-ECD L33Y T69Y | ETRECIYYNANWELERTNQSGLERCEGEQDKRYHCYASWRNSSGTIELVKK GCWLDDFNCYDRQECVAYEENPQVYFCCCEGNFCNERFTHLPEAGGPEVTY EPPPT | 26 |
| ActRIIB-ECD L33Y T69W | ETRECIYYNANWELERTNQSGLERCEGEQDKRYHCYASWRNSSGTIELVKK GCWLDDFNCYDRQECVAWEENPQVYFCCCEGNFCNERFTHLPEAGGPEVTY EPPPT | 27 |
| ActRIIB-ECD L33Y T69H | ETRECIYYNANWELERTNQSGLERCEGEQDKRYHCYASWRNSSGTIELVKK GCWLDDFNCYDRQECVAHEENPQVYFCCCEGNFCNERFTHLPEAGGPEVTY EPPPT | 28 |
| ActRIIB-ECD L33Y T69Q | ETRECIYYNANWELERTNQSGLERCEGEQDKRYHCYASWRNSSGTIELVKK GCWLDDFNCYDRQECVAQEENPQVYFCCCEGNFCNERFTHLPEAGGPEVTY EPPPT | 29 |
| ActRIIB-ECD L33Y T69E | ETRECIYYNANWELERTNQSGLERCEGEQDKRYHCYASWRNSSGTIELVKK GCWLDDFNCYDRQECVAEEENPQVYFCCCEGNFCNERFTHLPEAGGPEVTY EPPPT | 30 |
| ActRIIB-ECD L33F T69Q | ETRECIYYNANWELERTNQSGLERCEGEQDKRFHCYASWRNSSGTIELVKK GCWLDDFNCYDRQECVAQEENPQVYFCCCEGNFCNERFTHLPEAGGPEVTY EPPPT | 31 |
| ActRIIB-ECD G27E L33Y | ETRECIYYNANWELERTNQSGLERCEEQDKRYHCYASWRNSSGTIELVKK GCWLDDFNCYDRQECVATEENPQVYFCCCEGNFCNERFTHLPEAGGPEVTY EPPPT | 32 |
| ActRIIB-ECD G27D L33Y | ETRECIYYNANWELERTNQSGLERCEDEQDKRYHCYASWRNSSGTIELVKK GCWLDDFNCYDRQECVATEENPQVYFCCCEGNFCNERFTHLPEAGGPEVTY EPPPT | 33 |
| ActRIIB-ECD G27N L33Y | ETRECIYYNANWELERTNQSGLERCENEQDKRYHCYASWRNSSGTIELVKK GCWLDDFNCYDRQECVATEENPQVYFCCCEGNFCNERFTHLPEAGGPEVTY EPPPT | 34 |
| ActRIIB-ECD G27Q L33Y | ETRECIYYNANWELERTNQSGLERCEQEQDKRYHCYASWRNSSGTIELVKK GCWLDDFNCYDRQECVATEENPQVYFCCCEGNFCNERFTHLPEAGGPEVTY EPPPT | 35 |
| ActRIIB-ECD G27K L33Y | ETRECIYYNANWELERTNQSGLERCEKEQDKRYHCYASWRNSSGTIELVKK GCWLDDFNCYDRQECVATEENPQVYFCCCEGNFCNERFTHLPEAGGPEVTY EPPPT | 36 |
| ActRIIB-ECD G27T L33Y | ETRECIYYNANWELERTNQSGLERCETEQDKRYHCYASWRNSSGTIELVKK GCWLDDFNCYDRQECVATEENPQVYFCCCEGNFCNERFTHLPEAGGPEVTY EPPPT | 37 |
| ActRIIB-ECD G27M L33Y | ETRECIYYNANWELERTNQSGLERCEMEQDKRYHCYASWRNSSGTIELVKK GCWLDDFNCYDRQECVATEENPQVYFCCCEGNFCNERFTHLPEAGGPEVTY EPPPT | 38 |
| ActRIIB-ECD G27D L33Y | ETRECIYYNANWELERTNQSGLERCEDEQDKRYHCYASWRNSSGTIELVKK GCWLDDFNCYDRQECVATEENPQVYFCCCEGNFCNERFTHLPEAGGPEVTY EPPPT | 39 |
| ActRIIB-ECD G27E L33Y | ETRECIYYNANWELERTNQSGLERCEEQDKRYHCYASWRNSSGTIELVKK GCWLDDFNCYDRQECVATEENPQVYFCCCEGNFCNERFTHLPEAGGPEVTY EPPPT | 40 |
| ActRIIB-ECD G27N L33Y | ETRECIYYNANWELERTNQSGLERCENEQDKRYHCYASWRNSSGTIELVKK GCWLDDFNCYDRQECVATEENPQVYFCCCEGNFCNERFTHLPEAGGPEVTY EPPPT | 41 |
| ActRIIB-ECD G27Q L33Y | ETRECIYYNANWELERTNQSGLERCEQEQDKRYHCYASWRNSSGTIELVKK GCWLDDFNCYDRQECVATEENPQVYFCCCEGNFCNERFTHLPEAGGPEVTY EPPPT | 42 |
| ActRIIB-ECD G27D L33W | ETRECIYYNANWELERTNQSGLERCEDEQDKRWHCYASWRNSSGTIELVKK GCWLDDFNCYDRQECVATEENPQVYFCCCEGNFCNERFTHLPEAGGPEVTY EPPPT | 43 |
| ActRIIB-ECD G27E L33W | ETRECIYYNANWELERTNQSGLERCEEEQDKRWHCYASWRNSSGTIELVKK GCWLDDFNCYDRQECVATEENPQVYFCCCEGNFCNERFTHLPEAGGPEVTY EPPPT | 44 |
| ActRIIB-ECD G27N L33W | ETRECIYYNANWELERTNQSGLERCENEQDKRWHCYASWRNSSGTIELVKK GCWLDDFNCYDRQECVATEENPQVYFCCCEGNFCNERFTHLPEAGGPEVTY EPPPT | 45 |
| ActRIIB-ECD G27Q L33W | ETRECIYYNANWELERTNQSGLERCEQEQDKRWHCYASWRNSSGTIELVKK GCWLDDFNCYDRQECVATEENPQVYFCCCEGNFCNERFTHLPEAGGPEVTY EPPPTGGGGSGGGGSGGGGSGGGG | 46 |
| ActRIIB-ECD G27T L33W | ETRECIYYNANWELERTNQSGLERCETEQDKRWHCYASWRNSSGTIELVKK GCWLDDFNCYDRQECVATEENPQVYFCCCEGNFCNERFTHLPEAGGPEVTY EPPPT | 47 |
| ActRIIB-ECD G27M L33W | ETRECIYYNANWELERTNQSGLERCEMEQDKRWHCYASWRNSSGTIELVKK GCWLDDFNCYDRQECVATEENPQVYFCCCEGNFCNERFTHLPEAGGPEVTY EPPPT | 48 |
| ActRIIB-ECD L14Q L33Y | ETRECIYYNANWEQERTNQSGLERCEGEQDKRYHCYASWRNSSGTIELVKK GCWLDDFNCYDRQECVATEENPQVYFCCCEGNFCNERFTHLPEAGGPEVTY EPPPT | 49 |
| ActRIIB-ECD L14D L33Y | ETRECIYYNANWEDERTNQSGLERCEGEQDKRYHCYASWRNSSGTIELVKK GCWLDDFNCYDRQECVATEENPQVYFCCCEGNFCNERFTHLPEAGGPEVTY EPPPT | 50 |
| ActRIIB-ECD L14N L33Y | ETRECIYYNANWENERTNQSGLERCEGEQDKRYHCYASWRNSSGTIELVKK GCWLDDFNCYDRQECVATEENPQVYFCCCEGNFCNERFTHLPEAGGPEVTY EPPPT | 51 |
| ActRIIB-ECD L14E L33Y | ETRECIYYNANWEEERTNQSGLERCEGEQDKRYHCYASWRNSSGTIELVKK GCWLDDFNCYDRQECVATEENPQVYFCCCEGNFCNERFTHLPEAGGPEVTY EPPPT | 52 |
| ActRIIB-ECD L14H L33Y | ETRECIYYNANWEHERTNQSGLERCEGEQDKRYHCYASWRNSSGTIELVKK GCWLDDFNCYDRQECVATEENPQVYFCCCEGNFCNERFTHLPEAGGPEVTY EPPPT | 53 |
| ActRIIB-ECD L14S L33Y | ETRECIYYNANWESERTNQSGLERCEGEQDKRYHCYASWRNSSGTIELVKK GCWLDDFNCYDRQECVATEENPQVYFCCCEGNFCNERFTHLPEAGGPEVTY EPPPT | 54 |
| ActRIIB-ECD L14E L33W | ETRECIYYNANWEEERTNQSGLERCEGEQDKRWHCYASWRNSSGTIELVKK GCWLDDFNCYDRQECVATEENPQVYFCCCEGNFCNERFTHLPEAGGPEVTY EPPPT | 55 |
| ActRIIB-ECD L14D L33W | ETRECIYYNANWEDERTNQSGLERCEGEQDKRWHCYASWRNSSGTIELVKK GCWLDDFNCYDRQECVATEENPQVYFCCCEGNFCNERFTHLPEAGGPEVTY EPPPT | 56 |
| ActRIIB-ECD L14N L33W | ETRECIYYNANWENERTNQSGLERCEGEQDKRWHCYASWRNSSGTIELVKK GCWLDDFNCYDRQECVATEENPQVYFCCCEGNFCNERFTHLPEAGGPEVTY EPPPT | 57 |
| ActRIIB-ECD L14Q L33W | ETRECIYYNANWEQERTNQSGLERCEGEQDKRWHCYASWRNSSGTIELVKK GCWLDDFNCYDRQECVATEENPQVYFCCCEGNFCNERFTHLPEAGGPEVTY EPPPT | 58 |
| ActRIIB-ECD L33Y L55Y | ETRECIYYNANWELERTNQSGLERCEGEQDKRYHCYASWRNSSGTIELVKK GCWYDDFNCYDRQECVATEENPQVYFCCCEGNFCNERFTHLPEAGGPEVTY EPPPT | 59 |
| ActRIIB-ECD L33Y L55Q | ETRECIYYNANWELERTNQSGLERCEGEQDKRYHCYASWRNSSGTIELVKK GCWQDDFNCYDRQECVATEENPQVYFCCCEGNFCNERFTHLPEAGGPEVTY EPPPT | 60 |
| ActRIIB-ECD L33Y L55M | ETRECIYYNANWELERTNQSGLERCEGEQDKRYHCYASWRNSSGTIELVKK GCWMDDFNCYDRQECVATEENPQVYFCCCEGNFCNERFTHLPEAGGPEVTY EPPPT | 61 |
| ActRIIB-ECD L33Y L55I | ETRECIYYNANWELERTNQSGLERCEGEQDKRYHCYASWRNSSGTIELVKK GCWIDDFNCYDRQECVATEENPQVYFCCCEGNFCNERFTHLPEAGGPEVTY EPPPT | 62 |

In some embodiments, an ActRIIB-ECD variant of the disclosure further includes an extension of up to 5 amino acids at the N-terminus. In some embodiments, the ActRIIB-ECD variant of the disclosure further includes an extension of 5 amino acids at the N-terminus, e.g., of GRGEA (SEQ ID NO: 63). In some embodiments, the ActRIIB-ECD variant of the disclosure further includes an extension at the N-terminus of 4 amino acids, 3 amino acids, 2 amino acids, or 1 amino acid, for example and without limitation, RGEA, GEA, EA, or A. In some embodiments, an ActRIIB-ECD variant of the disclosure further includes an extension of 3 amino acids at the C-terminus. In some embodiments, the ActRIIB-ECD variant of the disclosure further includes an extension of 3 amino acids at the C-terminus, e.g., of APT.

Exemplary ActRIIB ECDs with N-terminal and C-terminal extensions are provided in **Table 2.** The extension amino acids are indicated in bold and italicized text. In some embodiments, any one of SEQ ID NOs: 4-62 can further comprise an N- or C-terminal extension.

**Table 2: ActRIIB-ECDs with N-terminal extensions**

| **ECD** | **AA Sequence** | **SEQ ID** |
|---|---|---|
| WT ActRIIB-ECD + N and C terminal extensions | GRGEAETRECIYYNANWELERTNQSGLERCEGEQDKRLHCYASWRN SSGTIELVKKGCWLDDFNCYDRQECVATEENPQVYFCCCEGNFCNE RFTHLPEAGGPEVTYEPPPTAPT | 65 |
| WT ActRIIB-ECD + N terminal extension | GRGEAETRECIYYNANWELERTNQSGLERCEGEQDKRLHCYASWRN SSGTIELVKKGCWLDDFNCYDRQECVATEENPQVYFCCCEGNFCNE RFTHLPEAGGPEVTYEPPPT | 66 |
| WT ActRIIB-ECD + C terminal extension | ETRECIYYNANWELERTNQSGLERCEGEQDKRLHCYASWRNSSGTI ELVKKGCWLDDFNCYDRQECVATEENPQVYFCCCEGNFCNERFTHL PEAGGPEVTYEPPPTAPT | 67 |

ActRIIB-ECD variants of the disclosure have been designed to maximize therapeutic efficacy in certain disease indications while minimizing adverse effects, specifically to prevent or reduce disruption of endogenous BMP-9 and/or BMP-10 signaling, while maintaining and/or increasing neutralization potency for other TGFβ superfamily ligands such as activin A, activin B, GDF-8, and/or GDF-11. ActRIIB-ECD variants of the disclosure exhibit: (1) similar or improved binding to activin A, activin B, GDF-8, and/or GDF-11 compared to wild type ActRIIB-ECD, which allows them to compete with endogenous receptors for ligand binding and reduce or inhibit endogenous receptor signaling; and (2) reduced binding to BMP-9 compared to wild type ActRIIB-ECD, which avoids toxicity associated with inhibition of BMP-9 signaling; and optionally (3) similar or reduced binding to or inhibition of BMP-10, which avoids toxicity associated with inhibition of BMP-10 signaling. These variants can be used to treat a wide range of diseases and conditions in which activin receptor signaling is elevated, such as pulmonary hypertension (PH) (e.g., PAH, venous PH, hypoxic PH, thromboembolic PH, or miscellaneous PH), metabolic disorders and cardiometabolic disease (e.g., obesity, Type 1 diabetes, Type 2 diabetes, pre-diabetes, heart failure), bone disease (e.g., diseases or conditions involving bone damage), muscle disease, fibrosis, and low red blood cell levels (e.g., anemia, blood loss), as further described herein. The variants can, for example and without limitation, lead to a reduction in the symptoms or progression of PH (e.g., PAH, venous PH, hypoxic PH, thromboembolic PH, or miscellaneous PH), a reduction in bone resorption or osteoclast activity, an increase in bone formation or bone mineral density, an increase in muscle mass or strength, a reduction in fibrosis (e.g., reduced fibrosis or a slowing or stopping of the progression of fibrosis), and/or an increase in red blood cell levels (e.g., an increase in hemoglobin levels, hematocrit, or red blood cell counts, e.g., an increase in red blood cell production), as described further herein.

In some embodiments, ActRIIB-ECD variants of the disclosure bind to one or more ligand selected from activin A, activin B, GDF-8, and GDF-11 and inhibit signaling of the one or more ligand through their respective receptors, without substantially binding to BMP-9 or BMP-10 and/or inhibiting BMP-9 or BMP-10 signaling through their receptor(s).

In some embodiments, the inhibition potency of an ActRIIB-ECD variant of the disclosure for human BMP-9 signaling is reduced by about 5-fold compared to the inhibition potency of the human wild type ActRIIB-ECD for human BMP-9 signaling.

In some embodiments, the inhibition potency of an ActRIIB-ECD variant of the disclosure for human BMP-9 signaling is reduced by about 10-fold compared to the inhibition potency of the human wild type ActRIIB-ECD for human BMP-9 signaling.

In some embodiments, the inhibition potency of an ActRIIB-ECD variant of the disclosure for human BMP-9 signaling is reduced by about 100-fold compared to the inhibition potency of the human wild type ActRIIB-ECD for human BMP-9 signaling.

In some embodiments, the inhibition potency of an ActRIIB-ECD variant of the disclosure for human BMP-10 signaling is reduced by about 5-fold compared to the inhibition potency of the human wild type ActRIIB-ECD for human BMP-10 signaling.

In some embodiments, the inhibition potency of an ActRIIB-ECD variant of the disclosure for human BMP-10 signaling is reduced by about 10-fold compared to the inhibition potency of the human wild type ActRIIB-ECD for human BMP-10 signaling.

In some embodiments, the inhibition potency of an ActRIIB-ECD variant of the disclosure for human BMP-10 signaling is reduced by about 100-fold compared to the inhibition potency of the human wild type ActRIIB-ECD for human BMP-10 signaling.

In some embodiments, the inhibition potency of ActRIIB-ECD variant of the disclosure for the one or more ligand selected from activin A, activin B, GDF-8, and GDF-11 is increased or is substantially the same as the inhibition potency of the human wild type ActRIIB-ECD for the same one or more ligand.

In some embodiments, the ActRIIB-ECD variant of the disclosure has greater inhibition potency for activin A and lower inhibition potency for BMP-9 and/or BMP-10 compared to the human wild type ActRIIB-ECD.

In some embodiments, the ActRIIB-ECD variant of the disclosure has greater inhibition potency for activin B and lower inhibition potency for BMP-9 and/or BMP-10 compared to the human wild type ActRIIB-ECD.

In some embodiments, the ActRIIB-ECD variant of the disclosure has greater inhibition potency for both activin A and activin B and lower inhibition potency for BMP-9 and/or BMP-10 compared to the human wild type ActRIIB-ECD.

In some embodiments, the ActRIIB-ECD variant of the disclosure has greater inhibition potency for GDF-8 and lower inhibition potency for BMP-9 and/or BMP-10 compared to the human wild type ActRIIB-ECD.

In some embodiments, the ActRIIB-ECD variant of the disclosure has greater inhibition potency for GDF-11 and lower inhibition potency for BMP-9 and/or BMP-10 compared to the human wild type ActRIIB-ECD.

In some embodiments, the ActRIIB-ECD variant of the disclosure has reduced inhibition potency for BMP-10 compared to the human wild type ActRIIB-ECD.

In some embodiments, the ActRIIB-ECD variant of the disclosure has greater inhibition potency for both activin A and/or activin B; lower inhibition potency for BMP-9; and lower inhibition potency for BMP-10 compared to the human wild type ActRIIB-ECD.

In some embodiments, the ActRIIB-ECD variant of the disclosure does not cause a vascular complication in a subject. In some embodiments, the ActRIIB-ECD variant of the disclosure does not increase vascular permeability or leakage in a subject.

Consequently, in accordance with the disclosure there are provided herein novel polypeptides comprising an Activin receptor type IIB (ActRIIB) ectodomain (ECD) variant, the variant having one or more amino acid substitution relative to the sequence of the human wild type ActRIIB-ECD, having a tailored TGFβ superfamily ligand specificity in order to maximize therapeutic efficacy while minimizing adverse effects, specifically with the goal of preventing or reducing disruption of endogenous BMP-9 and/or BMP-10 signaling, while maintaining and/or increasing neutralization potency for other TGFβ superfamily ligands such as activin A, activin B, GDF-8, and/or GDF-11.

### Polypeptides comprising ActRIIB ECD variants

In some embodiments, the present disclosure provides polypeptides comprising an ActRIIB ECD variant fused, via a linker, to an Fc domain monomer. In some embodiments, the polypeptides comprise, from N-terminus to C-terminus, an ActRIIB ECD variant - peptide linker - Fc domain monomer. The polypeptides comprising ActRIIB ECDs can dimerize via cysteine bonds between Fc domain monomers to form the TGFβ superfamily ligand binding agents described herein.

### Linkers

In some embodiments, the ActRIIB ECD variants described herein are fused to a heterologous domain by way of a linker. In some embodiments, the heterologous domain increases stability of the polypeptide. In some embodiments, the heterologous domain is selected from the group consisting of an Fc domain monomer (e.g., a wild-type Fc domain monomer, an Fc domain monomer with one or more amino acid substitutions), an albumin-binding peptide, a fibronectin domain, or a human serum albumin domain.

As used herein, the terms "peptide linker" and "linker" are used interchangeably to refer to a short stretch of amino acids used to connect two functional domains together in a polypeptide chain. For example, in some embodiments of the polypeptides or binding agents of the disclosure, the ActRIIB-ECD variant and the Fc domain monomer are linked together on a polypeptide chain via one or more peptide linkers. Peptide linkers can also be used to attach other domains or modules or regions (such as half-life extending domains) to the polypeptides or binding agents of the disclosure. The term "long linker" as used herein refers to a linker that is at least 10 amino acids in length (*i.e*., 10, 11, 12, 13, 14, 15, 16, 17, 18, 19, 20 or more amino acids in length). The term "short linker" as used herein refers to a linker that is less than 10 amino acids in length (*i.e*., 9, 8, 7, 6, 5, 4, 3, 2, or 1 amino acids in length)

Suitable peptide linkers are known in the art, and include, for example, peptide linkers containing flexible amino acid residues such as glycine, alanine, and serine. In some embodiments, a linker can contain motifs, e.g., multiple or repeating motifs, of GA, GS, GG, GGA, GGS, GGG, GGGA (SEQ ID NO: 126), GGGS (SEQ ID NO: 125), GGGG (SEQ ID NO: 104), GGGGA (SEQ ID NO: 124), GGGGS (SEQ ID NO: 103), GGGGG (SEQ ID NO: 123), GGAG (SEQ ID NO: 122), GGSG (SEQ ID NO: 121), AGGG (SEQ ID NO: 120), or SGGG (SEQ ID NO: 110).

In some embodiments, a linker can contain 2 to 12 amino acids including motifs of GA or GS, e.g., GA, GS, GAGA (SEQ ID NO: 137), GSGS (SEQ ID NO: 129), GAGAGA (SEQ ID NO: 130), GSGSGS (SEQ ID NO: 131), GAGAGAGA (SEQ ID NO: 132), GSGSGSGS (SEQ ID NO: 133), GAGAGAGAGA (SEQ ID NO: 134), GSGSGSGSGS (SEQ ID NO: 135), GAGAGAGAGAGA (SEQ ID NO: 136), and GSGSGSGSGSGS (SEQ ID NO: 138). In some embodiments, a linker can contain 3 to 12 amino acids including motifs of GGA or GGS, e.g., GGA, GGS, GGAGGA (SEQ ID NO: 139), GGSGGS (SEQ ID NO: 140), GGAGGAGGA (SEQ ID NO: 141), GGSGGSGGS (SEQ ID NO: 142), GGAGGAGGAGGA (SEQ ID NO: 143), and GGSGGSGGSGGS (SEQ ID NO: 144). In some embodiments, a linker can contain 4 to 12 amino acids including motifs of GGAG (SEQ ID NO: 145), GGSG (SEQ ID NO: 146), GGAGGGAG (SEQ ID NO: 147), GGSGGGSG (SEQ ID NO: 148), GGAGGGAGGGAG (SEQ ID NO: 149), and GGSGGGSGGGSG (SEQ ID NO: 150). In some embodiments, a linker can contain motifs of GGGGA (SEQ ID NO: 124) or GGGGS (SEQ ID NO: 103), e.g, GGGGAGGGGAGGGGA (SEQ ID NO: 151) and GGGGSGGGGSGGGGS (SEQ ID NO: 93). In some embodiments, an amino acid linker between an ActRIIB-ECD variant and a heterologous domain (*e.g.*, an Fc domain monomer (*e.g*., a wild-type Fc domain monomer, an Fc domain monomer with one or more amino acid substitutions), an albumin-binding peptide, a fibronectin domain, or a human serum albumin domain) may be GGG, GGGA (SEQ ID NO: 126), GGGG (SEQ ID NO: 104), GGGAG (SEQ ID NO: 168), GGGAGG (SEQ ID NO: 169), or GGGAGGG (SEQ ID NO: 170).

In the event that a linker is used, the linker is generally of a length and sequence sufficient to ensure that each of the domains can, independently from one another, retain their differential binding specificities and/or functions. In some embodiments, peptide linkers which furthermore do not promote any secondary structures are selected. The linkage of said domains to each other can be provided, e.g., by genetic engineering, as described herein. Methods for preparing fused and operatively linked polypeptide constructs and expressing them in mammalian cells or bacteria are well-known in the art (*e.g*., WO 99/54440 or Sambrook et al., Molecular Cloning: A Laboratory Manual, Cold Spring Harbor Laboratory Press, Cold Spring Harbor, N.Y., 2001).

In some embodiments, the linker comprises various permutations of amino acid sequences containing Gly and Ser. In some embodiments, the linkers are glycine and serine rich linkers. In some embodiments, the linker may be rich in glycine (e.g, 2-10, 2-5, 2-4, 2-3 glycine residues) or glycine and proline residues and may, for example, contain a single sequence of threonine/serine and glycines or repeating sequences of threonine/serine and/or glycines, e.g., GGG, GGGG (SEQ ID NO: 104), GGGS (SEQ ID NO: 125), TGGGG (SEQ ID NO: 108), SGGGG (SEQ ID NO: 109), TGGG (SEQ ID NO: 107), or SGGG (SEQ ID NO: 110) singlets, or repeats. Other near neutral amino acids, such as, but not limited to, Thr, Asn, Pro and Ala, may also be used in the linker sequence.

In some embodiments, the linker is 10 amino acids in length. In some embodiments, the linker is greater than 10 amino acids in length. In some embodiments, the linker has a length of at least 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 amino acids. In some embodiments, the linker is less than 40, 35, 30, 25, 22 or 20 amino acids. In some embodiments, the linker is 10-50, 10-40, 10-30, 10-25, 10-21, 10-15, 10-14, 12-14, 15-25, 17-22, 20, or 21 amino acids in length. In some embodiments, the linker is 14-40, 14-39, 14-35, 14-30, 14-25, or 14-20 amino acids in length. In some embodiments, the linker is at least 10 amino acids in length. In some embodiments, the linker is at least 14 amino acids in length. In some embodiments, the linker is at least 19 amino acids in length. In some embodiments, the linker is at least 39 amino acids in length. In some embodiments, the linker is 14 amino acids in length. In some embodiments, the linker is 19 amino acids in length. In some embodiments, the linker is 39 amino acids in length. In some embodiments, the linker is 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 amino acids in length. In further embodiments, the linkers have a length of at least 12, 14, 15, 20, 21, 25, 30, 35, 40, 45 or 50 amino acids.

In other embodiments, the linker is less than 10 amino acids in length. In some embodiments, the linker is 3 amino acids in length. In some embodiments, the linker is 6 amino acids in length. In some embodiments, the linker is 9 amino acids in length.

In some embodiments, the linker comprises SEQ ID NO: 98. In some embodiments, the linker comprises SEQ ID NO: 94. In some embodiments, the linker comprises SEQ ID NO: 89.

In some embodiments, the linker consists of SEQ ID NO: 98. In some embodiments, the linker consists of SEQ ID NO: 94. In some embodiments, the linker consists of SEQ ID NO: 89.

In some embodiments, the linker comprises or consists of the sequence set forth in any one of SEQ ID NOs: 68-170.

In some embodiments, linkers are Glycine-rich, often Glycine/Serine-rich, peptides of up to 40 amino acids, or from 1 to 40 amino acids, from 2 to 39 amino acids, from 3 to 39 amino acids, from 3 to 14 amino acids, from 3 to 19 amino acids, from 5 to 25 amino acids, from 5 to 20 amino acids, from 5 to 15 amino acids, or from 15 to 25 amino acids. In some embodiments, peptide linkers comprise only a relatively small number of amino acid residues, e.g., 39 amino acids or less, 19 amino acids or less, 14 amino acids or less, 5 amino acids or less, or 3 amino acids of less. In certain embodiments, Gly-rich linkers are used. In one embodiment, a peptide linker may consist of the single amino acid Glycine (Gly). In another embodiment, a peptide linker comprises or consists of the amino acid sequence Gly-Gly-Gly-Gly-Ser, i.e. Gly4Ser, or polymers thereof, i.e. (Gly4Ser)n, where n is an integer of 1 or greater, or n is from 1 to 8 (e.g. 1, 2, 3, 4, 5, 6, 7, or 8).

In some embodiments, the linker comprises the amino acid sequence GlyGlyGlyGlySer (GGGGS) (SEQ ID NO: 103), or repetitions thereof (GGGGS)n, where n > 2. In particular embodiments n > 3, or n = 3-10. In some embodiments, n > 4, or n = 4-10. In some embodiments, n is not greater than 4 in a (GGGGS)n linker. In some embodiments, n = 4-10, 4-9, 4-8, 4-7, 4-6, 4-5, 5-8, 5-7, or 5-6. In some embodiments, n = 3, 4, 5, 6, or 7. In some embodiments, n = 4. In some embodiments, a linker comprising a (GGGGS)n sequence also comprises an N-terminal threonine.

In some embodiments, a linker can also contain amino acids other than glycine, alanine, and serine, e.g., AAAL (SEQ ID NO: 152), AAAK (SEQ ID NO: 153), AAAR (SEQ ID NO: 154), EGKSSGSGSESKST (SEQ ID NO: 155), GSAGSAAGSGEF (SEQ ID NO: 156), AEAAAKEAAAKA (SEQ ID NO: 157), KESGSVSSEQLAQFRSLD (SEQ ID NO: 158), GENLYFQSGG (SEQ ID NO: 159), SACYCELS (SEQ ID NO: 160), RSIAT (SEQ ID NO: 161), RPACKIPNDLKQKVMNH (SEQ ID NO: 162), GGSAGGSGSGSSGGSSGASGTGTAGGTGSGSGTGSG (SEQ ID NO: 163), AAANSSIDLISVPVDSR (SEQ ID NO: 164), or GGSGGGSEGGGSEGGGSEGGGSEGGGSEGGGSGGGS (SEQ ID NO: 165). In some embodiments, a linker can contain motifs, e.g., multiple or repeating motifs, of EAAAK (SEQ ID NO: 166). In some embodiments, a linker can contain motifs, e.g., multiple or repeating motifs, of praline-rich sequences such as (XP)n, in which X may be any amino acid (e.g., A, K, or E) and n is from 1-5, and PAPAP (SEQ ID NO: 167).

The length of the peptide linker and the amino acids used can be adjusted depending on the two proteins involved and the degree of flexibility desired in the final protein fusion polypeptide. The length of the linker can be adjusted to ensure proper protein folding and avoid aggregate formation.

Non-limiting examples of linkers are depicted in **Table 3**. It should be understood that the linkers are not meant to be particularly limited and any suitable linker may be used, as long as the desired functions (binding, neutralization, etc.) of the polypeptide or binding agent are provided.

**Table 3: Exemplary linker sequences**

| **SEQ ID** | **Linker sequence** |
|---|---|
| 68 | GGGGSGGGGSGGGGSGGGGSGGGGSGGGGSGGGGSGGGGS |
| 69 | GGGGSGGGGSGGGGSGGGGSGGGGSGGGGSGGGGSGGGG |
| 70 | GGGGSGGGGSGGGGSGGGGSGGGGSGGGGSGGGGSGGG |
| 71 | GGGGSGGGGSGGGGSGGGGSGGGGSGGGGSGGGGSGG |
| 72 | GGGGSGGGGSGGGGSGGGGSGGGGSGGGGSGGGGSG |
| 73 | GGGGSGGGGSGGGGSGGGGSGGGGSGGGGSGGGGS |
| 74 | GGGGSGGGGSGGGGSGGGGSGGGGSGGGGSGGGG |
| 75 | GGGGSGGGGSGGGGSGGGGSGGGGSGGGGSGGG |
| 76 | GGGGSGGGGSGGGGSGGGGSGGGGSGGGGSGG |
| 77 | GGGGSGGGGSGGGGSGGGGSGGGGSGGGGSG |
| 78 | GGGGSGGGGSGGGGSGGGGSGGGGSGGGGS |
| 79 | GGGGSGGGGSGGGGSGGGGSGGGGSGGGG |
| 80 | GGGGSGGGGSGGGGSGGGGSGGGGSGGG |
| 81 | GGGGSGGGGSGGGGSGGGGSGGGGSGG |
| 82 | GGGGSGGGGSGGGGSGGGGSGGGGSG |
| 83 | GGGGSGGGGSGGGGSGGGGSGGGGS |
| 84 | GGGGSGGGGSGGGGSGGGGSGGGG |
| 85 | GGGGSGGGGSGGGGSGGGGSGGG |
| 86 | GGGGSGGGGSGGGGSGGGGSGG |
| 87 | GGGGSGGGGSGGGGSGGGGSG |
| 88 | GGGGSGGGGSGGGGSGGGGS |
| 89 | GGGGSGGGGSGGGGSGGGG |
| 90 | GGGGSGGGGSGGGGSGGG |
| 91 | GGGGSGGGGSGGGGSGG |
| 92 | GGGGSGGGGSGGGGSG |
| 93 | GGGGSGGGGSGGGGS |
| 94 | GGGGSGGGGSGGGG |
| 95 | GGGGSGGGGSGGG |
| 96 | GGGGSGGGGSGG |
| 97 | GGGGSGGGGSG |
| 98 | GGGGSGGGGS |
| 99 | GGGGSGGGG |
| 100 | GGGGSGGG |
| 101 | GGGGSGG |
| 102 | GGGGSG |
| 103 | GGGGS |
| 104 | GGGG |
| 105 | GGG |
| 106 | GG |
| 107 | TGGG |
| 108 | TGGGG |
| 109 | SGGGG |
| 110 | SGGG |
| 111 | TGGGGSGGGGS |
| 112 | TGGGGSGGGGSGGGGS |
| 113 | TGGGGSGGGGSGGGGSGGGGS |
| 114 | TGGGGSGGGGSGGGGSGGGGSGGGGS |
| 115 | TGGGGSGGGGSGGGGSGGGGSGGGGSGGGGS |
| 116 | TGGGGSGGGGSGGGGSGGGGSGGGGSGGGGSGGGGS |
| 117 | TGGGPKSCDK |
| 118 | GA |
| 119 | GS |
| 120 | AGGG |
| 121 | GGSG |
| 122 | GGAG |
| 123 | GGGGG |
| 124 | GGGGA |
| 125 | GGGS |
| 126 | GGGA |
| 127 | GGS |
| 128 | GGA |
| 129 | GSGS |
| 130 | GAGAGA |
| 131 | GSGSGS |
| 132 | GAGAGAGA |
| 133 | GSGSGSGS |
| 134 | GAGAGAGAGA |
| 135 | GSGSGSGSGS |
| 136 | GAGAGAGAGAGA |
| 137 | GAGA |
| 138 | GSGSGSGSGSGS |
| 139 | GGAGGA |
| 140 | GGSGGS |
| 141 | GGAGGAGGA |
| 142 | GGSGGSGGS |
| 143 | GGAGGAGGAGGA |
| 144 | GGSGGSGGSGGS |
| 145 | GGAG |
| 146 | GGSG |
| 147 | GGAGGGAG |
| 148 | GGSGGGSG |
| 149 | GGAGGGAGGGAG |
| 150 | GGSGGGSGGGSG |
| 151 | GGGGAGGGGAGGGGA |
| 152 | AAAL |
| 153 | AAAK |
| 154 | AAAR |
| 155 | EGKSSGSGSESKST |
| 156 | GSAGSAAGSGEF |
| 157 | AEAAAKEAAAKA |
| 158 | KESGSVSSEQLAQFRSLD |
| 159 | GENLYFQSGG |
| 160 | SACYCELS |
| 161 | RSIAT |
| 162 | RPACKIPNDLKQKVMNH |
| 163 | GGSAGGSGSGSSGGSSGASGTGTAGGTGSGSGTGSG |
| 164 | AAANSSIDLISVPVDSR |
| 165 | GGSGGGSEGGGSEGGGSEGGGSEGGGSEGGGSGGGS |
| 166 | EAAAK |
| 167 | PAPAP |
| 168 | GGGAG |
| 169 | GGGAGG |
| 170 | GGGAGGG |

In some embodiments, the ActRIIB ECD variant polypeptide or binding agent of the disclosure comprises one or more linkers having the sequence set forth in any one of SEQ ID NOs: 89, 94, or 98. In some embodiments, the ActRIIB ECD variant polypeptide or binding agent comprises a Glycine-rich linker at the C-terminus of the ActRIIB ECD variant polypeptide that is 2, 3, 6, 10, 14, 19, or 39 amino acids long. In some embodiments, the ActRIIB ECD variant polypeptide or binding agent of the disclosure comprises a linker of SEQ ID NO: 89 at the C-terminus of the ActRIIB ECD variant polypeptide. In some embodiments, the ActRIIB ECD variant polypeptide or binding agent of the disclosure comprises a linker of SEQ ID NO: 94 at the C-terminus of the ActRIIB ECD variant polypeptide. In some embodiments, the ActRIIB ECD variant polypeptide or binding agent of the disclosure comprises a linker of SEQ ID NO: 98 at the C-terminus of the ActRIIB ECD variant polypeptide.

### Fc domain monomers and Fc domains

In some embodiments, the present disclosure provides polypeptides comprising an ActRIIB-ECD variant described herein fused, via linker, to an Fc domain monomer. In some embodiments, the ActRIIB-ECD variant is fused at the C-terminus, via a linker, to the N-terminus of the Fc domain monomer.

As used herein, "Fc domain monomer" describes the single chain protein that, when associated with another Fc domain monomer, forms a functional Fc domain. The association of two Fc domain monomers creates one Fc domain. As used herein, "Fc domain" describes the minimum region (in the context of a larger polypeptide) or smallest protein folded structure (in the context of an isolated protein) that can bind to or be bound by an Fc receptor (FcR). When two Fc domain monomers associate, the resulting Fc domain has Fc receptor binding activity. Thus, an Fc domain is a dimeric structure that can bind an Fc receptor. Unless otherwise noted, all references herein to a "variant Fc domain" are to be understood as referring to a dimeric Fc domain, in which each Fc domain monomer comprises the referenced mutation.

It will be understood that Fc domain as used herein includes the polypeptides comprising the constant region of an antibody excluding the first constant region immunoglobulin domain. Thus, Fc refers to the last two constant region immunoglobulin domains (CH2, CH3) of IgG and optionally the flexible hinge N-terminal to these domains. Although the boundaries of the Fc domain monomer may vary, the human IgG heavy chain Fc domain monomer is usually defined to comprise residues C226 or P230 to its carboxyl-terminus. Unless otherwise noted, all references to amino acid positions in Fc domains and Fc domain monomers are according to the EU index as set forth in Kabat (1991, NIH Publication 91-3242, National Technical Information Service, Springfield, Va.). Fc may refer to this region in isolation, or this region in the context of a polypeptide construct. It is noted that polymorphisms have been observed at a number of Fc positions, including but not limited to Kabat 270, 272, 312, 315, 356, and 358, and thus slight differences between the sequences provided herein and sequences in the art may exist. The Fc domain monomer included in the polypeptides or binding agents of the present disclosure may be an IgG1, IgG2, IgG3, or IgG4 domain.

In exemplary embodiments, the polypeptides of the disclosure comprise one or more constant region of an antibody, e.g., the second constant domain (CH2) and/or the third constant domain (CH3) of an antibody heavy chain, or an Fc domain monomer of an antibody heavy chain. The antibody may be, for example and without limitation, an IgG antibody such as an IgG1, IgG2, IgG3 or IgG4 antibody. In particular embodiments, the antibody is a human antibody, e.g., the Fc domain monomer comprises a constant region of the heavy chain of a human IgG1, IgG2, IgG3 or IgG4. In some embodiments, the Fc domain monomer has at least 85%, at least 90%, at least 95%, at least 96%, at least 97%, at least 98%, or at least 99% sequence identity with a human IgG1, IgG2, IgG3 or IgG4 constant region. In a particular embodiment, the Fc domain monomer comprises or consists of an Fc domain monomer of a human IgG1 antibody. In another particular embodiment, the Fc domain monomer comprises or consists of an Fc domain monomer of a human IgG2 antibody. In another particular embodiment, the Fc domain monomer comprises or consists of an Fc domain monomer of a human IgG4 antibody. Exemplary Fc domain sequences (including both wild type sequences, polymorphisms thereof, and variant sequences) are provided in **Table 4.**

**Table 4: Exemplary Fc domain sequences**

| **Isotype** | **AA Sequence** | **SEQ ID** |
|---|---|---|
| IgG1 - EEM polymorph ("IgG1-EM") | THTCPPCPAPELLGGPSVFLFPPKPKDTLMISRTPEVTCVVVDVSHEDPEVK FNWYVDGVEVHNAKTKPREEQYNSTYRVVSVLTVLHQDWLNGKEYKCKVSNK ALPAPIEKTISKAKGQPREPQVYTLPPSREEMTKNQVSLTCLVKGFYPSDIA VEWESNGQPENNYKTTPPVLDSDGSFFLYSKLTVDKSRWQQGNVFSCSVMHE ALHNHYTQKSLSLSPG | 252 |
| IgG1 - DEL polymorph ("IgG1 -DL") | THTCPPCPAPELLGGPSVFLFPPKPKDTLMISRTPEVTCVVVDVSHEDPEVK FNWYVDGVEVHNAKTKPREEQYNSTYRVVSVLTVLHQDWLNGKEYKCKVSNK ALPAPIEKTISKAKGQPREPQVYTLPPSRDELTKNQVSLTCLVKGFYPSDIA VEWESNGQPENNYKTTPPVLDSDGSFFLYSKLTVDKSRWQQGNVFSCSVMHE ALHNHYTQKSLSLSPG | 253 |
| IgG1 - YTE-EM | THTCPPCPAPELLGGPSVFLFPPKPKDTLYITREPEVTCVVVDVSHEDPEVK FNWYVDGVEVHNAKTKPREEQYNSTYRVVSVLTVLHQDWLNGKEYKCKVSNK ALPAPIEKTISKAKGQPREPQVYTLPPSREEMTKNQVSLTCLVKGFYPSDIA VEWESNGQPENNYKTTPPVLDSDGSFFLYSKLTVDKSRWQQGNVFSCSVMHE ALHNHYTQKSLSLSPG | 254 |
| IgG1 - YTE-DL | THTCPPCPAPELLGGPSVFLFPPKPKDTLYITREPEVTCVVVDVSHEDPEVK FNWYVDGVEVHNAKTKPREEQYNSTYRVVSVLTVLHQDWLNGKEYKCKVSNK ALPAPIEKTISKAKGQPREPQVYTLPPSRDELTKNQVSLTCLVKGFYPSDIA VEWESNGQPENNYKTTPPVLDSDGSFFLYSKLTVDKSRWQQGNVFSCSVMHE ALHNHYTQKSLSLSPG | 255 |
| IgG1 - Y-DL | THTCPPCPAPELLGGPSVFLFPPKPKDTLYISRTPEVTCVVVDVSHEDPEVK FNWYVDGVEVHNAKTKPREEQYNSTYRVVSVLTVLHQDWLNGKEYKCKVSNK ALPAPIEKTISKAKGQPREPQVYTLPPSRDELTKNQVSLTCLVKGFYPSDIA VEWESNGQPENNYKTTPPVLDSDGSFFLYSKLTVDKSRWQQGNVFSCSVMHE ALHNHYTQKSLSLSPG | 256 |
| IgG1 - Y-EM | THTCPPCPAPELLGGPSVFLFPPKPKDTLYISRTPEVTCVVVDVSHEDPEVK FNWYVDGVEVHNAKTKPREEQYNSTYRVVSVLTVLHQDWLNGKEYKCKVSNK ALPAPIEKTISKAKGQPREPQVYTLPPSREEMTKNQVSLTCLVKGFYPSDIA VEWESNGQPENNYKTTPPVLDSDGSFFLYSKLTVDKSRWQQGNVFSCSVMHE ALHNHYTQKSLSLSPG | 257 |
| IgG1 - DL | APELLGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEK TISKAKGQPREPQVYTLPPSRDELTKNQVSLTCLVKGFYPSDIAVEWESNGQ PENNYKTTPPVLDSDGSFFLYSKLTVDKSRWQQGNVFSCSVMHEALHNHYTQ KSLSLSPGK | 258 |
| IgG1 - DL | APELLGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEK TISKAKGQPREPQVYTLPPSRDELTKNQVSLTCLVKGFYPSDIAVEWESNGQ PENNYKTTPPVLDSDGSFFLYSKLTVDKSRWQQGNVFSCSVMHEALHNHYTQ KSLSLSPG | 259 |
| IgG1 - DL | PPCPAPELLGGPSVFLFPPKPKDTLMISRTPEVTCVVVDVSHEDPEVKFNWY VDGVEVHNAKTKPREEQYNSTYRVVSVLTVLHQDWLNGKEYKCKVSNKALPA PIEKTISKAKGQPREPQVYTLPPSRDELTKNQVSLTCLVKGFYPSDIAVEWE SNGQPENNYKTTPPVLDSDGSFFLYSKLTVDKSRWQQGNVFSCSVMHEALHN HYTQKSLSLSPG | 260 |
| IgG1 - DL | DKTHTCPPCPAPELLGGPSVFLFPPKPKDTLMISRTPEVTCVVVDVSHEDPE VKFNWYVDGVEVHNAKTKPREEQYNSTYRVVSVLTVLHQDWLNGKEYKCKVS NKALPAPIEKTISKAKGQPREPQVYTLPPSRDELTKNQVSLTCLVKGFYPSD IAVEWESNGQPENNYKTTPPVLDSDGSFFLYSKLTVDKSRWQQGNVFSCSVM HEALHNHYTQKSLSLSPG | 261 |
| IgG1 - DL | EPKSSDKTHTCPPCPAPELLGGPSVFLFPPKPKDTLMISRTPEVTCVVVDVS HEDPEVKFNWYVDGVEVHNAKTKPREEQYNSTYRVVSVLTVLHQDWLNGKEY KCKVSNKALPAPIEKTISKAKGQPREPQVYTLPPSRDELTKNQVSLTCLVKG FYPSDIAVEWESNGQPENNYKTTPPVLDSDGSFFLYSKLTVDKSRWQQGNVF SCSVMHEALHNHYTQKSLSLSPG | 262 |
| IgG1 - DL | EPKSSDKTHTSPPSPAPELLGGPSVFLFPPKPKDTLMISRTPEVTCVVVDVS HEDPEVKFNWYVDGVEVHNAKTKPREEQYNSTYRVVSVLTVLHQDWLNGKEY KCKVSNKALPAPIEKTISKAKGQPREPQVYTLPPSRDELTKNQVSLTCLVKG FYPSDIAVEWESNGQPENNYKTTPPVLDSDGSFFLYSKLTVDKSRWQQGNVF SCSVMHEALHNHYTQKSLSLSPG | 263 |
| IgG1 - EM | THTCPPCPAPELLGGPSVFLFPPKPKDTLYISRTPEVTCVVVDVSHEDPEVK FNWYVDGVEVHNAKTKPREEQYNSTYRVVSVLTVLHQDWLNGKEYKCKVSNK ALPAPIEKTISKAKGQPREPQVYTLPPSREEMTKNQVSLTCLVKGFYPSDIA VEWESNGQPENNYKTTPPVLDSDGSFFLYSKLTVDKSRWQQGNVFSCSVMHE ALHNHYTQKSLSLSPG | 264 |
| IgG1 - DL | THTCPPCPAPELLGGPSVFLFPPKPKDTLMISRTPEVTCVVVDVSHEDPEVK FNWYVDGVEVHNAKTKPREEQYNSTYRVVSVLTVLHQDWLNGKEYKCKVSNK ALPVPIEKTISKAKGQPREPQVYTLPPSRDELTKNQVSLTCLVKGFYPSDIA VEWESNGQPENNYKTTPPVLDSDGSFFLYSKLTVDKSRWQQGNVFSCSVMHE ALHNHYTQKSLSLSPG | 265 |
| IgG1 - EM | THTCPPCPAPELLGGPSVFLFPPKPKDTLMISRTPEVTCVVVDVSHEDPEVK FNWYVDGVEVHNAKTKPREEQYNSTYRVVSVLTVLHQDWLNGKEYKCKVSNK ALPVPIEKTISKAKGQPREPQVYTLPPSREEMTKNQVSLTCLVKGFYPSDIA VEWESNGQPENNYKTTPPVLDSDGSFFLYSKLTVDKSRWQQGNVFSCSVMHE ALHNHYTQKSLSLSPG | 266 |
| IgG2 | APPVAGPSVFLFPPKPKDTLMISRTPEVTCVVVDVSHEDPEVQFNWYVDGVE VHNAKTKPREEQFNSTFRVVSVLTVVHQDWLNGKEYKCKVSNKGLPAPIEKT ISKTKGQPREPQVYTLPPSREEMTKNQVSLTCLVKGFYPSDISVEWESNGQP ENNYKTTPPMLDSDGSFFLYSKLTVDKSRWQQGNVFSCSVMHEALHNHYTQK SLSLSPGK | 267 |
| IgG2 | VECPPCPAPPVAGPSVFLFPPKPKDTLMISRTPEVTCVVVDVSHEDPEVQFN WYVDGVEVHNAKTKPREEQFNSTFRVVSVLTVVHQDWLNGKEYKCKVSNKGL PAPIEKTISKTKGQPREPQVYTLPPSREEMTKNQVSLTCLVKGFYPSDIAVE WESNGQPENNYKTTPPMLDSDGSFFLYSKLTVDKSRWQQGNVFSCSVMHEAL HNHYTQKSLSLSPGK | 268 |
| IgG2 | ERKCCVECPPCPAPPVAGPSVFLFPPKPKDTLMISRTPEVTCVVVDVSHEDP EVQFNWYVDGVEVHNAKTKPREEQFNSTFRVVSVLTVVHQDWLNGKEYKCKV SNKGLPAPIEKTISKTKGQPREPQVYTLPPSREEMTKNQVSLTCLVKGFYPS DISVEWESNGQPENNYKTTPPMLDSDGSFFLYSKLTVDKSRWQQGNVFSCSV MHEALHNHYTQKSLSLSPGK | 269 |
| IgG2 | ERKSSVECPPCPAPPVAGPSVFLFPPKPKDTLMISRTPEVTCVVVDVSHEDP EVQFNWYVDGVEVHNAKTKPREEQFNSTFRVVSVLTVVHQDWLNGKEYKCKV SNKGLPAPIEKTISKTKGQPREPQVYTLPPSREEMTKNQVSLTCLVKGFYPS DISVEWESNGQPENNYKTTPPMLDSDGSFFLYSKLTVDKSRWQQGNVFSCSV MHEALHNHYTQKSLSLSPG | 270 |
| IgG2 | ERKSSVESPPCPAPPVAGPSVFLFPPKPKDTLMISRTPEVTCVVVDVSHEDP EVQFNWYVDGVEVHNAKTKPREEQFNSTFRVVSVLTVVHQDWLNGKEYKCKV SNKGLPAPIEKTISKTKGQPREPQVYTLPPSREEMTKNQVSLTCLVKGFYPS DISVEWESNGQPENNYKTTPPMLDSDGSFFLYSKLTVDKSRWQQGNVFSCSV MHEALHNHYTQKSLSLSPG | 271 |
| IgG2 | ERKSSVESPPSPAPPVAGPSVFLFPPKPKDTLMISRTPEVTCVVVDVSHEDP EVQFNWYVDGVEVHNAKTKPREEQFNSTFRVVSVLTVVHQDWLNGKEYKCKV SNKGLPAPIEKTISKTKGQPREPQVYTLPPSREEMTKNQVSLTCLVKGFYPS DISVEWESNGQPENNYKTTPPMLDSDGSFFLYSKLTVDKSRWQQGNVFSCSV MHEALHNHYTQKSLSLSPG | 272 |
| IgG2 | APPVAGPSVFLFPPKPKDTLMISRTPEVTCVVVDVSHEDPEVQFNWYVDGVE VHNAKTKPREEQFNSTFRVVSVLTVVHQDWLNGKEYKCKVSNKGLPAPIEKT ISKTKGQPREPQVYTLPPSREEMTKNQVSLTCLVKGFYPSDISVEWESNGQP ENNYKTTPPMLDSDGSFFLYSKLTVDKSRWQQGNVFSCSVMHEALHNHYTQK SLSLSPG | 273 |
| IgG2 | PPCPAPPVAGPSVFLFPPKPKDTLMISRTPEVTCVVVDVSHEDPEVQFNWYV DGVEVHNAKTKPREEQFNSTFRVVSVLTVVHQDWLNGKEYKCKVSNKGLPAP IEKTISKTKGQPREPQVYTLPPSREEMTKNQVSLTCLVKGFYPSDISVEWES NGQPENNYKTTPPMLDSDGSFFLYSKLTVDKSRWQQGNVFSCSVMHEALHNH YTQKSLSLSPG | 274 |
| IgG2 | VECPPCPAPPVAGPSVFLFPPKPKDTLMISRTPEVTCVVVDVSHEDPEVQFN WYVDGVEVHNAKTKPREEQFNSTFRVVSVLTVVHQDWLNGKEYKCKVSNKGL PAPIEKTISKTKGQPREPQVYTLPPSREEMTKNQVSLTCLVKGFYPSDISVE WESNGQPENNYKTTPPMLDSDGSFFLYSKLTVDKSRWQQGNVFSCSVMHEAL HNHYTQKSLSLSPG | 275 |
| IgG2 | PCPPCKCPAPNLLGGPSVFIFPPKIKDVLMISLSPIVTCVVVDVSEDDPDVQ ISWFVNNVEVHTAQTQTHREDYNSTLRVVSALPIQHQDWMSGKEFKCKVNNK DLPAPIERTISKPKGSVRAPQVYVLPPPEEEMTKKQVTLTCMVTDFMPEDIY VEWTNNGKTELNYKNTEPVLDSDGSYFMYSKLRVEKKNWVERNSYSCSVVHE GLHNHHTTKSFSRTPG | 276 |
| IgG3 | APELLGGPSVFLFPPKPKDTLMISRTPEVTCVVVDVSHEDPEVQFKWYVDGV EVHNAKTKPREEQYNSTFRVVSVLTVLHQDWLNGKEYKCKVSNKALPAPIEK TISKTKGQPREPQVYTLPPSREEMTKNQVSLTCLVKGFYPSDIAVEWESSGQ PENNYNTTPPMLDSDGSFFLYSKLTVDKSRWQQGNIFSCSVMHEALHNRFTQ KSLSLSPGK | 277 |
| IgG3 | APELLGGPSVFLFPPKPKDTLMISRTPEVTCVVVDVSHEDPEVQFKWYVDGV EVHNAKTKPREEQYNSTFRVVSVLTVLHQDWLNGKEYKCKVSNKALPAPIEK TISKTKGQPREPQVYTLPPSREEMTKNQVSLTCLVKGFYPSDIAVEWESSGQ PENNYNTTPPMLDSDGSFFLYSKLTVDKSRWQQGNIFSCSVMHEALHNRFTQ KSLSLSPG | 278 |
| IgG3 | PRCPAPELLGGPSVFLFPPKPKDTLMISRTPEVTCVVVDVSHEDPEVQFKWY VDGVEVHNAKTKPREEQYNSTFRVVSVLTVLHQDWLNGKEYKCKVSNKALPA PIEKTISKTKGQPREPQVYTLPPSREEMTKNQVSLTCLVKGFYPSDIAVEWE SSGQPENNYNTTPPMLDSDGSFFLYSKLTVDKSRWQQGNIFSCSVMHEALHN RFTQKSLSLSPG | 279 |
| IgG3 | DTPPPCPRCPAPELLGGPSVFLFPPKPKDTLMISRTPEVTCVVVDVSHEDPE VQFKWYVDGVEVHNAKTKPREEQYNSTFRVVSVLTVLHQDWLNGKEYKCKVS NKALPAPIEKTISKTKGQPREPQVYTLPPSREEMTKNQVSLTCLVKGFYPSD IAVEWESSGQPENNYNTTPPMLDSDGSFFLYSKLTVDKSRWQQGNIFSCSVM HEALHNRFTQKSLSLSPG | 280 |
| IgG3 | EPKSSDTPPPCPRCPAPELLGGPSVFLFPPKPKDTLMISRTPEVTCVVVDVS HEDPEVQFKWYVDGVEVHNAKTKPREEQYNSTFRVVSVLTVLHQDWLNGKEY KCKVSNKALPAPIEKTISKTKGQPREPQVYTLPPSREEMTKNQVSLTCLVKG FYPSDIAVEWESSGQPENNYNTTPPMLDSDGSFFLYSKLTVDKSRWQQGNIF SCSVMHEALHNRFTQKSLSLSPG | 281 |
| IgG3 | EPKSSDTPPPSPRCPAPELLGGPSVFLFPPKPKDTLMISRTPEVTCVVVDVS HEDPEVQFKWYVDGVEVHNAKTKPREEQYNSTFRVVSVLTVLHQDWLNGKEY KCKVSNKALPAPIEKTISKTKGQPREPQVYTLPPSREEMTKNQVSLTCLVKG FYPSDIAVEWESSGQPENNYNTTPPMLDSDGSFFLYSKLTVDKSRWQQGNIF SCSVMHEALHNRFTQKSLSLSPG | 282 |
| IgG3 | EPKSSDTPPPSPRSPAPELLGGPSVFLFPPKPKDTLMISRTPEVTCVVVDVS HEDPEVQFKWYVDGVEVHNAKTKPREEQYNSTFRVVSVLTVLHQDWLNGKEY KCKVSNKALPAPIEKTISKTKGQPREPQVYTLPPSREEMTKNQVSLTCLVKG FYPSDIAVEWESSGQPENNYNTTPPMLDSDGSFFLYSKLTVDKSRWQQGNIF SCSVMHEALHNRFTQKSLSLSPG | 283 |
| IgG4 | APEFLGGPSVFLFPPKPKDTLMISRTPEVTCVVVDVSQEDPEVQFNWYVDGV EVHNAKTKPREEQFNSTYRVVSVLTVLHQDWLNGKEYKCKVSNKGLPSSIEK TISKAKGQPREPQVYTLPPSQEEMTKNQVSLTCLVKGFYPSDIAVEWESNGQ PENNYKTTPPVLDSDGSFFLYSRLTVDKSRWQEGNVFSCSVMHEALHNHYTQ KSLSLSLGK | 284 |
| IgG4 | GPPCPPCPAPEFLGGPSVFLFPPKPKDTLMISRTPEVTCVVVDVSQEDPEVQ FNWYVDGVEVHNAKTKPREEQFNSTYRVVSVLTVLHQDWLNGKEYKCKVSNK GLPSSIEKTISKAKGQPREPQVYTLPPSQEEMTKNQVSLTCLVKGFYPSDIA VEWESNGQPENNYKTTPPVLDSDGSFFLYSRLTVDKSRWQEGNVFSCSVMHE ALHNHYTQKSLSLSLG | 285 |
| IgG4 | APEFLGGPSVFLFPPKPKDTLMISRTPEVTCVVVDVSQEDPEVQFNWYVDGV EVHNAKTKPREEQFNSTYRVVSVLTVLHQDWLNGKEYKCKVSNKGLPSSIEK TISKAKGQPREPQVYTLPPSQEEMTKNQVSLTCLVKGFYPSDIAVEWESNGQ PENNYKTTPPVLDSDGSFFLYSRLTVDKSRWQEGNVFSCSVMHEALHNHYTQ KSLSLSLG | 286 |
| IgG4 | PSCPAPEFLGGPSVFLFPPKPKDTLMISRTPEVTCVVVDVSQEDPEVQFNWY VDGVEVHNAKTKPREEQFNSTYRVVSVLTVLHQDWLNGKEYKCKVSNKGLPS SIEKTISKAKGQPREPQVYTLPPSQEEMTKNQVSLTCLVKGFYPSDIAVEWE SNGQPENNYKTTPPVLDSDGSFFLYSRLTVDKSRWQEGNVFSCSVMHEALHN HYTQKSLSLSLG | 287 |
| IgG4 | ESKYGPPCPSCPAPEFLGGPSVFLFPPKPKDTLMISRTPEVTCVVVDVSQED PEVQFNWYVDGVEVHNAKTKPREEQFNSTYRVVSVLTVLHQDWLNGKEYKCK VSNKGLPSSIEKTISKAKGQPREPQVYTLPPSQEEMTKNQVSLTCLVKGFYP SDIAVEWESNGQPENNYKTTPPVLDSDGSFFLYSRLTVDKSRWQEGNVFSCS VMHEALHNHYTQKSLSLSLG | 288 |
| IgG4 | ESKYGPPCPPCPAPEFLGGPSVFLFPPKPKDTLMISRTPEVTCVVVDVSQED PEVQFNWYVDGVEVHNAKTKPREEQFNSTYRVVSVLTVLHQDWLNGKEYKCK VSNKGLPSSIEKTISKAKGQPREPQVYTLPPSQEEMTKNQVSLTCLVKGFYP SDIAVEWESNGQPENNYKTTPPVLDSDGSFFLYSRLTVDKSRWQEGNVFSCS VMHEALHNHYTQKSLSLSLG | 289 |
| IgG4 | ESKYGPPCPPCPAPEFLGGPSVFLFPPKPKDTLMISRTPEVTCVVVDVSQED PEVQFNWYVDGVEVHNAKTKPREEQFNSTYRVVSVLTVLHQDWLNGKEYKCK VSNKGLPSSIEKTISKAKGQPREPQVYTLPPSQEEMTKNQVSLTCLVKGFYP SDIAVEWESNGQPENNYKTTPPVLDSDGSFFLYSKLTVDKSRWQEGNVFSCS VMHEALHNHYTQKSLSLSLG | 290 |
| IgG4 | ESKYGPPSPSCPAPEFLGGPSVFLFPPKPKDTLMISRTPEVTCVVVDVSQED PEVQFNWYVDGVEVHNAKTKPREEQFNSTYRVVSVLTVLHQDWLNGKEYKCK VSNKGLPSSIEKTISKAKGQPREPQVYTLPPSQEEMTKNQVSLTCLVKGFYP SDIAVEWESNGQPENNYKTTPPVLDSDGSFFLYSRLTVDKSRWQEGNVFSCS VMHEALHNHYTQKSLSLSLG | 291 |
| IgG4 | ESKYGPPSPSSPAPEFLGGPSVFLFPPKPKDTLMISRTPEVTCVVVDVSQED PEVQFNWYVDGVEVHNAKTKPREEQFNSTYRVVSVLTVLHQDWLNGKEYKCK VSNKGLPSSIEKTISKAKGQPREPQVYTLPPSQEEMTKNQVSLTCLVKGFYP SDIAVEWESNGQPENNYKTTPPVLDSDGSFFLYSRLTVDKSRWQEGNVFSCS VMHEALHNHYTQKSLSLSLG | 292 |

In some embodiments, an IgG1 sequence of the disclosure further includes an extension of 2 amino acids, e.g., of DK, at the N-terminus.

In general, ActRIIB-ECD polypeptides are organized such that the Fc domain monomer is linked at its N-terminus to the C-terminus of the ActRIIB-ECD variant, so that for each ActRIIB-ECD polypeptide, the orientation of the construct is, from N-terminus to C-terminus, a single chain of (ActRIIB-ECD variant)-(linker)-(Fc domain monomer). However, the orientation of constructs is not particularly limited, and other orientations are contemplated. For example, in some embodiments the Fc domain monomer may be linked at its C-terminus to the N-terminus of the ActRIIB-ECD variant.

In an exemplary embodiment, the Fc domain monomer allows assembly of two or more polypeptide chains in a covalent manner, for example by disulfide linking between cysteine residues. In this way the Fc domain monomer acts as a dimerization domain, allowing assembly of two ActRIIB-ECD polypeptide chains to form a dimer. In accordance with the present disclosure, such dimers generally comprise two polypeptides, each polypeptide including an ActIIRB-ECD variant linked to the Fc domain monomer as described herein, thereby forming a divalent TGFβ superfamily ligand binding agent. The binding agents described herein therefore comprise two ActIIRB-ECD variants, a linker domain, and an Fc domain.

The Fc domain monomer generally comprises one or more cysteine residue for crosslinking of a first polypeptide with a second polypeptide in a homodimeric construct. For example, the Fc domain monomer may include at least two cysteine residues for forming a disulfide bridge between two polypeptides, thereby forming a dimer. In some embodiments of the present technology, the Fc domain monomer comprises or consists of the sequence set forth in any one of SEQ ID NOs: 252-292, or a sequence at least 85%, at least 90%, at least 95%, at least 96%, at least 97%, at least 98%, or at least 99% identical thereto. In a particular embodiment, the Fc domain monomer comprises or consists of the amino acid sequence set forth in SEQ ID NO: 253. In a particular embodiment, the Fc domain monomer comprises or consists of the amino acid sequence set forth in SEQ ID NO: 266. In a particular embodiment, the Fc domain monomer comprises or consists of the amino acid sequence set forth in SEQ ID NO: 256. In a particular embodiment, the Fc domain monomer comprises or consists of the amino acid sequence set forth in SEQ ID NO: 255.

In some embodiments, the present disclosure provides binding agents comprising a variant Fc domain, i.e., a non-naturally occurring Fc domain, for example an Fc domain comprising one or more non-naturally occurring amino acid residue, substitution, addition, deletion, etc.

In some embodiments of the technology, the Fc domain is a variant Fc domain that forms a variant Fc domain with a desirable property, such as increased half-life, on the polypeptide or binding agent compared to naturally occurring (wild-type) Fc sequences. As used herein, a "variant Fc domain" refers to a non-naturally occurring Fc domain, for example an Fc domain comprising one or more non-naturally occurring amino acid residues, one or more amino acid substitutions relative to a wild-type human constant domain, or one or more amino acid deletion, addition and/or modification.

There are many known polymorphs for the IgG1 Fc domain, including the "DEL" polymorph and the "EEM" polymorph. The DEL polymorph comprises the amino acids D-EL at positions 356, 357, and 358, respectively (also referred to herein as "Fc-DL", *e.g.,* SEQ ID NO: 253). The EEM polymorph comprises the amino acids E-E-M at positions 356, 357, and 358, respectively (also referred to herein as "Fc-EM", *e.g.,* SEQ ID NO: 252). Two binding agents that are otherwise identical except for the presence of a DEL Fc domain or an EEM Fc domain are expected to demonstrate similar properties in terms of ligand binding and therapeutic efficacy. In some embodiments of the technology, the Fc domain is a DEL Fc domain ("DL"). In some embodiments of the technology, the Fc domain is an EEM Fc domain ("EM"). Other polymorphs may also be used, *e.g.,* IgG1 polymorphs of SEQ ID NOs: 252-253 and 258-266, IgG2 polymorphs of SEQ ID NOs: 267-276, IgG3 polymorphs of SEQ ID NOs: 277-283, and IgG4 polymorphs of SEQ ID NOs: 284-292.

In some embodiments, a variant Fc domain formed by two variant Fc domain monomers has altered binding properties for an Fc receptor such as FcRn, relative to a comparable molecule (e.g., a protein having the same amino acid sequence except having a wild type Fc domain monomer). The serum half-life of proteins comprising Fc domains may be increased by increasing the binding affinity of the Fc domain for FcRn. In one embodiment, the Fc domain variant has enhanced serum half-life relative to a comparable molecule. In a particular embodiment, the Fc domain variant comprises at least one amino acid substitution at one or more positions selected from the group consisting of M252Y, S254T and T256 (referred to herein as "YTE"; e.g., SEQ ID NO: 254 and 255). In another embodiment, the Fc domain variant comprises a Y at position 252 (e.g., SEQ ID NO: 256 and 257, referred to herein as "Fc-Y"). In another embodiment, the Fc domain variant comprises a T at position 254. In another embodiment, the Fc domain variant comprises an E at position 256.

Consequently, in some embodiments of the present technology, the ActRIIB-ECD polypeptide comprises a variant Fc domain monomer that forms an Fc domain with increased in vivo half-life relative to a comparable molecule. In some such embodiments, the Fc domain monomer of the ActRIIB-ECD polypeptide comprises at least one substitution of an amino acid residue selected from the group consisting of: residue 252, 254, and 256.

In some embodiments, an ActRIIB-ECD polypeptide comprises a variant Fc domain monomer comprising at least one amino acid substitution selected from the group consisting of M252Y, S254T, and T256E. In such embodiments, the variant Fc domain monomer may further comprise one or more additional amino acid substitution(s) such as, without limitation, E356D and M358L.

In some embodiments, an ActRIIB-ECD polypeptide comprises a variant Fc domain monomer comprising the following amino acid substitutions: M252Y, S254T, and T256E, referred to herein as "FcYTE" or "YTE". In some embodiments, the FcYTE domain monomer is a DEL polymorph (referred to herein as YTE-DL, e.g., SEQ ID NO: 255). In some embodiments, the FcYTE domain monomer is an EEM polymorph (referred to herein as YTE-EM, *e.g.,* SEQ ID NO: 254).

In some embodiments, an ActRIIB-ECD polypeptide comprises a variant Fc domain monomer comprising the following amino acid substitutions: M252Y, referred to herein as "FcY". In some embodiments, the FcY domain monomer is a DEL polymorph (referred to herein as Y-DL, *e.g.,* SEQ ID NO: 256). In some embodiments, the FcY domain monomer is an EEM polymorph (referred to herein as Y-EM, *e.g.,* SEQ ID NO: 257).

In some embodiments, an ActRIIB-ECD polypeptide comprises an Fc domain monomer comprising a Lysine residue (K) at the C-terminus.

In some embodiments, a variant Fc domain (e.g., an Fc domain formed by two variant Fc domain monomers) for use in the ActRIIB-ECD polypeptides of the disclosure comprises one or more amino acid substitution that reduces aggregation and/or increases stability and/or increases half-life of the ActRIIB-ECD polypeptide compared to naturally occurring Fc sequences. In some embodiments, the Fc domain is selected to provide one or more effector function such as antibody dependent cellular cytotoxicity (ADCC), complement activation (complement dependent cytotoxicity or CDC), opsonization, and the like. In an embodiment, a variant Fc domain has enhanced binding to an Fc receptor relative to a comparable molecule. In a specific embodiment, a variant Fc domain has enhanced binding to the neonatal Fc receptor FcRn. In another embodiment, the variant Fc domain and/or the polypeptide or binding agent containing the variant Fc domain has a binding affinity for FcRn that is at least 2 fold, or at least 3 fold, or at least 5 fold, or at least 7 fold, or at least 10 fold, or at least 20 fold, or at least 30 fold, or at least 40 fold, or at least 50 fold, or at least 60 fold, or at least 70 fold, or at least 80 fold, or at least 90 fold, or at least 100 fold, or at least 200 fold greater than that of a comparable molecule. The serum half-life of proteins comprising Fc domain may be increased by increasing the binding affinity of the Fc domain monomer for FcRn. Consequently, in one embodiment the polypeptide or binding agent comprising the variant Fc domain has an enhanced serum half-life relative to a comparable molecule.

Examples for means to extend serum half-life of the polypeptides and binding agents of the disclosure include peptides, proteins or domains of proteins, which are fused or otherwise attached to the polypeptides and binding agents. The group of peptides, proteins or protein domains includes peptides binding to other proteins with preferred pharmacokinetic profile in the human body such as serum albumin (see WO 2009/127691). An alternative concept of such half-life extending peptides includes peptides binding to the neonatal Fc receptor (FcRn, see WO 2007/098420), which can also be used in the polypeptides and binding agents of the present disclosure. The concept of attaching larger domains of proteins or complete proteins includes e.g. the fusion of human serum albumin, variants or mutants of human serum albumin (see WO 2011/051489, WO 2012/059486, WO 2012/150319, WO 2013/135896, WO 2014/072481, WO 2013/075066) or domains thereof as well as the fusion of constant region of immunoglobulins (Fc domains) and variants thereof, as described herein. Such variants of Fc domains may be optimized/modified in order to allow the desired pairing of dimers or multimers, to abolish Fc receptor binding (e.g., the Fcg receptor), to enhance binding to FcRn, or for other reasons. A further concept known in the art to extend the half-life of small protein compounds in the human body is the pegylation of those compounds such as the polypeptide or binding agent of the present disclosure.

In one embodiment, the present disclosure provides binding agents, wherein the Fc domain comprises a non-naturally occurring amino acid residue at one or more positions selected from the group consisting of 234, 235, 236, 237, 238, 239, 240, 241, 243, 244, 245, 247, 251, 252, 254, 255, 256, 262, 263, 264, 265, 266, 267, 268, 269, 279, 280, 284, 292, 296, 297, 298, 299, 305, 313, 316, 325, 326, 327, 328, 329, 330, 332, 333, 334, 339, 341, 343, 370, 373, 378, 392, 416, 419, 421, 440 and 443 as numbered by the EU index as set forth in Kabat. Optionally, the Fc domain may comprise a non-naturally occurring amino acid residue at additional and/or alternative positions known to one skilled in the art (see, e.g., U.S. Pat. Nos. 5,624,821; 6,277,375; 6,737,056; PCT Patent Publications WO 01/58957; WO 02/06919; WO 04/016750; WO 04/029207; WO 04/035752; WO 04/074455; WO 04/099249; WO 04/063351; WO 05/070963; WO 05/040217, WO 05/092925 and WO 06/020114). In a specific embodiment, the present disclosure provides an Fc variant protein composition, wherein the Fc domain comprises at least one amino acid substitution selected from the group consisting of 234D, 234E, 234N, 234Q, 234T, 234H, 234Y, 2341, 234V, 234F, 235A, 235D, 235R. 235W, 235P, 235S, 235N, 235Q, 235T, 235H, 235Y, 2351, 235V, 235F, 236E, 239D, 239E, 239N, 239Q, 239F, 239T, 239H, 239Y, 240I, 240A, 240T, 240M, 241W, 241 L, 241Y, 241E, 241R. 243W, 243L 243Y, 243R, 243Q, 244H, 245A, 247L, 247V, 247G, 251F, 252Y, 254T, 255L, 256E, 256M, 262I, 262A, 262T, 262E, 2631, 263A, 263T, 263M, 264L, 2641, 264W, 264T, 264R, 264F, 264M, 264Y, 264E, 265G, 265N, 265Q, 265Y, 265F, 265V, 265I, 265L, 265H, 265T, 266I, 266A, 266T, 266M, 267Q, 267L, 268E, 269H, 269Y, 269F, 269R, 270E, 280A, 284M, 292P, 292L, 296E, 296Q, 296D, 296N, 296S, 296T, 296L, 296I, 296H, 269G, 297S, 297D, 297E, 298H, 298I, 298T, 298F, 299I, 299L, 299A, 299S, 299V, 299H, 299F, 299E, 305I, 313F, 316D, 325Q, 325L, 325I, 325D, 325E, 325A, 325T, 325V, 325H, 327G, 327W, 327N, 327L, 328S, 328M, 328D, 328E, 328N, 328Q, 328F, 3281, 328V, 328T, 328H, 328A, 329F, 329H, 329Q, 330K, 330G, 330T, 330C, 330L, 330Y, 330V, 330I, 330F, 330R, 330H, 332D, 332S, 332W, 332F, 332E, 332N, 332Q, 332T, 332H, 332Y, 332A, 339T, 370E, 370N, 378D, 392T, 396L, 416G, 419H, 421K, 440Y and 434W as numbered by the EU index as set forth in Kabat. Optionally, the Fc domain may comprise additional and/or alternative amino acid substitutions known to one skilled in the art (see, e.g., U.S. Pat. Nos. 5,624,821; 6,277,375; 6,737,056; PCT Patent Publications WO 01/58957; WO 02/06919; WO 04/016750; WO 04/029207; WO 04/035752 and WO 05/040217).

### Additional domains

It is envisaged that the ActRIIB-ECD polypeptide and/or binding agent of the disclosure may have, in addition to its function to bind to the target TGFβ superfamily ligand(s) as specified, a further binding specificity or a further function. In some embodiments of the present technology, a ActRIIB-ECD polypeptide or binding agent may be conjugated with a targeting agent, a therapeutic moiety, a detectable moiety and/or a diagnostic moiety. In some embodiments, a polypeptide may possess a further function such as a fully functional Fc constant domain mediating antibody-dependent cellular cytotoxicity through recruitment of effector cells like NK cells, by providing a label (fluorescent etc.), by providing a therapeutic agent such as a toxin or radionuclide, and/or by providing means to enhance serum half-life, etc.

In some embodiments, the ActRIIB-ECD polypeptides described herein comprise an ActRIIB-ECD, a linker, an Fc domain monomer, and one or more additional domains. In some embodiments, the one or more additional domains is selected from a fibronectin domain, a human serum albumin domain, As used herein, the term "fibronectin domain" refers to a high molecular weight glycoprotein of the extracellular matrix, or a fragment thereof, that binds to, e.g., membrane-spanning receptor proteins such as integrins and extracellular matrix components such as collagens and fibrins. In some embodiments, a fibronectin domain is a fibronectin type III domain having amino acids 610-702 of the sequence of UniProt ID NO: P02751. In other embodiments, a fibronectin domain is an adnectin protein.

In some embodiments, a polypeptide or binding agent of the disclosure includes an ActRIIB-ECD variant fused to one or more fibronectin domain. Binding to fibronectin domains can improve the pharmacokinetics of protein pharmaceuticals. A fibronectin domain is a high molecular weight glycoprotein of the extracellular matrix, or a fragment thereof, that binds to, e.g., membrane-spanning receptor proteins such as integrins and extracellular matrix components such as collagens and fibrins. In some embodiments of the present invention, a fibronectin domain is joined to the N- or C-terminus (e.g., C-terminus) of an ActRIIB-ECD variant described herein (e.g., an ActRIIB-ECD variant having the amino acid sequence set forth in any one of SEQ ID NOs: 4-62) to increase the serum half-life of the ActRIIB-ECD variant. A fibronectin domain can be joined, either directly or through a linker, to the N- or C-terminus of an ActRIIB-ECD variant, or a polypeptide thereof, or a binding agent thereof. In some embodiments, a polypeptide or binding agent of the disclosure may be fused to the Nor C-terminus of a fibronectin domain, e.g., through conventional genetic or chemical means, e.g., chemical conjugation. If desired, a linker (e.g., a spacer) can be inserted between the ActRIIB-ECD variant and the fibronectin domain. Without being bound by theory, it is expected that in some embodiments inclusion of a fibronectin domain in an ActRIIB-ECD variant described herein may lead to prolonged retention of the therapeutic protein through its binding to integrins and extracellular matrix components such as collagens and fibrins.

As one example, fibronectin domains that can be used in the methods and compositions and polypeptides of the disclosure are generally known in the art. In one embodiment, the fibronectin domain is a fibronectin type III domain having amino acids 610-702 of the sequence of UniProt ID NO: P02751. In another embodiment, the fibronectin domain is an adnectin protein.

As used herein, the term "human serum albumin" refers to the albumin protein present in human blood plasma. Human serum albumin is the most abundant protein in the blood. It constitutes about half of the blood serum protein. In some embodiments, a human serum albumin has the sequence of UniProt ID NO: P02768.

In some embodiments, an ActRIIB variant or a polypeptide or a binding agent described herein may be fused to serum albumin. Binding to serum albumins can improve the pharmacokinetics of protein pharmaceuticals. Serum albumin is a globular protein that is the most abundant blood protein in mammals. Serum albumin is produced in the liver and constitutes about half of the blood serum proteins. It is monomeric and soluble in the blood. Some of the most crucial functions of serum albumin include transporting hormones, fatty acids, and other proteins in the body, buffering pH, and maintaining osmotic pressure needed for proper distribution of bodily fluids between blood vessels and body tissues. In some embodiments, serum albumin is human serum albumin. In some embodiments, a human serum albumin is joined to the N- or C-terminus (e.g., C-terminus) of an ActRIIB-ECD variant described herein (e.g., an ActRIIB-ECD variant having the amino acid sequence set forth in any one of SEQ ID NOs: 4-62) to increase the serum half-life of the ActRIIB-ECD variant. A human serum albumin can be joined, either directly or through a linker, to the N- or C-terminus of an ActRIIB-ECD variant.

As one example, serum albumins that can be used in the polypeptides and methods and compositions described herein are generally known in the art. In one embodiment, the serum albumin includes the sequence of UniProt ID NO: P02768. In some embodiments, a polypeptide or binding agent of the disclosure may be fused to the N- or C-terminus of a human serum albumin, e.g., through conventional genetic or chemical means, e.g., chemical conjugation. If desired, a linker (e.g., a spacer) can be inserted between the ActRIIB-ECD variant and the human serum albumin. Without being bound by theory, it is expected that in some embodiments inclusion of a human serum albumin in an ActRIIB-ECD variant described herein may lead to prolonged retention of the therapeutic protein.

In some embodiments, a polypeptide or binding agent of the disclosure further includes a moiety (e.g., Fc domain monomer, a wild-type Fc domain, an Fc domain with amino acid substitutions (e.g., one or more substitutions that reduce dimerization, an albumin-binding peptide, a fibronectin domain, or a human serum albumin), which may be fused to the N- or C-terminus (e.g., C-terminus) of the ActRIIB-ECD variant, the polypeptide, or the binding agent by way of a linker or other covalent bonds. A polypeptide including an ActRIIB-ECD variant fused to an Fc domain monomer may form a dimer (e.g., homodimer or heterodimer) through the interaction between two Fc domain monomers, which combine to form an Fc domain in the dimer. Furthermore, in some embodiments, a polypeptide or binding agent described herein has a serum half-life of at least 7 days in humans.

### Exemplary TGFβ superfamily binding agents

The overall structures of exemplary binding agents described herein are provided in **Table 5.** The amino acid sequences of each binding agent are provided in **Table 6.**

**Table 5: Structure of exemplary binding agents**

| **Binding agent** | **ActRII ECD** | **Linker** | **Fc domain** | **SEQ ID** |
|---|---|---|---|---|
| P75 | WT hActRIIB (SEQ ID: 2) | 3 aa (GGG) | IgG1-DL (SEQ ID: 253) | 171 |
| P757 | WT hActRIIB (SEQ ID: 2) | 14 aa (SEQ ID:94) | IgG1-DL (SEQ ID: 253) | 172 |
| P444 | WT hActRIIA (SEQ ID:3) | 3 aa (GGG) | IgG1 (SEQ ID: 266) | 173 |
| P739 | hActRIIB G27E (SEQ ID: 4) | 14 aa (SEQ ID: 94) | IgG1-DL (SEQ ID: 253) | 174 |
| P750 | hActRIIB L33R (SEQ ID: 15) | 14 aa (SEQ ID: 94) | IgG1-DL (SEQ ID: 253) | 175 |
| P751 | hActRIIB L33Y (SEQ ID: 12) | 14 aa (SEQ ID: 94) | IgG1-DL (SEQ ID: 253) | 176 |
| P753 | hActRIIB T69H (SEQ ID: 19) | 14 aa (SEQ ID: 94) | IgG1-DL (SEQ ID: 253) | 177 |
| P754 | hActRIIB T69Q (SEQ ID: 20) | 14 aa (SEQ ID: 94) | IgG1-DL (SEQ ID: 253) | 178 |
| P765 | hActRIIB G27E (SEQ ID: 4) | 3 aa (GGG) | IgG1-DL (SEQ ID: 253) | 179 |
| P777 | hActRIIB L33Y (SEQ ID: 12) | 3 aa (GGG) | IgG1-DL (SEQ ID: 253) | 180 |
| P779 | hActRIIB T69H (SEQ ID: 19) | 3 aa (GGG) | IgG1-DL (SEQ ID: 253) | 181 |
| P780 | hActRIIB T69Q (SEQ ID: 20) | 3 aa (GGG) | IgG1-DL (SEQ ID: 253) | 182 |
| P1171 | hActRIIB L33Y T69H (SEQ ID: 28) | 3 aa (GGG) | IgG1-DL (SEQ ID: 253) | 183 |
| P1172 | hActRIIB L33Y T69H (SEQ ID: 28) | 10 aa (SEQ ID: 98) | IgG1-DL (SEQ ID: 253) | 184 |
| P1173 | hActRIIB L33Y T69H (SEQ ID: 28) | 14 aa (SEQ ID: 94) | IgG1-DL (SEQ ID: 253) | 185 |
| P1174 | hActRIIB L33Y T69Q (SEQ ID: 29) | 3 aa (GGG) | IgG1-DL (SEQ ID: 253) | 186 |
| P1175 | hActRIIB L33Y T69Q (SEQ ID: 29) | 10 aa (SEQ ID: 98) | IgG1-DL (SEQ ID: 253) | 187 |
| P1176 | hActRIIB L33Y T69Q (SEQ ID: 29) | 14 aa (SEQ ID: 94) | IgG1-DL (SEQ ID: 253) | 188 |
| P1177 | hActRIIB L33Y T69E (SEQ ID: 30) | 3 aa (GGG) | IgG1-DL (SEQ ID: 253) | 189 |
| P1178 | hActRIIB L33Y T69E (SEQ ID: 30) | 10 aa (SEQ ID: 98) | IgG1-DL (SEQ ID: 253) | 190 |
| P1179 | hActRIIB L33Y T69E (SEQ ID: 30) | 14 aa (SEQ ID: 94) | IgG1-DL (SEQ ID: 253) | 191 |
| P1180 | hActRIIB L33Y G27D (SEQ ID: 33) | 3 aa (GGG) | IgG1-DL (SEQ ID: 253) | 192 |
| P1181 | hActRIIB L33Y G27D (SEQ ID: 33) | 10 aa (SEQ ID: 98) | IgG1-DL (SEQ ID: 253) | 193 |
| P1182 | hActRIIB L33Y G27D (SEQ ID: 33) | 14 aa (SEQ ID: 94) | IgG1-DL (SEQ ID: 253) | 194 |
| P1183 | hActRIIB L33Y G27E (SEQ ID: 32) | 3 aa (GGG) | IgG1-DL (SEQ ID: 253) | 195 |
| P1184 | hActRIIB L33Y G27E (SEQ ID: 32) | 10 aa (SEQ ID: 98) | IgG1-DL (SEQ ID: 253) | 196 |
| P1185 | hActRIIB L33Y G27E (SEQ ID: 32) | 14 aa (SEQ ID: 94) | IgG1-DL (SEQ ID: 253) | 197 |
| P1186 | hActRIIB L33F T69Q (SEQ ID: 31) | 3 aa (GGG) | IgG1-DL (SEQ ID: 253) | 198 |
| P1187 | hActRIIB L33F T69Q (SEQ ID: 31) | 10 aa (SEQ ID: 98) | IgG1-DL (SEQ ID: 253) | 199 |
| P1188 | hActRIIB L33F T69Q (SEQ ID: 31) | 14 aa (SEQ ID: 94) | IgG1-DL (SEQ ID: 253) | 200 |
| P1201 | hActRIIB T69E (SEQ ID: 21) | 3 aa (GGG) | IgG1-DL (SEQ ID: 253) | 201 |
| P1202 | hActRIIB T69E (SEQ ID: 21) | 10 aa (SEQ ID: 98) | IgG1-DL (SEQ ID: 253) | 202 |
| P1203 | hActRIIB T69E (SEQ ID: 21) | 14 aa (SEQ ID: 94) | IgG1-DL (SEQ ID: 253) | 203 |
| P1204 | hActRIIB L33F (SEQ ID: 10) | 3 aa (GGG) | IgG1-DL (SEQ ID: 253) | 204 |
| P1205 | hActRIIB L33F (SEQ ID: 10) | 10 aa (SEQ ID: 98) | IgG1-DL (SEQ ID: 253) | 205 |
| P1206 | hActRIIB L33F (SEQ ID: 10) | 14 aa (SEQ ID: 94) | IgG1-DL (SEQ ID: 253) | 206 |
| P1207 | hActRIIB L33Q (SEQ ID: 11) | 3 aa (GGG) | IgG1-DL (SEQ ID: 253) | 207 |
| P1208 | hActRIIB L33Q (SEQ ID: 11) | 10 aa (SEQ ID: 98) | IgG1-DL (SEQ ID: 253) | 208 |
| P1209 | hActRIIB L33Q (SEQ ID: 11) | 14 aa (SEQ ID: 94) | IgG1-DL (SEQ ID: 253) | 209 |
| P1210 | hActRIIB L33Y (SEQ ID: 12) | 10 aa (SEQ ID: 98) | IgG1-DL (SEQ ID: 253) | 210 |
| P1229 | hActRIIB L33W (SEQ ID: 13) | 14 aa (SEQ ID: 94) | IgG1-DL (SEQ ID: 253) | 211 |
| P1230 | hActRIIB L33E (SEQ ID: 16) | 14 aa (SEQ ID:94) | IgG1-DL (SEQ ID: 253) | 212 |
| P1231 | hActRIIB L33K (SEQ ID: 17) | 14 aa (SEQ ID: 94) | IgG1-DL (SEQ ID: 253) | 213 |
| P1232 | hActRIIB L33M (SEQ ID: 18) | 14 aa (SEQ ID: 94) | IgG1-DL (SEQ ID: 253) | 214 |
| P1235 | hActRIIB L33Y G27N (SEQ ID: 34) | 14 aa (SEQ ID: 94) | IgG1-DL (SEQ ID: 253) | 215 |
| P1236 | hActRIIB L33Y G27Q (SEQ ID: 35) | 14 aa (SEQ ID: 94) | IgG1-DL (SEQ ID: 253) | 216 |
| P1237 | hActRIIB L33Y G27K (SEQ ID: 36) | 14 aa (SEQ ID: 94) | IgG1-DL (SEQ ID: 253) | 217 |
| P1238 | hActRIIB L33Y G27T (SEQ ID: 37) | 14 aa (SEQ ID: 94) | IgG1-DL (SEQ ID: 253) | 218 |
| P1239 | hActRIIB L33Y G27M (SEQ ID: 38) | 14 aa (SEQ ID: 94) | IgG1-DL (SEQ ID: 253) | 219 |
| P1240 | hActRIIB L33Y T69R (SEQ ID: 25) | 14 aa (SEQ ID: 94) | IgG1-DL (SEQ ID: 253) | 220 |
| P1241 | hActRIIB L33Y T69Y (SEQ ID: 26) | 14 aa (SEQ ID: 94) | IgG1-DL (SEQ ID: 253) | 221 |
| P1242 | hActRIIB L33Y T69W (SEQ ID: 27) | 14 aa (SEQ ID: 94) | IgG1-DL (SEQ ID: 253) | 222 |
| P1269 | hActRIIB L33Y, L14E (SEQ ID: 52) | 14 aa (SEQ ID: 94) | IgG1-DL (SEQ ID: 253) | 223 |
| P1270 | hActRIIB L33Y, L14H (SEQ ID: 53) | 14 aa (SEQ ID: 94) | IgG1-DL (SEQ ID: 253) | 224 |
| P1271 | hActRIIB L33Y, L14S (SEQ ID: 54) | 14 aa (SEQ ID: 94) | IgG1-DL (SEQ ID: 253) | 225 |
| P1272 | hActRIIB L33Y, L55Y (SEQ ID: 59) | 14 aa (SEQ ID: 94) | IgG1-DL (SEQ ID: 253) | 226 |
| P1273 | hActRIIB L33Y, L55Q (SEQ ID: 60) | 14 aa (SEQ ID: 94) | IgG1-DL (SEQ ID: 253) | 227 |
| P1274 | hActRIIB L33Y, L55M (SEQ ID: 61) | 14 aa (SEQ ID: 94) | IgG1-DL (SEQ ID: 253) | 228 |
| P1275 | hActRIIB L33Y, L55I (SEQ ID: 62) | 14 aa (SEQ ID: 94) | IgG1-DL (SEQ ID: 253) | 229 |
| P1371 | hActRIIB L33W (SEQ ID: 13) | 14 aa (SEQ ID: 94) | IgG1 Y-DL (SEQ ID: 256) | 230 |
| P1372 | hActRIIB L33W (SEQ ID: 13) | 14 aa (SEQ ID: 94) | IgG1 YTE-DL (SEQ ID: 255) | 231 |
| P1373 | hActRIIB L33W (SEQ ID: 13) | 19 aa (SEQ ID: 89) | IgG1-DL (SEQ ID: 253) | 232 |
| P1374 | hActRIIB L33W (SEQ ID: 13) | 19 aa (SEQ ID: 89) | IgG1 Y-DL (SEQ ID: 256) | 233 |
| P1375 | hActRIIB L33W (SEQ ID: 13) | 19 aa (SEQ ID: 89) | IgG1 YTE-DL (SEQ ID: 255) | 234 |
| P1385 | hActRIIB G27D, L33W (SEQ ID: 43) | 19 aa (SEQ ID: 89) | IgG1-DL (SEQ ID: 253) | 235 |
| P1386 | hActRIIB G27E, L33W (SEQ ID: 44) | 19 aa (SEQ ID: 89) | IgG1-DL (SEQ ID: 253) | 236 |
| P1387 | hActRIIB G27N, L33W (SEQ ID: 45) | 19 aa (SEQ ID: 89) | IgG1-DL (SEQ ID: 253) | 237 |
| P1388 | hActRIIB G27Q, L33W (SEQ ID: 46) | 19 aa (SEQ ID: 89) | IgG1-DL (SEQ ID: 253) | 238 |
| P1389 | hActRIIB G27T, L33W (SEQ ID: 47) | 19 aa (SEQ ID: 89) | IgG1-DL (SEQ ID: 253) | 239 |
| P1390 | hActRIIB G27M, L33W (SEQ ID: 48) | 19 aa (SEQ ID: 89) | IgG1-DL (SEQ ID: 253) | 240 |
| P1391 | hActRIIB G27D, L33Y (SEQ ID: 39) | 19 aa (SEQ ID: 89) | IgG1-DL (SEQ ID: 253) | 241 |
| P1392 | hActRIIB G27E, L33Y (SEQ ID: 40) | 19 aa (SEQ ID: 89) | IgG1-DL (SEQ ID: 253) | 242 |
| P1395 | hActRIIB G27N, L33Y (SEQ ID: 41) | 19 aa (SEQ ID: 89) | IgG1-DL (SEQ ID: 253) | 243 |
| P1396 | hActRIIB G27Q, L33Y (SEQ ID: 42) | 19 aa (SEQ ID: 89) | IgG1-DL (SEQ ID: 253) | 244 |
| P1406 | hActRIIB L14E, L33W (SEQ ID: 55) | 19 aa (SEQ ID: 89) | IgG1-DL (SEQ ID: 253) | 245 |
| P1407 | hActRIIB L14D, L33W (SEQ ID: 56) | 19 aa (SEQ ID: 89) | IgG1-DL (SEQ ID: 253) | 246 |
| P1408 | hActRIIB L14N, L33W (SEQ ID: 57) | 19 aa (SEQ ID: 89) | IgG1-DL (SEQ ID: 253) | 247 |
| P1409 | hActRIIB L14Q, L33W (SEQ ID: 58) | 19 aa (SEQ ID: 89) | IgG1-DL (SEQ ID: 253) | 248 |
| P1410 | hActRIIB L14Q, L33Y (SEQ ID: 49) | 19 aa (SEQ ID: 89) | IgG1-DL (SEQ ID: 253) | 249 |
| P1411 | hActRIIB L14D, L33Y (SEQ ID: 50) | 19 aa (SEQ ID: 89) | IgG1-DL (SEQ ID: 253) | 250 |
| P1412 | hActRIIB L14N, L33Y (SEQ ID: 51) | 19 aa (SEQ ID: 89) | IgG1-DL (SEQ ID: 253) | 251 |

**Table 6: Exemplary binding agent amino acid sequences**

| **Agent** | **AA Sequence** | **SEQ ID** |
|---|---|---|
| P75 | ETRECIYYNANWELERTNQSGLERCEGEQDKRLHCYASWRNSSGTIELVKKGCWLDDFNC YDRQECVATEENPQVYFCCCEGNFCNERFTHLPEAGGPEVTYEPPPTGGGTHTCPPCPAP ELLGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLP PSRDELTKNQVSLTCLVKGFYPSDIAVEWESNGQPENNYKTTPPVLDSDGSFFLYSKLTV DKSRWQQGNVFSCSVMHEALHNHYTQKSLSLSPG | 171 |
| P757 | ETRECIYYNANWELERTNQSGLERCEGEQDKRLHCYASWRNSSGTIELVKKGCWLDDFNC YDRQECVATEENPQVYFCCCEGNFCNERFTHLPEAGGPEVTYEPPPTGGGGSGGGGSGGG GTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPG | 172 |
| P444 | ILGRSETQECLFFNANWEKDRTNQTGVEPCYGDKDKRRHCFATWKNISGSIEIVKQGCWL DDINCYDRTDCVEKKDSPEVYFCCCEGNMCNEKFSYFPEMEVTQPTSNPVTPKPPTGGGT HTCPPCPAPELLGGPSVFLFPPKPKDTLMISRTPEVTCVVVDVSHEDPEVKFNWYVDGVE VHNAKTKPREEQYNSTYRVVSVLTVLHQDWLNGKEYKCKVSNKALPVPIEKTISKAKGQP REPQVYTLPPSREEMTKNQVSLTCLVKGFYPSDIAVEWESNGQPENNYKTTPPVLDSDGS FFLYSKLTVDKSRWQQGNVFSCSVMHEALHNHYTQKSLSLSPG | 173 |
| P739 | ETRECIYYNANWELERTNQSGLERCEEEQDKRLHCYASWRNSSGTIELVKKGCWLDDFNC YDRQECVATEENPQVYFCCCEGNFCNERFTHLPEAGGPEVTYEPPPTGGGGSGGGGSGGG GTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPG | 174 |
| P750 | ETRECIYYNANWELERTNQSGLERCEGEQDKRRHCYASWRNSSGTIELVKKGCWLDDFNC YDRQECVATEENPQVYFCCCEGNFCNERFTHLPEAGGPEVTYEPPPTGGGGSGGGGSGGG GTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPG | 175 |
| P751 | ETRECIYYNANWELERTNQSGLERCEGEQDKRYHCYASWRNSSGTIELVKKGCWLDDFNC YDRQECVATEENPQVYFCCCEGNFCNERFTHLPEAGGPEVTYEPPPTGGGGSGGGGSGGG GTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPG | 176 |
| P753 | ETRECIYYNANWELERTNQSGLERCEGEQDKRLHCYASWRNSSGTIELVKKGCWLDDFNC YDRQECVAHEENPQVYFCCCEGNFCNERFTHLPEAGGPEVTYEPPPTGGGGSGGGGSGGG GTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPG | 177 |
| P754 | ETRECIYYNANWELERTNQSGLERCEGEQDKRLHCYASWRNSSGTIELVKKGCWLDDFNC YDRQECVAQEENPQVYFCCCEGNFCNERFTHLPEAGGPEVTYEPPPTGGGGSGGGGSGGG GTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPG | 178 |
| P765 | ETRECIYYNANWELERTNQSGLERCEEEQDKRLHCYASWRNSSGTIELVKKGCWLDDFNC YDRQECVATEENPQVYFCCCEGNFCNERFTHLPEAGGPEVTYEPPPTGGGTHTCPPCPAP ELLGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLP PSRDELTKNQVSLTCLVKGFYPSDIAVEWESNGQPENNYKTTPPVLDSDGSFFLYSKLTV DKSRWQQGNVFSCSVMHEALHNHYTQKSLSLSPG | 179 |
| P777 | ETRECIYYNANWELERTNQSGLERCEGEQDKRYHCYASWRNSSGTIELVKKGCWLDDFNC YDRQECVATEENPQVYFCCCEGNFCNERFTHLPEAGGPEVTYEPPPTGGGTHTCPPCPAP ELLGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLP PSRDELTKNQVSLTCLVKGFYPSDIAVEWESNGQPENNYKTTPPVLDSDGSFFLYSKLTV DKSRWQQGNVFSCSVMHEALHNHYTQKSLSLSPG | 180 |
| P779 | ETRECIYYNANWELERTNQSGLERCEGEQDKRLHCYASWRNSSGTIELVKKGCWLDDFNC YDRQECVAHEENPQVYFCCCEGNFCNERFTHLPEAGGPEVTYEPPPTGGGTHTCPPCPAP ELLGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLP PSRDELTKNQVSLTCLVKGFYPSDIAVEWESNGQPENNYKTTPPVLDSDGSFFLYSKLTV DKSRWQQGNVFSCSVMHEALHNHYTQKSLSLSPG | 181 |
| P780 | ETRECIYYNANWELERTNQSGLERCEGEQDKRLHCYASWRNSSGTIELVKKGCWLDDFNC YDRQECVAQEENPQVYFCCCEGNFCNERFTHLPEAGGPEVTYEPPPTGGGTHTCPPCPAP ELLGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLP PSRDELTKNQVSLTCLVKGFYPSDIAVEWESNGQPENNYKTTPPVLDSDGSFFLYSKLTV DKSRWQQGNVFSCSVMHEALHNHYTQKSLSLSPG | 182 |
| P1171 | ETRECIYYNANWELERTNQSGLERCEGEQDKRYHCYASWRNSSGTIELVKKGCWLDDFNC YDRQECVAHEENPQVYFCCCEGNFCNERFTHLPEAGGPEVTYEPPPTGGGTHTCPPCPAP ELLGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLP PSRDELTKNQVSLTCLVKGFYPSDIAVEWESNGQPENNYKTTPPVLDSDGSFFLYSKLTV DKSRWQQGNVFSCSVMHEALHNHYTQKSLSLSPG | 183 |
| P1172 | ETRECIYYNANWELERTNQSGLERCEGEQDKRYHCYASWRNSSGTIELVKKGCWLDDFNC YDRQECVAHEENPQVYFCCCEGNFCNERFTHLPEAGGPEVTYEPPPTGGGGSGGGGSTHT CPPCPAPELLGGPSVFLFPPKPKDTLMISRTPEVTCVVVDVSHEDPEVKFNWYVDGVEVH NAKTKPREEQYNSTYRVVSVLTVLHQDWLNGKEYKCKVSNKALPAPIEKTISKAKGQPRE PQVYTLPPSRDELTKNQVSLTCLVKGFYPSDIAVEWESNGQPENNYKTTPPVLDSDGSFF LYSKLTVDKSRWQQGNVFSCSVMHEALHNHYTQKSLSLSPG | 184 |
| P1173 | ETRECIYYNANWELERTNQSGLERCEGEQDKRYHCYASWRNSSGTIELVKKGCWLDDFNC YDRQECVAHEENPQVYFCCCEGNFCNERFTHLPEAGGPEVTYEPPPTGGGGSGGGGSGGG GTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPG | 185 |
| P1174 | ETRECIYYNANWELERTNQSGLERCEGEQDKRYHCYASWRNSSGTIELVKKGCWLDDFNC YDRQECVAQEENPQVYFCCCEGNFCNERFTHLPEAGGPEVTYEPPPTGGGTHTCPPCPAP ELLGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLP PSRDELTKNQVSLTCLVKGFYPSDIAVEWESNGQPENNYKTTPPVLDSDGSFFLYSKLTV DKSRWQQGNVFSCSVMHEALHNHYTQKSLSLSPG | 186 |
| P1175 | ETRECIYYNANWELERTNQSGLERCEGEQDKRYHCYASWRNSSGTIELVKKGCWLDDFNC YDRQECVAQEENPQVYFCCCEGNFCNERFTHLPEAGGPEVTYEPPPTGGGGSGGGGSTHT CPPCPAPELLGGPSVFLFPPKPKDTLMISRTPEVTCVVVDVSHEDPEVKFNWYVDGVEVH NAKTKPREEQYNSTYRVVSVLTVLHQDWLNGKEYKCKVSNKALPAPIEKTISKAKGQPRE PQVYTLPPSRDELTKNQVSLTCLVKGFYPSDIAVEWESNGQPENNYKTTPPVLDSDGSFF LYSKLTVDKSRWQQGNVFSCSVMHEALHNHYTQKSLSLSPG | 187 |
| P1176 | ETRECIYYNANWELERTNQSGLERCEGEQDKRYHCYASWRNSSGTIELVKKGCWLDDFNC YDRQECVAQEENPQVYFCCCEGNFCNERFTHLPEAGGPEVTYEPPPTGGGGSGGGGSGGG GTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPG | 188 |
| P1177 | ETRECIYYNANWELERTNQSGLERCEGEQDKRYHCYASWRNSSGTIELVKKGCWLDDFNC YDRQECVAEEENPQVYFCCCEGNFCNERFTHLPEAGGPEVTYEPPPTGGGTHTCPPCPAP ELLGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLP PSRDELTKNQVSLTCLVKGFYPSDIAVEWESNGQPENNYKTTPPVLDSDGSFFLYSKLTV DKSRWQQGNVFSCSVMHEALHNHYTQKSLSLSPG | 189 |
| P1178 | ETRECIYYNANWELERTNQSGLERCEGEQDKRYHCYASWRNSSGTIELVKKGCWLDDFNC YDRQECVAEEENPQVYFCCCEGNFCNERFTHLPEAGGPEVTYEPPPTGGGGSGGGGSTHT CPPCPAPELLGGPSVFLFPPKPKDTLMISRTPEVTCVVVDVSHEDPEVKFNWYVDGVEVH NAKTKPREEQYNSTYRVVSVLTVLHQDWLNGKEYKCKVSNKALPAPIEKTISKAKGQPRE PQVYTLPPSRDELTKNQVSLTCLVKGFYPSDIAVEWESNGQPENNYKTTPPVLDSDGSFF LYSKLTVDKSRWQQGNVFSCSVMHEALHNHYTQKSLSLSPG | 190 |
| P1179 | ETRECIYYNANWELERTNQSGLERCEGEQDKRYHCYASWRNSSGTIELVKKGCWLDDFNC YDRQECVAEEENPQVYFCCCEGNFCNERFTHLPEAGGPEVTYEPPPTGGGGSGGGGSGGG GTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPG | 191 |
| P1180 | ETRECIYYNANWELERTNQSGLERCEDEQDKRYHCYASWRNSSGTIELVKKGCWLDDFNC YDRQECVATEENPQVYFCCCEGNFCNERFTHLPEAGGPEVTYEPPPTGGGTHTCPPCPAP ELLGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLP PSRDELTKNQVSLTCLVKGFYPSDIAVEWESNGQPENNYKTTPPVLDSDGSFFLYSKLTV DKSRWQQGNVFSCSVMHEALHNHYTQKSLSLSPG | 192 |
| P1181 | ETRECIYYNANWELERTNQSGLERCEDEQDKRYHCYASWRNSSGTIELVKKGCWLDDFNC YDRQECVATEENPQVYFCCCEGNFCNERFTHLPEAGGPEVTYEPPPTGGGGSGGGGSTHT CPPCPAPELLGGPSVFLFPPKPKDTLMISRTPEVTCVVVDVSHEDPEVKFNWYVDGVEVH NAKTKPREEQYNSTYRVVSVLTVLHQDWLNGKEYKCKVSNKALPAPIEKTISKAKGQPRE PQVYTLPPSRDELTKNQVSLTCLVKGFYPSDIAVEWESNGQPENNYKTTPPVLDSDGSFF LYSKLTVDKSRWQQGNVFSCSVMHEALHNHYTQKSLSLSPG | 193 |
| P1182 | ETRECIYYNANWELERTNQSGLERCEDEQDKRYHCYASWRNSSGTIELVKKGCWLDDFNC YDRQECVATEENPQVYFCCCEGNFCNERFTHLPEAGGPEVTYEPPPTGGGGSGGGGSGGG GTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPG | 194 |
| P1183 | ETRECIYYNANWELERTNQSGLERCEEEQDKRYHCYASWRNSSGTIELVKKGCWLDDFNC YDRQECVATEENPQVYFCCCEGNFCNERFTHLPEAGGPEVTYEPPPTGGGTHTCPPCPAP ELLGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLP PSRDELTKNQVSLTCLVKGFYPSDIAVEWESNGQPENNYKTTPPVLDSDGSFFLYSKLTV DKSRWQQGNVFSCSVMHEALHNHYTQKSLSLSPG | 195 |
| P1184 | ETRECIYYNANWELERTNQSGLERCEEEQDKRYHCYASWRNSSGTIELVKKGCWLDDFNC YDRQECVATEENPQVYFCCCEGNFCNERFTHLPEAGGPEVTYEPPPTGGGGSGGGGSTHT CPPCPAPELLGGPSVFLFPPKPKDTLMISRTPEVTCVVVDVSHEDPEVKFNWYVDGVEVH NAKTKPREEQYNSTYRVVSVLTVLHQDWLNGKEYKCKVSNKALPAPIEKTISKAKGQPRE PQVYTLPPSRDELTKNQVSLTCLVKGFYPSDIAVEWESNGQPENNYKTTPPVLDSDGSFF LYSKLTVDKSRWQQGNVFSCSVMHEALHNHYTQKSLSLSPG | 196 |
| P1185 | ETRECIYYNANWELERTNQSGLERCEEEQDKRYHCYASWRNSSGTIELVKKGCWLDDFNC YDRQECVATEENPQVYFCCCEGNFCNERFTHLPEAGGPEVTYEPPPTGGGGSGGGGSGGG GTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPG | 197 |
| P1186 | ETRECIYYNANWELERTNQSGLERCEGEQDKRFHCYASWRNSSGTIELVKKGCWLDDFNC YDRQECVAQEENPQVYFCCCEGNFCNERFTHLPEAGGPEVTYEPPPTGGGTHTCPPCPAP ELLGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLP PSRDELTKNQVSLTCLVKGFYPSDIAVEWESNGQPENNYKTTPPVLDSDGSFFLYSKLTV DKSRWQQGNVFSCSVMHEALHNHYTQKSLSLSPG | 198 |
| P1187 | ETRECIYYNANWELERTNQSGLERCEGEQDKRFHCYASWRNSSGTIELVKKGCWLDDFNC YDRQECVAQEENPQVYFCCCEGNFCNERFTHLPEAGGPEVTYEPPPTGGGGSGGGGSTHT CPPCPAPELLGGPSVFLFPPKPKDTLMISRTPEVTCVVVDVSHEDPEVKFNWYVDGVEVH NAKTKPREEQYNSTYRVVSVLTVLHQDWLNGKEYKCKVSNKALPAPIEKTISKAKGQPRE PQVYTLPPSRDELTKNQVSLTCLVKGFYPSDIAVEWESNGQPENNYKTTPPVLDSDGSFF LYSKLTVDKSRWQQGNVFSCSVMHEALHNHYTQKSLSLSPG | 199 |
| P1188 | ETRECIYYNANWELERTNQSGLERCEGEQDKRFHCYASWRNSSGTIELVKKGCWLDDFNC YDRQECVAQEENPQVYFCCCEGNFCNERFTHLPEAGGPEVTYEPPPTGGGGSGGGGSGGG GTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPG | 200 |
| P1201 | ETRECIYYNANWELERTNQSGLERCEGEQDKRLHCYASWRNSSGTIELVKKGCWLDDFNC YDRQECVAEEENPQVYFCCCEGNFCNERFTHLPEAGGPEVTYEPPPTGGGTHTCPPCPAP ELLGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLP PSRDELTKNQVSLTCLVKGFYPSDIAVEWESNGQPENNYKTTPPVLDSDGSFFLYSKLTV DKSRWQQGNVFSCSVMHEALHNHYTQKSLSLSPG | 201 |
| P1202 | ETRECIYYNANWELERTNQSGLERCEGEQDKRLHCYASWRNSSGTIELVKKGCWLDDFNC YDRQECVAEEENPQVYFCCCEGNFCNERFTHLPEAGGPEVTYEPPPTGGGGSGGGGSTHT CPPCPAPELLGGPSVFLFPPKPKDTLMISRTPEVTCVVVDVSHEDPEVKFNWYVDGVEVH NAKTKPREEQYNSTYRVVSVLTVLHQDWLNGKEYKCKVSNKALPAPIEKTISKAKGQPRE PQVYTLPPSRDELTKNQVSLTCLVKGFYPSDIAVEWESNGQPENNYKTTPPVLDSDGSFF LYSKLTVDKSRWQQGNVFSCSVMHEALHNHYTQKSLSLSPG | 202 |
| P1203 | ETRECIYYNANWELERTNQSGLERCEGEQDKRLHCYASWRNSSGTIELVKKGCWLDDFNC YDRQECVAEEENPQVYFCCCEGNFCNERFTHLPEAGGPEVTYEPPPTGGGGSGGGGSGGG GTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPG | 203 |
| P1204 | ETRECIYYNANWELERTNQSGLERCEGEQDKRFHCYASWRNSSGTIELVKKGCWLDDFNC YDRQECVATEENPQVYFCCCEGNFCNERFTHLPEAGGPEVTYEPPPTGGGTHTCPPCPAP ELLGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLP PSRDELTKNQVSLTCLVKGFYPSDIAVEWESNGQPENNYKTTPPVLDSDGSFFLYSKLTV DKSRWQQGNVFSCSVMHEALHNHYTQKSLSLSPG | 204 |
| P1205 | ETRECIYYNANWELERTNQSGLERCEGEQDKRFHCYASWRNSSGTIELVKKGCWLDDFNC YDRQECVATEENPQVYFCCCEGNFCNERFTHLPEAGGPEVTYEPPPTGGGGSGGGGSTHT CPPCPAPELLGGPSVFLFPPKPKDTLMISRTPEVTCVVVDVSHEDPEVKFNWYVDGVEVH NAKTKPREEQYNSTYRVVSVLTVLHQDWLNGKEYKCKVSNKALPAPIEKTISKAKGQPRE PQVYTLPPSRDELTKNQVSLTCLVKGFYPSDIAVEWESNGQPENNYKTTPPVLDSDGSFF LYSKLTVDKSRWQQGNVFSCSVMHEALHNHYTQKSLSLSPG | 205 |
| P1206 | ETRECIYYNANWELERTNQSGLERCEGEQDKRFHCYASWRNSSGTIELVKKGCWLDDFNC YDRQECVATEENPQVYFCCCEGNFCNERFTHLPEAGGPEVTYEPPPTGGGGSGGGGSGGG GTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPG | 206 |
| P1207 | ETRECIYYNANWELERTNQSGLERCEGEQDKRQHCYASWRNSSGTIELVKKGCWLDDFNC YDRQECVATEENPQVYFCCCEGNFCNERFTHLPEAGGPEVTYEPPPTGGGTHTCPPCPAP ELLGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLP PSRDELTKNQVSLTCLVKGFYPSDIAVEWESNGQPENNYKTTPPVLDSDGSFFLYSKLTV DKSRWQQGNVFSCSVMHEALHNHYTQKSLSLSPG | 207 |
| P1208 | ETRECIYYNANWELERTNQSGLERCEGEQDKRQHCYASWRNSSGTIELVKKGCWLDDFNC YDRQECVATEENPQVYFCCCEGNFCNERFTHLPEAGGPEVTYEPPPTGGGGSGGGGSTHT CPPCPAPELLGGPSVFLFPPKPKDTLMISRTPEVTCVVVDVSHEDPEVKFNWYVDGVEVH NAKTKPREEQYNSTYRVVSVLTVLHQDWLNGKEYKCKVSNKALPAPIEKTISKAKGQPRE PQVYTLPPSRDELTKNQVSLTCLVKGFYPSDIAVEWESNGQPENNYKTTPPVLDSDGSFF LYSKLTVDKSRWQQGNVFSCSVMHEALHNHYTQKSLSLSPG | 208 |
| P1209 | ETRECIYYNANWELERTNQSGLERCEGEQDKRQHCYASWRNSSGTIELVKKGCWLDDFNC YDRQECVATEENPQVYFCCCEGNFCNERFTHLPEAGGPEVTYEPPPTGGGGSGGGGSGGG GTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPG | 209 |
| P1210 | ETRECIYYNANWELERTNQSGLERCEGEQDKRYHCYASWRNSSGTIELVKKGCWLDDFNC YDRQECVATEENPQVYFCCCEGNFCNERFTHLPEAGGPEVTYEPPPTGGGGSGGGGSTHT CPPCPAPELLGGPSVFLFPPKPKDTLMISRTPEVTCVVVDVSHEDPEVKFNWYVDGVEVH NAKTKPREEQYNSTYRVVSVLTVLHQDWLNGKEYKCKVSNKALPAPIEKTISKAKGQPRE PQVYTLPPSRDELTKNQVSLTCLVKGFYPSDIAVEWESNGQPENNYKTTPPVLDSDGSFF LYSKLTVDKSRWQQGNVFSCSVMHEALHNHYTQKSLSLSPG | 210 |
| P1229 | ETRECIYYNANWELERTNQSGLERCEGEQDKRWHCYASWRNSSGTIELVKKGCWLDDFNC YDRQECVATEENPQVYFCCCEGNFCNERFTHLPEAGGPEVTYEPPPTGGGGSGGGGSGGG GTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPG | 211 |
| P1230 | ETRECIYYNANWELERTNQSGLERCEGEQDKREHCYASWRNSSGTIELVKKGCWLDDFNC YDRQECVATEENPQVYFCCCEGNFCNERFTHLPEAGGPEVTYEPPPTGGGGSGGGGSGGG GTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPG | 212 |
| P1231 | ETRECIYYNANWELERTNQSGLERCEGEQDKRHCYASWRNSSGTIELVKKGCWLDDFNC YDRQECVATEENPQVYFCCCEGNFCNERFTHLPEAGGPEVTYEPPPTGGGGSGGGGSGGG GTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPG | 213 |
| P1232 | ETRECIYYNANWELERTNQSGLERCEGEQDKRMHCYASWRNSSGTIELVKKGCWLDDFNC YDRQECVATEENPQVYFCCCEGNFCNERFTHLPEAGGPEVTYEPPPTGGGGSGGGGSGGG GTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPG | 214 |
| P1235 | ETRECIYYNANWELERTNQSGLERCENEQDKRYHCYASWRNSSGTIELVKKGCWLDDFNC YDRQECVATEENPQVYFCCCEGNFCNERFTHLPEAGGPEVTYEPPPTGGGGSGGGGSGGG GTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPG | 215 |
| P1236 | ETRECIYYNANWELERTNQSGLERCEQEQDKRYHCYASWRNSSGTIELVKKGCWLDDFNC YDRQECVATEENPQVYFCCCEGNFCNERFTHLPEAGGPEVTYEPPPTGGGGSGGGGSGGG GTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPG | 216 |
| P1237 | ETRECIYYNANWELERTNQSGLERCEKEQDKRYHCYASWRNSSGTIELVKKGCWLDDFNC YDRQECVATEENPQVYFCCCEGNFCNERFTHLPEAGGPEVTYEPPPTGGGGSGGGGSGGG GTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPG | 217 |
| P1238 | ETRECIYYNANWELERTNQSGLERCETEQDKRYHCYASWRNSSGTIELVKKGCWLDDFNC YDRQECVATEENPQVYFCCCEGNFCNERFTHLPEAGGPEVTYEPPPTGGGGSGGGGSGGG GTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPG | 218 |
| P1239 | ETRECIYYNANWELERTNQSGLERCEMEQDKRYHCYASWRNSSGTIELVKKGCWLDDFNC YDRQECVATEENPQVYFCCCEGNFCNERFTHLPEAGGPEVTYEPPPTGGGGSGGGGSGGG GTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPG | 219 |
| P1240 | ETRECIYYNANWELERTNQSGLERCEGEQDKRYHCYASWRNSSGTIELVKKGCWLDDFNC YDRQECVAREENPQVYFCCCEGNFCNERFTHLPEAGGPEVTYEPPPTGGGGSGGGGSGGG GTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPG | 220 |
| P1241 | ETRECIYYNANWELERTNQSGLERCEGEQDKRYHCYASWRNSSGTIELVKKGCWLDDFNC YDRQECVAYEENPQVYFCCCEGNFCNERFTHLPEAGGPEVTYEPPPTGGGGSGGGGSGGG GTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPG | 221 |
| P1242 | ETRECIYYNANWELERTNQSGLERCEGEQDKRYHCYASWRNSSGTIELVKKGCWLDDFNC YDRQECVAWEENPQVYFCCCEGNFCNERFTHLPEAGGPEVTYEPPPT*GGGGSGGGGSGGG* GTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPG | 222 |
| P1269 | ETRECIYYNANWEEERTNQSGLERCEGEQDKRYHCYASWRNSSGTIELVKKGCWLDDFNC YDRQECVATEENPQVYFCCCEGNFCNERFTHLPEAGGPEVTYEPPPTGGGGSGGGGSGGG GTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPG | 223 |
| P1270 | ETRECIYYNANWEHERTNQSGLERCEGEQDKRYHCYASWRNSSGTIELVKKGCWLDDFNC YDRQECVATEENPQVYFCCCEGNFCNERFTHLPEAGGPEVTYEPPPTGGGGSGGGGSGGG GTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPG | 224 |
| P1271 | ETRECIYYNANWESERTNQSGLERCEGEQDKRYHCYASWRNSSGTIELVKKGCWLDDFNC YDRQECVATEENPQVYFCCCEGNFCNERFTHLPEAGGPEVTYEPPPTGGGGSGGGGSGGG GTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPG | 225 |
| P1272 | ETRECIYYNANWELERTNQSGLERCEGEQDKRYHCYASWRNSSGTIELVKKGCWYDDFNC YDRQECVATEENPQVYFCCCEGNFCNERFTHLPEAGGPEVTYEPPPTGGGGSGGGGSGGG GTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPG | 226 |
| P1273 | ETRECIYYNANWELERTNQSGLERCEGEQDKRYHCYASWRNSSGTIELVKKGCWQDDFNC YDRQECVATEENPQVYFCCCEGNFCNERFTHLPEAGGPEVTYEPPPTGGGGSGGGGSGGG GTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPG | 227 |
| P1274 | ETRECIYYNANWELERTNQSGLERCEGEQDKRYHCYASWRNSSGTIELVKKGCWMDDFNC YDRQECVATEENPQVYFCCCEGNFCNERFTHLPEAGGPEVTYEPPPTGGGGSGGGGSGGG GTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPG | 228 |
| P1275 | ETRECIYYNANWELERTNQSGLERCEGEQDKRYHCYASWRNSSGTIELVKKGCWIDDFNC YDRQECVATEENPQVYFCCCEGNFCNERFTHLPEAGGPEVTYEPPPTGGGGSGGGGSGGG GTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPG | 229 |
| P1371 | ETRECIYYNANWELERTNQSGLERCEGEQDKRWHCYASWRNSSGTIELVKKGCWLDDFNC YDRQECVATEENPQVYFCCCEGNFCNERFTHLPEAGGPEVTYEPPPTGGGGSGGGGSGGG GTHTCPPCPAPELLGGPSVFLFPPKPKDTLYISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPG | 230 |
| P1372 | ETRECIYYNANWELERTNQSGLERCEGEQDKRWHCYASWRNSSGTIELVKKGCWLDDFNC YDRQECVATEENPQVYFCCCEGNFCNERFTHLPEAGGPEVTYEPPPTGGGGSGGGGSGGG GTHTCPPCPAPELLGGPSVFLFPPKPKDTLYITREPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPG | 231 |
| P1373 | ETRECIYYNANWELERTNQSGLERCEGEQDKRWHCYASWRNSSGTIELVKKGCWLDDFNC YDRQECVATEENPQVYFCCCEGNFCNERFTHLPEAGGPEVTYEPPPTGGGGSGGGGSGGG GSGGGGTHTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLSPG | 232 |
| P1374 | ETRECIYYNANWELERTNQSGLERCEGEQDKRWHCYASWRNSSGTIELVKKGCWLDDFNC YDRQECVATEENPQVYFCCCEGNFCNERFTHLPEAGGPEVTYEPPPTGGGGSGGGGSGGG GSGGGGTHTCPPCPAPELLGGPSVFLFPPKPKDTLYISRTPEVTCVVVDVSHEDPEVKFN WYVDGVEVHNAKTKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLSPG | 233 |
| P1375 | ETRECIYYNANWELERTNQSGLERCEGEQDKRWHCYASWRNSSGTIELVKKGCWLDDFNC YDRQECVATEENPQVYFCCCEGNFCNERFTHLPEAGGPEVTYEPPPTGGGGSGGGGSGGG GSGGGGTHTCPPCPAPELLGGPSVFLFPPKPKDTLYITREPEVTCVVVDVSHEDPEVKFN WYVDGVEVHNAKTKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLSPG | 234 |
| P1385 | ETRECIYYNANWELERTNQSGLERCEDEQDKRWHCYASWRNSSGTIELVKKGCWLDDFNC YDRQECVATEENPQVYFCCCEGNFCNERFTHLPEAGGPEVTYEPPPTGGGGSGGGGSGGG GSGGGGTHTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLSPG | 235 |
| P1386 | ETRECIYYNANWELERTNQSGLERCEEEQDKRWHCYASWRNSSGTIELVKKGCWLDDFNC YDRQECVATEENPQVYFCCCEGNFCNERFTHLPEAGGPEVTYEPPPTGGGGSGGGGSGGG GSGGGGTHTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLSPG | 236 |
| P1387 | ETRECIYYNANWELERTNQSGLERCENEQDKRWHCYASWRNSSGTIELVKKGCWLDDFNC YDRQECVATEENPQVYFCCCEGNFCNERFTHLPEAGGPEVTYEPPPTGGGGSGGGGSGGG GSGGGGTHTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLSPG | 237 |
| P1388 | ETRECIYYNANWELERTNQSGLERCEQEQDKRWHCYASWRNSSGTIELVKKGCWLDDFNC YDRQECVATEENPQVYFCCCEGNFCNERFTHLPEAGGPEVTYEPPPTGGGGSGGGGSGGG GSGGGGTHTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLSPG | 238 |
| P1389 | ETRECIYYNANWELERTNQSGLERCETEQDKRWHCYASWRNSSGTIELVKKGCWLDDFNC YDRQECVATEENPQVYFCCCEGNFCNERFTHLPEAGGPEVTYEPPPTGGGGSGGGGSGGG GSGGGGTHTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLSPG | 239 |
| P1390 | ETRECIYYNANWELERTNQSGLERCEMEQDKRWHCYASWRNSSGTIELVKKGCWLDDFNC YDRQECVATEENPQVYFCCCEGNFCNERFTHLPEAGGPEVTYEPPPTGGGGSGGGGSGGG GSGGGGTHTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLSPG | 240 |
| P1391 | ETRECIYYNANWELERTNQSGLERCEDEQDKRYHCYASWRNSSGTIELVKKGCWLDDFNC YDRQECVATEENPQVYFCCCEGNFCNERFTHLPEAGGPEVTYEPPPTGGGGSGGGGSGGG GSGGGGTHTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLSPG | 241 |
| P1392 | ETRECIYYNANWELERTNQSGLERCEEEQDKRYHCYASWRNSSGTIELVKKGCWLDDFNC YDRQECVATEENPQVYFCCCEGNFCNERFTHLPEAGGPEVTYEPPPTGGGGSGGGGSGGG GSGGGGTHTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLSPG | 242 |
| P1395 | ETRECIYYNANWELERTNQSGLERCENEQDKRYHCYASWRNSSGTIELVKKGCWLDDFNC YDRQECVATEENPQVYFCCCEGNFCNERFTHLPEAGGPEVTYEPPPTGGGGSGGGGSGGG GSGGGGTHTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLSPG | 243 |
| P1396 | ETRECIYYNANWELERTNQSGLERCEQEQDKRYHCYASWRNSSGTIELVKKGCWLDDFNC YDRQECVATEENPQVYFCCCEGNFCNERFTHLPEAGGPEVTYEPPPTGGGGSGGGGSGGG GSGGGGTHTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLSPG | 244 |
| P1406 | ETRECIYYNANWEEERTNQSGLERCEGEQDKRWHCYASWRNSSGTIELVKKGCWLDDFNC YDRQECVATEENPQVYFCCCEGNFCNERFTHLPEAGGPEVTYEPPPTGGGGSGGGGSGGG GSGGGGTHTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLSPG | 245 |
| P1407 | ETRECIYYNANWEDERTNQSGLERCEGEQDKRWHCYASWRNSSGTIELVKKGCWLDDFNC YDRQECVATEENPQVYFCCCEGNFCNERFTHLPEAGGPEVTYEPPPTGGGGSGGGGSGGG GSGGGGTHTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLSPG | 246 |
| P1408 | ETRECIYYNANWENERTNQSGLERCEGEQDKRWHCYASWRNSSGTIELVKKGCWLDDFNC YDRQECVATEENPQVYFCCCEGNFCNERFTHLPEAGGPEVTYEPPPTGGGGSGGGGSGGG GSGGGGTHTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLSPG | 247 |
| P1409 | ETRECIYYNANWEQERTNQSGLERCEGEQDKRWHCYASWRNSSGTIELVKKGCWLDDFNC YDRQECVATEENPQVYFCCCEGNFCNERFTHLPEAGGPEVTYEPPPTGGGGSGGGGSGGG GSGGGGTHTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLSPG | 248 |
| P1410 | ETRECIYYNANWEQERTNQSGLERCEGEQDKRYHCYASWRNSSGTIELVKKGCWLDDFNC YDRQECVATEENPQVYFCCCEGNFCNERFTHLPEAGGPEVTYEPPPTGGGGSGGGGSGGG GSGGGGTHTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLSPG | 249 |
| P1411 | ETRECIYYNANWEDERTNQSGLERCEGEQDKRYHCYASWRNSSGTIELVKKGCWLDDFNC YDRQECVATEENPQVYFCCCEGNFCNERFTHLPEAGGPEVTYEPPPTGGGGSGGGGSGGG GSGGGGTHTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLSPG | 250 |
| P1412 | ETRECIYYNANWENERTNQSGLERCEGEQDKRYHCYASWRNSSGTIELVKKGCWLDDFNC YDRQECVATEENPQVYFCCCEGNFCNERFTHLPEAGGPEVTYEPPPTGGGGSGGGGSGGG GSGGGGTHTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLSPG | 251 |

| | | |
|---|---|---|
| *Bold and italicized text indicates the linker sequence; Bold text indicates the N-terminal extension amino acids | | |

In some embodiments, the binding agent comprises, from N-terminus to C-terminus, an ActRIIB ECD, a peptide linker, and an Fc domain. In some embodiments, the ActRIIB ECD comprises one or more amino acid substitutions. In some embodiments, the one or more amino acid substitutions are substitutions at a position selected from L14, G27, L33, L55, and L69, wherein the amino acid numbering is based on SEQ ID NO: 2. In some embodiments, the amino acid substitution at position L14 is selected from L14E, L14H, L14S, L14N, L14Q, and L14D. In some embodiments, the amino acid substitution at position G27 is selected from G27E, G27D, G27N, G27Q, G27Q, G27K, G27T, and G27M. In some embodiments, the amino acid substitution at position L33 is selected from L33R, L33Y, L33F, L33Q, L33W, L33E, L33K, and L33M. In some embodiments, the amino acid substitution at position L55 is selected from L55Y, L55Q, L55M, and L55I. In some embodiments, the amino acid substitution at position T69 is selected from T69H, T69Q, T69E, T69R, T69Y, and T69W.

In some embodiments the TGFβ superfamily ligand binding agent is a homodimeric polypeptide, wherein each polypeptide chain comprises from N-terminus to C-terminus, an ActRIIB ECD comprising the amino acid substitution of G27E, a peptide linker that is 14aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 4, a peptide linker that is 14aa in length, and an IgG1-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 4, a peptide linker that is 14aa in length, and an IgG1-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 174 (P739). In some embodiments the TGFβ superfamily ligand binding agent is a homodimeric polypeptide, wherein each polypeptide chain comprises or consists of the amino acid sequence of SEQ ID NO: 174 (P739).

In some embodiments the TGFβ superfamily ligand binding agent is a homodimeric polypeptide, wherein each polypeptide chain comprises from N-terminus to C-terminus, an ActRIIB ECD comprising the amino acid substitution of L33R, a peptide linker that is 14aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 15, a peptide linker that is 14aa in length, and an IgG1-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 15, a peptide linker that is 14aa in length, and an IgG1-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 175 (P750). In some embodiments the TGFβ superfamily ligand binding agent is a homodimeric polypeptide, wherein each polypeptide chain comprises or consists of the amino acid sequence of SEQ ID NO: 175 (P750).

In some embodiments the TGFβ superfamily ligand binding agent is a homodimeric polypeptide, wherein each polypeptide chain comprises from N-terminus to C-terminus, an ActRIIB ECD comprising the amino acid substitution of L33Y, a peptide linker that is 14aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 12, a peptide linker that is 14aa in length, and an IgG1-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 12, a peptide linker that is 14aa in length, and an IgG1-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 176 (P751). In some embodiments, the TGFβ superfamily ligand binding agent is a homodimeric polypeptide, wherein each polypeptide chain comprises or consists of the amino acid sequence of SEQ ID NO: 176 (P751).

In some embodiments the TGFβ superfamily ligand binding agent is a homodimeric polypeptide, wherein each polypeptide chain comprises from N-terminus to C-terminus, an ActRIIB ECD comprising the amino acid substitution of T69H, a peptide linker that is 14aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 19, a peptide linker that is 14aa in length, and an IgG1-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 19, a peptide linker that is 14aa in length, and an IgG1-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 177 (P753). In some embodiments, the TGFβ superfamily ligand binding agent is a homodimeric polypeptide, wherein each polypeptide chain comprises or consists of the amino acid sequence of SEQ ID NO: 177 (P753).

In some embodiments the TGFβ superfamily ligand binding agent is a homodimeric polypeptide, wherein each polypeptide chain comprises from N-terminus to C-terminus, an ActRIIB ECD comprising the amino acid substitution of T69Q, a peptide linker that is 14aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 20, a peptide linker that is 14aa in length, and an IgG1-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 20, a peptide linker that is 14aa in length, and an IgG1-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 178 (P754). In some embodiments, the TGFβ superfamily ligand binding agent is a homodimeric polypeptide, wherein each polypeptide chain comprises or consists of the amino acid sequence of SEQ ID NO: 178 (P754).

In some embodiments the TGFβ superfamily ligand binding agent is a homodimeric polypeptide, wherein each polypeptide chain comprises from N-terminus to C-terminus, an ActRIIB ECD comprising the amino acid substitution of G27E, a peptide linker that is 3aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 4, a peptide linker that is 3aa in length, and an IgG1-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 4, a peptide linker that is 3aa in length, and an IgG1-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 179 (P765). In some embodiments, the TGFβ superfamily ligand binding agent is a homodimeric polypeptide, wherein each polypeptide chain comprises or consists of the amino acid sequence of SEQ ID NO: 179 (P765).

In some embodiments the TGFβ superfamily ligand binding agent is a homodimeric polypeptide, wherein each polypeptide chain comprises from N-terminus to C-terminus, an ActRIIB ECD comprising the amino acid substitution of L33Y, a peptide linker that is 3aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 12, a peptide linker that is 3aa in length, and an IgG1-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 12, a peptide linker that is 3aa in length, and an IgG1-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 180 (P777). In some embodiments, the TGFβ superfamily ligand binding agent is a homodimeric polypeptide, wherein each polypeptide chain comprises or consists of the amino acid sequence of SEQ ID NO: 180 (P777).

In some embodiments the TGFβ superfamily ligand binding agent is a homodimeric polypeptide, wherein each polypeptide chain comprises from N-terminus to C-terminus, an ActRIIB ECD comprising the amino acid substitution of T69H, a peptide linker that is 3aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 19, a peptide linker that is 3aa in length, and an IgG1-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 19, a peptide linker that is 3aa in length, and an IgG1-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 181 (P779). In some embodiments, the TGFβ superfamily ligand binding agent is a homodimeric polypeptide, wherein each polypeptide chain comprises or consists of the amino acid sequence of SEQ ID NO: 181 (P779).

In some embodiments the TGFβ superfamily ligand binding agent is a homodimeric polypeptide, wherein each polypeptide chain comprises from N-terminus to C-terminus, an ActRIIB ECD comprising the amino acid substitution of T69Q, a peptide linker that is 3aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 20, a peptide linker that is 3aa in length, and an IgG1-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 20, a peptide linker that is 3aa in length, and an IgG1-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 182 (P780). In some embodiments, the TGFβ superfamily ligand binding agent is a homodimeric polypeptide, wherein each polypeptide chain comprises or consists of the amino acid sequence of SEQ ID NO: 182 (P780).

In some embodiments the TGFβ superfamily ligand binding agent is a homodimeric polypeptide, wherein each polypeptide chain comprises from N-terminus to C-terminus, an ActRIIB ECD comprising the amino acid substitutions of L33Y and T69H, a peptide linker that is 3aa, 10aa, or 14aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 28, a peptide linker that is 3aa, 10aa, or 14aa in length, and an IgG1-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 28, a peptide linker that is 3aa, 10aa, or 14aa in length, and an IgG1-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 183 (P1171). In some embodiments, the TGFβ superfamily ligand binding agent is a homodimeric polypeptide, wherein each polypeptide chain comprises or consists of the amino acid sequence of SEQ ID NO: 183 (P1171). In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 184 (P1172). In some embodiments, the TGFβ superfamily ligand binding agent is a homodimeric polypeptide, wherein each polypeptide chain comprises or consists of the amino acid sequence of SEQ ID NO: 184 (P1172). In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 185 (P1173). In some embodiments, the TGFβ superfamily ligand binding agent is a homodimeric polypeptide, wherein each polypeptide chain comprises or consists of the amino acid sequence of SEQ ID NO: 185 (P1173).

In some embodiments the TGFβ superfamily ligand binding agent is a homodimeric polypeptide, wherein each polypeptide chain comprises from N-terminus to C-terminus, an ActRIIB ECD comprising the amino acid substitutions of L33Y and T69Q, a peptide linker that is 3aa, 10aa, or 14aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 29, a peptide linker that is 3aa, 10aa, or 14aa in length, and an IgG1-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 29, a peptide linker that is 3aa, 10aa, or 14aa in length, and an IgG1-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 186 (P1174). In some embodiments, the TGFβ superfamily ligand binding agent is a homodimeric polypeptide, wherein each polypeptide chain comprises or consists of the amino acid sequence of SEQ ID NO: 186 (P1174). In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 187 (P1175). In some embodiments, the TGFβ superfamily ligand binding agent is a homodimeric polypeptide, wherein each polypeptide chain comprises or consists of the amino acid sequence of SEQ ID NO: 187 (P1175). In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 188 (P1176). In some embodiments, the TGFβ superfamily ligand binding agent is a homodimeric polypeptide, wherein each polypeptide chain comprises or consists of the amino acid sequence of SEQ ID NO: 188 (P1176).

In some embodiments the TGFβ superfamily ligand binding agent is a homodimeric polypeptide, wherein each polypeptide chain comprises from N-terminus to C-terminus, an ActRIIB ECD comprising the amino acid substitutions of L33Y and T69E, a peptide linker that is 3aa, 10aa, or 14aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 30, a peptide linker that is 3aa, 10aa, or 14aa in length, and an IgG1-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 30, a peptide linker that is 3aa, 10aa, or 14aa in length, and an IgG1-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 189 (P1177). In some embodiments, the TGFβ superfamily ligand binding agent is a homodimeric polypeptide, wherein each polypeptide chain comprises or consists of the amino acid sequence of SEQ ID NO: 189 (P1177). In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 190 (P1178). In some embodiments, the TGFβ superfamily ligand binding agent is a homodimeric polypeptide, wherein each polypeptide chain comprises or consists of the amino acid sequence of SEQ ID NO: 190 (P1178). In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 191 (P1179). In some embodiments, the TGFβ superfamily ligand binding agent is a homodimeric polypeptide, wherein each polypeptide chain comprises or consists of the amino acid sequence of SEQ ID NO: 191 (P1179).

In some embodiments the TGFβ superfamily ligand binding agent is a homodimeric polypeptide, wherein each polypeptide chain comprises from N-terminus to C-terminus, an ActRIIB ECD comprising the amino acid substitutions of L33Y and G27D, a peptide linker that is 3aa, 10aa, or 14aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 33, a peptide linker that is 3aa, 10aa, or 14aa in length, and an IgG1-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 33, a peptide linker that is 3aa, 10aa, or 14aa in length, and an IgG1-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 192 (P1180). In some embodiments, the TGFβ superfamily ligand binding agent is a homodimeric polypeptide, wherein each polypeptide chain comprises or consists of the amino acid sequence of SEQ ID NO: 192 (P1180). In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 193 (P1181). In some embodiments, the TGFβ superfamily ligand binding agent is a homodimeric polypeptide, wherein each polypeptide chain comprises or consists of the amino acid sequence of SEQ ID NO: 193 (P1181). In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 194 (P1182). In some embodiments, the TGFβ superfamily ligand binding agent is a homodimeric polypeptide, wherein each polypeptide chain comprises or consists of the amino acid sequence of SEQ ID NO: 194 (P1182).

In some embodiments the TGFβ superfamily ligand binding agent is a homodimeric polypeptide, wherein each polypeptide chain comprises from N-terminus to C-terminus, an ActRIIB ECD comprising the amino acid substitutions of L33Y and G27E, a peptide linker that is 3aa, 10aa, or 14aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 32, a peptide linker that is 3aa, 10aa, or 14aa in length, and an IgG1-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 32, a peptide linker that is 3aa, 10aa, or 14aa in length, and an IgG1-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 195 (P1183). In some embodiments, the TGFβ superfamily ligand binding agent is a homodimeric polypeptide, wherein each polypeptide chain comprises or consists of the amino acid sequence of SEQ ID NO: 195 (P1183). In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 196 (P1184). In some embodiments, the TGFβ superfamily ligand binding agent is a homodimeric polypeptide, wherein each polypeptide chain comprises or consists of the amino acid sequence of SEQ ID NO: 196 (P1184). In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 197 (P1185). In some embodiments, the TGFβ superfamily ligand binding agent is a homodimeric polypeptide, wherein each polypeptide chain comprises or consists of the amino acid sequence of SEQ ID NO: 197 (P1185).

In some embodiments the TGFβ superfamily ligand binding agent is a homodimeric polypeptide, wherein each polypeptide chain comprises from N-terminus to C-terminus, an ActRIIB ECD comprising the amino acid substitutions of L33F and T69Q, a peptide linker that is 3aa, 10aa, or 14aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 31, a peptide linker that is 3aa, 10aa, or 14aa in length, and an IgG1-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 31, a peptide linker that is 3aa, 10aa, or 14aa in length, and an IgG1-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 198 (P1186). In some embodiments, the TGFβ superfamily ligand binding agent is a homodimeric polypeptide, wherein each polypeptide chain comprises or consists of the amino acid sequence of SEQ ID NO: 198 (P1186). In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 199 (P1187). In some embodiments, the TGFβ superfamily ligand binding agent is a homodimeric polypeptide, wherein each polypeptide chain comprises or consists of the amino acid sequence of SEQ ID NO: 199 (P1187). In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 200 (P1188). In some embodiments, the TGFβ superfamily ligand binding agent is a homodimeric polypeptide, wherein each polypeptide chain comprises or consists of the amino acid sequence of SEQ ID NO: 200 (P1188).

In some embodiments the TGFβ superfamily ligand binding agent is a homodimeric polypeptide, wherein each polypeptide chain comprises from N-terminus to C-terminus, an ActRIIB ECD comprising the amino acid substitution of T69E, a peptide linker that is 3aa, 10aa, or 14aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 21, a peptide linker that is 3aa, 10aa, or 14aa in length, and an IgG1-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 21, a peptide linker that is 3aa, 10aa, or 14aa in length, and an IgG1-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 201 (P1201). In some embodiments, the TGFβ superfamily ligand binding agent is a homodimeric polypeptide, wherein each polypeptide chain comprises or consists of the amino acid sequence of SEQ ID NO: 201 (P1201). In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 202 (P1202). In some embodiments, the TGFβ superfamily ligand binding agent is a homodimeric polypeptide, wherein each polypeptide chain comprises or consists of the amino acid sequence of SEQ ID NO: 202 (P1202). In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 203 (P1203). In some embodiments, the TGFβ superfamily ligand binding agent is a homodimeric polypeptide, wherein each polypeptide chain comprises or consists of the amino acid sequence of SEQ ID NO: 203 (P1203).

In some embodiments the TGFβ superfamily ligand binding agent is a homodimeric polypeptide, wherein each polypeptide chain comprises from N-terminus to C-terminus, an ActRIIB ECD comprising the amino acid substitution of L33F, a peptide linker that is 3aa, 10aa, or 14aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 10, a peptide linker that is 3aa, 10aa, or 14aa in length, and an IgG1-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 10, a peptide linker that is 3aa, 10aa, or 14aa in length, and an IgG1-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 204 (P1204). In some embodiments, the TGFβ superfamily ligand binding agent is a homodimeric polypeptide, wherein each polypeptide chain comprises or consists of the amino acid sequence of SEQ ID NO: 204 (P1204). In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 205 (P1205). In some embodiments, the TGFβ superfamily ligand binding agent is a homodimeric polypeptide, wherein each polypeptide chain comprises or consists of the amino acid sequence of SEQ ID NO: 205 (P1205). In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 206 (P1206). In some embodiments, the TGFβ superfamily ligand binding agent is a homodimeric polypeptide, wherein each polypeptide chain comprises or consists of the amino acid sequence of SEQ ID NO: 206 (P1206).

In some embodiments the TGFβ superfamily ligand binding agent is a homodimeric polypeptide, wherein each polypeptide chain comprises from N-terminus to C-terminus, an ActRIIB ECD comprising the amino acid substitution of L33Q, a peptide linker that is 3aa, 10aa, or 14aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 11, a peptide linker that is 3aa, 10aa, or 14aa in length, and an IgG1-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 11, a peptide linker that is 3aa, 10aa, or 14aa in length, and an IgG1-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 207 (P1207). In some embodiments, the TGFβ superfamily ligand binding agent is a homodimeric polypeptide, wherein each polypeptide chain comprises or consists of the amino acid sequence of SEQ ID NO: 207 (P1207). In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 208 (P1208). In some embodiments, the TGFβ superfamily ligand binding agent is a homodimeric polypeptide, wherein each polypeptide chain comprises or consists of the amino acid sequence of SEQ ID NO: 208 (P1208). In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 209 (P1209). In some embodiments, the TGFβ superfamily ligand binding agent is a homodimeric polypeptide, wherein each polypeptide chain comprises or consists of the amino acid sequence of SEQ ID NO: 209 (P1209).

In some embodiments the TGFβ superfamily ligand binding agent is a homodimeric polypeptide, wherein each polypeptide chain comprises from N-terminus to C-terminus, an ActRIIB ECD comprising the amino acid substitution of L33Y, a peptide linker that is 10aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 12, a peptide linker that is 10aa in length, and an IgG1-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 12, a peptide linker that is 10aa in length, and an IgG1-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 210 (P1210). In some embodiments, the TGFβ superfamily ligand binding agent is a homodimeric polypeptide, wherein each polypeptide chain comprises or consists of the amino acid sequence of SEQ ID NO: 210 (P1210).

In some embodiments the TGFβ superfamily ligand binding agent is a homodimeric polypeptide, wherein each polypeptide chain comprises from N-terminus to C-terminus, an ActRIIB ECD comprising the amino acid substitution of L33W, a peptide linker that is 14aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 13, a peptide linker that is 14aa in length, and an IgG1-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 13, a peptide linker that is 14aa in length, and an IgG1-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 211 (P1229). In some embodiments, the TGFβ superfamily ligand binding agent is a homodimeric polypeptide, wherein each polypeptide chain comprises or consists of the amino acid sequence of SEQ ID NO: 211 (P1229).

In some embodiments the TGFβ superfamily ligand binding agent is a homodimeric polypeptide, wherein each polypeptide chain comprises from N-terminus to C-terminus, an ActRIIB ECD comprising the amino acid substitution of L33E, a peptide linker that is 14aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 16, a peptide linker that is 14aa in length, and an IgG1-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 16, a peptide linker that is 14aa in length, and an IgG1-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 212 (P1230). In some embodiments, the TGFβ superfamily ligand binding agent is a homodimeric polypeptide, wherein each polypeptide chain comprises or consists of the amino acid sequence of SEQ ID NO: 212 (P1230).

In some embodiments the TGFβ superfamily ligand binding agent is a homodimeric polypeptide, wherein each polypeptide chain comprises from N-terminus to C-terminus, an ActRIIB ECD comprising the amino acid substitution of L33K, a peptide linker that is 14aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 17, a peptide linker that is 14aa in length, and an IgG1-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 17, a peptide linker that is 14aa in length, and an IgG1-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 213 (P1231). In some embodiments, the TGFβ superfamily ligand binding agent is a homodimeric polypeptide, wherein each polypeptide chain comprises or consists of the amino acid sequence of SEQ ID NO: 213 (P1231).

In some embodiments the TGFβ superfamily ligand binding agent is a homodimeric polypeptide, wherein each polypeptide chain comprises from N-terminus to C-terminus, an ActRIIB ECD comprising the amino acid substitution of L33M, a peptide linker that is 14aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 18, a peptide linker that is 14aa in length, and an IgG1-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 18, a peptide linker that is 14aa in length, and an IgG1-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 214 (P1232). In some embodiments, the TGFβ superfamily ligand binding agent is a homodimeric polypeptide, wherein each polypeptide chain comprises or consists of the amino acid sequence of SEQ ID NO: 214 (P1232).

In some embodiments the TGFβ superfamily ligand binding agent is a homodimeric polypeptide, wherein each polypeptide chain comprises from N-terminus to C-terminus, an ActRIIB ECD comprising the amino acid substitutions of L33Y and G27N, a peptide linker that is 14aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 34, a peptide linker that is 14aa in length, and an IgG1-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 34, a peptide linker that is 14aa in length, and an IgG1-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 215 (P1235). In some embodiments, the TGFβ superfamily ligand binding agent is a homodimeric polypeptide, wherein each polypeptide chain comprises or consists of the amino acid sequence of SEQ ID NO: 215 (P1235).

In some embodiments the TGFβ superfamily ligand binding agent is a homodimeric polypeptide, wherein each polypeptide chain comprises from N-terminus to C-terminus, an ActRIIB ECD comprising the amino acid substitutions of L33Y and G27Q, a peptide linker that is 14aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 35, a peptide linker that is 14aa in length, and an IgG1-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 35, a peptide linker that is 14aa in length, and an IgG1-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 216 (P1236). In some embodiments, the TGFβ superfamily ligand binding agent is a homodimeric polypeptide, wherein each polypeptide chain comprises or consists of the amino acid sequence of SEQ ID NO: 216 (P1236).

In some embodiments the TGFβ superfamily ligand binding agent is a homodimeric polypeptide, wherein each polypeptide chain comprises from N-terminus to C-terminus, an ActRIIB ECD comprising the amino acid substitutions of L33Y and G27K, a peptide linker that is 14aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 36, a peptide linker that is 14aa in length, and an IgG1-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 36, a peptide linker that is 14aa in length, and an IgG1-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 217 (P1237). In some embodiments, the TGFβ superfamily ligand binding agent is a homodimeric polypeptide, wherein each polypeptide chain comprises or consists of the amino acid sequence of SEQ ID NO: 217 (P1237).

In some embodiments the TGFβ superfamily ligand binding agent is a homodimeric polypeptide, wherein each polypeptide chain comprises from N-terminus to C-terminus, an ActRIIB ECD comprising the amino acid substitutions of L33Y and G27Y, a peptide linker that is 14aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 37, a peptide linker that is 14aa in length, and an IgG1-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 37, a peptide linker that is 14aa in length, and an IgG1-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 218 (P1238). In some embodiments, the TGFβ superfamily ligand binding agent is a homodimeric polypeptide, wherein each polypeptide chain comprises or consists of the amino acid sequence of SEQ ID NO: 218 (P1238).

In some embodiments the TGFβ superfamily ligand binding agent is a homodimeric polypeptide, wherein each polypeptide chain comprises from N-terminus to C-terminus, an ActRIIB ECD comprising the amino acid substitutions of L33Y and G27M, a peptide linker that is 14aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 38, a peptide linker that is 14aa in length, and an IgG1-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 38, a peptide linker that is 14aa in length, and an IgG1-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 219 (P1239). In some embodiments, the TGFβ superfamily ligand binding agent is a homodimeric polypeptide, wherein each polypeptide chain comprises or consists of the amino acid sequence of SEQ ID NO: 219 (P1239).

In some embodiments the TGFβ superfamily ligand binding agent is a homodimeric polypeptide, wherein each polypeptide chain comprises from N-terminus to C-terminus, an ActRIIB ECD comprising the amino acid substitutions of L33Y and T69R, a peptide linker that is 14aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 25, a peptide linker that is 14aa in length, and an IgG1-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 25, a peptide linker that is 14aa in length, and an IgG1-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 220 (P1240). In some embodiments, the TGFβ superfamily ligand binding agent is a homodimeric polypeptide, wherein each polypeptide chain comprises or consists of the amino acid sequence of SEQ ID NO: 220 (P1240).

In some embodiments the TGFβ superfamily ligand binding agent is a homodimeric polypeptide, wherein each polypeptide chain comprises from N-terminus to C-terminus, an ActRIIB ECD comprising the amino acid substitutions of L33Y and T69Y, a peptide linker that is 14aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 26, a peptide linker that is 14aa in length, and an IgG1-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 26, a peptide linker that is 14aa in length, and an IgG1-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 221 (P1241). In some embodiments, the TGFβ superfamily ligand binding agent is a homodimeric polypeptide, wherein each polypeptide chain comprises or consists of the amino acid sequence of SEQ ID NO: 221 (P1241).

In some embodiments the TGFβ superfamily ligand binding agent is a homodimeric polypeptide, wherein each polypeptide chain comprises from N-terminus to C-terminus, an ActRIIB ECD comprising the amino acid substitutions of L33Y and T69W, a peptide linker that is 14aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 27, a peptide linker that is 14aa in length, and an IgG1-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 27, a peptide linker that is 14aa in length, and an IgG1-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 222 (P1242). In some embodiments, the TGFβ superfamily ligand binding agent is a homodimeric polypeptide, wherein each polypeptide chain comprises or consists of the amino acid sequence of SEQ ID NO: 222 (P1242).

In some embodiments the TGFβ superfamily ligand binding agent is a homodimeric polypeptide, wherein each polypeptide chain comprises from N-terminus to C-terminus, an ActRIIB ECD comprising the amino acid substitutions of L33Y and L14E, a peptide linker that is 14aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 52, a peptide linker that is 14aa in length, and an IgG1-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 52, a peptide linker that is 14aa in length, and an IgG1-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 223 (P1269). In some embodiments, the TGFβ superfamily ligand binding agent is a homodimeric polypeptide, wherein each polypeptide chain comprises or consists of the amino acid sequence of SEQ ID NO: 223 (P1269).

In some embodiments the TGFβ superfamily ligand binding agent is a homodimeric polypeptide, wherein each polypeptide chain comprises from N-terminus to C-terminus, an ActRIIB ECD comprising the amino acid substitutions of L33Y and L14H, a peptide linker that is 14aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 53, a peptide linker that is 14aa in length, and an IgG1-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 53, a peptide linker that is 14aa in length, and an IgG1-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 224 (P1270). In some embodiments, the TGFβ superfamily ligand binding agent is a homodimeric polypeptide, wherein each polypeptide chain comprises or consists of the amino acid sequence of SEQ ID NO: 224 (P1270).

In some embodiments the TGFβ superfamily ligand binding agent is a homodimeric polypeptide, wherein each polypeptide chain comprises from N-terminus to C-terminus, an ActRIIB ECD comprising the amino acid substitutions of L33Y and L14S, a peptide linker that is 14aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 54, a peptide linker that is 14aa in length, and an IgG1-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 54, a peptide linker that is 14aa in length, and an IgG1-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 225 (P1271). In some embodiments, the TGFβ superfamily ligand binding agent is a homodimeric polypeptide, wherein each polypeptide chain comprises or consists of the amino acid sequence of SEQ ID NO: 225 (P1271).

In some embodiments the TGFβ superfamily ligand binding agent is a homodimeric polypeptide, wherein each polypeptide chain comprises from N-terminus to C-terminus, an ActRIIB ECD comprising the amino acid substitutions of L33Y and L55Y, a peptide linker that is 14aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 59, a peptide linker that is 14aa in length, and an IgG1-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 59, a peptide linker that is 14aa in length, and an IgG1-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 226 (P1272). In some embodiments, the TGFβ superfamily ligand binding agent is a homodimeric polypeptide, wherein each polypeptide chain comprises or consists of the amino acid sequence of SEQ ID NO: 226 (P1272).

In some embodiments the TGFβ superfamily ligand binding agent is a homodimeric polypeptide, wherein each polypeptide chain comprises from N-terminus to C-terminus, an ActRIIB ECD comprising the amino acid substitutions of L33Y and L55Q, a peptide linker that is 14aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 60, a peptide linker that is 14aa in length, and an IgG1-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 60, a peptide linker that is 14aa in length, and an IgG1-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 227 (P1273). In some embodiments, the TGFβ superfamily ligand binding agent is a homodimeric polypeptide, wherein each polypeptide chain comprises or consists of the amino acid sequence of SEQ ID NO: 227 (P1273).

In some embodiments the TGFβ superfamily ligand binding agent is a homodimeric polypeptide, wherein each polypeptide chain comprises from N-terminus to C-terminus, an ActRIIB ECD comprising the amino acid substitutions of L33Y and L55M, a peptide linker that is 14aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 61, a peptide linker that is 14aa in length, and an IgG1-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 61, a peptide linker that is 14aa in length, and an IgG1-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 228 (P1274). In some embodiments, the TGFβ superfamily ligand binding agent is a homodimeric polypeptide, wherein each polypeptide chain comprises or consists of the amino acid sequence of SEQ ID NO: 228 (P1274).
In some embodiments the TGFβ superfamily ligand binding agent is a homodimeric polypeptide, wherein each polypeptide chain comprises from N-terminus to C-terminus, an ActRIIB ECD comprising the amino acid substitutions of L33Y and L55I, a peptide linker that is 14aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 62, a peptide linker that is 14aa in length, and an IgG1-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 62, a peptide linker that is 14aa in length, and an IgG1-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 229 (P1275). In some embodiments, the TGFβ superfamily ligand binding agent is a homodimeric polypeptide, wherein each polypeptide chain comprises or consists of the amino acid sequence of SEQ ID NO: 229 (P1275).

In some embodiments the TGFβ superfamily ligand binding agent is a homodimeric polypeptide, wherein each polypeptide chain comprises from N-terminus to C-terminus, an ActRIIB ECD comprising the amino acid substitution of L33W, a peptide linker that is 14aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 13, a peptide linker that is 14aa in length, and an IgG1 Y-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 13, a peptide linker that is 14aa in length, and an IgG1 Y-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 230 (P1371). In some embodiments, the TGFβ superfamily ligand binding agent is a homodimeric polypeptide, wherein each polypeptide chain comprises or consists of the amino acid sequence of SEQ ID NO: 230 (P1371).

In some embodiments the TGFβ superfamily ligand binding agent is a homodimeric polypeptide, wherein each polypeptide chain comprises from N-terminus to C-terminus, an ActRIIB ECD comprising the amino acid substitution of L33W, a peptide linker that is 14aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 13, a peptide linker that is 14aa in length, and an IgG1 YTE-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 13, a peptide linker that is 14aa in length, and an IgG1 YTE-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 231 (P1372). In some embodiments, the TGFβ superfamily ligand binding agent is a homodimeric polypeptide, wherein each polypeptide chain comprises or consists of the amino acid sequence of SEQ ID NO: 231 (P1372).

In some embodiments the TGFβ superfamily ligand binding agent is a homodimeric polypeptide, wherein each polypeptide chain comprises from N-terminus to C-terminus, an ActRIIB ECD comprising the amino acid substitution of L33W, a peptide linker that is 19aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 13, a peptide linker that is 19aa in length, and an IgG1 DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 13, a peptide linker that is 19aa in length, and an IgG1 DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 232 (P1373). In some embodiments, the TGFβ superfamily ligand binding agent is a homodimeric polypeptide, wherein each polypeptide chain comprises or consists of the amino acid sequence of SEQ ID NO: 232 (P1373).

In some embodiments the TGFβ superfamily ligand binding agent is a homodimeric polypeptide, wherein each polypeptide chain comprises from N-terminus to C-terminus, an ActRIIB ECD comprising the amino acid substitution of L33W, a peptide linker that is 19aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 13, a peptide linker that is 19aa in length, and an IgG1 Y-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 13, a peptide linker that is 19aa in length, and an IgG1 Y-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 233 (P1374). In some embodiments, the TGFβ superfamily ligand binding agent is a homodimeric polypeptide, wherein each polypeptide chain comprises or consists of the amino acid sequence of SEQ ID NO: 233 (P1374).

In some embodiments the TGFβ superfamily ligand binding agent is a homodimeric polypeptide, wherein each polypeptide chain comprises from N-terminus to C-terminus, an ActRIIB ECD comprising the amino acid substitution of L33W, a peptide linker that is 19aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 13, a peptide linker that is 19aa in length, and an IgG1 YTE-DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 13, a peptide linker that is 19aa in length, and an IgG1 YTE-DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 234 (P1375). In some embodiments, the TGFβ superfamily ligand binding agent is a homodimeric polypeptide, wherein each polypeptide chain comprises or consists of the amino acid sequence of SEQ ID NO: 234 (P1375).

In some embodiments the TGFβ superfamily ligand binding agent is a homodimeric polypeptide, wherein each polypeptide chain comprises from N-terminus to C-terminus, an ActRIIB ECD comprising the amino acid substitutions of G27D and L33W, a peptide linker that is 19aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 43, a peptide linker that is 19aa in length, and an IgG1 DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 43, a peptide linker that is 19aa in length, and an IgG1 DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 235 (P1385). In some embodiments, the TGFβ superfamily ligand binding agent is a homodimeric polypeptide, wherein each polypeptide chain comprises or consists of the amino acid sequence of SEQ ID NO: 235 (P1385).

In some embodiments the TGFβ superfamily ligand binding agent is a homodimeric polypeptide, wherein each polypeptide chain comprises from N-terminus to C-terminus, an ActRIIB ECD comprising the amino acid substitutions of G27E and L33W, a peptide linker that is 19aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 44, a peptide linker that is 19aa in length, and an IgG1 DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 44, a peptide linker that is 19aa in length, and an IgG1 DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 236 (P1386). In some embodiments, the TGFβ superfamily ligand binding agent is a homodimeric polypeptide, wherein each polypeptide chain comprises or consists of the amino acid sequence of SEQ ID NO: 236 (P1386).

In some embodiments the TGFβ superfamily ligand binding agent is a homodimeric polypeptide, wherein each polypeptide chain comprises from N-terminus to C-terminus, an ActRIIB ECD comprising the amino acid substitutions of G27N and L33W, a peptide linker that is 19aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 45, a peptide linker that is 19aa in length, and an IgG1 DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 45, a peptide linker that is 19aa in length, and an IgG1 DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 237 (P1387). In some embodiments, the TGFβ superfamily ligand binding agent is a homodimeric polypeptide, wherein each polypeptide chain comprises or consists of the amino acid sequence of SEQ ID NO: 237 (P1387).

In some embodiments the TGFβ superfamily ligand binding agent is a homodimeric polypeptide, wherein each polypeptide chain comprises from N-terminus to C-terminus, an ActRIIB ECD comprising the amino acid substitutions of G27Q and L33W, a peptide linker that is 19aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 46, a peptide linker that is 19aa in length, and an IgG1 DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 46, a peptide linker that is 19aa in length, and an IgG1 DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 238 (P1388). In some embodiments, the TGFβ superfamily ligand binding agent is a homodimeric polypeptide, wherein each polypeptide chain comprises or consists of the amino acid sequence of SEQ ID NO: 238 (P1388).

In some embodiments the TGFβ superfamily ligand binding agent is a homodimeric polypeptide, wherein each polypeptide chain comprises from N-terminus to C-terminus, an ActRIIB ECD comprising the amino acid substitutions of G27T and L33W, a peptide linker that is 19aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 47, a peptide linker that is 19aa in length, and an IgG1 DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 47, a peptide linker that is 19aa in length, and an IgG1 DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 239 (P1389). In some embodiments, the TGFβ superfamily ligand binding agent is a homodimeric polypeptide, wherein each polypeptide chain comprises or consists of the amino acid sequence of SEQ ID NO: 239 (P1389).

In some embodiments the TGFβ superfamily ligand binding agent is a homodimeric polypeptide, wherein each polypeptide chain comprises from N-terminus to C-terminus, an ActRIIB ECD comprising the amino acid substitutions of G27M and L33W, a peptide linker that is 19aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 48, a peptide linker that is 19aa in length, and an IgG1 DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 48, a peptide linker that is 19aa in length, and an IgG1 DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 240 (P1390). In some embodiments, the TGFβ superfamily ligand binding agent is a homodimeric polypeptide, wherein each polypeptide chain comprises or consists of the amino acid sequence of SEQ ID NO: 240 (P1390).

In some embodiments the TGFβ superfamily ligand binding agent is a homodimeric polypeptide, wherein each polypeptide chain comprises from N-terminus to C-terminus, an ActRIIB ECD comprising the amino acid substitutions of G27D and L33Y, a peptide linker that is 19aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 39, a peptide linker that is 19aa in length, and an IgG1 DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 39, a peptide linker that is 19aa in length, and an IgG1 DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 241 (P1391). In some embodiments, the TGFβ superfamily ligand binding agent is a homodimeric polypeptide, wherein each polypeptide chain comprises or consists of the amino acid sequence of SEQ ID NO: 241 (P1391).

In some embodiments the TGFβ superfamily ligand binding agent is a homodimeric polypeptide, wherein each polypeptide chain comprises from N-terminus to C-terminus, an ActRIIB ECD comprising the amino acid substitutions of G27E and L33Y, a peptide linker that is 19aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 40, a peptide linker that is 19aa in length, and an IgG1 DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 40, a peptide linker that is 19aa in length, and an IgG1 DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 242 (P1392). In some embodiments, the TGFβ superfamily ligand binding agent is a homodimeric polypeptide, wherein each polypeptide chain comprises or consists of the amino acid sequence of SEQ ID NO: 242 (P1392).

In some embodiments the TGFβ superfamily ligand binding agent is a homodimeric polypeptide, wherein each polypeptide chain comprises from N-terminus to C-terminus, an ActRIIB ECD comprising the amino acid substitutions of G27N and L33Y, a peptide linker that is 19aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 41, a peptide linker that is 19aa in length, and an IgG1 DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 41, a peptide linker that is 19aa in length, and an IgG1 DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 243 (P1395). In some embodiments, the TGFβ superfamily ligand binding agent is a homodimeric polypeptide, wherein each polypeptide chain comprises or consists of the amino acid sequence of SEQ ID NO: 243 (P1395).

In some embodiments the TGFβ superfamily ligand binding agent is a homodimeric polypeptide, wherein each polypeptide chain comprises from N-terminus to C-terminus, an ActRIIB ECD comprising the amino acid substitutions of G27Q and L33Y, a peptide linker that is 19aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 42, a peptide linker that is 19aa in length, and an IgG1 DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 42, a peptide linker that is 19aa in length, and an IgG1 DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 244 (P1396). In some embodiments, the TGFβ superfamily ligand binding agent is a homodimeric polypeptide, wherein each polypeptide chain comprises or consists of the amino acid sequence of SEQ ID NO: 244 (P1396).

In some embodiments the TGFβ superfamily ligand binding agent is a homodimeric polypeptide, wherein each polypeptide chain comprises from N-terminus to C-terminus, an ActRIIB ECD comprising the amino acid substitutions of L14E and L33W, a peptide linker that is 19aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 55, a peptide linker that is 19aa in length, and an IgG1 DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 55, a peptide linker that is 19aa in length, and an IgG1 DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 245 (P1406). In some embodiments, the TGFβ superfamily ligand binding agent is a homodimeric polypeptide, wherein each polypeptide chain comprises or consists of the amino acid sequence of SEQ ID NO: 245 (P1406).

In some embodiments the TGFβ superfamily ligand binding agent is a homodimeric polypeptide, wherein each polypeptide chain comprises from N-terminus to C-terminus, an ActRIIB ECD comprising the amino acid substitutions of L14D and L33W, a peptide linker that is 19aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 56, a peptide linker that is 19aa in length, and an IgG1 DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 56, a peptide linker that is 19aa in length, and an IgG1 DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 246 (P1407). In some embodiments, the TGFβ superfamily ligand binding agent is a homodimeric polypeptide, wherein each polypeptide chain comprises or consists of the amino acid sequence of SEQ ID NO: 246 (P1407).

In some embodiments the TGFβ superfamily ligand binding agent is a homodimeric polypeptide, wherein each polypeptide chain comprises from N-terminus to C-terminus, an ActRIIB ECD comprising the amino acid substitutions of L14N and L33W, a peptide linker that is 19aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 57, a peptide linker that is 19aa in length, and an IgG1 DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 57, a peptide linker that is 19aa in length, and an IgG1 DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 247 (P1408). In some embodiments, the TGFβ superfamily ligand binding agent is a homodimeric polypeptide, wherein each polypeptide chain comprises or consists of the amino acid sequence of SEQ ID NO: 247 (P1408).

In some embodiments the TGFβ superfamily ligand binding agent is a homodimeric polypeptide, wherein each polypeptide chain comprises from N-terminus to C-terminus, an ActRIIB ECD comprising the amino acid substitutions of L14Q and L33W, a peptide linker that is 19aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 58, a peptide linker that is 19aa in length, and an IgG1 DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 58, a peptide linker that is 19aa in length, and an IgG1 DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 248 (P1409). In some embodiments, the TGFβ superfamily ligand binding agent is a homodimeric polypeptide, wherein each polypeptide chain comprises or consists of the amino acid sequence of SEQ ID NO: 248 (P1409).

In some embodiments the TGFβ superfamily ligand binding agent is a homodimeric polypeptide, wherein each polypeptide chain comprises from N-terminus to C-terminus, an ActRIIB ECD comprising the amino acid substitutions of L14Q and L33Y, a peptide linker that is 19aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 49, a peptide linker that is 19aa in length, and an IgG1 DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 49, a peptide linker that is 19aa in length, and an IgG1 DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 249 (P1410). In some embodiments, the TGFβ superfamily ligand binding agent is a homodimeric polypeptide, wherein each polypeptide chain comprises or consists of the amino acid sequence of SEQ ID NO: 249 (P1410).

In some embodiments the TGFβ superfamily ligand binding agent is a homodimeric polypeptide, wherein each polypeptide chain comprises from N-terminus to C-terminus, an ActRIIB ECD comprising the amino acid substitutions of L14D and L33Y, a peptide linker that is 19aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 50, a peptide linker that is 19aa in length, and an IgG1 DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 50, a peptide linker that is 19aa in length, and an IgG1 DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 250 (P1411). In some embodiments, the TGFβ superfamily ligand binding agent is a homodimeric polypeptide, wherein each polypeptide chain comprises or consists of the amino acid sequence of SEQ ID NO: 250 (P1411).

In some embodiments the TGFβ superfamily ligand binding agent is a homodimeric polypeptide, wherein each polypeptide chain comprises from N-terminus to C-terminus, an ActRIIB ECD comprising the amino acid substitutions of L14N and L33Y, a peptide linker that is 19aa in length, and an IgG1 Fc domain monomer. In some embodiments, the TGFβ superfamily ligand binding agent is a homodimeric polypeptide, wherein each polypeptide chain comprises from N-terminus to C-terminus, an ActRIIB ECD comprising an amino acid sequence that is at least 95%, 96%, 97%, 98%, or 99% identical to SEQ ID NO: 51, a peptide linker that is 19aa in length, and an IgG1 DL Fc domain monomer. In some embodiments, the TGFβ superfamily ligand binding agent is a homodimeric polypeptide, wherein each polypeptide chain comprises from N-terminus to C-terminus, an ActRIIB ECD comprising or consisting of the amino acid sequence of SEQ ID NO: 51, a peptide linker that is 19aa in length, and an IgG1 DL Fc domain monomer. In some embodiments, the TGFβ superfamily ligand binding agent is a homodimeric polypeptide, wherein each polypeptide chain comprises an amino acid sequence that is at least 95%, 96%, 97%, 98%, or 99% identical to SEQ ID NO: 251 (P1412). In some embodiments, the TGFβ superfamily ligand binding agent is a homodimeric polypeptide, wherein each polypeptide chain comprises or consists of the amino acid sequence of SEQ ID NO: 251 (P1412).

In some embodiments, the polypeptides or binding agents of the disclosure are "isolated" or "substantially pure". "Isolated" or "substantially pure", when used to describe the polypeptides or binding agents disclosed herein, means a polypeptide or binding agent that has been identified, separated and/or recovered from a component of its production environment. Preferably, the polypeptide or binding agent is free or substantially free of association with all other components from its production environment. Contaminant components of its production environment, such as that resulting from recombinant transfected cells, are materials that would typically interfere with diagnostic or therapeutic uses for the polypeptide, and may include enzymes, hormones, and other proteinaceous or non-proteinaceous solutes. The polypeptides or binding agents may, e.g., constitute at least about 5%, or at least about 50% by weight of the total protein in a given sample. It is understood that the isolated protein may constitute from 5% to 99.9% by weight of the total protein content, depending on the circumstances. The polypeptide or binding agent may be made at a significantly higher concentration through the use of an inducible promoter or high expression promoter, such that it is made at increased concentration levels. The definition includes the production of a polypeptide or binding agent in a wide variety of organisms and/or host cells that are known in the art. In preferred embodiments, the polypeptide or binding agent will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Ordinarily, however, an isolated polypeptide or binding agent will be prepared by at least one purification step, such as, for example and without limitation, affinity and/or ionexchange chromatography, e.g., binding to a Protein A column.

In some embodiments, polypeptides and binding agents of the present disclosure are characterized, for example, by one or more of the following: a particularly high affinity for one or more of activin A, activin B, GDF-8, and GDF-11; high neutralization potency (low IC50 values) for one or more of activin A, activin B, GDF-8, and GDF-11; a particularly low or undetectable affinity for BMP-9 and/or BMP-10; low or not detectable neutralization potency (high IC50 value) for BMP-9 and/or BMP-10; high thermostability; high plasma stability; long or extended half-life, low turbidity; high protein homogeneity; and/or high manufacturability.

The biological activity of a polypeptide or binding agent, or pharmaceutical composition thereof, of the disclosure can be determined for instance by cellular neutralization assays, binding assays, competition assays and the like. "Efficacy" or "in vivo efficacy" as used herein refers to the response to therapy using a polypeptide or binding agent or pharmaceutical composition of the disclosure. The success or in vivo efficacy of the therapy using a polypeptide or binding agent or pharmaceutical composition of the disclosure refers to the effectiveness of the a polypeptide or binding agent or composition for its intended purpose, e.g., the ability of the a polypeptide or binding agent or composition to cause its desired effect, i.e. treatment, amelioration, or prevention of a TGFβ superfamily-associated disease or disorder as defined herein. The in vivo efficacy may be monitored by established standard methods for the respective disease entities. In addition, various disease specific clinical chemistry parameters and other established standard methods may be used.

Another major challenge in the development of drugs such as a pharmaceutical composition of the disclosure is the predictable modulation of pharmacokinetic properties. To this end, a pharmacokinetic profile of the drug candidate, i.e., a profile of the pharmacokinetic parameters that affect the ability of a particular drug to treat a given condition, can be established. Pharmacokinetic parameters of the drug influencing the ability of a drug for treating a certain disease entity include, but are not limited to: half-life, volume of distribution, hepatic first-pass metabolism and the degree of blood serum binding. The efficacy of a given drug agent can be influenced by each of the parameters mentioned above.

Pharmacokinetic parameters also include bioavailability, lag time (Tlag), Tmax, absorption rates, more onset and/or Cmax for a given amount of drug administered. "Bioavailability" means the amount of a drug in the blood compartment. "Lag time" means the time delay between the administration of the drug and its detection and measurability in blood or plasma. "Tmax" is the time after which maximal blood concentration of the drug is reached, and "Cmax" is the blood concentration maximally obtained with a given drug. The time to reach a blood or tissue concentration of the drug which is required for its biological effect is influenced by all parameters.

In some embodiments, the polypeptide or binding agent of the present disclosure has a half-life of about 3 days or longer, about 5 days or longer, about 1 week or longer, about 2 weeks or longer, about 3 weeks or longer, about 4 weeks or longer, about 5 weeks or longer, about 6 weeks or longer, or about two months or longer.

In some embodiments, the polypeptides or binding agents of the present disclosure may show a favorable thermostability with aggregation temperatures of about 45 °C or higher, about 45 to about 50 °C, about 52-about 54 °C, about 56 to about 60 °C, or about 60 °C or higher. The thermostability parameter can be determined in terms of polypeptide aggregation temperature as follows: Protein solution at a test concentration (e.g., 100 µg/ml, 250 µg/ml) is transferred into a single use cuvette and placed in a Dynamic Light Scattering (DLS) device. The sample is heated from 40 °C to 70 °C at a heating rate of 0.5 °C/min with constant acquisition of the measured radius. Increase of radius indicating melting of the protein and aggregation is used to calculate the aggregation temperature of the polypeptide. Other methods known in the art may be used.

In an embodiment the polypeptide or binding agent according to the disclosure is stable at 2-8 °C for at least 1 month, 2 months, or 3 months. In an embodiment the polypeptide or binding agent according to the disclosure is stable at 25-40 °C for at least 4 weeks. In an embodiment the polypeptide or binding agent according to the disclosure is stable after undergoing 3 Freeze/Thaw cycles. In an embodiment the polypeptide or binding agent according to the disclosure is stable at -20 °C for 1 month, 2 month, 3 months or longer.

Alternatively, temperature melting curves can be determined by Differential Scanning Calorimetry (DSC) to determine intrinsic biophysical protein stabilities of the polypeptides or binding agents. These experiments may be performed using a MicroCal LLC (Northampton, Mass., U.S.A) VP-DSC device. The energy uptake of a sample containing a polypeptide or binding agent is recorded from 20 °C to 90 °C compared to a sample containing only the formulation buffer. For recording of the respective melting curve, the overall sample temperature is increased stepwise. At each temperature T energy uptake of the sample and the formulation buffer reference is recorded. The difference in energy uptake Cp (kcal/mole/ °C) of the sample minus the reference is plotted against the respective temperature. The melting temperature is defined as the temperature at the first maximum of energy uptake.

In a further embodiment the polypeptides or binding agents according to the disclosure are stable at acidic pH. The more tolerant the polypeptide or binding agent behaves at unphysiologic pH such as pH 5.5 (a pH which is required to run e.g. a cation exchange chromatography), the higher is the recovery of the polypeptide or binding agent eluted from an ion exchange column relative to the total amount of loaded protein. Recovery of the polypeptides or binding agents from an ion (e.g., cation) exchange column at pH 5.5 may be 50% or more, 60% or more, 65% or more, 70% or more, 72% or more, 74% or more, 76% or more, 78% or more, 80% or more, 90% or more, 95% or more, or 99% or more.

### Amino acid sequence modifications

Amino acid sequence modifications of the polypeptides and binding agents described herein are contemplated. For example, it may be desirable to improve the binding affinity, effector functions, half-life and/or other biological properties of the polypeptide or binding agent. Amino acid sequence modification/variants of the polypeptides and binding agents are generally prepared by introducing appropriate nucleotide changes into the encoding nucleic acid, or by peptide synthesis. All of the below described amino acid sequence modifications should result in a polypeptide or binding agent which still retains the desired biological activity (e.g., binding to one or more of activin A, activin B, GDF-8, GDF-11, without substantial binding to BMP-9 and BMP-10) of the unmodified parental molecule.

As used herein, the term "functionally equivalent" refers to modified sequences that have the same or substantially the same biological activity or function as the original sequence from which it is derived, e.g., no significant change in physiological, chemical, physicochemical or functional properties compared to the original sequence. The term "substantially identical" refers to sequences that are functionally equivalent to the original or reference sequence and have a high degree of sequence identity thereto. Generally, a substantially identical sequence is at least 85%, at least about 90%, at least about 95%, at least about 96%, at least about 97%, at least about 98%, or at least about 99% identical to the original or reference sequence and has the same function. In some cases when referring to nucleic acid sequences, a substantially identical sequence hybridizes to the original sequence under high stringency conditions, for example at salt and temperature conditions substantially equivalent to 0.5 X SSC to about 5 X SSC and 65 °C for both hybridization and wash. In general, modified sequences that are substantially identical or functionally equivalent to sequences provided in accordance with the present disclosure are meant to be encompassed.

Amino acid modifications include, for example, deletions from, and/or insertions into, and/or substitutions of, residues within the amino acid sequences of the polypeptides or binding agents. Any combination of deletion, insertion, and substitution is made to arrive at the final construct, provided that the final construct possesses the desired characteristics. The amino acid changes also may alter post-translational processes of the polypeptides or binding agents, such as changing the number or position of glycosylation sites. In a particular embodiment, one or more amino acid is changed to alter a glycosylation site.

For example, 1, 2, 3, 4, 5, or 6 amino acids may be inserted or deleted in a polypeptide or binding agent. Preferably, amino acid sequence insertions include amino- and/or carboxyl-terminal fusions ranging in length from 1, 2, 3, 4, 5, 6, 7, 8, 9 or 10 residues to polypeptides containing a hundred or more residues, as well as intra-sequence insertions of single or multiple amino acid residues. An insertional variant of the polypeptides or binding agents of the disclosure includes fusion to the N-terminus or to the C-terminus of the polypeptides or binding agents of an enzyme or fusion to a polypeptide which increases the serum half-life of the polypeptides or binding agents.

It is envisioned that modifications of the polypeptides or binding agents described herein are also encompassed. Modifications encompassed by the present disclosure include those having a variation in the amino acid sequence of the polypeptides or binding agents. Modifications of the polypeptides or binding agents include, for example, those having similar or improved binding affinity, avidity, ligand specificity, potency of inhibition, stability, manufacturability, half-life, and/or reduced aggregation in comparison with the polypeptides or binding agents disclosed herein.

One site of interest for substitutional mutagenesis includes the Fc domain monomer, as described hereinabove. Exemplary embodiments of modified polypeptides or binding agents of the present disclosure may comprise those having a modified IgG1, IgG2, IgG3, or IgG4 constant region or a portion thereof. In an embodiment, the polypeptides or binding agents comprise an IgG1 constant region (modified or unmodified). In an embodiment, the polypeptides or binding agents comprise an IgG2 constant region (modified or unmodified). In an embodiment, the polypeptides or binding agents comprise an IgG3 constant region (modified or unmodified). In an embodiment, the polypeptides or binding agents comprise an IgG4 constant region (modified or unmodified).

Modifications encompassed by the present disclosure include those which may comprise an insertion, a deletion or an amino acid substitution (conservative or non-conservative). These modifications may have at least one amino acid residue in its amino acid sequence removed and a different residue inserted in its place. It should be understood that variation may occur in multiple regions of the polypeptides or binding agents, as long as the desired binding or biological activity is maintained.

It is known in the art that modifications and variants may be generated by substitutional mutagenesis and retain the biological activity (i.e., functional equivalence) of the polypeptides of the present disclosure. These modifications or variants have at least one amino acid residue in the amino acid sequence removed and a different residue inserted in its place, e.g., one or more conservative amino acid substitution. In general, a conservative amino acid substitution is the substitution of an amino acid residue for another amino acid residue with similar chemical properties (e.g., size, charge, or polarity).

Generally, the degree of similarity and identity between variant polypeptide chains is determined herein using the Blast2 sequence program (Tatusova, T.A. and Madden, T.L., 1999) using default settings, i.e., blastp program, BLOSUM62 matrix (open gap 11 and extension gap penalty 1; gapx dropoff 50, expect 10.0, word size 3) and activated filters.

However, the level of identity may also be determined over the entire length of a given sequence. Percent identity will therefore be indicative of amino acids which are identical in comparison with the original peptide and which may occupy the same or similar position. Percent similarity will be indicative of amino acids which are identical and those which are replaced with conservative amino acid substitution in comparison with the original peptide at the same or similar position.

In some embodiments, modifications of the polypeptides or binding agents of the present disclosure therefore comprise amino acid sequences which have at least 85%, at least 90%, at least 91%, at least 92%, at least 93%, at least 94%, at least 95%, at least 96%, at least 97%, at least 98%, or at least 99% sequence identity with an original sequence or a portion of an original sequence.

In some embodiments, substitutions are conservative substitutions. However, any substitution (including non-conservative substitution) is envisaged as long as the polypeptides or binding agents retain their capability to bind and/or inhibit the desired TGFβ superfamily ligands, without substantially binding or inhibiting BMP-9 and/or BMP-10.

Generally, the nucleic acid sequence homology, similarity, or identity between the nucleotide sequences encoding polypeptides or binding agents of the disclosure and the nucleotide sequences depicted herein are at least 60%, and more typically with preferably increasing homologies or identities of at least 65%, 70%, 75%, 80%, 81%, 82%, 83%, 84%, 85%, 86%, 87%, 88%, 89%, 90%, 91%, 92%, 93%, 94%, 95%, 96%, 97%, 98%, or 99%, and almost 100%.

### Nucleic acids and Production of polypeptides and binding agents

The disclosure further provides a polynucleotide encoding an ActRIIB-ECD polypeptide provided herein. In some embodiments, the polynucleotide comprises a nucleic acid sequence that is at least 80%, 85%, 90%, 95%, 96%, 97%, 98%, or 99% identical to one of SEQ ID NOs: 293-296 or 298-375. In some embodiments, the polynucleotide comprises or consists of any one of SEQ ID NOs: 293-296 or 298-375. In some embodiments, the polynucleotide encodes an amino acid sequence comprising 95%, 96%, 97%, 98%, or 99% identity to any one of SEQ ID NOs: 174-251. In some embodiments, the polynucleotide encodes an amino acid sequence comprising or consisting of any one of SEQ ID NOs: 174-251.

A polynucleotide is a biopolymer composed of nucleotide monomers covalently bonded in a chain. DNA (such as cDNA) and RNA (such as mRNA) are examples of polynucleotides with distinct biological function. Nucleotides are organic molecules that serve as the monomers or subunits of nucleic acid molecules like DNA or RNA. The nucleic acid molecule or polynucleotide can be double stranded or single stranded, linear or circular. In some embodiments, the nucleic acid molecule or polynucleotide is comprised in a vector. In some embodiments, the vector is comprised in a host cell. The host cell is, e.g. after transformation or transfection with the vector or the polynucleotide of the disclosure, capable of expressing the polypeptide or binding agent. For that purpose the polynucleotide or nucleic acid molecule is usually operatively linked with control sequences.

Furthermore, the disclosure provides a vector comprising a polynucleotide/nucleic acid molecule coding for a polypeptide or binding agent provided herein.

A vector is a nucleic acid molecule used as a vehicle to transfer (foreign) genetic material into a cell. The term "vector" encompasses--but is not restricted to--plasmids, viruses, cosmids and artificial chromosomes. In general, engineered vectors comprise an origin of replication, a multicloning site and a selectable marker. The vector itself is generally a nucleotide sequence, commonly a DNA sequence, that comprises an insert (transgene) and a larger sequence that serves as the "backbone" of the vector. Modern vectors may encompass additional features besides the transgene insert and a backbone: promoter, genetic marker, antibiotic resistance, reporter gene, targeting sequence, protein purification tag. Vectors called expression vectors (expression constructs) specifically are for the expression of the transgene in the target cell, and generally have control sequences.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

A nucleic acid is "operably linked" when it is placed into a functional relationship with another nucleic acid sequence. For example, DNA for a presequence or secretory leader (signal sequence) is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

"Transfection" is the process of deliberately introducing nucleic acid molecules or polynucleotides (including vectors) into target cells. The term is mostly used for non-viral methods in eukaryotic cells. Transduction is often used to describe virus-mediated transfer of nucleic acid molecules or polynucleotides. Transfection of animal cells typically involves opening transient pores or "holes" in the cell membrane, to allow the uptake of material. Transfection can be carried out using calcium phosphate, by electroporation, by cell squeezing or by mixing a cationic lipid with the material to produce liposomes, which fuse with the cell membrane and deposit their cargo inside.

The term "transformation" is used to describe non-viral transfer of nucleic acid molecules or polynucleotides (including vectors) into bacteria, and also into non-animal eukaryotic cells, including plant cells. Transformation is hence the genetic alteration of a bacterial or non-animal eukaryotic cell resulting from the direct uptake through the cell membrane(s) from its surroundings and subsequent incorporation of exogenous genetic material (nucleic acid molecules). Transformation can be effected by artificial means. For transformation to happen, cells or bacteria must be in a state of competence, which might occur as a time-limited response to environmental conditions such as starvation and cell density.

Moreover, the disclosure provides a host cell transformed or transfected with the polynucleotide/nucleic acid molecule or with the vector of the technology.

As used herein, the terms "host cell" and "recipient cell" are intended to include any individual cell or cell culture that can be or has/have been recipients of vectors, exogenous nucleic acid molecules, and polynucleotides encoding the polypeptide or binding agent of the present disclosure; and/or recipients of the polypeptide or binding agent itself. The introduction of the respective material into the cell is carried out by way of transformation, transfection and the like. The term "host cell" is also intended to include progeny or potential progeny of a single cell. Because certain modifications may occur in succeeding generations due to either natural, accidental, or deliberate mutation or due to environmental influences, such progeny may not, in fact, be completely identical (in morphology or in genomic or total DNA complement) to the parent cell but is still included within the scope of the term as used herein. Suitable host cells include prokaryotic or eukaryotic cells, and also include but are not limited to bacteria, yeast cells, fungi cells, plant cells, and animal cells such as insect cells and mammalian cells, e.g., murine, rat, macaque or human.

The polypeptide or binding agent of the disclosure can be produced in bacteria. After expression, the polypeptide or binding agent is isolated from the E. coli cell paste in a soluble fraction and can be purified through, e.g., affinity chromatography and/or size exclusion. Final purification can be carried out similar to the process for purifying protein expressed e.g., in CHO cells.

In addition to prokaryotes, eukaryotic microbes such as filamentous fungi or yeast are suitable cloning or expression hosts for the polypeptide or binding agent. Saccharomyces cerevisiae, or common baker's yeast, is the most commonly used among lower eukaryotic host microorganisms. However, a number of other genera, species, and strains are commonly available and useful herein, such as Schizosaccharomyces pombe, Kluyveromyces hosts such as K. lactis, K. fragilis (ATCC 12424), K. bulgaricus (ATCC 16045), K. wickeramii (ATCC 24178), K. waltii (ATCC 56500), K. drosophilarum (ATCC 36906), K. thermotolerans, and K. marxianus; yarrowia (EP 402 226); Pichia pastoris (EP 183 070); Candida; Trichoderma reesia (EP 244 234); Neurospora crassa; Schwanniomyces such as Schwanniomyces occidentalis; and filamentous fungi such as Neurospora, Penicillium, Tolypocladium, and Aspergillus hosts such as A. nidulans and A. niger.

Suitable host cells for the expression of glycosylated polypeptide or binding agent of the disclosure are derived from multicellular organisms. Examples of invertebrate cells include plant and insect cells. Numerous baculoviral strains and variants and corresponding permissive insect host cells from hosts such as Spodoptera frugiperda (caterpillar), Aedes aegypti (mosquito), Aedes albopictus (mosquito), Drosophila melanogaster (fruit fly), and Bombyx mori have been identified. A variety of viral strains for transfection are publicly available, e.g., the L-1 variant of Autographa californica NPV and the Bm-5 strain of Bombyx mori NPV, and such viruses may be used as the virus herein according to the present disclosure, particularly for transfection of Spodoptera frugiperda cells.

Plant cell cultures of cotton, corn, potato, soybean, petunia, tomato, Arabidopsis and tobacco can also be used as hosts. Cloning and expression vectors useful in the production of proteins in plant cell culture are known to those of skill in the art. See, e.g., Hiatt et al., Nature (1989) 342: 76-78, Owen et al. (1992) Bio/Technology 10: 790-794, Artsaenko et al. (1995) The Plant J 8: 745-750, and Fecker et al. (1996) Plant Mol Biol 32: 979-986.

However, interest has been greatest in vertebrate cells, and propagation of vertebrate cells in culture (tissue culture) has become a routine procedure. Examples of useful mammalian host cell lines are monkey kidney CV1 line transformed by SV40 (COS-7, ATCC CRL 1651); human embryonic kidney line (293 or 293 cells subcloned for growth in suspension culture, Graham et al., 1977); baby hamster kidney cells (BHK, ATCC CCL 10); Chinese hamster ovary cells/-DHFR (CHO, Urlaub et al., 1980); mouse sertoli cells (TM4, Mather, 1980); monkey kidney cells (CVI ATCC CCL 70); African green monkey kidney cells (VERO-76, ATCC CRL1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2,1413 8065); mouse mammary tumor (MMT 060562, ATCC CCL5 1); TRI cells (Mather et al., Annals N. Y Acad. Sci. (1982) 383: 44-68); MRC 5 cells; FS4 cells; and a human hepatoma line (Hep G2).

In a further embodiment the disclosure provides a process for the production of a polypeptide or binding agent, said process comprising culturing a host cell under conditions allowing the expression of the polypeptide or binding agent and recovering the produced polypeptide or binding agent from the culture.

As used herein, the term "culturing" refers to the in vitro maintenance, differentiation, growth, proliferation and/or propagation of cells under suitable conditions in a medium. The term "expression" includes any step involved in the production of a polypeptide or binding agent of the disclosure including, but not limited to, transcription, post-transcriptional modification, translation, post-translational modification, and secretion.

When using recombinant techniques, the polypeptide or binding agent can be produced intracellularly, in the periplasmic space, or directly secreted into the medium. If the polypeptide or binding agent is produced intracellularly, as a first step, the particulate debris, either host cells or lysed fragments, are removed, for example, by centrifugation or ultrafiltration. Carter et al., Bio/Technology 10: 163-167 (1992) describe a procedure for isolating antibodies which are secreted to the periplasmic space of E. coli. Briefly, cell paste is thawed in the presence of sodium acetate (pH 3.5), EDTA, and phenylmethylsulfonylfluoride (PMSF) over about 30 min. Cell debris can be removed by centrifugation. Where the antibody is secreted into the medium, supernatants from such expression systems are generally first concentrated using a commercially available protein concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants.

The polypeptide or binding agent of the disclosure prepared from the host cells can be recovered or purified using, for example, hydroxylapatite chromatography, gel electrophoresis, dialysis, and affinity chromatography. Other techniques for protein purification such as fractionation on an ion-exchange column, ethanol precipitation, Reverse Phase HPLC, chromatography on silica, chromatography on heparin SEPHAROSE^{™}, chromatography on an anion or cation exchange resin (such as a polyaspartic acid column), chromato-focusing, SDS-PAGE, and ammonium sulfate precipitation are also available depending on the antibody to be recovered. Where the polypeptide or binding agent of the disclosure comprises a CH3 domain, the Bakerbond ABX resin (J. T. Baker, Phillipsburg, N.J.) may be useful for purification.

Affinity chromatography is a common purification technique. The matrix to which the affinity ligand is attached is most often agarose, but other matrices are available. Mechanically stable matrices such as controlled pore glass or poly (styrenedivinyl) benzene allow for faster flow rates and shorter processing times than can be achieved with agarose.

The polypeptide or binding agent disclosed herein may be made by a variety of methods familiar to those skilled in the art, including by recombinant DNA methods.

In order to express the polypeptides or binding agents, nucleotide sequences able to encode the polypeptide chain described herein may be inserted into an expression vector, i.e., a vector that contains the elements for transcriptional and translational control of the inserted coding sequence in a particular host. These elements may include regulatory sequences, such as enhancers, constitutive and inducible promoters, and 5' and 3' un-translated regions. Methods that are well known to those skilled in the art may be used to construct such expression vectors. These methods include in vitro recombinant DNA techniques, synthetic techniques, in vivo genetic recombination and the like.

A variety of expression vector and host cell systems known to those of skill in the art may be used to express the polypeptide chains described herein. These include, but are not limited to, microorganisms such as bacteria transformed with recombinant bacteriophage, plasmid, or cosmid DNA expression vectors; yeast transformed with yeast expression vectors; insect cell systems infected with baculovirus vectors; plant cell systems transformed with viral or bacterial expression vectors; and animal cell systems. For long-term production of recombinant proteins in mammalian systems, stable expression in mammalian cell lines may be used. For example, nucleotide sequences able to encode any one of the polypeptide chains described herein may be transformed into cell lines using expression vectors that may contain viral origins of replication and/or endogenous expression elements and a selectable or visible marker gene on the same or on a separate vector. The present disclosure is not to be limited by the vector or host cell employed. In certain embodiments disclosed herein, nucleic acids able to encode polypeptide chains described herein may be ligated into expression vectors. In the event that the binding agent is composed of distinct polypeptide chains (i.e., the first polypeptide and the second polypeptide are not identical), each of such polypeptide chain may be ligated into separate vectors or into the same vector. In accordance with the present disclosure, the polypeptide chains of the binding agent may be encoded by a single vector or by separate vectors (e.g., a vector set). Cells are transformed with the desired vector or vector sets.

Alternatively, the polypeptide chains may be expressed from an in vitro transcription system or a coupled in vitro transcription/translation system respectively or any such cell-free system.

Host cells comprising nucleotide sequences may be cultured under conditions for the transcription of the corresponding RNA (mRNA, etc.) and/or the expression and secretion of the polypeptide(s) from cell culture. In an exemplary embodiment, expression vectors containing nucleotide sequences able to encode the polypeptide chains described herein may be designed to contain signal sequences that direct secretion of the polypeptide through a prokaryotic or eukaryotic cell membrane.

Due to the inherent degeneracy of the genetic code, DNA sequences that encode the same, substantially the same or a functionally equivalent amino acid sequence may be produced and used. The nucleotide sequences of the present disclosure may be engineered using methods generally known in the art in order to alter the nucleotide sequences for a variety of purposes including, but not limited to, modification of the cloning, processing, and/or expression of the gene product. DNA shuffling by random fragmentation and PCR reassembly of gene fragments and synthetic oligonucleotides may be used to engineer the nucleotide sequences. For example, oligonucleotide-mediated site-directed mutagenesis may be used to introduce mutations that create new restriction sites, alter glycosylation patterns, change codon preference, produce splice variants, and so forth. Codon-optimized nucleic acids encoding the polypeptide chains described herein are encompassed by the present disclosure.

In addition, a host cell strain may be chosen for its ability to modulate expression of the inserted sequences or to process the expressed polypeptide in the desired fashion. Different host cells that have specific cellular machinery and characteristic mechanisms for post-translational activities (e.g., CHO, HeLa, MDCK, HEK293, and W138) are available commercially and from the American Type Culture Collection (ATCC) and may be chosen to ensure the correct modification and processing of the expressed polypeptide.

Those of skill in the art will also readily recognize that the nucleic acid and polypeptide sequences may be synthesized, in whole or in part, using chemical or enzymatic methods well known in the art. For example, peptide synthesis may be performed using various solid-phase techniques and machines such as the ABI 431A Peptide synthesizer (PE Biosystems) may be used to automate synthesis. If desired, the amino acid sequence may be altered during synthesis and/or combined with sequences from other proteins to produce a variant protein.

### Pharmaceutical compositions

Pharmaceutical compositions comprising the polypeptides or TGFβ superfamily ligand binding agents disclosed herein are provided by the present disclosure. The pharmaceutical composition generally comprises the polypeptide or binding agent disclosed herein and a pharmaceutically acceptable carrier.

The preparation of pharmaceutical compositions can be carried out as known in the art (see, for example, Remington: The Science and Practice of Pharmacy, 20th Edition, 2000). For example, a therapeutic compound and/or composition, together with one or more solid or liquid pharmaceutical carrier substances and/or additives (or auxiliary substances) and, if desired, in combination with other pharmaceutically active compounds having therapeutic or prophylactic action, are brought into a suitable administration form or dosage form which can then be used as a pharmaceutical in human or veterinary medicine. Pharmaceutical preparations can also contain additives, of which many are known in the art, for example fillers, disintegrants, binders, lubricants, wetting agents, stabilizers, emulsifiers, dispersants, preservatives, sweeteners, colorants, flavorings, aromatizers, thickeners, diluents, buffer substances, solvents, solubilizers, agents for achieving a depot effect, salts for altering the osmotic pressure, coating agents or antioxidants.

The term "pharmaceutical composition" means a composition comprising a polypeptide or binding agent as described herein and at least one component comprising pharmaceutically acceptable carriers, diluents, adjuvants, excipients, or vehicles, such as preserving agents, fillers, disintegrating agents, wetting agents, emulsifying agents, suspending agents, sweetening agents, flavoring agents, perfuming agents, antibacterial agents, antifungal agents, lubricating agents and dispensing agents, depending on the nature of the mode of administration and dosage forms.

The term "pharmaceutically acceptable carrier" is used to mean any carrier, diluent, adjuvant, excipient, or vehicle, as described herein or as known in the art. Examples of suspending agents include ethoxylated isostearyl alcohols, polyoxyethylene sorbitol and sorbitan esters, microcrystalline cellulose, aluminum metahydroxide, bentonite, agar-agar and tragacanth, or mixtures of these substances. Prevention of the action of microorganisms can be ensured by various antibacterial and antifungal agents, for example, parabens, chlorobutanol, phenol, sorbic acid, and the like. It may also be desirable to include isotonic agents, for example sugars, sodium chloride, and the like. Prolonged absorption of the injectable pharmaceutical form can be brought about by the use of agents delaying absorption, for example, aluminum monosterate and gelatin. Non-limiting examples of suitable carriers, diluents, solvents, or vehicles include water, salt solutions, phosphate buffered saline (PBS), gelatins, oils, alcohols, polyols, suitable mixtures thereof, vegetable oils (such as olive oil), and injectable organic esters such as ethyl oleate. Non-limiting examples of excipients include lactose, milk sugar, sodium citrate, calcium carbonate, and dicalcium phosphate. Non-limiting examples of disintegrating agents include starch, alginic acids, and certain complex silicates. Non-limiting examples of lubricants include magnesium stearate, sodium lauryl sulphate, talc, as well as high molecular weight polyethylene glycols.

The term "pharmaceutically acceptable" means it is, within the scope of sound medical judgment, suitable for use in contact with the cells of a subject, e.g., humans and animals, without undue toxicity, irritation, allergic response, and the like, and are commensurate with a reasonable benefit/risk ratio.

As used herein "pharmaceutically acceptable carrier" or "pharmaceutical carrier" are known in the art and include, but are not limited to, 0.01-0.1 M or 0.05 M phosphate buffer or 0.8 % saline. Additionally, such pharmaceutically acceptable carriers may be aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's or fixed oils. Intravenous vehicles include fluid and nutrient replenishers, electrolyte replenishers such as those based on Ringer's dextrose, and the like. Preservatives and other additives may also be present, such as, for example, antimicrobials, antioxidants, collating agents, inert gases and the like.

A pharmaceutically acceptable carrier may include any and all solvents, dispersion media, coatings, antibacterial and antifungal agents, isotonic and absorption delaying agents, and the like that are physiologically compatible. In one embodiment, the carrier is suitable for parenteral administration. The carrier may be suitable for intravenous, intraperitoneal, subcutaneous or intramuscular administration. Alternatively, the carrier may be suitable for sublingual or oral administration. In other embodiments, the carrier is suitable for topical administration or for administration via inhalation. Pharmaceutically acceptable carriers include sterile aqueous solutions or dispersions and sterile powders for the extemporaneous preparation of sterile injectable solutions or dispersion. The use of such media and agents for pharmaceutically active substances is well known in the art. Except insofar as any conventional media or agent is incompatible with the active compound, use thereof in the pharmaceutical compositions provided herein is contemplated. Supplementary active compounds can also be incorporated into the compositions. For example, a pharmaceutical composition provided herein may further comprise at least one additional therapeutic agent, as discussed further below.

The pharmaceutical compositions described herein may be administered by any number of routes including, but not limited to, oral, intravenous, intramuscular, intra-arterial, intramedullary, intrathecal, intraventricular, transdermal, subcutaneous, intraperitoneal, intranasal, enteral, topical, sublingual, or rectal means.

In some embodiments, a pharmaceutical composition provided herein can be administered orally, for example in the form of pills, tablets, lacquered tablets, sugar-coated tablets, granules, hard and soft gelatin capsules, aqueous, alcoholic or oily solutions, syrups, emulsions or suspensions, or rectally, for example in the form of suppositories.

In other embodiments, a pharmaceutical composition provided herein can be administered parenterally, for example subcutaneously, intramuscularly or intravenously in the form of solutions for injection or infusion. Other suitable administration forms are, for example, percutaneous or topical administration, for example in the form of ointments, creams, tinctures, sprays or transdermal therapeutic systems, or the inhalative administration in the form of nasal sprays or aerosol mixtures, or, for example, microcapsules, implants or wafers.

Pharmaceutical compositions typically must be sterile and stable under the conditions of manufacture and storage. A composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as mannitol, sorbitol, or sodium chloride in the composition. Prolonged absorption of injectable compositions can be brought about by including in the composition an agent which delays absorption, for example, monostearate salts and gelatin. Moreover, a compound can be administered in a time release formulation, for example in a composition which includes a slow release polymer. The compound can be prepared with carriers that will protect against rapid release, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, polylactic acid and polylactic, polyglycolic copolymers (PLG).

Many methods for the preparation of such formulations are generally known to those skilled in the art. Sterile injectable solutions can be prepared by incorporating an active compound, such as a polypeptide or binding agent provided herein, in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, common methods of preparation are vacuum drying and freeze-drying which yields a powder of the active ingredient plus any additional desired ingredient from a previously sterile-filtered solution thereof. Compounds may also be formulated with one or more additional compounds that enhance their solubility.

In some embodiments, a pharmaceutical composition of the disclosure comprises a sterile injectable solution. In some embodiments, the sterile injectable solution comprises a sterile powder which is reconstituted with an acceptable solution, e.g., water.

It is often advantageous to formulate compositions (such as parenteral compositions) in dosage unit form for ease of administration and uniformity of dosage. The term "unit dosage form" refers to a physically discrete unit suitable as unitary dosages for human subjects and other animals, each unit containing a predetermined quantity of active material calculated to produce the desired therapeutic effect, in association with a suitable pharmaceutical carrier. The specification for the dosage unit forms of the invention may vary and are dictated by and directly dependent on (a) the unique characteristics of the therapeutic compound and the particular therapeutic effect to be achieved, and (b) the limitations inherent in the art of compounding such a therapeutic compound for the prevention or treatment of a TGFβ-superfamily associated disease or condition. Dosages are discussed further below.

The dosage regimen will be determined by the attending physician and clinical factors. As is well known in the medical arts, dosages for any one patient depend upon many factors, including the patient's size, body surface area, age, the particular compound to be administered, sex, time and route of administration, general health, and other drugs being administered concurrently.

For any compound, the therapeutically effective dose may be estimated initially either in cell culture assays or in animal models such as mice, rats, rabbits, dogs, or pigs. An animal model may also be used to determine the concentration range and route of administration. Such information may then be used to determine useful doses and routes for administration in humans. These techniques are well known to one skilled in the art and a therapeutically effective dose refers to that amount of active ingredient that ameliorates the symptoms or condition. Therapeutic efficacy and toxicity may be determined by standard pharmaceutical procedures in cell cultures or with experimental animals, such as by calculating and contrasting the ED50 (the dose therapeutically effective in 50% of the population) and LD50 (the dose lethal to 50% of the population). Any of the pharmaceutical compositions described herein may be applied to any subject in need of therapy, including, but not limited to, mammals such as dogs, cats, cows, horses, rabbits, monkeys, and especially humans.

The terms "effective dose", "effective dosage" and "effective amount" are used interchangeably to refer to an amount sufficient to achieve or at least partially achieve the desired effect. The term "therapeutically effective" dose or amount is defined as an amount sufficient to treat or to ameliorate or at least partially arrest the disease and its complications in a patient already suffering from the disease. A therapeutically effective amount of a polypeptide or binding agent or pharmaceutical composition of the disclosure generally results in a decrease in severity of disease symptoms, an increase in frequency or duration of disease symptom-free periods or a prevention of impairment or disability due to the disease affliction. In some embodiments, a therapeutically effective amount is an amount or dose of a polypeptide or binding agent or composition that prevents or treats a TGFβ superfamily-associated disease or disorder in a subject, as described herein. In some embodiments, an effective amount is an amount or dose of a polypeptide or binding agent or composition that inhibits one or more of activin A, activin B, GDF-8, and GDF-11 in a subject, as described herein.

Amounts or doses effective for this use will depend on the disorder to be treated (the indication), the delivered polypeptide or binding agent, the therapeutic context and objectives, the severity of the disease, prior therapy, the patient's clinical history and response to the therapeutic agent, the route of administration, the size (body weight, body surface or organ size) and/or condition (the age and general health) of the patient, and the general state of the patient's own immune system. The proper dose can be adjusted according to the judgment of the attending physician such that it can be administered to the patient once or over a series of administrations, and in order to obtain the optimal therapeutic effect.

A typical dosage may range from about 0.1 µg/kg to up to about 30 mg/kg or more, depending on the factors mentioned above. In specific embodiments, the dosage may range from 1.0 µg/kg up to about 20 mg/kg, optionally from 10 µg/kg up to about 10 mg/kg or from 100 µg/kg up to about 5 mg/kg. Dosages are discussed further below.

### Methods of Use

Formulations described herein are useful as pharmaceutical compositions in the treatment, amelioration and/or prevention of a disease/disorder as described herein in a subject in need thereof. The term "treatment" refers to both therapeutic treatment and prophylactic or preventative measures. Treatment includes the application or administration of the formulation to the body, an isolated tissue, or cell from a patient who has a disease/disorder, a symptom of a disease/disorder, or a predisposition toward a disease/disorder, with the purpose to cure, heal, alleviate, relieve, alter, remedy, ameliorate, improve, or affect the disease, the symptom of the disease, or the predisposition toward the disease.

Any reference herein to methods of treatment can also be interpreted as a reference to the ActRIIB-ECD polypeptide described herein, or TGFβ superfamily ligand binding agent comprising the same, for use in such a method of treatment.

The term "amelioration" as used herein refers to any improvement of the disease state of a patient having a disease/disorder as specified herein below, by the administration of a polypeptide or binding agent or pharmaceutical composition according to the disclosure to a subject. For example, the term "amelioration" means to decrease, suppress, attenuate, diminish, arrest, or stabilize the development or progression of a disease. Such an improvement may also be seen as a slowing or stopping of the progression of disease/disorder of the patient. The term "prevention" as used herein means the avoidance of the occurrence or re-occurrence of a patient having a disease/disorder as specified herein below, by the administration of polypeptide or binding agent or pharmaceutical composition according to the disclosure to a subject in need thereof.

The term "disease or condition" (or "disease/condition") refers to any pathological medical condition that would benefit from treatment with the polypeptide or binding agent or the pharmaceutic composition described herein. This includes chronic and acute diseases or conditions including those pathological conditions that predispose the mammal to the disease/disorder in question. In some embodiments, the polypeptide or binding agent of the disclosure and compositions thereof are useful in the prevention, treatment or amelioration of a TGFβ superfamily associated disease or condition. As such, there are provided methods for prevention or treatment of a TGFβ superfamily associated disease or condition in a subject, the methods comprising administering a therapeutically effective amount of the polypeptide or binding agent or pharmaceutical composition described herein. Polypeptides and binding agents are generally administered in the form of a pharmaceutical composition. A subject may be in need of such treatment, i.e., having, suspected of having, or at risk of having a disease or condition associated with TGFβ superfamily signaling or mediated by one or more member of the TGFβ superfamily, as described herein.

The term "inhibition" or "inhibiting" is used herein to refer generally to reducing, slowing, restricting, delaying, suppressing, blocking, neutralizing, hindering, or preventing a process, such as without limitation reducing or slowing progression, growth, or spread of a disease or condition.

In some embodiments of the present disclosure, "treating" refers to neutralizing the biologic activity of excess activin A, activin B, GDF-8, and/or GDF-11. It may be determined by suitable clinical variables of improvement; by pathologic evaluation of the effects on a disease or condition; by a direct inhibition of activin A, activin B, GDF-8, and/or GDF-11 signaling (without substantially inhibiting BMP-9 and/or BMP-10); or by another measure suitable for the disease or condition being treated.

In some embodiments, improvement is determined by comparing clinical variables to measurements made before treatment, or alternatively to typical values measured in healthy adults. In some embodiments, treatment or prevention are within the context of the present disclosure if there is a measurable difference between the performances of subjects treated using the polypeptides, binding agents, compositions and methods provided herein as compared to members of a placebo group, historical control, or between subsequent tests given to the same subject.

The term "subject" includes living organisms, in particular mammals. Examples of subjects include mammals, e.g., humans, monkeys, cows, rabbits, sheep, goats, pigs, dogs, cats, rats, mice, and transgenic species thereof. The term "subject" generally includes animals susceptible to states characterized by TGFβ superfamily associated diseases or conditions such as pulmonary hypertension, fibrosis, muscle weakness and atrophy, metabolic disorders and/or cardiometabolic disease, bone damage, or low red blood cell levels, e.g., mammals, e.g., primates, e.g., humans. The animal can also be an animal model for a disorder, e.g., a mouse model, a xenograft recipient, and the like. In certain embodiments, the subject is a human.

There are no particular limitations on the dose of the polypeptide or binding agent for use in the compositions and methods of the disclosure. Exemplary doses include milligram or microgram amounts of the polypeptide or binding agent per kilogram of subject or sample weight (e.g., about 50 micrograms per kilogram to about 500 milligrams per kilogram, about 1 milligram per kilogram to about 100 milligrams per kilogram, about 1 milligram per kilogram to about 50 milligram per kilogram, about 1 milligram per kilogram to about 10 milligrams per kilogram, or about 3 milligrams per kilogram to about 5 milligrams per kilogram). Additional exemplary doses include doses of about 5 to about 500 mg, about 25 to about 300 mg, about 25 to about 200 mg, about 50 to about 150 mg, or about 50, about 100, about 150 mg, about 200 mg or about 250 mg, and, for example, daily or twice daily, or lower or higher amounts.

In some embodiments, the dose range for adult humans is generally from 0.005 mg to 10 g/day. Polypeptide or binding agent and compositions thereof may be provided in Unit dosage form, e.g., in a unit which is effective at such dosage or as a multiple of the same, for instance, units containing 5 mg to 500 mg, usually around 10 mg to 200 mg. A dosage unit can include from, for example, 1 to 30 mg, 1 to 40 mg, 1 to 100 mg, 1 to 300 mg, 1 to 500 mg, 2 to 500 mg, 3 to 100 mg, 5 to 20 mg, 5 to 100 mg (e.g. 1 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 150 mg, 200 mg, 250 mg, 300 mg, 350 mg, 400 mg, 450 mg, or 500 mg) of a polypeptide or binding agent or composition described herein.

It should be understood that the effective amount of a polypeptide or binding agent for therapeutic treatment of a disease or condition varies depending upon the manner of administration, the age, body weight, and general health of the subject. Ultimately, the attending physician or veterinarian decides the appropriate amount and dosage regimen. It should be understood that the dosage or amount of a polypeptide or binding agent used, alone or in combination with one or more active compounds to be administered, depends on the individual case and is, as is customary, to be adapted to the individual circumstances to achieve an optimum effect. Dosing and administration regimens are within the purview of the skilled artisan, and appropriate doses depend upon a number of factors within the knowledge of the ordinarily skilled physician, veterinarian, or researcher (e.g., see Wells et al. eds., Pharmacotherapy Handbook, 2nd Edition, Appleton and Lange, Stamford, Conn. (2000); PDR Pharmacopoeia, Tarascon Pocket Pharmacopoeia 2000, Deluxe Edition, Tarascon Publishing, Loma Linda, Calif. (2000)). For example, dosing and administration regimens depend on the nature and the severity of the disease or condition to be treated, and also on the sex, age, weight and individual responsiveness of the human or animal to be treated, on the efficacy and duration of action of the compounds used, on whether the therapy is acute or chronic or prophylactic, and/or on whether other active compounds are administered in addition to the therapeutic molecule(s).

Administration of polypeptides or binding agents or compositions provided herein can be carried out using known procedures, at dosages and for periods of time effective to achieve the desired purpose. Dosage regimens can be adjusted to provide the optimum therapeutic response. For example, several divided doses may be administered daily, weekly, biweekly, or monthly, or the dose may be proportionally reduced as indicated by the exigencies of the therapeutic situation. In some embodiments, a polypeptide or binding agent or composition is administered at a therapeutically effective dosage sufficient to prevent or treat a TGFβ superfamily associated disease or condition, e.g., pulmonary hypertension, fibrosis, muscle weakness and atrophy, metabolic disorders and/or cardiometabolic disease, bone damage, and/or low red blood cell levels, in a subject.

In some embodiments, in accordance with the methods of the present disclosure, one or more symptom of development or progression of a TGFβ superfamily associated disease or condition is reduced by at least 5%, e.g., at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or 100% in a subject.

In some embodiments of methods provided herein, the polypeptide or binding agent is administered in combination with one or more additional therapy or therapeutic agent. The additional therapy or therapeutic agent can be administered before, after or simultaneously with the administration of the polypeptide or binding agent or composition described herein. In some embodiments, the additional therapy or therapeutic agent is formulated together with the polypeptide or binding agent in the same composition. In other embodiments, the additional therapy or therapeutic agent is administered separately. Examples of additional therapies and therapeutic agents include, without limitation, an anti-fibrotic agent; an anticancer agent; an anti-inflammatory agent; an anti-obesity agent; an anti-diabetes agent; another TGFβ superfamily ligand binding agent or inhibitor, such as an antibody, antibody fragment, antigen-binding fragment, soluble TGFβ superfamily ligand trap, and the like; another agent that binds or inhibits one or more additional target, and the like.

Alternatively, in some embodiments, the polypeptide or binding agent may be conjugated with a detectable moiety or a diagnostic moiety that is useful for tracking the polypeptide or binding agent or cells or tissues expressing TGFβ superfamily ligands and/or another target. In some such embodiments, there are provided methods of diagnosis of a TGFβ superfamily associated disease or condition comprising administering to a subject a polypeptide or binding agent of the present disclosure conjugated with a detectable moiety or a diagnostic moiety, and detecting the polypeptide or binding agent such that a disease or disorder associated with TGFβ superfamily signaling (e.g., overexpression of activin A, activin B, GDF-8, or GDF-11) is diagnosed.

### TGFβ superfamily associated diseases and conditions

The polypeptide or binding agent described herein, and pharmaceutical compositions thereof, are useful for prevention or treatment of TGFβ superfamily associated diseases and conditions. As such, there are provided methods for prevention or treatment of a disease or condition associated with TGFβ-superfamily ligand signaling in a subject, the methods comprising administering a therapeutically effective amount of the polypeptide, binding agent, or pharmaceutical composition described herein. Polypeptides and binding agents are generally administered in the form of a pharmaceutical composition. A subject may be in need of such treatment, i.e., having, suspected of having, or at risk of developing a disease or condition associated with TGFβ superfamily signaling, as described herein.

As used herein, the term "TGFβ-superfamily ligand signaling associated disease or condition" refers to diseases and conditions that may be ameliorated through inhibition of one or more TGFβ superfamily ligand, particularly activin A, activin B, GDF-8, and/or GDF-11 activity. TGFβ-superfamily associated diseases or conditions include, without limitation, diseases and conditions associated with over-expression or over-activation of TGFβ superfamily ligands, particularly activin A, activin B, GDF-8, and/or GDF-11. In some embodiments, a TGFβ-superfamily associated disease or condition is mediated by activin A and/or activin B. In one embodiment, the disease or condition to be treated is mediated by activin A. In one embodiment, the disease or condition to be treated is mediated by activin B. In one embodiment, the disease or condition to be treated is mediated by GDF-8. In one embodiment, the disease or condition to be treated is mediated by GDF-11. In another embodiment, the disease or condition to be treated is mediated by a combination of activin A, activin B, GDF-8, and GDF-11.

Examples of TGFβ-superfamily associated diseases or conditions that may be prevented or treated in accordance with the present disclosure include, without limitation: pulmonary hypertension, fibrosis, muscle weakness and atrophy, metabolic disorders and/or cardiometabolic disease, bone damage, and/or low red blood cell levels, as well as vascular diseases and vasculopathies.

In some embodiments, the subject does not suffer from a TGFβ superfamily associated disease described below. In some embodiments, an ActRIIB-ECD polypeptide described herein or TGFβ superfamily ligand binding agent comprising the same is used for improvement of body composition in a healthy subject. In some embodiments, the methods described herein decrease fat mass in a healthy subject. In some embodiments, the methods described herein increase lean muscle mass in a healthy subject.

In some embodiments, an ActRIIB-ECD polypeptide described herein or TGFβ superfamily ligand binding agent comprising the same is used for improvement in exercise tolerance in a healthy subject. In some embodiments, the methods described herein increase left ventricular end-diastolic pressure in a healthy subject. In some embodiments, the methods described herein increase (improve) exercise capacity (ability, tolerance) in a healthy subject. For example, the method may increase 6-minute walk distance in a healthy subject, optionally increasing 6-minute walk distance by at least 10 meters (e.g., at least 10, at least 20, at least 30, at least 40, at least 50, at least 60, at least 70, at least 80, at least 90, at least 100 or more meters).

### Metabolic disorders and Cardiometabolic disease

In some embodiments, a polypeptide or binding agent described herein is used for treatment or prevention of a metabolic disorder and/or a cardiometabolic disease. Non-limiting examples of metabolic disorders include diabetes (including Type 1 Diabetes and Type 2 Diabetes), pre-diabetes, diabetic complications (such as, for example, retinopathy, nephropathy or neuropathies, diabetic foot, ulcus, macroangiopathies), metabolic acidosis or ketosis, hyperglycemia, reactive hypoglycemia, hyperinsulinemia, glucose metabolism disorder, insulin resistance, metabolic syndrome, Dyslipidaemias, atherosclerosis and related diseases, obesity, pre-diabetes, hypertension, chronic heart failure, acute renal failure, edema, and hyperuricemia. Non-limiting examples of cardiometabolic diseases include heart attack, stroke, diabetes, pre-diabetes, insulin resistance, non-alcoholic fatty liver disease, chronic renal failure, heart failure and/or low ejection fraction, including heart failure with reduced ejection fraction (HFrEF) and heart failure with preserved ejection fraction (HFpEF). In some embodiments, the cardiometabolic disease is HFrEF. In some embodiments, the cardiometabolic disease is HFpEF.

Treatment of obesity or overweight condition and their related comorbidities, such as Type II Diabetes, dyslipidemia, hyperglycemia, cardiovascular disease, and other metabolic disorders, represent a substantial, unmet medical need. Obesity is a risk factor of overall mortality and is estimated to have caused 3.4 million deaths worldwide in 2010 (Lim et al 2012). Obesity, diabetes, high blood sugar and related conditions are high risk factors for serious or life-threatening complications such as heart disease, stroke, kidney problems, nerve damage, and eye disorders. In particular, cardiometabolic diseases such as stroke, heart attack, and heart and blood vessel diseases can be ameliorated by lowering blood sugar levels or achieving weight loss in patients with type 2 diabetes or obesity. Pharmacotherapeutics that can improve body composition, by changing the ratio of lean mass versus fat mass and thereby achieve weight loss or decrease of central adiposity, and improve patient glycemic status, are highly sought. There is also a need for therapies that address heart failure associated with left ventricular dysfunction, such as Heart Failure with preserved Ejection Fraction (HFpEF). HFpEF represents more than half of heart failure cases worldwide and therapies to address HFpEF and associated risk factors such as obesity are highly desired.

There is a need for pharmacotherapeutics that can improve body composition, by changing the ratio of lean mass versus fat mass and thereby achieve weight loss or decrease of central adiposity and improve patient glycemic status. It has been reported that muscle mass increases in myostatin knockout mice and in wild type mice where myostatin is sequestered by a soluble ActRIIB, and that this increase is associated with improved whole body insulin sensitivity and with resistance to diet-induced and genetic obesity (Guo et al, 2009, Akpan et al, 2009). Inhibition of ActRIIB with an antibody has been shown to reduce white adipose tissue in mice on a normal or high-fat diet, while increasing skeletal muscle mass (Fournier et al 2012).

In some embodiments, the disclosure provides for a method of treating a subject having a metabolic disorder comprising administering to a subject in need thereof an effective amount of an ActRIIB-ECD polypeptide described herein or TGFβ superfamily ligand binding agent comprising the same. For example, methods of treating Type 2 Diabetes, Metabolic Syndrome, hyperglycemia, and obesity are provided. In some embodiments therefore, there is provided a method of preventing or treating a metabolic disorder or condition such as, without limitation, Type 1 Diabetes, Type 2 Diabetes, diabetic complications (such as, for example, retinopathy, nephropathy or neuropathies, diabetic foot, ulcus, macroangiopathies), metabolic acidosis or ketosis, reactive hypoglycemia, hyperinsulinemia, glucose metabolism disorder, insulin resistance, metabolic syndrome, Dyslipidaemias, atherosclerosis and related diseases, obesity, pre-diabetes, hypertension, chronic heart failure, acute renal failure, edema, or hyperuricemia.

In some embodiments, the disclosure provides for a method of increasing lean mass in a subject having a metabolic disorder, comprising administering to a subject in need thereof an effective amount of an ActRIIB-ECD polypeptide described herein or TGFβ superfamily ligand binding agent comprising the same.

In some embodiments, the disclosure provides for a method of increasing muscle mass in a subject having a metabolic disorder, comprising administering to a subject in need thereof an effective amount of an ActRIIB-ECD polypeptide described herein or TGFβ superfamily ligand binding agent comprising the same.

In some embodiments, the disclosure provides for a method of decreasing white fat and/or increasing brown fat in a subject in need thereof, e.g., a subject having a metabolic disorder, comprising administering to a subject in need thereof an effective amount of an ActRIIB-ECD polypeptide described herein or TGFβ superfamily ligand binding agent comprising the same.

In some embodiments, the disclosure provides for a method of decreasing total body fat mass (FM) in a subject in need thereof, e.g., a subject having a metabolic disorder, comprising administering to a subject in need thereof an effective amount of an ActRIIB-ECD polypeptide described herein or TGFβ superfamily ligand binding agent comprising the same.

In some embodiments, the disclosure provides for a method of decreasing total body fat mass (FM) while at the same time increasing muscle mass in a subject in need thereof, e.g., a subject having a metabolic disorder, e.g., obesity, comprising administering to a subject in need thereof an effective amount of an ActRIIB-ECD polypeptide described herein or TGFβ superfamily ligand binding agent comprising the same.

In some embodiments, the disclosure provides for a method of improving exercise tolerance in a subject having a metabolic disorder (*e*.*g*., obesity). In some embodiments, the methods described herein increase left ventricular end-diastolic pressure in a healthy subject. In some embodiments, the methods described herein increase (improve) exercise capacity (ability, tolerance) in a subject having a metabolic disorder (*e.g*., obesity). For example, the method may increase 6-minute walk distance in a subject suffering from a metabolic or cardiometabolic disorder, optionally increasing 6-minute walk distance by at least 10 meters (e.g., at least 10, at least 20, at least 30, at least 40, at least 50, at least 60, at least 70, at least 80, at least 90, at least 100 or more meters).

In some embodiments, the disclosure provides for a method of improving insulin sensitivity in a subject in need thereof, e.g., a subject having a metabolic disorder, comprising administering to a subject in need thereof an effective amount of an ActRIIB-ECD polypeptide described herein or TGFβ superfamily ligand binding agent comprising the same.

In some embodiments, the disclosure provides for a method of increasing energy expenditure in a subject in need thereof, e.g., a subject having a metabolic disorder, comprising administering to a subject in need thereof an effective amount of an ActRIIB-ECD polypeptide described herein or TGFβ superfamily ligand binding agent comprising the same.

In some embodiments, the disclosure provides for a method of treating a subject having a cardiometabolic disease comprising administering to a subject in need thereof an effective amount of an ActRIIB-ECD polypeptide described herein or TGFβ superfamily ligand binding agent comprising the same. Non-limiting examples of cardiometabolic disease include heart attack, stroke, diabetes, insulin resistance, non-alcoholic fatty liver disease, and chronic renal failure. In some such embodiments, the disclosure provides for a method of reducing the risk of cardiovascular death in subjects with type 2 Diabetes and established cardiovascular disease. In some such embodiments, the disclosure provides for a method of reducing the risk of death and/or hospitalization in people with heart failure and/or low ejection fraction. In some such embodiments, the disclosure provides for a method of reducing the risk of death and/or hospitalization in people with heart failure with a reduced ejection fraction (HFrEF) or with heart failure with preserved ejection fraction (HFpEF). In some such embodiments, the disclosure provides for a method of reducing the risk of cardiovascular death and/or hospitalization for heart failure in adults. In some such embodiments, the disclosure provides for a method of reducing glucose levels in subjects with type 2 Diabetes. In some such embodiments, the disclosure provides for a method of treating or preventing metabolic disorders such as diabetes and/or improving glycemic control in a subject with moderate renal impairment or stage 3 chronic kidney disease.

In some embodiments, the disclosure provides for a method of improving body composition, e.g., for the treatment of central adiposity, obesity or overweight condition and related comorbidities, comprising administering to a subject in need thereof an effective amount of an ActRIIB-ECD polypeptide described herein or TGFβ superfamily ligand binding agent comprising the same.

In some embodiments, the disclosure provides for a method of treating Type II diabetes, e.g., by improving glycemic control or increasing insulin sensitivity, comprising administering to a subj ect in need thereof an effective amount of an ActRIIB-ECD polypeptide described herein or TGFβ superfamily ligand binding agent comprising the same.

In some embodiments, the disclosure provides for a method of improving body composition in a subject, wherein the improvement comprises an increase in lean mass and a decrease in fat mass, comprising administering to the subject an effective amount of a polypeptide or binding agent described herein. Body composition may be determined by, for example, body mass index (BMI), body shape index (ABSI), dual energy absorptiometry (DXA), magnetic resonance imaging (MM), and/or bioelectrical impedance techniques.

In some embodiments, the disclosure provides for a method of treating obesity or overweight disorder, e.g., by improving body composition whereby lean mass is increased and fat mass is reduced, comprising administering to a subject in need thereof an effective amount of an ActRIIB-ECD polypeptide described herein or TGFβ superfamily ligand binding agent comprising the same.

In some embodiments, the disclosure provides for a method of treating, preventing or reducing obesity or overweight condition related comorbidities, comprising administering to a subject in need thereof an effective amount of a polypeptide or binding agent described herein. "Obesity or overweight condition related comorbidities" refers to serious chronic disorders associated with obesity, including but not limited to type 2 diabetes or glucose intolerance, prediabetes, high triglycerides, physical impairment, osteoporosis, renal disease, obstructive sleep apnea, sexual hormones impairment, endocrine reproductive disorders such as polycystic ovary syndrome or male hypogonadism, osteoarthritis, gastrointestinal cancers, dyslipidaemia, hypertension, heart failure, coronary heart disease and stroke, gallstones, hypertension, and altered gonadal hormone profile. In some such embodiments, the disclosure provides a method of improving glycemic control, e.g., in a patient suffering from Type II Diabetes, e.g., by increasing insulin sensitivity.

### Pulmonary hypertension

In some embodiments, an ActRIIB-ECD polypeptide described herein or TGFβ superfamily ligand binding agent comprising the same is used for treatment or prevention of pulmonary hypertension (PH). For example, a polypeptide or binding agent described herein may be used to treat, prevent or reduce the progression rate and/or severity of a variety of conditions including, but not limited to, pulmonary vascular remodeling, pulmonary fibrosis, and right ventricular hypertrophy, or of one or more PH-associated complications, e.g., to reduce right ventricular systolic pressure in a subject in need thereof.

Pulmonary hypertension is a disease characterized by pulmonary vascular remodeling which results in high pulmonary artery pressure. An imbalance between the growth-promoting activin/growth differentiation factor pathway and the growth-inhibiting BMP pathway promotes the vascular remodeling observed in this disease. For example, elevated activin A has been observed in clinical and experimental pulmonary hypertension. Myostatin and activins are known to play a role in the regulation of skeletal muscle growth. For example, mice without myostatin show a large increase in skeletal muscle mass. Myostatin has also been implicated in promoting fibrosis. For example, mice without myostatin show a reduction in muscle fibrosis. Mice overexpressing activin A also exhibit increased fibrosis. In addition, activins are expressed abundantly in bone tissues and regulate bone formation by controlling both osteoblast and osteoclast functions. Activin has been reported to be upregulated in bone disease. Myostatin is also implicated in bone homeostasis through increasing osteogenesis and inhibiting osteoblast activity. Methods that reduce or inhibit activin and/or GDF signaling could therefore be useful in the treatment of diseases and conditions such as pulmonary hypertension (e.g., PAH, venous PH, hypoxic PH, thromboembolic PH, or miscellaneous PH), muscle atrophy or weakness, fibrosis, bone damage, or low red blood cell levels (e.g., anemia).

PH can be categorized into five major types: arterial (PAH), venous (PH secondary to left-sided heart disease), hypoxic (PH caused by lung disease), thromboembolic (PH caused by chronic arterial obstruction, e.g., blood clots), or miscellaneous (PH with unclear or multifactorial mechanisms), also known as WHO groups I-V. PAH features increased pressure in blood vessels of the lungs caused by obstruction in, or narrowing of, small blood vessels due to remodeling. This leads to increased resistance to blood flow through the lungs which forces the right side of the heart to work harder, which may lead to heart failure. PAH can be idiopathic, heritable, or may be related to drug use, infection, liver cirrhosis, congenital heart abnormalities, or connective tissue/autoimmune disorders (e.g., scleroderma or lupus). Treatments for PH include vasodilators, anticoagulants, and supplemental oxygen, but these treatments manage disease symptoms rather than targeting the biological mechanisms that cause the disease.

Pulmonary hypertension (PH) is a disease characterized by high blood pressure in lung vasculature, including pulmonary arteries, pulmonary veins, and pulmonary capillaries. In general, PH is defined as a mean pulmonary arterial (PA) pressure ≥25 mm Hg at rest or ≥30 mm Hg with exercise (Hill et al., 2009). The main PH symptom is difficulty in breathing or shortness of breath, and other symptoms include fatigue, dizziness, fainting, peripheral edema

(swelling in foot, legs or ankles), bluish lips and skin, chest pain, angina pectoris, light-headedness during exercise, non-productive cough, racing pulse and palpitations. PH can be a severe disease causing heart failure, which is one of the most common causes of death in people who have pulmonary hypertension. Postoperative pulmonary hypertension may complicate many types of surgeries or procedures, and present a challenge associated with high mortality.

PH may be grouped based on different manifestations of the disease sharing similarities in pathophysiologic mechanisms, clinical presentation, and therapeutic approaches (Simonneau et al., 2009). Clinical classification of PH was first proposed in 1973, and a recent updated clinical classification was endorsed by the World Health Organization (WHO) in 2008. According to the updated PH clinical classification, there are five main groups of PH: pulmonary arterial hypertension (PAH), characterized by a PA wedge pressure ≤15 mm Hg; PH owing to a left heart disease (also known as pulmonary venous hypertension or congestive heart failure), characterized by a PA wedge pressure >15 mm Hg; PH owing to lung diseases and/or hypoxia; chronic thromboemboli PH; and PH with unclear or multifactorial etiologies (Simonneau et al., 2009; Hill et al., 2009). PAH is further classified into idiopathic PAH (IPAH), a sporadic disease in which there is neither a family history of PAH nor an identified risk factor; heritable PAH; PAH induced by drugs and toxins; PAH associated with connective tissue diseases, HIV infection, portal hypertension, congenital heart diseases, schistosomiasis, and chronic hemolytic anemia; and persistent PH of newborns (Simonneau et al., 2009). Diagnosis of various types of PH requires a series of tests.

In general, PH treatment depends on the cause or classification of the PH. Where PH is caused by a known medicine or medical condition, it is known as a secondary PH, and its treatment is usually directed at the underlying disease. Treatment of pulmonary venous hypertension generally involves optimizing left ventricular function by administering diuretics, beta blockers, and ACE inhibitors, or repairing or replacing a mitral valve or aortic valve. PAH therapies include pulmonary vasodilators, digoxin, diuretics, anticoagulants, and oxygen therapy. Pulmonary vasodilators target different pathways, including prostacyclin pathway (e.g., prostacyclins, including intravenous epoprostenol, subcutaneous or intravenous treprostinil, and inhaled iloprost), nitric oxide pathway (e.g., phosphodiesterase-5 inhibitors, including sildenafil and tadalafil), and endotheline-1 pathway (e.g., endothelin receptor antagonists, including oral bosentan and oral ambrisentan) (Humbert, M., 2009; Hill et al., 2009). However, currently available therapies provide no cure for PH, and they do not directly treat the underlying vascular remodeling and muscularization of blood vessels observed in many PH patients.

In some embodiments, the present disclosure relates to methods of treating PH comprising administering to a subject in need thereof an effective amount of an ActRIIB-ECD polypeptide described herein or TGFβ superfamily ligand binding agent comprising the same. In some embodiments, the disclosure relates to methods of preventing PH comprising administering to a patient in need thereof an effective amount of an ActRIIB-ECD polypeptide described herein or TGFβ superfamily ligand binding agent comprising the same. In some embodiments, the disclosure relates to methods of reducing the progression rate of PH comprising administering to a patient in need thereof comprising administering an effective amount of an ActRIIB-ECD polypeptide described herein or TGFβ superfamily ligand binding agent comprising the same. In some embodiments, the disclosure relates to methods of treating PH-induced heart failure comprising administering to a subject in need thereof an effective amount of an ActRIIB-ECD polypeptide described herein or TGFβ superfamily ligand binding agent comprising the same.

In some embodiments, the disclosure provides for a method of treating an interstitial lung disease, comprising administering to a subject in need thereof an effective amount of an ActRIIB-ECD polypeptide described herein or TGFβ superfamily ligand binding agent comprising the same, wherein the an ActRIIB-ECD polypeptide described herein or TGFβ superfamily ligand binding agent comprising the same inhibits one or more of activin A, activin B, GDF-8, and GDF-11. In some embodiments, one or more additional TGFβ superfamily ligands such as, without limitation, GDF-3, BMP-6, BMP-15, ALK-4, ALK-5, and ALK-7, may also be inhibited.

In some embodiments, the disclosure provides for a method of treating, preventing, or reducing the progression rate and/or severity of one or more complications of an interstitial lung disease, comprising administering to a patient in need thereof an effective amount of an ActRIIB-ECD polypeptide described herein or TGFβ superfamily ligand binding agent comprising the same, wherein the polypeptide or binding agent inhibits one or more of activin A, activin B, GDF-8, and GDF-11. In some embodiments, the interstitial lung disease is idiopathic pulmonary fibrosis. In some embodiments, the disclosure relates to methods of reducing the severity of PH comprising administering to a patient in need thereof an effective amount of an ActRIIB-ECD polypeptide described herein or TGFβ superfamily ligand binding agent comprising the same. In certain aspects, the disclosure relates to methods of treating one or more complications (e.g., smooth muscle and/or endothelial cell proliferation in the pulmonary artery, angiogenesis in the pulmonary artery, dyspnea, chest pain, pulmonary vascular remodeling, right ventricular hypertrophy, and pulmonary fibrosis) of pulmonary hypertension comprising administering to a patient in need thereof an effective amount of a polypeptide or binding agent described herein.

In certain embodiments, the disclosure relates to methods of preventing one or more complication of PH (e.g., smooth muscle and/or endothelial cell proliferation in the pulmonary artery, angiogenesis in the pulmonary artery, dyspnea, chest pain, pulmonary vascular remodeling, right ventricular hypertrophy, and pulmonary fibrosis) comprising administering to a patient in need thereof an effective amount of an ActRIIB-ECD polypeptide described herein or TGFβ superfamily ligand binding agent comprising the same. In certain embodiments, the disclosure relates to methods of reducing the progression rate of one or more complication of PH (e.g., smooth muscle and/or endothelial cell proliferation in the pulmonary artery, angiogenesis in the pulmonary artery, dyspnea, chest pain, pulmonary vascular remodeling, right ventricular hypertrophy, and pulmonary fibrosis) comprising administering to a patient in need thereof an effective amount of a polypeptide or binding agent described herein. In certain aspects, the disclosure relates to methods of reducing the severity of one or more complication of pulmonary hypertension (e.g., smooth muscle and/or endothelial cell proliferation in the pulmonary artery, angiogenesis in the pulmonary artery, dyspnea, chest pain, pulmonary vascular remodeling, right ventricular hypertrophy, and pulmonary fibrosis) comprising administering to a patient in need thereof an effective amount of an ActRIIB-ECD polypeptide described herein or TGFβ superfamily ligand binding agent comprising the same.

In certain preferred embodiments, methods described herein relate to a subject having pulmonary arterial hypertension (PAH). In some embodiments, methods described herein relate to a subject having a resting pulmonary arterial pressure (PAP) of at least 25 mm Hg (e.g., at least 25, at least 30, at least 35, at least 40, at least 45, or at least 50 mm Hg). In some embodiments, the methods described herein reduce PAP in a subject having pulmonary hypertension. For example, the method may reduce PAP by at least 3 mmHg (e.g., at least 3, at least 5, at least 7, at least 10, at least 12, at least 15, at least 20, or at least 25 mm Hg) in a subject having pulmonary hypertension. In some embodiments, the methods described herein reduce pulmonary vascular resistance in a subject having pulmonary hypertension.

In some embodiments, the methods described herein increase pulmonary capillary wedge pressure in a subject having pulmonary hypertension. In some embodiments, the methods described herein increase left ventricular end-diastolic pressure in a subject having pulmonary hypertension. In some embodiments, the methods described herein increase (improve) exercise capacity (ability, tolerance) in a subject having pulmonary hypertension. For example, the method may increase 6-minute walk distance in a subject having pulmonary hypertension, optionally increasing 6-minute walk distance by at least 10 meters (e.g., at least 10, at least 20, at least 30, at least 40, at least 50, at least 60, at least 70, at least 80, at least 90, at least 100 or more meters). In addition, the method may reduce the subject's Borg dyspnea index (BDI), which optionally may be assessed after a 6-minute walk test. In some embodiments, the method reduces the subject's Borg dyspnea index (BDI) by at least 0.5 index points (e.g., at least 0.5, 1, 1.5, 2, 2.5, 3, 3.5, 4, 4.5, 5, 5.5, 6, 6.5, 7, 7.5, 8, 8.5, 9, 9.5, or 10 index points). In some embodiments, the methods described herein relate to a subject having Class I, Class II, Class III, or Class IV pulmonary hypertension as recognized by the World Health Organization. In some embodiments, the methods described herein relate to delaying clinical progression (worsening) of pulmonary hypertension (e.g., progression as measured by the World Health Organization standard). In some embodiments, the method prevents or delays pulmonary hypertension Class progression (e.g., prevents or delays progression from Class I to Class II, Class II to Class III, or Class III to Class IV pulmonary hypertension as recognized by the World Health Organization). In some embodiments, the method promotes or increases pulmonary hypertension Class regression (e.g., promotes or increases regression from Class IV to Class III, Class III to Class II, or Class II to Class I pulmonary hypertension as recognized by the World Health Organization).

In some embodiments, the subject is further administered one or more supportive therapies or active agents for treating pulmonary hypertension in addition to the one or more polypeptide or binding agent of the disclosure. For example, the subject also may be administered one or more supportive therapies or active agents selected from the group consisting of: prostacyclin and derivatives thereof (e.g., epoprostenol, treprostinil, and iloprost); prostacyclin receptor agonists (e.g., selexipag); endothelin receptor antagonists (e.g., thelin, ambrisentan, macitentan, and bosentan); calcium channel blockers (e.g., amlodipine, diltiazem, and nifedipine; anticoagulants (e.g., warfarin); diuretics; oxygen therapy; atrial septostomy; pulmonary thromboendarterectomy; phosphodiesterase type 5 inhibitors (e.g., sildenafil and tadalafil); activators of soluble guanylate cyclase (e.g., cinaciguat and riociguat); ASK-1 inhibitors (e.g., CIIA; SCH79797; GS-4997; MSC2032964A; 3H-naphtho[1,2,3-de]quiniline-2,7-diones, NQDI-1; 2-thioxo-thiazolidines, 5-bromo-3-(4-oxo-2-thioxo-thiazolidine-5-ylidene)-1,3-dihydro-indol-2-one-); NF-κB antagonists (e.g., dh404, CDDO-epoxide; 2.2-difluoropropionamide; C28 imidazole (CDDO-Im); 2-cyano-3,12-dioxoolean-1,9-dien-28-oic acid (CDDO); 3-Acetyloleanolic Acid; 3-Triflouroacetyloleanolic Acid; 28-Methyl-3-acetyloleanane; 28-Methyl-3-trifluoroacetyloleanane; 28-Methyloxyoleanolic Acid; SZCO14; SCZ015; SZCO17; PEGylated derivatives of oleanolic acid; 3-O-(beta-D-glucopyranosyl) oleanolic acid; 3-O-[beta-D-glucopyranosyl-(1→3)-beta-D-glucopyranosyl]oleanolic acid; 3-O-[beta-D-glucopyranosyl-(1→2)-beta-D-glucopyranosyl]oleanolic acid; 3-O-[beta-D-glucopyranosyl-(1->3)-beta-D-glucopyranosyl]oleanolic acid 28-O-beta-D-glucopyranosyl ester; 3-O-[beta-D-glucopyranosyl-(1→2)-beta-D-glucopyranosyl]oleanolic acid 28-O-beta-D-glucopyranosyl ester; 3-O-[a-L-rhamnopyranosyl-(1→3)-beta-D-glucuronopyranosyl]oleanolic acid; 3-O-[alpha-L-rhamnopyranosyl-(1→3)-beta-D-glucuronopyranosyl-]oleanolic acid 28-O-beta-D-glucopyranosyl ester; 28-O-β-D-glucopyranosyl-oleanolic acid; 3-O-β-D-glucopyranosyl (1→3)-β-D-glucopyranosiduronic acid (CS1); oleanolic acid 3-O-β-D-glucopyranosyl (1→3)-β-D-glucopyranosiduronic acid (CS2); methyl 3,11-dioxoolean-12-en-28-olate (DIOXOL); ZCVI4-2; Benzyl 3-dehydroxy-1,2,5-oxadiazolo[3',4':2,3]oleanolate) lung and/or heart transplantation.

In some embodiments, the subject may also be administered a BMP-9 polypeptide. In some embodiments the BMP-9 polypeptide is a mature BMP-9 polypeptide. In some embodiments, the BMP-9 polypeptide comprises a BMP-9 prodomain polypeptide. In some embodiments, the BMP-9 polypeptide is administered in a pharmaceutical preparation, which optionally may comprise a BMP-9 prodomain polypeptide. In such BMP-9 pharmaceutical preparations comprising a BMP-9 prodomain polypeptide, the BMP-9 polypeptide may be noncovalently associated with the BMP-9 prodomain polypeptide. In some embodiments, BMP-9 pharmaceutical preparations are substantially free, or do not comprise, of BMP9 prodomain polypeptide. In some embodiments, the subject may also be administered oleanolic acid or a derivative thereof.

### Fibrosis

In some embodiments, a polypeptide or binding agent described herein is used for treatment or prevention of fibrosis. For example, an ActRIIB-ECD polypeptide described herein or TGFβ superfamily ligand binding agent comprising the same may be used to treat, prevent or reduce the progression rate and/or severity of a variety of conditions including, but not limited to, fibrotic disease of tissues and/or organs, fibrotic scarring, and fibroproliferative disorders, in a subject in need thereof. Non-limiting examples of fibrotic diseases or conditions include pulmonary fibrosis (e.g., idiopathic pulmonary fibrosis), renal fibrosis, liver fibrosis (e.g., hepatic cirrhosis), systemic sclerosis, scleroderma, skin fibrosis, heart fibrosis, bone marrow fibrosis, ocular fibrosis, and myelofibrosis. In one particular embodiment, systemic sclerosis (SSc) is treated or prevented. In another particular embodiment, scleroderma is treated or prevented. In another particular embodiment, myelofibrosis (MF) is treated or prevented.

Fibrosis is the formation of excess connective tissue in an organ or tissue. The connective tissue, which can form in response to damage (e.g., injury) or as part of an immune response (e.g., an inflammatory response), can disrupt the structure and function of the organ or tissue in which it forms, leading to an increase in tissue stiffness. Fibrosis can occur in many organs and tissues within the body, including the lung (e.g., pulmonary fibrosis, cystic fibrosis), liver (e.g., cirrhosis), heart (e.g., endomyocardial fibrosis or fibrosis after myocardial infarction), brain (e.g., glial scar formation), skin (e.g., formation of keloids), kidney (e.g., renal fibrosis), and eye (e.g., corneal fibrosis), among others; and is known to be associated with certain medical treatments (e.g., chemotherapy, radiation therapy, and surgery). However, there are limited treatment options currently available for subjects with fibrosis, and most available treatments are focused on improving quality of life or temporarily slowing disease progression.

Other exemplary embodiments of fibrosis that may be prevented or treated include, for example and without limitation: interstitial lung disease; human fibrotic lung disease (e.g., obliterative bronchiolitis, idiopathic pulmonary fibrosis, pulmonary fibrosis from a known etiology, tumor stroma in lung disease, systemic sclerosis affecting lungs, Hermansky-Pudlak syndrome, coal worker's pneumoconiosis, asbestosis, silicosis, chronic pulmonary hypertension); AIDS-associated treatable types of fibrosis, including lung fibrosis, cystic fibrosis, liver fibrosis, heart fibrosis, mediastinal fibrosis, retroperitoneal cavity fibrosis, bone marrow fibrosis, skin fibrosis; scleroderma; and systemic sclerosis. Specific forms of fibrosis that can be treated or prevented include those that affect any organ or tissue or cell of the body, such as human tenon's fibroblasts, kidney, lung, intestine, liver, heart, bone marrow, genitalia, skin and eye. These diseases include, but are not limited to, cystic fibrosis, systemic sclerosis, chronic obstructive pulmonary disease (COPD), Dupuytren's contracture, glomerulonephritis, liver fibrosis, post-infarction cardiac fibrosis, restenosis, ocular surgery-induced fibrosis, and scarring. Genetic disorders of connective tissue can also be treated, and include but are not limited to, Marfan syndrome (MFS) and Osteogenesis imperfecta.

In some embodiments, an ActRIIB-ECD polypeptide described herein or TGFβ superfamily ligand binding agent comprising the same is used for inhibiting differentiation of fibroblasts into myofibroblasts.

In some embodiments, an ActRIIB-ECD polypeptide described herein or TGFβ superfamily ligand binding agent comprising the same is used for treatment or prevention of a fibroproliferative disorder. Fibroproliferative disorders are characterized by proliferation of fibroblasts plus the corresponding overexpression of extracellular matrix such as fibronectin, laminin and collagen.

In some embodiments, the disclosure provides for a method of decreasing or preventing fibrosis in a subject in need thereof by administering to the subject a therapeutically effective amount of an ActRIIB-ECD polypeptide described herein or TGFβ superfamily ligand binding agent comprising the same or a pharmaceutical composition described herein.

In some embodiments, the disclosure provides for a method of slowing or inhibiting the progression of fibrosis in a subject in need thereof by administering to the subject a therapeutically effective amount of an ActRIIB-ECD polypeptide described herein or TGFβ superfamily ligand binding agent comprising the same or a pharmaceutical composition described herein.

In some embodiments, the disclosure provides for a method of reducing the risk of developing fibrosis in a subject in need thereof by administering to the subject a therapeutically effective amount of an ActRIIB-ECD polypeptide described herein or TGFβ superfamily ligand binding agent comprising the same described herein or a pharmaceutical composition described herein.

In some embodiments, the disclosure provides for a method of treating a subject having or at risk of developing fibrosis by administering to the subject a therapeutically effective amount of a polypeptide or binding agent described herein or a pharmaceutical composition described herein.

In some embodiments, the fibrosis is chemotherapeutic drug-induced fibrosis, radiation-induced fibrosis, pulmonary fibrosis, hepatic fibrosis, renal fibrosis (e.g., fibrosis related to chronic kidney disease), corneal fibrosis, heart fibrosis, bone marrow fibrosis, mediastinal fibrosis, retropertinoneal fibrosis, arthrofibrosis, osteoarticular fibrosis, tissue fibrosis, a tumor stroma, a desmoplastic tumor, a surgical adhesion, a hypertrophic scar, or a keloid.

In some embodiments, the fibrosis is fibrosis associated with wounds, burns, hepatitis B or C infection, fatty liver disease, Schistosoma infection, kidney disease (e.g., chronic kidney disease), heart disease, macular degeneration, Crohn's disease, retinal or vitreal retinopathy, systemic or local scleroderma, atherosclerosis, or restenosis. In some embodiments, the fibrosis results from chronic kidney disease. In some embodiments, the disease or condition is chemotherapeutic drug-induced fibrosis, radiation-induced fibrosis, pulmonary fibrosis, hepatic fibrosis, renal fibrosis (e.g., fibrosis related to chronic kidney disease), corneal fibrosis, heart fibrosis, bone marrow fibrosis, mediastinal fibrosis, retroperitoneal fibrosis, arthrofibrosis, osteoarticular fibrosis, tissue fibrosis, a tumor stroma, a desmoplastic tumor, a surgical adhesion, a hypertrophic scar, or a keloid. In some embodiments, the disease or condition is fibrosis associated with wounds, burns, hepatitis B or C infection, fatty liver disease, Schistosoma infection, kidney disease (e.g., chronic kidney disease), heart disease, macular degeneration, Crohn's disease, retinal or vitreal retinopathy, systemic or local scleroderma, atherosclerosis, or restenosis. In some embodiments, the fibrosis results from chronic kidney disease.

In some embodiments, the tissue fibrosis is fibrosis affecting a tissue selected from the group consisting of muscle tissue, skin epidermis, skin dermis, tendon, cartilage, pancreatic tissue, uterine tissue, neural tissue, heart tissue, testis, ovary, adrenal gland, artery, vein, colon, small intestine, large intestine, biliary tract, and gut.

In some embodiments, the disclosure provides for a method of improving the function of a fibrotic tissue or organ. In some embodiments, the disclosure provides for a method of slowing or inhibiting the progression of fibrosis. In some embodiments, the disclosure provides for a method of reducing one or more symptom of fibrosis, for example reducing the frequency or severity of one or more symptom of fibrosis.

In some embodiments, the disclosure provides for a method of treating a subject having or at risk of developing a fibrotic disease comprising administering to a subject in need thereof an effective amount of a polypeptide or binding agent or pharmaceutical composition described herein. In some such embodiments, activin A, activin B, GDF-8, and/or GDF-11 signaling is inhibited in the subject. For example, in some embodiments the disclosure relates to methods of reducing or inhibiting the binding of activin A, activin B, GDF-8, and/or GDF-11 to their endogenous receptors in a subject having or at risk of developing a disease or condition involving fibrosis, the method comprising administering to a subject in need thereof an effective amount of an ActRIIB-ECD polypeptide described herein or TGFβ superfamily ligand binding agent comprising the same or pharmaceutical composition described herein.

In some embodiments, in accordance with the methods of the present disclosure, fibrotic symptoms are reduced in a subject. For example, the polypeptide or binding agent or composition may reduce fibrosis, fibrotic scarring, or skin thickening in a subject by at least 5%, e.g., at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or 100%.

In some embodiments, in accordance with the methods of the present disclosure, the differentiation of fibroblasts into myofibroblasts is inhibited in a subject, e.g., by at least 5%, e.g., at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or 100%.

In some embodiments, in accordance with the methods of the present disclosure, tumor growth and/or metastasis is inhibited in a subject, e.g., by at least 5%, e.g., at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or 100%.

In some embodiments, in accordance with the methods of the present disclosure, hematopoietic colony formation and/or hematopoiesis in bone marrow hematopoietic stem or progenitor cells (HSPCs) is increased in the bone marrow of a subject, e.g., by at least 5%, e.g., at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or 100%.

In some embodiments, in accordance with the methods of the present disclosure, a positive response in lung fibrosis is revealed as a consistent slowing in the rate of decline in lung function, as measured by forced vital capacity.

In some embodiments, in accordance with the methods of the present disclosure, a positive response for skin fibrosis associated with systemic sclerosis is determined by an improvement in the Modified Rodnan Skin Score (MRSS). For example, the MRSS may improve in a subject by at least 5%, e.g., at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or 100%.

### Muscle weakness and atrophy

In some embodiments, an ActRIIB-ECD polypeptide described herein or TGFβ superfamily ligand binding agent comprising the same is used for treatment or prevention of diseases and conditions involving muscle disorders, such as without limitation, muscle weakness and atrophy. For example, an ActRIIB-ECD polypeptide described herein or TGFβ superfamily ligand binding agent comprising the same herein may be used to treat, prevent or reduce the progression rate and/or severity of a variety of conditions including, but not limited to, Duchenne muscular dystrophy (DMD), facioscapulohumeral muscular dystrophy (FSHD), inclusion body myositis (IBM), amyotrophic lateral sclerosis (ALS), sarcopenia, or cancer cachexia.

Duchenne muscular dystrophy (DMD), facioscapulohumeral muscular dystrophy (FSHD), inclusion body myositis (IBM), and amyotrophic lateral sclerosis (ALS) are examples of muscle diseases that involve weakness and atrophy of muscles and/or motor neurons that control voluntary muscle movements. DMD is caused by mutations in the X-linked dystrophin gene and characterized by progressive muscle degeneration and weakness in all skeletal muscles. FSHD particularly affects skeletal muscles of the face, shoulders, upper arms, and lower legs. IBM is an inflammatory muscle disease that mainly affects muscles of the thighs and muscles of the arms that control finger and wrist flexion. ALS is a motor neuron disease characterized by stiff muscles, muscle twitching, and muscle atrophy throughout the body due to the degeneration of the motor neurons. However, few treatment options are available for these devastating muscle diseases.

In some embodiments, the present disclosure relates to methods of treating DMD comprising administering to a subject in need thereof an effective amount of an ActRIIB-ECD polypeptide described herein or TGFβ superfamily ligand binding agent comprising the same. In some embodiments, the disclosure relates to methods of preventing FSHD comprising administering to a subject in need thereof an effective amount of an ActRIIB-ECD polypeptide described herein or TGFβ superfamily ligand binding agent comprising the same. In some embodiments, the disclosure relates to methods of treating IBM comprising administering to a patient in need thereof an effective amount of an ActRIIB-ECD polypeptide described herein or TGFβ superfamily ligand binding agent comprising the same. In some embodiments, the present disclosure relates to methods of treating ALS comprising administering to a subject in need thereof an effective amount of an ActRIIB-ECD polypeptide described herein or TGFβ superfamily ligand binding agent comprising the same. In some embodiments, the present disclosure relates to methods of treating sarcopenia comprising administering to a subject in need thereof an effective amount of an ActRIIB-ECD polypeptide described herein or TGFβ superfamily ligand binding agent comprising the same. In some embodiments, the present disclosure relates to methods of treating cancer cachexia comprising administering to a subject in need thereof an effective amount of an ActRIIB-ECD polypeptide described herein or TGFβ superfamily ligand binding agent comprising the same.

In some embodiments, the disclosure provides for a method of increasing muscle mass and/or strength in a subject in need thereof comprising administering to a subject in need thereof an effective amount of an ActRIIB-ECD polypeptide described herein or TGFβ superfamily ligand binding agent comprising the same.

In some embodiments, the disclosure provides for a method of increasing lean mass in a subject in need thereof comprising administering to a subject in need thereof an effective amount of an ActRIIB-ECD polypeptide described herein or TGFβ superfamily ligand binding agent comprising the same.

In some embodiments, the disclosure provides for a method of increasing muscle mass in a subject in need thereof comprising administering to a subject in need thereof an effective amount of an ActRIIB-ECD polypeptide described herein or TGFβ superfamily ligand binding agent comprising the same.

In some embodiments, the disclosure provides for a method of treating a subject having or at risk of developing a muscle disease comprising administering to a subject in need thereof an effective amount of an ActRIIB-ECD polypeptide described herein or TGFβ superfamily ligand binding agent comprising the same. In some embodiments, the muscle disease is DMD, FSHD, IBM, ALS, sarcopenia, or cancer cachexia. In some such embodiments, activin A, activin B, GDF-8, and/or GDF-11signaling is inhibited in the subject. For example, in some embodiments the disclosure relates to methods of reducing or inhibiting the binding of activin A, activin B, GDF-8, and/or GDF-11 to their endogenous receptors in a subject having or at risk of developing a disease or condition involving weakness and atrophy of muscles, the method comprising administering to a subject in need thereof an effective amount of an ActRIIB-ECD polypeptide described herein or TGFβ superfamily ligand binding agent comprising the same.

In some embodiments, the present disclosure relates to methods of treating muscle disorders, such as muscle wasting due to disease or disuse, comprising administering to a subject in need thereof an effective amount of an ActRIIB-ECD polypeptide described herein or TGFβ superfamily ligand binding agent comprising the same. The muscle disorder may be a musculoskeletal disease or disorder, such as muscle atrophy. There are many causes of muscle atrophy, including as a result of treatment with a glucocorticoid such as cortisol, dexamethasone, betamethasone, prednisone, methylprednisolone, or prednisolone. The muscle atrophy can also be a result of denervation due to nerve trauma or a result of degenerative, metabolic, or inflammatory neuropathy (e.g., Guillian-Barre syndrome, peripheral neuropathy, or exposure to environmental toxins or drugs).

In addition, the muscle atrophy can be a result of myopathy, such as myotonia; a congenital myopathy, including nemalene myopathy, multi/minicore myopathy and myotubular (centronuclear) myopathy; mitochondrial myopathy; familial periodic paralysis; inflammatory myopathy; metabolic myopathy, such as caused by a glycogen or lipid storage disease; dermatomyositisis; polymyositis; inclusion body myositis; myositis ossificans; rhabdomyolysis and myoglobinurias.

The myopathy may be caused by a muscular dystrophy syndrome, such as Duchenne, Becker, myotonic, fascioscapulohumeral, Emery-Dreifuss, oculopharyngeal, scapulohumeral, limb girdle, Fukuyama, a congenital muscular dystrophy, or hereditary distal myopathy. The musculoskeletal disease can also be osteoporosis, a bone fracture, short stature, or dwarfism.

In addition, the muscle atrophy can be a result of an adult motor neuron disease, infantile spinal muscular atrophy, amyotrophic lateral sclerosis, juvenile spinal muscular atrophy, autoimmune motor neuropathy with multifocal conductor block, paralysis due to stroke or spinal cord injury, skeletal immobilization due to trauma, prolonged bed rest, voluntary inactivity, involuntary inactivity, metabolic stress or nutritional insufficiency, cancer, AIDS, fasting, a thyroid gland disorder, diabetes, benign congenital hypotonia, central core disease, burn injury, chronic obstructive pulmonary disease, liver diseases (examples such as fibrosis, cirrhosis), sepsis, renal failure, congestive heart failure, ageing, space travel or time spent in a zero gravity environment.

In addition, the muscle atrophy can be associated with metabolic disorders, including without limitation Type II Diabetes, Metabolic Syndrome, hyperglycemia, and obesity.

### Bone damage

In some embodiments, a polypeptide or binding agent described herein is used for treatment or prevention of bone damage and associated conditions. For example, a polypeptide or binding agent described herein may be used to treat, prevent or reduce the progression rate and/or severity of a variety of diseases and conditions involving bone damage, including, but not limited to, primary osteoporosis, secondary osteoporosis, osteopenia, osteopetrosis, bone fracture, bone cancer or cancer metastasis-related bone loss, Paget's disease, renal osteodystrophy, treatment-related bone loss, diet-related bone loss, bone loss associated with the treatment of obesity, low gravity-related bone loss, or immobility.

Healthy bone undergoes a constant remodeling that involves both bone breakdown and bone growth. Bone growth is mediated by the osteoblast cell type whereas osteoclast cells resorb the bone. Pathology can occur when these systems fall out of balance either through downregulation of the anabolic program, upregulation of the catabolic system or a combination of both, resulting in a net bone loss. Controlling the balance in bone remodeling can therefore be useful for promoting the healing of damage to bone as well as the treatment of disorders, such as osteoporosis, associated with loss of bone mass and bone demineralization.

Bone damage can result from a range of root causes, including age- or cancer-related bone loss, genetic conditions, or adverse side effects of drug treatment. The World Health Organization estimates that osteoporosis alone affects 75 million people in the U.S., Europe and Japan, and is a significant risk factor in bone damage. In general, the whole of bone loss represents pathological states for which there are few effective treatments. Available treatments focus on immobilization, exercise and dietary modifications rather than agents that directly promote bone growth and increase bone density. With respect to osteoporosis, estrogen, calcitonin, osteocalcin with vitamin K, or high doses of dietary calcium are all used as therapeutic interventions. Other therapeutic approaches to osteoporosis include bisphosphonates, parathyroid hormone, parathyroid hormone related protein (PTHrP), calcimimetics, statins, anabolic steroids, lanthanum and strontium salts, and sodium fluoride, although these are often associated with undesirable side effects.

In some embodiments, a polypeptide or binding agent described herein is used to increase bone mass or bone mineral density in a subject having or at risk of developing a disease or condition involving bone damage.

In some embodiments, the disclosure provides a method of increasing bone mineral density in a subject in need thereof, comprising administering to the subject a therapeutically effective amount of an ActRIIB-ECD polypeptide described herein or TGFβ superfamily ligand binding agent comprising the same or pharmaceutical composition described herein.

In some embodiments, the disclosure provides a method of reducing bone resorption in a subject in need thereof, comprising administering to the subject a therapeutically effective amount of an ActRIIB-ECD polypeptide described herein or TGFβ superfamily ligand binding agent comprising the same or pharmaceutical composition described herein.

In some embodiments, the disclosure provides a method of increasing bone formation in a subject in need thereof, comprising administering to the subject a therapeutically effective amount of an ActRIIB-ECD polypeptide described herein or TGFβ superfamily ligand binding agent comprising the same or pharmaceutical composition described herein.

In some embodiments, the disclosure provides a method of increasing bone strength in a subject in need thereof, comprising administering to the subject a therapeutically effective amount of an ActRIIB-ECD polypeptide described herein or TGFβ superfamily ligand binding agent comprising the same or pharmaceutical composition described herein.

In some embodiments, the disclosure provides a method of reducing the risk of bone fracture in a subject in need thereof, comprising administering to the subject a therapeutically effective amount of an ActRIIB-ECD polypeptide described herein or TGFβ superfamily ligand binding agent comprising the same or pharmaceutical composition described herein.

In some embodiments, the disclosure provides a method of treating a subject having or at risk of developing bone disease, comprising administering to the subject a therapeutically effective amount of an ActRIIB-ECD polypeptide described herein or TGFβ superfamily ligand binding agent comprising the same or pharmaceutical composition described herein. Non-limiting examples of bone disease that may be treated in accordance with the present methods include primary osteoporosis, secondary osteoporosis, osteopenia, osteopetrosis, bone fracture, bone cancer or cancer metastasis related bone loss, Paget's disease, renal osteodystrophy, treatment-related bone loss, diet-related bone loss, bone loss associated with the treatment of obesity, low gravity-related bone loss, or immobility related bone loss. In some embodiments, the bone disease is osteoporosis. In some embodiments, the primary osteoporosis is age-related osteoporosis or hormone-related osteoporosis. In some embodiments of any of the above aspects, the secondary osteoporosis is immobilization-induced osteoporosis or glucocorticoid-induced osteoporosis. In some embodiments, the cancer is multiple myeloma. In some embodiments, the treatment-related bone loss occurs due to treatment with FGF-21, due to treatment with GLP-1, due to cancer therapy, e.g., chemotherapy or radiation, or due to treatment for obesity or Type-2 diabetes. In some embodiments, the diet-related bone loss is rickets, e.g., vitamin D deficiency. In some embodiments, the low gravity-related bone loss is lack of load-related bone loss. In some embodiments, the subject is at risk of bone fracture. In some embodiments, the polypeptide or binding agent described herein inhibits one or more of activin A, activin B, GDF-8, and GDF-11 in the subject, e.g., by reducing or inhibiting the binding of activin A, activin B, GDF-8, and/or GDF-11 to their receptors or inhibiting signaling through their receptors.

In some embodiments of methods of the disclosure, the method increases bone formation in the subject. In some embodiments of methods of the disclosure, the method decreases bone resorption in the subject. In some embodiments of methods of the disclosure, the method decreases bone loss in the subject. In some embodiments of methods of the disclosure, the method increases osteoblast activity or osteoblastogenesis in the subject. In some embodiments of methods of the disclosure, the method decreases osteoclast activity or decreases osteoclastogenesis in the subject. In some embodiments of methods of the disclosure, the method decreases the risk of bone fracture in the subject. In some embodiments of methods of the disclosure, the method increases bone strength in the subject. In some embodiments of methods of the disclosure, the bone is cortical bone. In some embodiments of methods of the disclosure, the bone is trabecular bone.

As used herein, the terms "bone mineral density (BMD)," "bone density," and "bone mass" refer to a measure of the amount of bone mineral (e.g., calcium) in bone tissue. BMD may be measured by well-established clinical techniques known to one of skill in the art (e.g., by single or dual-energy photon or X-ray absorptiometry). The concept of BMD relates to the mass of mineral per volume of bone, although clinically it is measured by proxy according to optical density per square centimeter of bone surface upon imaging. BMD measurement is used in clinical medicine as an indirect indicator of osteoporosis and fracture risk. In some embodiments, BMD test results are provided as a T-score, where the T-score represents the BMD of a subject compared to the ideal or peak bone mineral density of a healthy 30-year-old adult. A score of O indicates that the BMD is equal to the normal reference value for a healthy young adult. Differences between the measured BMD of subject and that of the reference value for a healthy young adult are measured in standard deviation units (SDs). Accordingly, a T-score of between +1 SD and -1 SD may indicate a normal BMD, a T-score of between -1 SD and -2.5 SD may indicate low bone mass (e.g., osteopenia), and a T-score lower than -2.5 SD may indicate osteoporosis or severe osteoporosis. In some embodiments of methods of the disclosure, a subject has low bone mass (e.g., a T-Score of between -1 SD and -2.5 SD). In some embodiments of methods of the disclosure, a subject has osteoporosis (e.g., a T-Score of less than -2.5 SD). In some embodiments of methods of the disclosure, the subject's BMD is increased by administration of a polypeptide or binding agent or pharmaceutical composition described herein. For example, treatment in accordance with the methods of the disclosure may increase the T-score of the subject, e.g., by 0.1 or more, 0.2 or more, 0.3 or more, 0.4 or more, 0.5 or more, 1.0 or more, or 2.0 or more.

In some embodiments, in bone marrow failure diseases including for example myelofibrosis, positive responses are revealed by improvements in anemia (for example, transfusion-independent patients exhibiting an increase in hemoglobin level, transfusion dependent patients become transfusion independent).

As used herein, the term "bone strength" refers to a measurement of bone that is determined by bone quality in addition to bone mineral density. Bone quality is influenced by bone geometry, microarchitecture, and the properties of constituent tissues. Bone strength can be used to assess the bone's risk of fracture.

As used herein, the term "bone disease" refers to a condition characterized by bone damage (e.g., decreased bone mineral density, decreased bone strength, and/or bone loss). Such diseases or conditions may be caused by an imbalance in osteoblast and/or osteoclast activity (e.g., increased bone resorption or reduced bone formation). Bone diseases include, without limitation, primary osteoporosis, secondary osteoporosis, osteopenia, osteopetrosis, bone fracture, bone cancer or cancer metastasis-related bone loss (e.g., bone loss associated with multiple myeloma), Paget's disease, renal osteodystrophy, treatment-related bone loss, diet-related bone loss, bone loss associated with the treatment of obesity, low gravity-related bone loss, and immobility-related bone loss.

As used herein, the terms "bone remodeling" or "bone metabolism" refer to the process for maintaining bone strength and ion homeostasis by replacing discrete parts of old bone with newly synthesized packets of proteinaceous matrix. Bone is resorbed by osteoclasts and is deposited by osteoblasts in a process called ossification. Osteocyte activity plays a key role in this process. Conditions that result in a decrease in bone mass, can either be caused by an increase in resorption, or a decrease in ossification. In a healthy individual, during childhood, bone formation exceeds resorption. As the aging process occurs, resorption exceeds formation. Bone resorption rates are also typically much higher in post-menopausal older women due to estrogen deficiency related to menopause.

As used herein, the terms "bone resorption" or "bone catabolic activity" refer to a process by which osteoclasts break down the tissue in bones and release the minerals, resulting in a transfer of the mineral (e.g., calcium) from bone tissue to the blood. Increased rates of bone resorption are associated with aging, including in post-menopausal women. High rates of bone resorption, or rates of bone resorption that exceed the rate of ossification, are associated with bone disorders, such as decreased bone mineral density, including osteopenia and osteoporosis, and can result in bone loss. In some embodiments of methods of the disclosure, bone resorption is decreased in the subject, e.g., bone loss is decreased in the subject, by administration of a polypeptide or binding agent or pharmaceutical composition described herein.

As used herein, the terms "bone formation," "ossification," "osteogenesis," or "bone anabolic activity" refer to the process of forming new bone tissue by osteoblasts. Reduced rates of bone formation, or rates of bone formation that are exceeded by the rate of bone resorption, can result in bone loss. In some embodiments of methods of the disclosure, bone formation is increased in the subject, e.g., the amount or rate of bone formation or osteogenesis is increased in the subject, by administration of a polypeptide or binding agent or pharmaceutical composition described herein.

In some embodiments of methods of the disclosure, administration of a polypeptide or binding agent or pharmaceutical composition described herein acts to increase bone mineral density, increase bone formation, increase bone strength, reduce the risk of bone fracture, and/or reduce bone resorption in the subject, compared to measurements obtained prior to treatment or compared to measurements typically observed in untreated subjects.

### Low red blood cell levels

In some embodiments, a polypeptide or binding agent described herein is used for treatment or prevention of a disease or condition associated with low red blood cell levels, such as anemia or blood loss. For example, a polypeptide or binding agent described herein may be used to treat, prevent or reduce the progression rate and/or severity of a variety of diseases and conditions involving low red blood cell levels, including, but not limited to, anemia or blood loss, e.g., by increasing red blood cell levels (for example by increasing hemoglobin levels, increasing red blood cell count, increasing hematocrit, or increasing red blood cell formation or production) in a subject in need thereof.

Anemia is a global health problem with health implications that affect both morbidity and mortality. In the United States alone, the prevalence of anemia nearly doubled from 2003 to 2012. Symptoms of anemia include fatigue, weakness, shortness of breath, heart palpitations, and reduced cognitive performance, and children, pregnant women, women of reproductive age, and the elderly have been found to have the highest risk of developing anemia. The most common form of anemia is iron deficiency anemia, but anemia can also be caused by chronic diseases, blood loss, and red blood cell destruction. While iron deficiency anemia can be treated with iron supplements, many other forms of anemia, such as aplastic anemia, anemia of chronic disease, and hemolytic anemia may require blood transfusions.

In some embodiments, the disclosure provides a method of treating low red blood cell levels in a subject in need thereof comprising administering to the subject an effective amount of an ActRIIB-ECD polypeptide described herein or TGFβ superfamily ligand binding agent comprising the same or pharmaceutical composition described herein.

In some embodiments, the disclosure provides a method of treating anemia in a subject in need thereof comprising administering to the subject an effective amount of an ActRIIB-ECD polypeptide described herein or TGFβ superfamily ligand binding agent comprising the same or pharmaceutical composition described herein.

In some embodiments, the disclosure provides a method of treating blood loss in a subject in need thereof comprising administering to the subject an effective amount of an ActRIIB-ECD polypeptide described herein or TGFβ superfamily ligand binding agent comprising the same or pharmaceutical composition described herein.

In some embodiments, the disclosure provides a method of increasing red blood cell levels in a subject in need thereof comprising administering to the subject an effective amount of an ActRIIB-ECD polypeptide described herein or TGFβ superfamily ligand binding agent comprising the same or pharmaceutical composition described herein.

In some embodiments, the disclosure provides a method of increasing hemoglobin levels in a subject in need thereof comprising administering to the subject an effective amount of a polypeptide or binding agent or pharmaceutical composition described herein.

In some embodiments, the disclosure provides a method of increasing red blood cell count in a subject in need thereof comprising administering to the subject an effective amount of an ActRIIB-ECD polypeptide described herein or TGFβ superfamily ligand binding agent comprising the same or pharmaceutical composition described herein.

In some embodiments, the disclosure provides a method of increasing hematocrit in a subject in need thereof comprising administering to the subject an effective amount of an ActRIIB-ECD polypeptide described herein or TGFβ superfamily ligand binding agent comprising the same or pharmaceutical composition described herein.

In some embodiments, the disclosure provides a method of increasing red blood cell formation in a subject in need thereof, comprising administering to the subject an effective amount of an ActRIIB-ECD polypeptide described herein or TGFβ superfamily ligand binding agent comprising the same or pharmaceutical composition described herein.

In some embodiments of methods of the disclosure, the subject may have or be at risk of developing anemia or blood loss. In some embodiments, the anemia is due to other diseases or conditions, such as chronic kidney disease, rheumatoid arthritis, cancer, or inflammatory diseases (e.g., Crohn's disease, SLE, or ulcerative colitis), or due to medical treatments, such as chemotherapy, radiation therapy, or surgery. In some embodiments, the polypeptide or binding agent described herein inhibits one or more of activin A, activin B, GDF-8, and/or GDF-11 in the subject, e.g., by reducing or inhibiting the binding of activin A, activin B, GDF-8, and/or GDF-11 to their receptors or inhibiting signaling through their receptors.

In some embodiments, the disclosure provides a method of increasing red blood cells (e.g., increasing hemoglobin levels, red blood cell count, or hematocrit) in a subject in need thereof, comprising administering to the subject a therapeutically effective amount of a polypeptide or binding agent or pharmaceutical composition described herein, wherein red blood cells are increased (e.g., hemoglobin levels, red blood cell count, or hematocrit are increased) compared to measurements obtained prior to treatment.

In some embodiments, the disclosure provides a method of increasing red blood cell levels in a subject who has or is at risk of developing anemia or blood loss. In some embodiments, the anemia or blood loss is associated with cancer, cancer treatment, renal disease or failure (e.g., chronic kidney disease or acute renal disease or failure), myelodysplastic syndrome, thalassemia, nutritional deficits, adverse reaction to medication, an inflammatory or autoimmune disease, splenomegaly, porphyria, vasculitis, hemolysis, bone marrow defects, bone marrow transplantation, diabetes, liver disease (e.g., acute liver disease or chronic liver disease), bleeding (e.g., acute or chronic bleeding), infection, hemoglobinopathy, drug use, alcohol abuse, advanced age, Churg-Strauss syndrome, Felty syndrome, graft versus host disease, hematopoietic stem cell transplantation, osteomyelofibrosis, pancytopenia, pure red-cell aplasia, purpura, Schoenlein Henoch, Shwachman syndrome (e.g., Shwachman-Diamond syndrome), contraindication to transfusion, surgery, trauma, a wound, an ulcer, urinary tract bleeding, digestive tract bleeding, frequent blood donation, or heavy menstrual bleeding.

In some embodiments of methods of the disclosure, the anemia is aplastic anemia, iron deficiency anemia, vitamin deficiency anemia, anemia of chronic disease, anemia associated with bone marrow disease, hemolytic anemia, sickle cell anemia, microcytic anemia, hypochromic anemia, sideroblastic anemia, Diamond Blackfan anemia, Fanconi's anemia, or refractory anemia with excess of blasts.

In some embodiments of methods of the disclosure, the subject in need of treatment is a subject who does not respond well to treatment with erythropoietin (EPO) or is susceptible to the adverse effects of EPO.

As used herein, the terms "increase red blood cell levels" and "promote red blood cell formation" refer to clinically observable metrics, such as hematocrit, red blood cell counts, and hemoglobin measurements, and are intended to be neutral as to the mechanism by which such changes occur. The term "low red blood cell levels" as used herein refers to red blood cell counts, hematocrit, and hemoglobin measurements that are below the range of values that is considered normal for the subject's age and gender.

As used herein, the terms "red blood cell formation" and "red blood cell production" refer to the generation of red blood cells, such as the process of erythropoiesis in which red blood cells are produced in the bone marrow.

As used herein, the term "anemia" refers to any abnormality in hemoglobin or red blood cells that leads to reduced oxygen levels in the blood. Anemia can be associated with abnormal production, processing, or performance of erythrocytes and/or hemoglobin. The term anemia refers to any reduction in the number of red blood cells and/or level of hemoglobin in blood relative to normal blood levels.

In some embodiments of methods of the disclosure, administration of a polypeptide or binding agent or pharmaceutical composition described herein acts to increase red blood cell formation, red blood cell count, hemoglobin levels, or hematocrit in the subject, e.g., compared to measurements obtained prior to treatment. In some embodiments of methods of the disclosure, administration of the polypeptide or binding agent of the disclosure reduces the subject's need for a blood transfusion.

In some embodiments, the disclosure provides a method of treating a vascular disease or vasculopathy in a subject in need thereof, comprising administering to the subject a therapeutically effective amount of an ActRIIB-ECD polypeptide described herein or TGFβ superfamily ligand binding agent comprising the same or pharmaceutical composition described herein. Non-limiting examples of vascular disease or vasculopathy include vasculitis, arteriosclerosis, aortic aneurysms and vascular calcification, which may present alone or in connection with other diseases such as, for example and without limitation, chronic kidney disease (CKD), diabetes, or rare disorders such as sickle cell anemia and Kawasaki disease.

In some embodiments, the present disclosure provides a method of treating diabetes in a subject in need thereof, comprising administering to the subject a therapeutically effective amount of an ActRIIB-ECD polypeptide described herein or TGFβ superfamily ligand binding agent comprising the same or pharmaceutical composition described herein.

In some embodiments, the present disclosure provides a method of treating pre-diabetes in a subject in need thereof, comprising administering to the subject a therapeutically effective amount of an ActRIIB-ECD polypeptide described herein or TGFβ superfamily ligand binding agent comprising the same or pharmaceutical composition described herein.

In some embodiments, the present disclosure provides a method of treating obesity in a subject in need thereof, comprising administering to the subject a therapeutically effective amount of an ActRIIB-ECD polypeptide described herein or TGFβ superfamily ligand binding agent comprising the same or pharmaceutical composition described herein.

In some embodiments, the present disclosure provides a method of treating metabolic syndrome in a subject in need thereof, comprising administering to the subject a therapeutically effective amount of an ActRIIB-ECD polypeptide described herein or TGFβ superfamily ligand binding agent comprising the same or pharmaceutical composition described herein.

In some embodiments, the present disclosure provides a method of treating a cardiometabolic disorder in a subject in need thereof, comprising administering to the subject a therapeutically effective amount of an ActRIIB-ECD polypeptide described herein or TGFβ superfamily ligand binding agent comprising the same or pharmaceutical composition described herein. In some embodiments, the cardiometabolic disorder is diabetes, pre-diabetes, obesity, metabolic syndrome, or a combination thereof.

In some embodiments, the present disclosure provides a method of treating cardiovascular disease in a subject in need thereof, comprising administering to the subject a therapeutically effective amount of an ActRIIB-ECD polypeptide described herein or TGFβ superfamily ligand binding agent comprising the same or pharmaceutical composition described herein. In some embodiments, the cardiovascular disease is heart failure. In some embodiments, the heart failure is with reduced, preserved or mildly reduced ejection fraction, with hypertension, with atherosclerosis, or with a combination thereof.

In some embodiments, the present disclosure provides a method of treating heart failure with reduced ejection fraction (HFrEF) in a subject in need thereof, comprising administering to the subject a therapeutically effective amount of an ActRIIB-ECD polypeptide described herein or TGFβ superfamily ligand binding agent comprising the same or pharmaceutical composition described herein.

In some embodiments, the present disclosure provides a method of treating heart failure with preserved ejection fraction (HFpEF) in a subject in need thereof, comprising administering to the subject a therapeutically effective amount of an ActRIIB-ECD polypeptide described herein or TGFβ superfamily ligand binding agent comprising the same or pharmaceutical composition described herein.

In some embodiments, the present disclosure provides a method of treating heart failure with mildly reduced ejection fraction (HFmrEF) in a subject in need thereof, comprising administering to the subject a therapeutically effective amount of an ActRIIB-ECD polypeptide described herein or TGFβ superfamily ligand binding agent comprising the same or pharmaceutical composition described herein.

In some embodiments, the present disclosure provides a method of treating hypertension in a subject in need thereof, comprising administering to the subject a therapeutically effective amount of an ActRIIB-ECD polypeptide described herein or TGFβ superfamily ligand binding agent comprising the same or pharmaceutical composition described herein.

In some embodiments, the present disclosure provides a method of treating atherosclerosis in a subject in need thereof, comprising administering to the subject a therapeutically effective amount of an ActRIIB-ECD polypeptide described herein or TGFβ superfamily ligand binding agent comprising the same or pharmaceutical composition described herein.

In some embodiments, the present disclosure provides a method of treating cardiac fibrosis in a subject in need thereof, comprising administering to the subject a therapeutically effective amount of an ActRIIB-ECD polypeptide described herein or TGFβ superfamily ligand binding agent comprising the same or pharmaceutical composition described herein.

In some embodiments, the present disclosure provides a method of treating a cardiomyopathy in a subject in need thereof, comprising administering to the subject a therapeutically effective amount of an ActRIIB-ECD polypeptide described herein or TGFβ superfamily ligand binding agent comprising the same or pharmaceutical composition described herein.

In some embodiments, the present disclosure provides a method of treating myocardial infarction in a subject in need thereof, comprising administering to the subject a therapeutically effective amount of an ActRIIB-ECD polypeptide described herein or TGFβ superfamily ligand binding agent comprising the same or pharmaceutical composition described herein.

In some embodiments, the present disclosure provides a method of treating pulmonary hypertension in a subject in need thereof, comprising administering to the subject a therapeutically effective amount of an ActRIIB-ECD polypeptide described herein or TGFβ superfamily ligand binding agent comprising the same or pharmaceutical composition described herein. In some embodiments, the pulmonary hypertension (PH) is WHO Group 1 PH. In some embodiments, the pulmonary hypertension (PH) is WHO Group 2 PH. In some embodiments, the pulmonary hypertension (PH) is WHO Group 3 PH. In some embodiments, the pulmonary hypertension (PH) is WHO Group 4 PH. In some embodiments, the pulmonary hypertension (PH) is WHO Group 5 PH.

In some embodiments, the present disclosure provides a method of treating sarcopenia in a subject in need thereof, comprising administering to the subject a therapeutically effective amount of an ActRIIB-ECD polypeptide described herein or TGFβ superfamily ligand binding agent comprising the same or pharmaceutical composition described herein. In some embodiments, the sarcopenia is related to age. In some embodiments, the sarcopenia is related to diabetes. In some embodiments, the sarcopenia is related to obesity.

In some embodiments, the present disclosure provides a method of treating chronic kidney disease in a subject in need thereof, comprising administering to the subject a therapeutically effective amount of an ActRIIB-ECD polypeptide described herein or TGFβ superfamily ligand binding agent comprising the same or pharmaceutical composition described herein. In some embodiments, the chronic kidney disease is cardio-renal syndrome.

In some embodiments, the present disclosure provides a method of treating liver disease in a subject in need thereof, comprising administering to the subject a therapeutically effective amount of an ActRIIB-ECD polypeptide described herein or TGFβ superfamily ligand binding agent comprising the same or pharmaceutical composition described herein. In some embodiments, the liver disease is non-alcoholic steatohepatitis (NASH), non-alcoholic fatty liver disease (NAFLD), liver fibrosis, liver cirrhosis, or a combination thereof.

In some embodiments, the present disclosure provides a method of treating non-alcoholic steatohepatitis (NASH) in a subject in need thereof, comprising administering to the subject a therapeutically effective amount of an ActRIIB-ECD polypeptide described herein or TGFβ superfamily ligand binding agent comprising the same or pharmaceutical composition described herein.

In some embodiments, the present disclosure provides a method of treating non-alcoholic fatty liver disease (NAFLD) in a subject in need thereof, comprising administering to the subject a therapeutically effective amount of an ActRIIB-ECD polypeptide described herein or TGFβ superfamily ligand binding agent comprising the same or pharmaceutical composition described herein.

In some embodiments, the present disclosure provides a method of treating liver fibrosis in a subject in need thereof, comprising administering to the subject a therapeutically effective amount of an ActRIIB-ECD polypeptide described herein or TGFβ superfamily ligand binding agent comprising the same or pharmaceutical composition described herein.

In some embodiments, the present disclosure provides a method of treating liver cirrhosis in a subject in need thereof, comprising administering to the subject a therapeutically effective amount of an ActRIIB-ECD polypeptide described herein or TGFβ superfamily ligand binding agent comprising the same or pharmaceutical composition described herein.

In some embodiments, the present disclosure provides a method of treating bone marrow failure in a subject in need thereof, comprising administering to the subject a therapeutically effective amount of an ActRIIB-ECD polypeptide described herein or TGFβ superfamily ligand binding agent comprising the same or pharmaceutical composition described herein.

### Kits

In accordance with the present disclosure, polypeptides, TGFβ superfamily ligand binding agents and pharmaceutical compositions described herein may be assembled into kits or pharmaceutical systems for use in treating or preventing TGFβ superfamily-associated diseases or conditions. Kits or pharmaceutical systems may comprise a container (e.g., packaging, a box, a carton, a vial, etc.), having in close confinement therein one or more container, such as vials, tubes, ampoules, bottles, and the like, that contains the polypeptide, binding agent or pharmaceutical composition. Additional kit components may include acids, bases, buffering agents, inorganic salts, solvents, antioxidants, preservatives, or metal chelators. The additional kit components may be present as pure compositions, or as aqueous or organic solutions that incorporate one or more additional kit components. Any or all of the kit components optionally further comprise buffers. Kits may also include tools for administration, such as needles, syringes, and the like. The kit may be used according to the methods described herein and may include instructions for use in such methods. Kits may also include instructions for administration and use of the polypeptide, binding agent or pharmaceutical composition.

In some embodiments, a kit comprises one or more recipients (such as vials, ampoules, containers, syringes, bottles, bags) of any appropriate shape, size and material (preferably waterproof, e.g., plastic or glass) containing the polypeptide or binding agent or pharmaceutical composition of the present disclosure in an appropriate dosage for administration (as discussed above). The kit may additionally contain directions for use (e.g., in the form of a leaflet or instruction manual), means for administering the polypeptide or binding agent of the present disclosure such as a syringe, pump, infuser or the like, means for reconstituting the polypeptide or binding agent of the disclosure and/or means for diluting the polypeptide or binding agent of the disclosure.

The disclosure also provides kits for a single-dose administration unit. The kit of the disclosure may also contain a first recipient comprising a dried/lyophilized polypeptide or binding agent and a second recipient comprising an aqueous formulation. In certain embodiments, kits containing single-chambered and multi-chambered pre-filled syringes (e.g., liquid syringes and lyosyringes) are provided.

### EXAMPLES

The present invention will be more readily understood by referring to the following examples, which are provided to illustrate the invention and are not to be construed as limiting the scope thereof in any manner.

Unless defined otherwise or the context clearly dictates otherwise, all technical and scientific terms used herein have the same meaning as commonly understood by one of ordinary skill in the art to which this invention belongs. It should be understood that any methods and materials similar or equivalent to those described herein can be used in the practice or testing of the invention.

### Example 1. Design and Characterization of ActRIIB-ECD fusion proteins that neutralize TGFβ superfamily ligands.

Characterization of the neutralization activities of the benchmark ActRIIB ECD-Fc fusion construct. Exemplary wild type human ActRIIB-ECD-Fc (also referred to as "ACVR2B-Fc"; referred to herein as P75 (SEQ ID NO: 171)) was produced, characterized, and used as a benchmark protein. P75 is comprised of the human wild type ActRIIB-ECD (also referred to as the ACVR2B ECD; SEQ ID NO: 2), linked to the human IgG1 Fc domain by a triple glycine linker. The sequence of P75 is shown in **Table 6**.

The inhibition potency (IC50) of the benchmark agent P75 (wild type ActRIIB) for activin A, activin B, GDF-8, and GDF-11 was determined using the TGFβ-reporter HEK293 cell-based assay described below. P75 efficiently neutralized all four ligands tested (activin A, activin B, GDF-8, and GDF-11) in a dose-dependent manner. IC50 values for P75 on activin A, activin B, GDF-8, and GDF-11 were 2.3, 1.4, 0.67, and 0.76 nM, respectively. The IC50 values are reported in **Table 7**.

The inhibition potency (IC50) of the benchmark agent P75 for BMP-9 and BMP-10 was determined using the BMP-reporter HepG2 cell-based assay described below. P75 neutralized BMP-9 and BMP-10 in a dose-dependent manner, with an average IC50 value of 2.7 and 2.0 nM, respectively. The IC50 values are reported in **Table 8**.

### Example 2. Design and characterization of ActRIIB-ECD polypeptide constructs that neutralize activin A, activin B, GDF-8, and GDF-11, but not BMP-9 and/or BMP-10 signaling.

A series of TGFβ superfamily ligand binding and neutralization agents were tested. The sequences of representative ActRIIB-ECD polypeptide constructs are listed in **Table 6**.

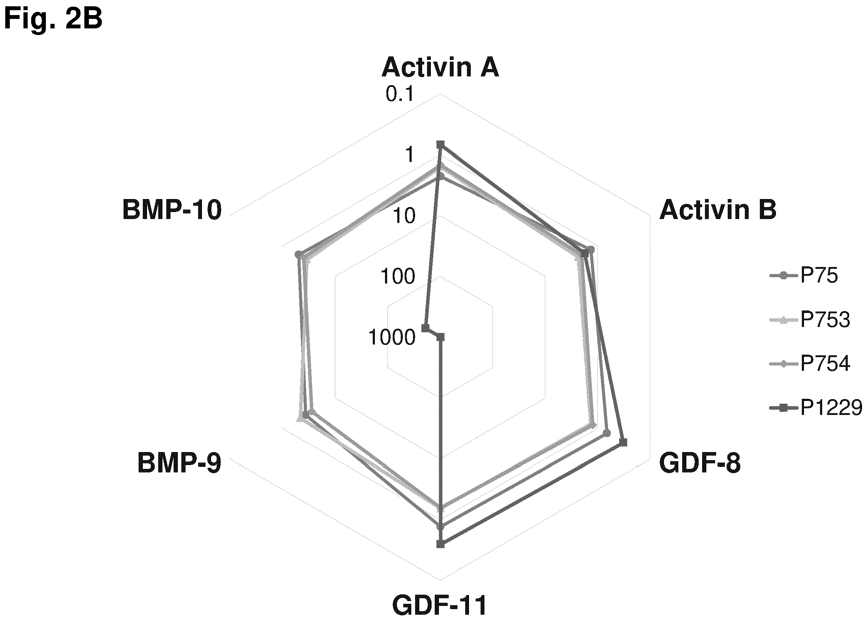

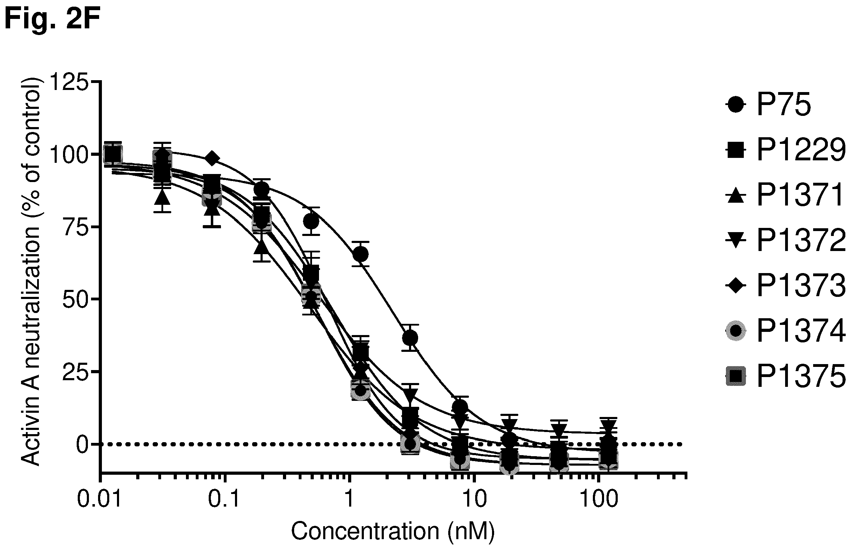

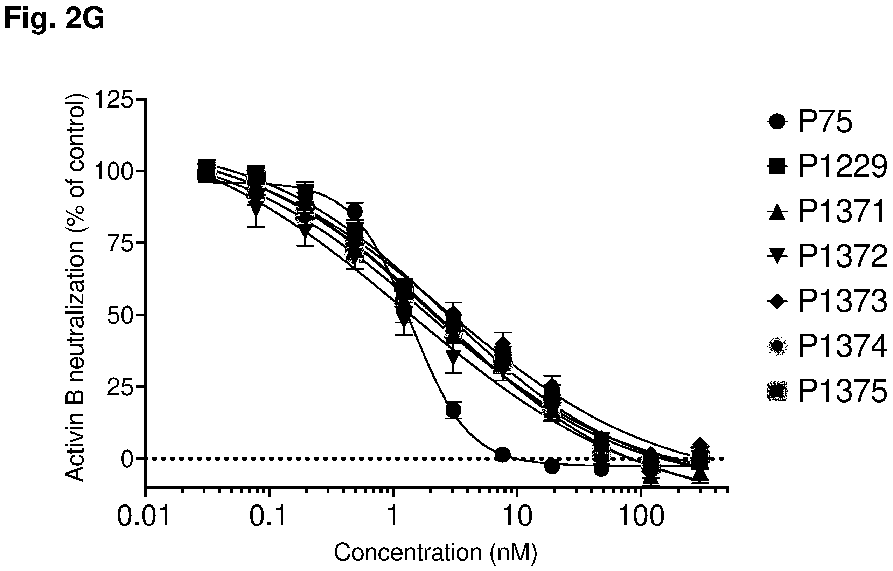

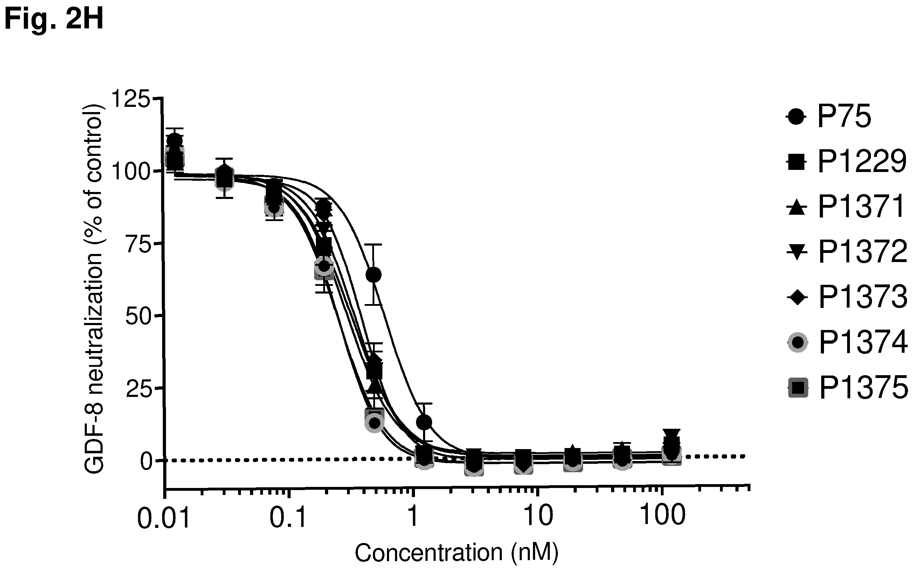

In order to assess the potency of these agents on TGFβ superfamily ligands known to be important in driving disease conditions, ActRIIB-ECD polypeptide variants were examined in the TGFβ-reporter HEK293 cells. Representative results for exemplary ActRIIB-ECD variants are shown in **FIGs. 2A-2I****.** Among these examples, P750, P751, P1229, and P1373 displayed increased potency on activin A compared to P75, with IC50 values of 0.76, 0.82, 0.70, and 0.53 nM, respectively (**FIGs. 2A-2C****,** **2F****,** and **Table 7**). P750 and P751 also showed increased potency relative to P75 on activin B, with IC50 values of 1.0 and 0.80 nM, respectively, relative to P75 (**FIG. 2A** and **Table 7**). In contrast, P1229 and P1373 showed comparable potency on activin B relative to P75, with IC50 values of 1.8 and 2.9 nM (**FIGs. 2B-2C****,** **2G** and **Table 7**). All four agents also demonstrated increased potency on GDF-8 compared to P75, with IC50 values of 0.58, 0.30, 0.31, and 0.46 nM, respectively; and on GDF-11, with IC50 values of 0.51, 0.25, 0.36, and 0.50 nM, respectively (**FIGs. 2A-C****,** **2H-2I****,** and **Table 7**). Other agents, notably P739, P753, and P754, displayed decreased potency relative to P75 on several ligands: activin B, with IC50 values of 6.0, 2.4, and 2.1 nM, respectively; GDF-8, with IC50 values of 2.0, 1.4, and 1.3 nM, respectively; and GDF-11, with IC50 values of 2.1, 1.5, and 1.6 nM, respectively (**FIGs. 2A-2B** and **Table 7**). Both P753 and P754 showed some increase in activin A potency, with IC50 values of 1.6 and 1.5 nM, respectively (**FIG. 2B** and **Table 7**). In contrast, P739 was less potent on activin A relative to P75, with an IC50 value of 3.5 nM (**FIG. 2A** and **Table 7**). These results indicate that it is possible to generate agents with distinct and unexpected neutralization profiles on activins and GDFs through mutation of the ActRIIB ECD.

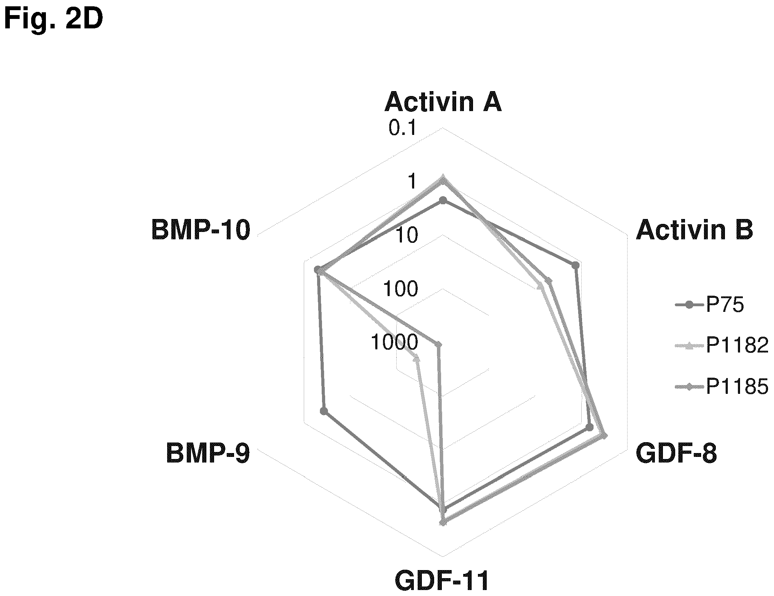

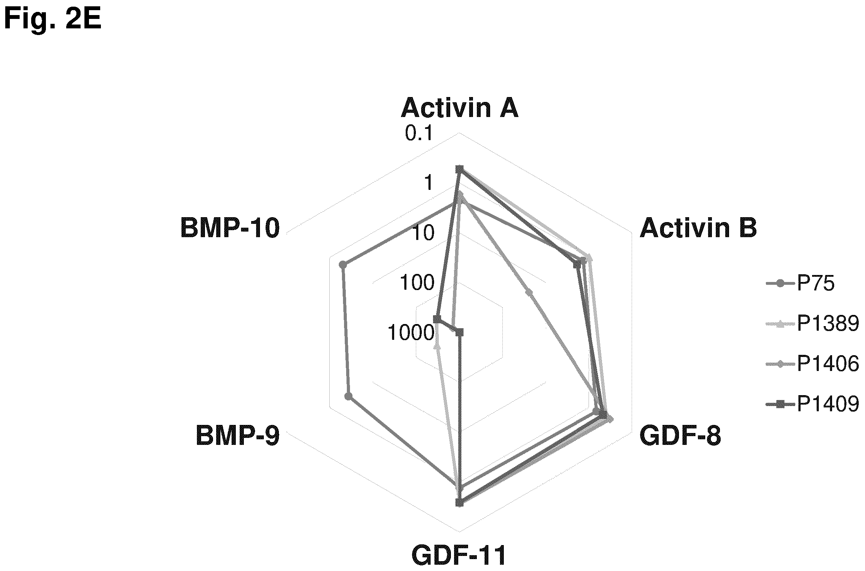

Additional exemplary agents with mutations in the ActRIIB ectodomain were further investigated: P1182, P1185, P1389, P1406, and P1409. All agents, excluding P1406, demonstrated increased potency compared to P75 on activin A, GDF-8, and GDF-11 (**FIGs. 2D-2E** and **Table 7**). Activin A: IC50 values of 0.85, 1.0, 0.52, and 0.55 nM for P1182, P1185, P1389, and P1409, respectively; GDF-8: IC50 values of 0.37, 0.33, 0.40, and 0.48 nM for P1182, P1185, P1389, and P1407, respectively; GDF-11: IC50 values of 0.46, 0.44, 0.39, and 0.39 nM for P1182, P1185, P1389, and P1407, respectively (**Table 7**). The agentP1406 gained potency on GDF-8 (IC50: 0.33 nM) and GDF-11 (IC50: 0.37 nM), but not on activin A (IC50: 1.7 nM) relative to P75 (**Table 7**). All agents with two mutations, except P1389, demonstrated decreased activin B potency relative to P75, with IC50 values of 7.7, 5.2, >20, and 1.9 nM for P1182, P1185, P1406, and P1409, respectively (**Table 7**). P1389 was the only agent with two mutations to show a potency gain on activin B relative to P75, with an IC50 value of 1.0 nM (**FIG. 2E** and **Table 7**).

The impact of modifications in the Fc sequence on activin A, activin B, GDF-8, and GDF-11 potency was investigated. For all ligands, P1371 and P1372 were comparable to P1229, and P1374 and P1375 were comparable to P1373 (**FIGs. 2B-2C**, **2F-2I**, and **Table 8**).

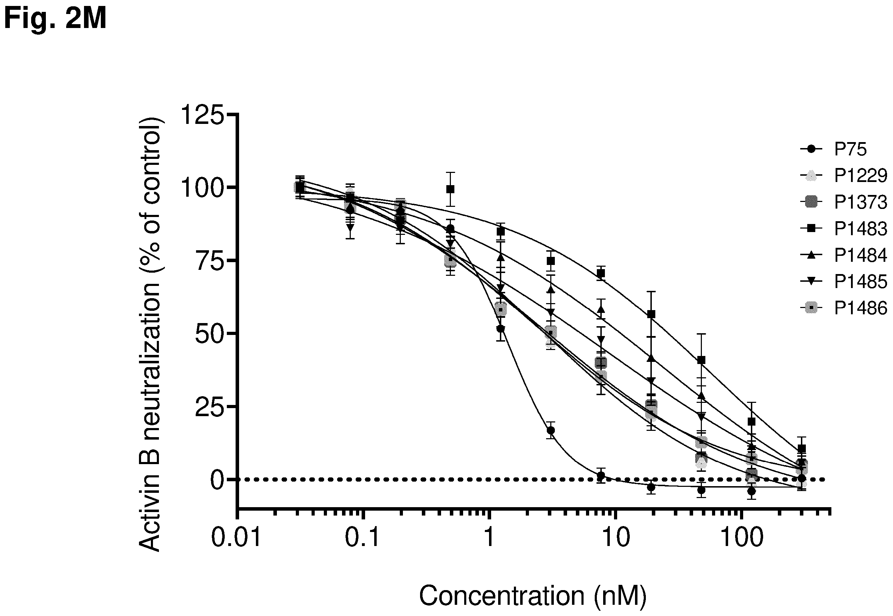

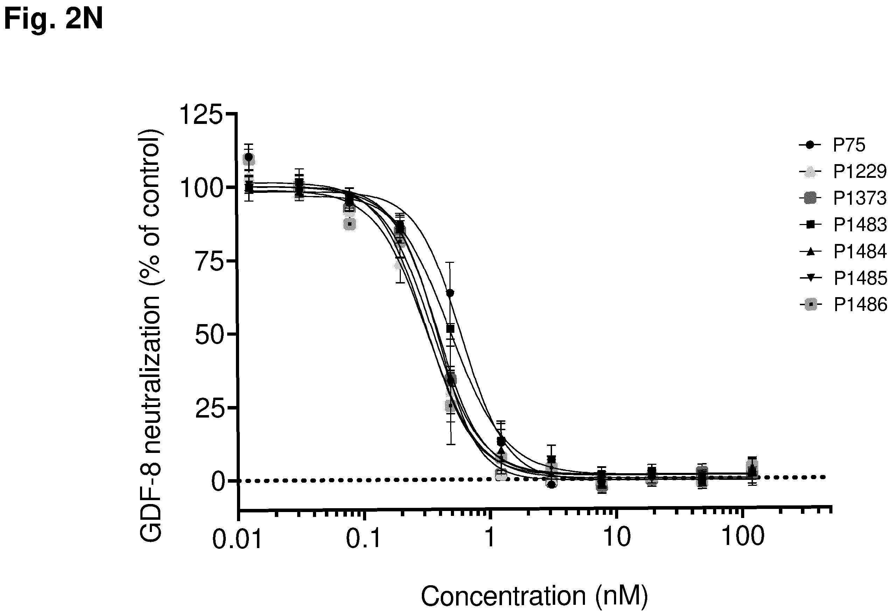

Finally, the impact of the length of the linker between the ectodomain and the Fc domain was investigated. Exemplary agents P1483, P1484, P1485, P1229, P1373, and P1486 contain linker length sequences of 3, 6, 10, 14, 19, and 36 amino acids, respectively. Potencies on activin A (**FIG. 2L**) and activin B (**FIG. 2M**) were linker dependent, i.e. activin A: IC50 values were 22, 5.1, 2.2, 0.70, 0.53, and 0.58 nM for P1483, P1484, P1485, P1229, P1373, and P1486, respectively; activin B: IC50 values were 57, 23, 8.8, 1.8, 2.9, and 2.2 nM for P1483, P1484, P1485, P1229, P1373, and P1486 (**Table 7**) . These data show that linker lengths of 3, 6, 10 and 14 amino acids resulted in a stepwise increase in potency on activin A and activin B. Beyond that length, there were no additional apparent gains in potency. This phenomenon was not observed with GDF-8 and GDF-11 (**FIGs 2N-2O**), where IC50 values were comparable between P1483, P1484, P1485, P1229, P1373, and P1486 (**Table 7**).

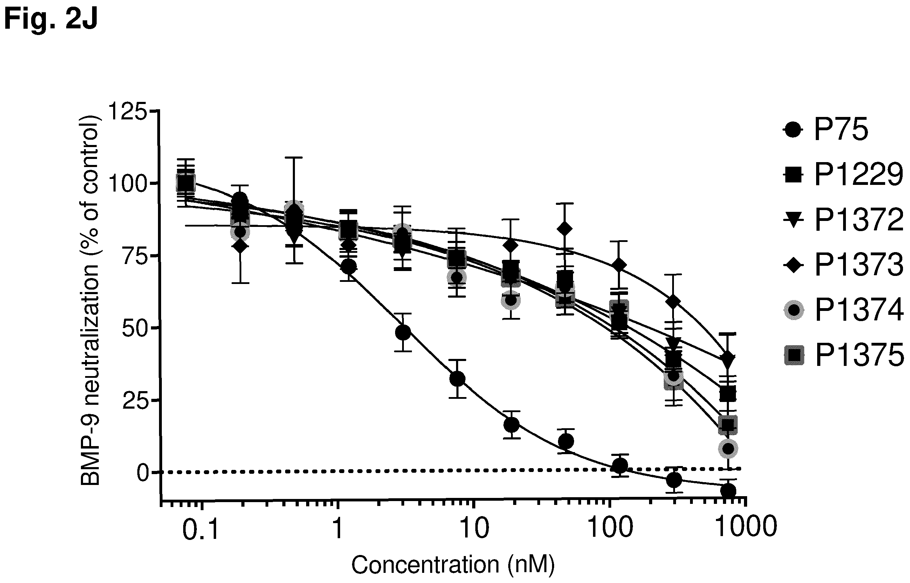

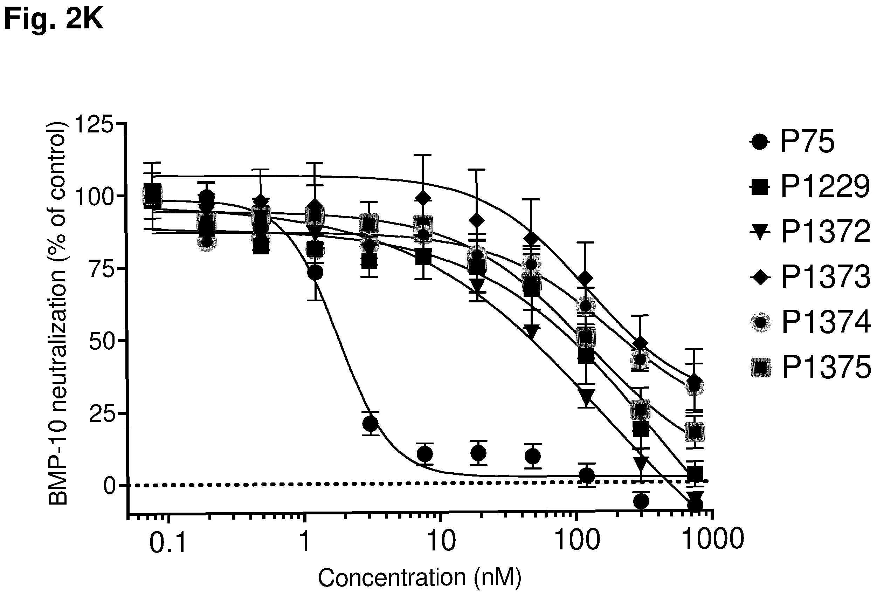

Characterization of the BMP-9 and BMP-10 neutralization activity of exemplary agents. The exemplary agents that were assessed for activin A, activin B, GDF-8, and GDF-11 neutralization were also tested in the BMP-reporter assays. The results obtained in these assays for the entire group of binding agents are presented in **FIGs. 2A-2E****,** **2J-2K**. Among these examples, P750, P751, P1229, and P1373 demonstrated significantly less neutralization of BMP-9 relative to P75, with IC50 values of 21, 18, >1,000, and >1,000 nM, respectively (**FIGs. 2A-2C****,** **2J** and **Table 8**). Moreover, these agents also demonstrated decreased potency on BMP-10, with IC50 values of 4.7, 2.4, 491, and 619 nM, respectively (**FIGs. 2A-2C****,** **2K****,** and **Table 8**). Two exemplary agents, P739 and P753, exhibited increased inhibition potency on BMP-9, with IC50 values of 1.1 and 2.1 nM, respectively, while having reduced inhibition potency on BMP-10, with IC50 values of 4.0 and 2.9 nM, respectively (FIGs. 2A-B and Table 4). Finally, one exemplary agent, P754, had negligible change in both BMP-9 and BMP-10 potency relative to P75, with IC50 values of 3.5 and 2.5 nM, respectively (FIG. 2B and Table 4). Agents P1182 and P1185 demonstrated decreased BMP-9 potency relative to P75 (IC50 values of 265 and 791 nM, respectively), with no changes in BMP-10 potency (IC50 values of 2.4 and 2.3 nM, respectively; **FIG. 2D** and **Table 8**). As for P1389, P1406 and P1409, both demonstrated minimal inhibition of either BMP-9 or BMP-10 (**FIG. 2E** and **Table 8**).

As was done with the other ligands, the impact of modifications in the Fc sequence on BMP-9 and BMP-10 potency was investigated. For both ligands, P1372 was comparable to P1229, and P1375 was comparable to P1373 (**FIGs. 2B-2C****,** **2J-2K** and **Table 8**).

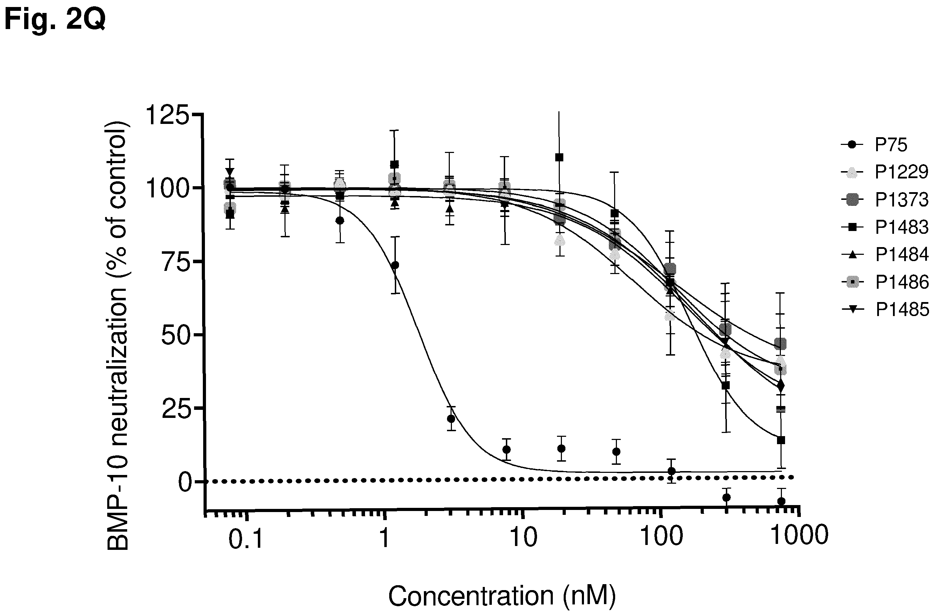

The impact of the length of the linker between the ectodomain and the Fc domain on BMP-9 and BMP-10 potency was also investigated. Exemplary agents P1483, P1484, and P1485, exhibited decreased apparent BMP-9 potency relative to P1229, P1373, and P1486 (**FIG. 2P**), though the IC50 differences could not be determined accurately (**Table 8**). All agents were still >100-fold less potent than P75 (ActRIIB-Fc wild-type). This was not observed with BMP-10, for which the potency was comparable between all six exemplary agents (**FIG. 2Q** and **Table 8**).

In summary, the results show that certain point mutations in the ActRIIB-ECD led to significant increases in inhibition potency for certain TGFβ superfamily ligands, particularly those shown to be involved in metabolic diseases, namely activin A, activin B, GDF-8, and GDF-11. Of note, the single point mutation in P750 and P751, and the double point mutations in P1182 and P1185, improved potency on activin A, activin B (except for P1182 and P1185), GDF-8, and GDF-11 relative to the wild-type ActRIIB-ECD (P75) by ~2-4-fold (**Table 7**), while decreasing the inhibition potency on BMP-9 by ~5-300-fold (**Table 8**); BMP-9 is considered a homeostatic ligand that should not be neutralized. For P750, P751, P1182, and P1185, inhibition potency on BMP-10 was unchanged or modestly reduced (**Table 4**). In contrast, the point mutation in P739 reduced neutralization of activin A, activin B, GDF-8, GDF-11, and BMP-10 by ~2-3-fold, while improving potency on BMP-9 by ~2-fold (**Tables 7** and **8**). Other point mutations (P753 and P754) reduced potency on most TGFβ superfamily ligands (e.g., activin B, GDF-8, GDF-11, BMP-9, and BMP-10) by ~2-fold, relative to P75, while specifically improving potency on activin A modestly, by ~1.5-fold (**Tables 7** and **8**). Furthermore, the single point mutation in P1229 and P1373, and double point mutations in P1409 improved potency on activin A, GDF-8, and GDF-11 relative to P75 by ~4-fold (**Table 7**), while simultaneously decreasing potency on both BMP-9 and BMP-10 by ~200-300-fold (**Table 8**). Unexpectedly, the single point mutation in P1229 and P1373 induced differential gains in potency on certain ligands, such as activin A and activin B, when the length of the linker was varied between the ectodomain and the Fc region. This was not the case for other ligands (for example, GDF-8 and -11). Taken together, the combination of the specific point mutation with an optimal linker length is required to provide the ideal ligand specificity profile for neutralization of disease-driving cytokines. Lastly, one exemplary test agent, P1406, demonstrated comparable activin A potency, increased GDF-8 and GDF-11 potency (~2-fold), and decreased activin B, BMP-9, and BMP-10 potency (~20-, ~300-, and ~350-fold, respectively) relative to P75. These data indicate that different point mutations (alone or in combination) had dramatically different and unexpected effects on the neutralization profile for TGFβ superfamily ligands.

**Table 7. IC₅₀ values for ectodomain constructs for activin A, activin B, GDF-8, and GDF-11 neutralization.**

| **Ectodomain construct** | **Activin A IC₅₀ (nM)** | **Activin B IC₅₀ (nM)** | **GDF-8 IC₅₀ (nM)** | **GDF-11 IC₅₀ (nM)** |
|---|---|---|---|---|
| P75 | 2.3 | 1.4 | 0.67 | 0.75 |
| P739 | 3.5 | 6.0 | 2.0 | 2.1 |
| P750 | 0.76 | 1.0 | 0.58 | 0.51 |
| P751 | 0.82 | 0.80 | 0.30 | 0.25 |
| P753 | 1.6 | 2.4 | 1.4 | 1.5 |
| P754 | 1.5 | 2.1 | 1.3 | 1.6 |
| P1229 | 0.70 | 1.8 | 0.33 | 0.39 |
| P1373 | 0.53 | 2.9 | 0.40 | 0.46 |
| P1371 | 0.56 | 2.7 | 0.23 | 0.60 |
| P1372 | 0.56 | 2.0 | 0.18 | 0.53 |
| P1374 | 0.53 | 1.9 | 0.37 | 0.40 |
| P1375 | 0.53 | 2.0 | 0.33 | 0.39 |
| P1182 | 0.85 | 7.7 | 0.37 | 0.46 |
| P1185 | 1.0 | 5.2 | 0.33 | 0.44 |
| P1389 | 0.52 | 1.0 | 0.40 | 0.39 |
| P1406 | 1.7 | > 20 | 0.33 | 0.37 |
| P1409 | 0.55 | 1.9 | 0.48 | 0.39 |
| P1483 | 22 | 57 | 0.49 | 0.58 |
| P1484 | 5.1 | 23 | 0.39 | 0.54 |
| P1485 | 2.2 | 8.8 | 0.35 | 0.53 |
| P1486 | 0.58 | 2.2 | 0.32 | 0.47 |

**Table 8. IC₅₀ values for ectodomain constructs for BMP-9 and BMP-10 neutralization.**

| **Ectodomain construct** | **BMP-9 IC₅₀ (nM)** | **BMP-10 IC₅₀ (nM)** |
|---|---|---|
| P75 | 3.0 | 2.0 |
| P739 | 1.1 | 4.0 |
| P750 | 21 | 4.7 |
| P751 | 18 | 2.4 |
| P753 | 2.1 | 2.9 |
| P754 | 3.5 | 2.5 |
| P1229 | >1,000 | >100 |
| P1373 | >1,000 | >100 |
| P1371 | NT | NT |
| P1372 | >1,000 | >100 |
| P1374 | NT | NT |
| P1375 | >1,000 | >100 |
| P1182 | 265 | 2.4 |
| P1185 | 791 | 2.3 |
| P1389 | >100 | >100 |
| P1406 | >1,000 | >100 |
| P1409 | >1,000 | >100 |
| P1483 | >1,000 | >100 |
| P1484 | >1,000 | >100 |
| P1485 | >100 | >100 |
| P1486 | >100 | >100 |

| | | |
|---|---|---|
| NT: not tested | | |

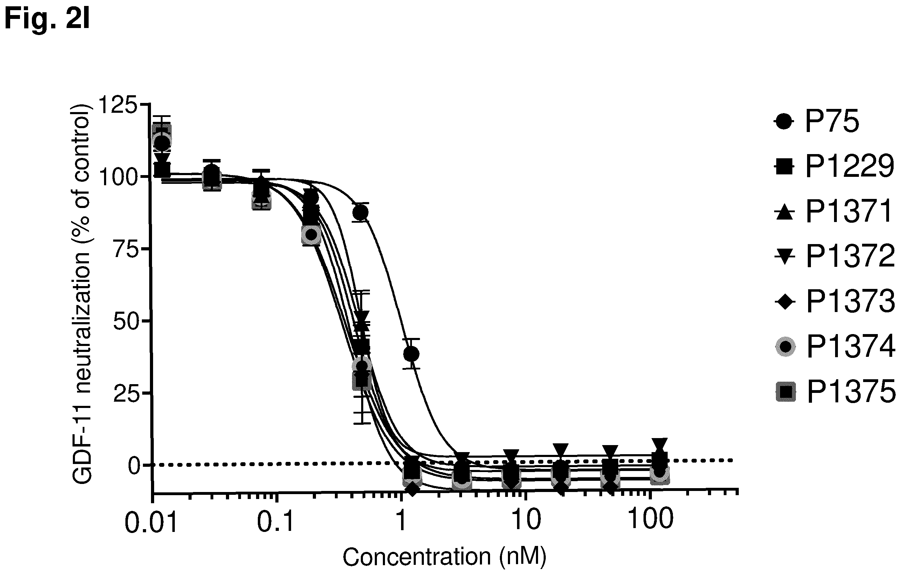

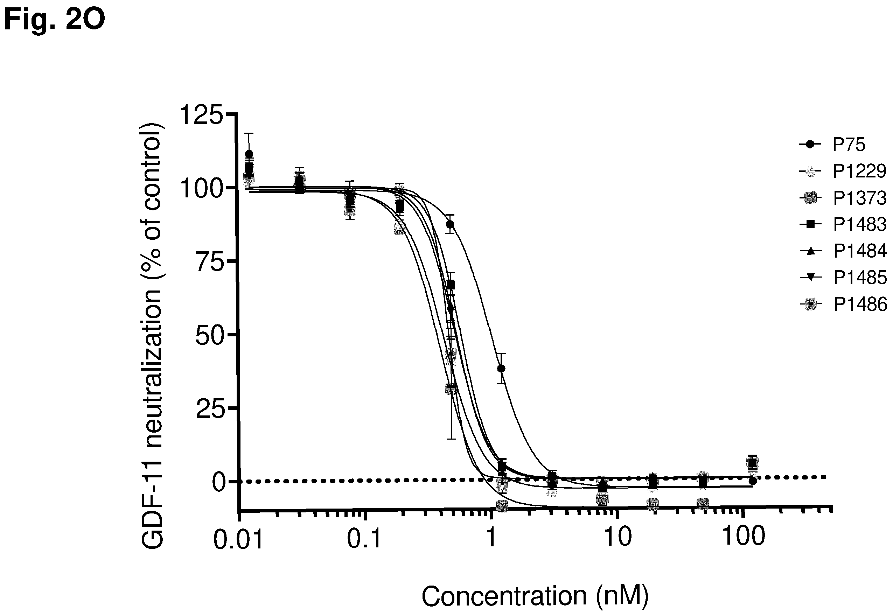

Additional exemplary agents were tested in binding assays to identify those with the desired ligand specificity profile, i.e., superior binding to activin A compared to the benchmark agent P75 (wild type ActRIIB-ECD-Fc), and decreased binding to BMP-9 and BMP-10 compared to P75. For activin A binding, our results showed that there was a broad spectrum of activin A binding potencies depending on the ActRIIB receptor modification. Several of the agents showed increased binding to activin A compared to P75 (i.e., decreased signal in the assay), whereas many did not bind to activin A as strongly as P75 (i.e., increased signal in the assay; see **FIG. 3**). For example, proteins P751, P1182, P1206, P1229, P1231, P1270, P1271, P1373, P1389, P1409, P1410, P1412, and others, had potent activin A binding. For P751, P1229, P1373, P1389, and P1409, this increased activin A binding relative to P75 is in agreement with its increased neutralization potency on activin A (**FIG. 2** and **Table 3**). On the other hand, P777, P1174, P1177, P1230, P1272, P1273, and others, had lower binding to activin A relative to P75. Moreover, some exemplary agents showed comparable binding to activin A such as P75, e.g., P1175, P1178, P1179, P1237, or P1275. Of note, P444 (wild-type ActRIIA-ECD-Fc) had a comparable activin A binding profile to P75 in this assay.

Our results also showed varying levels of BMP-9 binding depending on the ActRIIB-ECD modification. Of note, in the BMP-9 assay, P444 had near undetectable binding to BMP-9. All exemplary agents tested displayed decreased binding to BMP-9 as compared to P75 (i.e., decreased signal; see **FIG. 4**). Some proteins displayed near-undetectable binding to BMP-9 (like P444), such as P1273, P1372, P1373, P1374, P1375, P1389, P1406, P1409, and others (**FIG. 4**). Other examples of proteins with very low BMP-9 binding were P1181, P1229, P1239, and P1275. In contrast, some exemplary proteins demonstrated intermediate binding between P444 and P75, such as P1206, P1231, P1232, P1272, P1412, and others. For P751, P1229, P1373, P1389, P1406, and P1409, this decreased BMP-9 binding relative to P75 is in agreement with its decreased neutralization potency on BMP-9 (**Table 4**).

For BMP-10 neutralization, selected agents were analyzed for neutralization potency in the BMP-reporter cell-based assay (**FIG. 5A**) and/or for binding by bio-layer interferometry (BLI; **FIG. 5B**). At the concentration tested, P444 demonstrated less neutralization (i.e., greater signal) than P75 (**FIG. 5A**). In agreement with this, it exhibited a higher K_{D} (lower affinity) than P75 (**FIG. 5B**). In the cell-based assay, all agents tested demonstrated lower potency on BMP-10 than P75 (**FIG. 5A**). In agreement with this, by BLI, all agents exhibited higher K_{D}s than P75 (**FIG. 5B**). Results for exemplary agents P1371-P1392 and P1395-P1396 were well correlated between the two assays (**FIG. 5A** vs **FIG. 5B**). Other exemplary agents P1406-P1412 demonstrated comparable or greater K_{D} values relative to P444 (**FIG. 5B**).

Taken together, the results presented above show that certain exemplary agents have a desired ligand specificity profile, i.e., superior binding activity to activin A and significantly reduced binding and/or neutralization activity on BMP-9 and BMP-10. Such agents, including P750, P751, P1184, P1185, P1229, P1236, P1371, P1372, P1373, P1374, P1375, P1389, P1406, and P1409, are predicted to be useful in conditions where it is beneficial to inhibit the activity of TGFβ superfamily ligands that drive disease while not interfering with the activity of ligands associated with homeostasis.

### Example 3. Effects of exemplary agents in vivo on body weight gain.

To investigate the target engagement of exemplary ActRIIB-ECD polypeptide constructs *in vivo,* mice (n = 6/group) were injected with given agents (25 mg/kg, subcutaneous injection), and sacrificed after 4 days to assess body weight gain. Most agents tested (P75, P1185, P1373, and P1409) induced a statistically significant body weight gain over the course of 4 days, with P1409 exhibiting the greatest average weight gain (**FIG. 6**). Three agents, P1229 (p-value of 0.07), P1389, and P1406 induced an apparent body weight gain that did not reach statistical significance (**FIG. 6**).

Additional *in vivo* evaluation of exemplary agents, P750 and P1229, was conducted and compared to P75. P750 (1, 10, and 25 mg/kg), P1229 (5 and 25 mg/kg), and P75 (25 mg/kg) were injected subcutaneously twice weekly in wild-type male mice (n = 6/group), over the course of 11 days (P750; **FIG. 7A**) or 7 days (P1229 and P75; **FIG. 7B**). P750 (10 and 25 mg/kg), P1229 (5 and 25 mg/kg), and P75 (25 mg/kg) induced a significant body weight gain as early as Day 3 (between 5 and 10%), up until the end of the study (**FIGs. 7A-7B**). P750 at 1 mg/kg demonstrated a modest increase in body weight, which only reached statistical significance on Day 7 (**FIG. 7A**).

These data exemplify that the desired ligand specificity profile established *in vitro* translated to a significant body weight gain *in vivo* and show that most exemplary agents tested in these experiments demonstrated successful target engagement *in vivo.*

### Example 4. Exemplary agent effects on body composition and metabolism in lean mice

The effects of P1229 on body composition and metabolism in lean mice were further assessed. After seven days (twice weekly injections), P1229 and P75 at 25 mg/kg significantly increased tibialis anterior (**FIGs. 9A-B**) and gastrocnemius weight (**FIGs. 9C-D**), respectively.

Additional assays were performed to assess the relative expression level of *Mss51, a* skeletal muscle-specific gene. *Mss51* is a key target of myostatin signaling, with a role in fatty acid oxidation, glycolysis, and oxidative phosphorylation. Treatment with P1229 led to a dose-dependent decrease in *Mss51* expression in the tibialis muscle relative to vehicle (**FIG. 10**). The effects of P1229 were comparable to those of P75 (**FIG. 10**).

### Example 5. Exemplary agent effects on body composition and metabolism in obese mice

Experiments were performed to assess the effects of P1229 on body composition and metabolism in diet-induced obese (DIO) mice. DIO mice were purchased from Jackson Laboratories (#380050). Briefly, mice were fed a high-fat diet (HFD, 60% kcal from fat) from 6 weeks of age until 22 weeks of age (total of 16 weeks on the HFD). After a two-week acclimatization period, mice (n=8/group) were injected subcutaneously with vehicle, P75 (25 mg/kg twice weekly), P1229 (25 mg/kg twice weekly), P1373 (5, 20, or 50 mg/kg twice weekly), or 20 mg/kg of CDD866 (P1307), a murine version of bimagrumab (an antibody targeting ActRIIB and ActRIIA; once weekly, subcutaneously, as previously described in Morvan et al. (2017) PNAS 114(47): 12448-12453). After 3 weeks of treatments, some animals were sacrificed and scanned by EchoMRI (SkyScan1176) (**FIGs. 11A-F**), while others were sacrificed for tissue collection and RNA analysis. Animals were maintained on the HFD throughout the acclimatization and treatment periods. The murine version of bimagrumab used for these experiments comprises the heavy chain amino acid sequence of SEQ ID NO: 376 and the light chain sequence of SEQ ID NO: 377.

Importantly, treatment with P1229 or P1373 resulted in fat loss with concomitant lean mass gain. Treatment with bimagrumab, P1307 (CDD866), or P75 **FIGs. 11A-F****)** also promoted lean mass gain but less fat loss, with the result that the ratio of lean mass to fat mass was higher in the P1229- and P1373-treated animals. Fat loss with P1229 and P1373 was not caused by decreased food consumption (**FIGs. 12A-B**). As for individual skeletal muscles, all treated groups showed significant increases or trends towards increased muscle weight (**FIGs. 12C-H**).

Furthermore, treatment with P1229, P1373 and P1307 (but not P75), improved muscle metabolism as demonstrated by reduced *Mss51* gene expression in gastrocnemius muscle (**FIGs. 13A-B**). Additionally, P1373 led to a lower *Serpinel:Id1* expression ratio in soleus muscle (**FIG. 13C**); this ratio is indicative of reduced activin/GDF signaling *(Serpinel)* and greater BMP signaling (*Id1*).

Next, hepatic parameters were assessed in DIO animals treated with P75, P1229, P1373, or P1307. Exemplary agents P1229, P1373, and P1307, but not P75, reduced *Ahsg* expression (which encodes fetuin-A, a marker of obesity and decreased insulin sensitivity, See Mathews et al (2002) 10.2337/diabetes.51.8.2450) (**FIGs. 14A-B**), as well as *Fgf21* expression (a marker of increased triglycerides, adiposity, and insulin resistance, See Hale et al, Endocrinology, Volume 153, Issue 1, 1 January 2012, Pages 69-80) (**FIGs. 14C-D**). Finally, P1373 and P1307 (in one of the two studies) significantly decreased *Inhbe* expression (**FIGs. 14E-F**), which encodes activin E, a TGF-β superfamily member linked to obesity (See Akbari et al. Nat Commun 13, 4844 (2022) and Deaton et al. Nat Commun 13, 4319 (2022)).

Although bimagrumab decreased fat mass while preserving lean body mass in a phase 2 clinical trial (Heymsfield et al (2021) JAMA Netw Open), its mechanism of action may limit its muscle anabolic potential relative to that of a ligand trap such as ramatercept (ActRIIB-Fc) or the exemplary agents disclosed herein. Without wishing to be bound by theory, the relatively low affinity of ActRIIB-based traps for classic BMPs, (*e.g.*, BMP-2/4), which promote muscle anabolic effects, suggest that an ActRIIB-based trap may act primarily on activin A/GDF-8/GDF-11 without neutralizing positive BMP signaling to muscle. In contrast, the ability of BMPs to engage the cell surface activin type 2 receptors, which are the targets of bimagrumab, may render BMP signaling to muscle at least partially susceptible to inhibition by bimagrumab (see Lee et al. (2023) The Journals of Gerontology). Thus, the therapeutic strategy based on ActRIIB-Fc ligand traps, such as employed by the exemplary agents disclosed herein, may present advantages over direct targeting of the activin type 2 receptors (as done by bimagrumab).

### Example 6. Exemplary agents affect FSH production in obese mice

Follicle-stimulating hormone (FSH) is a known biomarker of *in vivo* target engagement for activin receptor-based traps (*See* Attie et al. (2013). Muscle Nerve, 47: 416-423). To further validate *in vivo* target engagement for exemplary agents, FSH production was evaluated in DIO mice, as described above. Agents P1229, P1373, and P1307, but not P75, demonstrated significant decreases in FSH production in DIO mice following 3 weeks of treatment (**FIGs. 15A-B**). Notably, P1373 demonstrated progressive dose-dependent target engagement between 5 and 50 mg/kg (**FIG. 15B**).

### Example 7. Exemplary agents demonstrate efficacy in a rat PAH model

In order to test the efficacy of exemplary agents in PAH, two rat models may be used. In the first model, male Sprague-Dawley rats (7-9 weeks of age) are injected with monocrotaline (MCT; 60 mg/kg) on Day 0. The next day, animals are randomly distributed between treatment groups with exemplary agents and injected twice weekly subcutaneously for four weeks. In the second model, male Sprague-Dawley rats (7-9 weeks of age) are injected with SUGEN5416 (20 mg/kg) on Day 0, followed by housing under normobaric hypoxia. After three weeks, rats are transferred back to normoxia and are injected with exemplary agents twice weekly subcutaneously for three weeks. Other variants of this model include a) combining the hypoxia and treatment phases, for a total duration of four weeks; b) adding a recovery period of two weeks at normoxia prior to the onset of treatment, while increasing the treatment phase to four weeks; and c) same paradigm as described in b), adding an additional 4 weeks of recovery after the end of the treatment period.

In both models, exemplary agents are injected at between 0.1-100 mg/kg, e.g., at 5, 10, and/or 20 mg/kg. At the end of the study, echocardiographic and hemodynamic assessments in the right ventricle (RV) are made for all animals. Lungs are also collected to investigate pulmonary remodeling and vessel muscularization.

### Example 8. Exemplary agents demonstrate efficacy in a mouse heart failure with reduced ejection fraction (HFrEF) model

In order to test the efficacy of exemplary agents in HFrEF, the murine transverse aortic constriction (TAC) model was used. Male C57BL/6 mice (8-10 weeks of age) underwent TAC surgery (27G needle size) on Day 0. Two weeks later, the animals were randomized into treatment groups based on their echocardiographic profiles. Exemplary agents were injected twice weekly, subcutaneously, for four weeks, at dose levels of 1.5, 5, and 15 mg/kg. At the end of the study, echocardiographic parameters were assessed to look at the efficacy of exemplary agents on left ventricle (LV) function. The lungs were also collected to assess pulmonary remodeling and vessel muscularization.

Total heart weight was significantly increased after TAC surgery confirming that the TAC mice showed evidence of heart failure compared to the Sham-Vehicle group (**FIGS. 17A-B**). Importantly, the highest dose level of P1436 showed a significant reduction in total heart weight (normalized with either tibia length or body weight) that was not statistically different from the weight of the sham-operated group (**FIGS. 17A-B**, respectively). Consistent with these results, left ventricle mass (LVM) was significantly increased in the TAC-vehicle group compared to the sham-operated animals (**FIG. 18A**). Treatment with P1436 led to a dose-dependent decrease in LVM with the largest decrease observed at the 15 mg/kg dose level. Furthermore, when comparing the LVM individually at baseline (Day -2, set to 100%) to the LVM on Day 28, virtually all animals in the TAC-vehicle group showed an increase in LVM (**FIG. 18B****,** second panel from the left). In the TAC animals treated with P1436, the number of animals in each group showing a smaller percent change in LVM between Days - 2 and 28 increased in a dose-dependent manner, with several exhibiting negative percent changes (third, fourth, and fifth panels in **FIG. 18B**). In addition, a significant correlation between the reduction of LVM and the serum concentration of P1436 on Day 28 was observed (**FIG. 18C**). Similar results were seen with intra-ventricular septum diameter at diastole (IVSd) (**FIGS. 18D-F**). These echocardiographic results indicate that P1436 was efficacious in reducing TAC-induced cardiac hypertrophy in a dose-dependent manner.

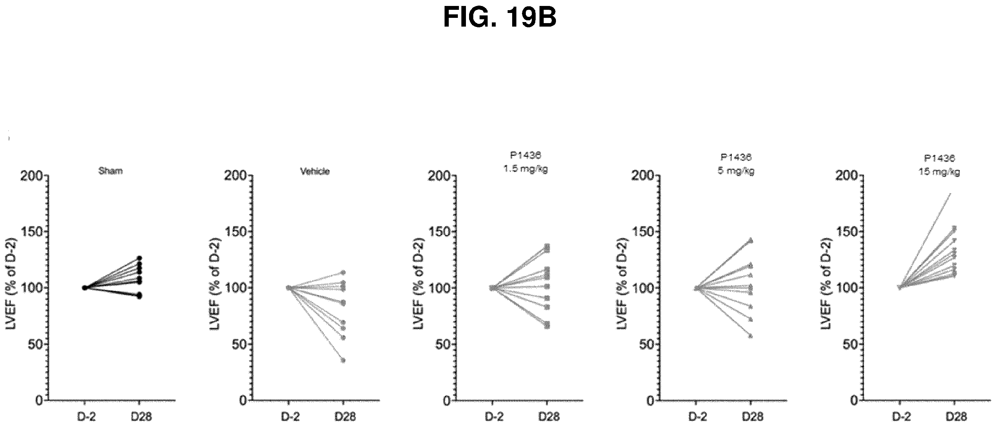

In addition, left ventricle ejection fraction (LVEF) was significantly decreased in the TAC-vehicle group compared to the sham-operated animals (**FIG. 19A**). There was a dose-dependent increase in LVEF in all P1436 treatment groups, with the 15 mg/kg group showing a LVEF value close to that seen in the sham-operated group (**FIG. 19A**). When comparing the LVEF measured on Day -2 (baseline, set at 100%) to the LVEF on Day 28 (end of study), P1436 induced a shift in the direction of the changes, i.e., from the downward-trending sham-vehicle animals to the upward-trending P1436 groups, especially in the high-dose group where all animals showed an increase in LVEF from Day -2 to Day 28 (**FIG. 19B**). As was seen in the cardiac hypertrophy results, there was a significant correlation between the percent increase in LVEF and the serum concentration of P1436 on Day 28 (**FIG. 19C**). Similar results were obtained with LV fractional shortening (FS) (**FIGS. 19D-F**). P1436 also improved other echocardiographic parameters, such as end systolic diameter (ESD), end diastolic diameter (EDD), end systolic volume (ESV) and end diastolic volume (EDD) (**FIGS 20A-D**). These echocardiographic results show that P1436 was efficacious in improving cardiac functional readouts in a dose-dependent manner.

TAC mice also showed an increase in average lung weight relative to the sham group (**FIGS. 21A-B**). Decreased lung weights were observed in all P1436-treated groups, with the highest dose level achieving close to the normal weights seen in sham-operated mice (**FIGS. 21A-B**). TAC also led to significant remodeling of pulmonary vessels, with an increase in the percentage of fully muscularized vessels mirrored by a decrease in the percentage of non-muscularized vessels (**FIG. 22**). P1436 partially rescued these effects, particularly at 5 mg/kg (**FIG. 22**).

In the TAC model, serum concentration of NT-proBNP, a biomarker associated with heart failure, was elevated at Day 28 (**FIG. 23**). Treatment with P1436 led to reductions in NT-proBNP across all dose levels (**FIG. 23**).

Parameters demonstrating target engagement of P1436 included body weight (BW), skeletal muscle (gastrocnemius, tibial anterior and soleus) weights, and circulating follicle-stimulating hormone (FSH) levels. Treatment with P1436 significantly and dose-dependently increased body weight (**FIG. 24A**) and skeletal muscle weights (**FIGS. 24B-D**). These observations indicate a positive effect on body composition. Finally, P1436 significantly and dose-dependently decreased circulating FSH levels (**FIG. 25**), an indicator of *in vivo* target engagement.

Taken together these data indicate that treatment with P1436 significantly ameliorated the cardiac and lung disease phenotypes in a murine model of HFrEF, with P1436 having an additional positive effect on body composition.

### Example 9. Exemplary agents demonstrate efficacy in a mouse heart failure with preserved ejection fraction (HFpEF) model

In order to test the efficacy of exemplary agents in HFpEF, two different murine models may be used. In the first model, male C57BL/6N mice (8-10 weeks of age) are fed a high-fat diet (HFD) and given L-NAME through the drinking water (0.5 g/L) *ad libitum.* Animals are maintained on this diet for 10 weeks, following which they are randomized into treatment groups based on their echocardiographic profiles. In the second model, female C57BL/6N mice (18 months of age) are fed a HFD for 8 weeks, at which point they undergo surgery and are implanted with a pump infusing angiotensin-II (1.44 ng/kg/day) for the rest of the study.

In both models, following the induction period, exemplary agents are injected twice weekly, subcutaneously, for four weeks, at doses of between 0.1-100 mg/kg, e.g., of 3, 10, and/or 30 mg/kg. Animals are still fed HFD during the treatment period (as well as L-NAME for the first model). At the end of the study, hemodynamic and echocardiographic parameters are assessed to look at the efficacy of exemplary agents on LV and RV function. Pulmonary congestion, exercise tolerance, and body composition are other key readouts to assess the efficacy of exemplary agents in HFpEF.

### Example 10. Administration of P1436 Improves Body composition in Mice Model of Heart Failure with preserved Ejection Fraction (HFpEF)

HFpEF mouse model was administered either P1436 or a vehicle control. The vehicle control was also administered to mice without HFpEF. The % fat mass and % lean mass was assessed in the three groups of mice. As shown in **FIG. 16A**, administration of P 143 6improved the body composition - that is, decreased the % fat mass and increased the % lean mass - in the mice model of HFpEF.

### Example 11. Administration of P1436P1372 Restores Left Ventricle Function and Improves Exercise Tolerance in Mice Model of Heart Failure with preserved Ejection Fraction (HFpEF)

HFpEF mouse model was administered either P1436 or a vehicle control. The vehicle control was also administered to mice without HFpEF. The left ventricular end-diastolic pressure (LVEDP) and exercise tolerance (measured by distance covered) was assessed in the three groups of mice. As shown in **FIG. 16B**, administration of P1436 to mice model of HFpEF restored the left ventricle function by decreasing the LVEDP, and improved exercise tolerance by increasing the distance covered.

### Experimental Procedures for Examples

*Production, Purification and Characterization of ActRIIB-ECD fusion proteins that neutralize TGFβ superfamily ligands.* All constructs included a secretion signal sequence MDWTWRILFLVAAATGTHA (SEQ ID NO: 1) at the N-terminus when expressed. The complementary cDNAs coding for constructs were prepared synthetically (Genscript, Piscataway, NJ). The cDNAs were cloned into the EcoR1 (5' end) and BamH1 (3' end) of the pTT5 mammalian expression plasmid vector (Durocher et al., 2002). The signal peptide is cleaved off in the cells during expression and was not included in the purified fusion proteins.

Fusion proteins were expressed by transient transfection of Chinese Hamster Ovary cells (ExpiCHO^{™}, Thermo Fisher, Waltham, MA) at 37°C, 8% CO₂. Transfection conditions were as follows: DNA was transfected according to the manufacturer's instructions (100% fusion protein expression plasmid, 0.5 µg/ml, following the recommended volumes for transfection at various scales from Gibco ExpiCHO^{™} Expression System user guide). At 18-22 hours post-transfection, feed was added (ExpiCHO^{™} feed) and ExpiFectamine CHO Enhancer, and the culture was transferred to a 32°C, 5% CO₂ incubator. This incubation was conducted to promote the production and secretion of the fusion protein. The culture was maintained for 6 days post-transfection (dpt) after which the cultures were harvested. In some instances, fusion proteins were expressed in Expi293^{™} (Thermo Fisher, Waltham, MA) cells following procedures similar to those described above. The harvest supernatant was filtered (0.22 µm) and purified by affinity chromatography using an Akta pure 25L (GE). Briefly, the supernatant was loaded onto an MabSelect PrismA protein A column, the column was washed with eight column volumes of PBS and the protein was eluted with five column volumes of 0.1 M sodium citrate, pH 3.2. Fractions containing the purified protein were pooled and buffer exchanged into formulation buffer (20 mM L-Histidine, 100 mM NaCl, pH 7.0) using a HiPrep 26/10 desalting column (GE). The protein was concentrated with a 30 kDa Amicon filter, followed by sterile filtration with a 0.22 um filter prior to further downstream applications.

Polyacrylamide gel electrophoresis (SDS-PAGE) analysis of the fusion proteins was carried out under both reducing and non-reducing conditions. Proteins (P) were electrophoresed on a 4-12% Bis-Tris acrylamide gel (NuPAGE^{™} 4-12% Bis-Tris Protein Gels, Cat# NP0321BOX, Life Technologies). To visualize the proteins, the gels were stained using Coomassie Blue (see FIG. 1A-1B).

Given that protein aggregation can be indicative of reduced conformational stability and decreased activity, Size-Exclusion Chromatography-High Performance Liquid Chromatography (SEC-HPLC) was used to determine the purity of each ActRIIB-ECD construct. This method measured the percentage of intact monomeric species as well as the presence of aggregates and/or degradation products.

All proteins expressed efficiently in CHO and/or 293 cells and purification efficiency of each using Protein A affinity chromatography was similar. Although many of the properties were very similar between variants, the potential for aggregation (or high molecular weight (HMW) content), as measured by SEC-HPLC, revealed some distinctions, which could be indicative of improper folding. For the benchmark protein, wild type ActRIIB-ECD-Fc (P75), the purity (i.e., percent monomer content) was 84% monomer and 16% high HMW content. Some of the exemplary agents had aggregate levels comparable to those of P75, namely P739 and P753 with 86 and 84% monomer content, respectively, and corresponding HMW content of 14 and 16%. Strikingly, other agents produced under the same conditions displayed much higher monomer content compared to P75 as exemplified by P750, P751, P754, P1182, P1185, P1229, P1371, P1372, P1373, P1374, P1375, P1389, P1406, and P1409 that showed 96, 98, 93, 97, 96, 97, 99, 99, 98, 99, 99, 97, 97, and 98% monomer content, respectively, and correspondingly lower HMW at 4, 2, 7, 2, 3, 3, 1, 1, 2, 0, 0, 3, 3, and 2% HMW.

These results demonstrate that point mutations in the ActRIIB ectodomain had unpredictable and unexpected consequences on the manufacturability profile of the variant. In particular, P750, P751 and P754 showed a superior monomer content compared to the Fc fusion containing the wild type receptor ectodomain (P75).

*Potency testing by neutralization on cell-based assays.* Characterization of the activin A, activin B, GDF-8, and GDF-11 inhibition potency (IC₅₀) of the fusion proteins was performed using a cell-based reporter assay. TGFβ-reporter HEK293 cells were obtained from InvivoGen (San Diego, CA, cat. no. hkb-tgfb) and were maintained following the manufacturer's instructions. Briefly, cells were grown in high-glucose DMEM, containing 10% heat-inactivated FBS, 30 µg/mL blasticidin, 250 µg/mL hygromycin B, and 100 µg/mL zeocin (hereafter, "growth medium"). Once the cells reached 70-80% confluence, they were detached in growth medium by gently tapping on the flask. For all assays, cells were seeded in 96-well plates at a density of 20,000 cells per well in growth medium. Cells were left to attach for 10 minutes at room temperature (RT) prior to being transferred to a 37°C incubator (5% CO₂) overnight. The next day, treatments were prepared by combining the appropriate concentration of agent with activin A, activin B, GDF-8, or GDF-11 for 30 min at RT (shaking at 150 rpm). Treatments were conducted in high-glucose DMEM (serum-free and with no selection agents), and the final concentration of cytokine (ligand) was 0.3 nM. Growth medium was removed from the culture plates, and cells were treated with the appropriate mixes of fusion protein and cytokine. Treatment duration was for 18-22 h at 37°C (5% CO₂). All treatments were performed in technical duplicates and at least one or two independent experiments were conducted for each cytokine. At the end of the treatment period, supernatants were transferred to a new plate, and centrifuged at 300 RPM for 3 min. Forty microlitres of supernatant from each condition was transferred to a new plate, into which 180 µL of Quanti-Blue reagent (InvivoGen, cat. no. rep-qbs) was added. Quanti-Blue reagent was prepared following the manufacturer's instructions. Samples were incubated for 45 min at 37°C. At the end of the incubation, absorbance was read at 650 nm. The data were plotted in GraphPad Prism, which was used to calculate IC₅₀ values as a non-linear regression.

Characterization of the BMP-9 and BMP-10 inhibition potency (IC₅₀) of the fusion proteins was performed using a second cell-based reporter assay. Human hepatic HepG2 cells were transfected with a SEAP-encoding plasmid, in which *SEAP* expression is under the control of the murine *Id1* promoter. A stable pool was generated from this transfection. Cells were grown in EMEM, containing 10% heat-inactivated FBS and 200 ug/mL zeocin (hereafter, "HepG2 growth medium"). Once cells reached 70% confluence, they were washed with PBS and detached using 0.25% trypsin. For all assays, cells were seeded in 96-well plates at a confluence of 100,000 cells per well in HepG2 growth medium. Cells were left to attach for 10 minutes at RT prior to being transferred to a 37°C incubator (5% CO₂) overnight. The next day, treatments were prepared in EMEM (2.5% heat-inactivated FBS and with no selection agents) by combining the appropriate concentration of agent with BMP-9 or BMP-10 for 30 min at RT (shaking at 150 rpm). Treatments were conducted in EMEM (2.5% heat-inactivated FBS and with no selection agents), and the final concentration of BMP-9 and BMP-10 was 2 ng/mL and 40 ng/mL, respectively. HepG2 growth medium was removed from the culture plates, and cells were treated with the appropriate mixes of fusion protein and cytokine. Treatment duration was for 20-24 h at 37°C (5% CO₂). All treatments were performed in technical duplicates and at least one or two independent experiments were conducted for each cytokine. At the end of the treatment period, supernatants were transferred to a new plate, and centrifuged at 300 RPM for 3 min. Forty microlitres of supernatant from each condition was transferred to a new plate, onto which 180 µL of Quanti-Blue reagent was added. Quanti-Blue reagent was prepared following the manufacturer's instructions. Samples were incubated for 5-6 h with Quanti-Blue reagent at 37°C. At the end of the incubation, absorbance was read at 650 nm. The data were plotted in GraphPad Prism, which was used to calculate IC₅₀ values as a non-linear regression.

*Small-scale testing of ActRIIB-ECD fusion proteins.* Additional investigation of a series of exemplary agents was performed using proteins expressed in CHO cells. Briefly, DNA encoding the ActRIIB-ECD-Fc fusion constructs was transfected in cells seeded in 12-well plates in a final volume of 1 ml. After 7 days, the supernatant was harvested, clarified, and the amount of expressed protein in each well was quantified using standard methods. The potency of each agent for inhibition of activin A, BMP-9 and BMP-10 was determined in three separate assays.

To determine potency on activin A, a 96-well plate was coated overnight at 4°C with a solution of anti-activin A antibody (R&D #MAB3381, 0.25 µg/mL in a carbonate-bicarbonate buffer, 100 µL/well). The next day, the plate was washed three times with buffer (Biolegend #421601). Blocking buffer (5% BSA in PBS, 200 µL/well) was added to each well and the plate was incubated for 1 hour at RT. Expressed proteins in the supernatants described above were diluted to 2 µg/mL in assay diluent (0.5% BSA, 0.05% tween-20 in PBS), mixed 1: 1 with recombinant human activin A (R&D #338-AC, 60 ng/mL, diluted in assay diluent). Complexes were immediately added to the 96-well plate in technical duplicates and incubated for 1 h at RT (concentration of expressed protein and activin A on the plate were 1 µg/mL and 30 ng/mL, respectively). The plate was washed three times and a solution of anti-activin A antibody conjugated to biotin (R&D #BAM3381, 1 ug/mL diluted in assay diluent, 100 µL/well) was added to each well and incubated for 1 hour at RT. After washing three times, binding was revealed using streptavidin-HRP using a standard colorimetric method. In this assay, a loss of signal indicates binding of the exemplary agent to activin A, which prevents activin A binding to the anti-activin A antibody on the plate. Results are expressed as a % of the signal obtained with wild-type ActRIIB-Fc (P75).

To determine potency on BMP-9, a 96-well plate was coated overnight at 4°C with a solution of anti-human IgG antibody (SouthernBiotech #2049-01, 1/6000 in a carbonate-bicarbonate buffer, 100 µL/well). The next day, the plate was washed three times. Blocking buffer (5% BSA in PBS, 200 µL/well) was added to each well and the plate was incubated for 1 hour at RT. Expressed proteins in the supernatants were diluted 1 in 2 in assay diluent (0.5% BSA, 0.05% tween-20 in PBS) and 100 µL was added to the 96-well plate in technical duplicates. After a 1.5-hour incubation at RT, the plate was washed three times. BMP-9 (R&D #3209-BP, 10 ng/mL diluted in assay diluent, 100 µL/well) was added to each well and incubated for 1.5 h at RT. The plate was washed three times, and a solution of anti-BMP-9 antibody conjugated to biotin (R&D #BAF3209, 0.4 ug/mL diluted in assay diluent, 100 µL/well) was added to each well and incubated for 1 hour at RT. After washing three times, binding was revealed using streptavidin-HRP using a standard colorimetric method. In this assay, a loss of signal indicates less binding of the exemplary agent to BMP-9. Results are expressed as a % of the signal obtained with wild-type ActRIIB-Fc (P75).

To determine potency on BMP-10, the same BMP-reporter cell line was used as described above. A final concentration of 2.5 nM of each expressed protein was tested in the presence of 40 ng/mL of BMP-10 (R&D #2926-BP). In this assay, a loss of signal indicates neutralization of BMP-10 by the exemplary agent. Results are expressed as a % of the signal obtained with the cytokine alone. A separate assay was used to estimate BMP-10 binding, using bio-layer interferometry (BLI) on a Gator^{®} Plus instrument (Gator Bio, Palo Alto, USA). An anti-Human IgG Fc Gen II probe (#160024, Gator Bio) was loaded with expressed proteins in CHO supernatants, following which BMP-10 binding kinetics (single concentration, 12.5 nM) were assessed.

Although this invention is described in detail with reference to embodiments thereof, these embodiments are offered to illustrate but not to limit the invention. It is possible to make other embodiments that employ the principles of the invention and that fall within its spirit and scope as defined by the claims appended hereto.

## Claims

1. A method of improving body composition in a subject comprising administering a TGFβ superfamily ligand binding agent comprising a first and a second polypeptide, wherein each of the first and second polypeptides comprise:
(a) an Activin receptor type IIB (ActRIIB) ectodomain (ECD) variant comprising an amino acid substitution at the position corresponding to position 33 of SEQ ID NO: 2;
(b) a peptide linker; and
(c) an Fc domain monomer.

2. The method of claim 1, wherein an improvement in body composition comprises an increase in lean muscle mass and/or decrease in fat mass.

3. A method of improving exercise tolerance in a subject comprising administering a TGFβ superfamily ligand binding agent comprising a first and a second polypeptide, wherein each of the first and second polypeptides comprise:
(a) an Activin receptor type IIB (ActRIIB) ectodomain (ECD) variant comprising an amino acid substitution at the position corresponding to position 33 of SEQ ID NO: 2;
(b) a peptide linker; and
(c) an Fc domain monomer

4. The method of claim 3, wherein an improvement in exercise tolerance comprises reduced Left ventricular end-diastolic pressure (LVEDP) and/or an increased 6-minute walk distance.

5. The method of any one of claims 1-4, wherein the subject suffers from a metabolic disorder.

6. The method of claim 5, wherein the metabolic disorder is obesity.

7. The method of any one of claims 1-4, wherein the subject does not suffer from a cardiometabolic disorder.

8. The method of any one of claims 1-7, wherein the ActRIIB ECD variant comprises the amino acid substitution L33E.

9. The method of claim 8, wherein the ActRIIB ECD variant
(a) comprises an amino acid sequence that is at least 85%, at least 90%, at least 95%, at least 96%, at least 97%, at least 98%, or at least 99% identical to SEQ ID NO: 16; or
(b) comprises or consists of the amino acid sequence of SEQ ID NO: 16.

10. The method of any one of claims 1-9, wherein the first and second polypeptides comprise the following structure, from N- to C-terminus: ActRIIB-ECD - peptide linker - Fc domain monomer.

11. The method of any one of claims 1-10, wherein the Fc domain monomer is an IgG1 isotype

12. The method of claim 11, wherein the Fc domain monomer comprises or consists of the amino acid sequence set forth in SEQ ID NO: 253, SEQ ID NO: 255, or SEQ ID NO: 256.

13. The method of any one of claims 1-12, wherein the Fc domain monomer forms a dimer.

14. The method of any one of claims 1-13, wherein the peptide linker is Glycine-rich.

15. The method of any one of claims 1-14, wherein the peptide linker is between 10 and 40 amino acids long.

16. The method of claim 15, wherein the peptide linker is 14 amino acids long.

17. The method of any one of claims 1-16, wherein the first and second polypeptides comprise or consist of the amino acid sequence of SEQ ID NO: 231, or an amino acid sequence that is at least 95%, 96%, 97%, 98%, or 99% identical thereto.
